# FASCICULE DE BREVET EUROPEEN

(11) **EP 3 708 566 B1**
(45) Date de publication et mention de la délivrance du brevet: **08.02.2023**
(21) Numéro de dépôt: 20172751.8
(22) Date de dépôt: 22.12.2015
(51) Int. Cl.: C07D 401/14, C07D 413/14, C07D 401/06, C07D 403/06, C07D 407/14, C07D 409/14, C07D 471/04, C07D 498/20, A61P 17/04, A61P 17/00, A61P 17/06, A61P 17/10, A61P 19/02, A61P 25/00, A61P 25/06, A61P 29/00, A61P 37/02, A61P 37/08, A61P 43/00

(54) **NOUVEAUX COMPOSÉS HÉTÉROCYCLIQUES ET LEUR UTILISATION EN MÉDECINE AINSI QU'EN COSMÉTIQUE**
NOUVEAUX COMPOSÉS HÉTÉROCYCLIQUES ET LEUR UTILISATION EN MÉDECINE AINSI QU'EN COSMÉTIQUE
NOVEL HETEROCYCLIC COMPOUNDS AND THE USE THEREOF IN MEDICINE AND IN COSMETICS

(30) Priorité: 23.12.2014 FR 1463212
(43) Date de publication de la demande: 16.09.2020
(62) Demande divisionnaire de: 15826043.0
(73) Titulaire: Galderma Research & Development, 06410 Biot (FR)
(72) Inventeur: Fournier, Jean-François, H4L 0B4 Saint Laurent (CA); Clary, Laurence, 06480 La Colle sur Loup (FR); Thoreau, Etienne, 06460 Saint Vallier de Thiey (FR)
(74) Mandataire: Nederlandsch Octrooibureau

(56) Documents cités:
- WO-A1-2004/083187
- WO-A1-2008/130512
- WO-A1-2010/139717
- WO-A2-2006/029153
- WO-A2-2008/112159

## Description

L'invention concerne de nouveaux composés hétérocycliques de formule générale (I) :

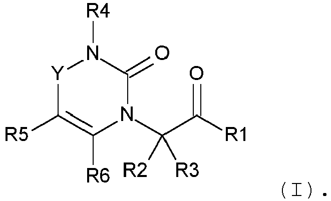

Elle concerne également composé pour son utilisation comme médicament, notamment dans la prévention et/ou le traitement de maladies inflammatoires avec une composante neurogène ou comme cosmétique. L'étendue de l'invention est celle des revendications. Toute autre matière n'est présente que comme référence et ne forme pas partie de l'invention.

Les composés de la présente invention agissent comme antagonistes du récepteur CGRP-R.

Le peptide relié au gène calcitonine (CGRP ou calcitonin gene-related peptide) est un neuropeptide de 37 acides aminés comportant un groupe amide en C-terminal et une structure cyclique formée avec un pont disulfure Cys-Cys en N-terminal (Poyner et al., « The mammalian calcitonin gene-related peptides, adrenomedullin, amylin, and calcitonin receptors », 2002) . CGRP est largement exprimé dans le système nerveux central et périphérique et est impliqué dans un certain nombre de fonctions biologiques (Edvinsson et al., « CGRP antagonism and migraine », 2010). Il y a de plus en plus de preuves que le CGRP joue un rôle important comme neuromodulateur (Ho et al., « CGRP and its receptors provide new insights into migraine pathophysiology », 2010), mais il est surtout connu comme un puissant vasodilatateur (Brain et al., « Calcitonin gene-related peptide is a potent vasodilatator », 1985).

Différentes composés utiles comme antagonistes des récepteurs CGRP sont connues. WO 2004/083187 et WO 2008/130512 concernent des composés des antagonistes du CGRP récepteurs et sont utiles dans le traitement ou la prévention de maladies dans lesquelles le CGRP est impliqués, comme la migraine.

WO 2008/112159 décrit des composés monocyclique anilide spirolactam CGRP récepteur antagonistes pour le traitement ou la prévention de maladies comme les céphalées et la migraine.

WO 2006/029153 divulgue des composés inhibiteurs du récepteur CGRP et utiles dans le traitement ou la prévention de maladies comme les céphalées, les migraines et les céphalées en grappe.

WO 2010/139717 divulgue des composes utiles comme antagonistes des récepteurs CGRP qui ont une stabilité accrue et des demi-vies plus longues dans la circulation sanguine.

CGRP signale principalement via un récepteur (CGRP-R) qui est un hétérodimère formé par l'association d'un récepteur couplé à une protéine G (RCPG) de classe B (récepteur de la calcitonine (CLR ou calcitonin-like receptor)) avec une protéine transmembranaire modifiant l'activité des récepteurs (RAMP1 ou receptor activity-modifying protein 1) (McLatchie et al., « RAMPs regulate the transport and ligand specificity of the calcitonin receptor-like receptor », 1998).

Le rôle de CGRP et l'activité du récepteur CGRP ont fait l'objet de nombreuses études.

On peut plus particulièrement citer à titre indicatif des études qui ont suggéré que la crise de migraine est associée à une libération de CGRP par les nocicepteurs méningés du ganglion trigéminé et que le CGRP puisse donc jouer un rôle actif dans la migraine (Edvinsson et al., « Functional rôle of perivascular peptides in the control of cérébral circulation », 1985 ; Goadsby et al., « Vasoactive peptide release in the extracerebral circulation of humans during migraine headache », 1990 ; Lassen et al., « CGRP may play a causative rôle in migraine », 2002).

Le nombre croissant de preuves reliant CGRP à la migraine a conduit à un intérêt dans le développement d'agents capables de bloquer les effets de ce neuropeptide. Des efforts ont notamment porté sur le développement de petites molécules antagonistes directs du récepteur CGRP (les gépants) et non vasoconstricteurs pour le traitement de la migraine.

Ces molécules sont décrites dans la littérature pour une utilisation par voie orale et présentent ainsi une stabilité métabolique au niveau hépatique (olcegepant, telcagepant et MK-3207) .

Toutefois, leur développement s'est heurté à des problèmes de toxicité hépatique.

Par ailleurs, outre son rôle dans la migraine, la libération de CGRP par le nerf trigéminal innervant la face pourrait être impliquée dans l'inflammation neurogène responsable notamment de l'érythème permanent dans la rosacée de type 1. Cette hypothèse est soutenue par des caractéristiques communes entre la rosacée et la migraine, qui comporte une composante neurogène avec des phénomènes vasoactifs, une activation du système nerveux trigéminé et la libération de peptides neuro-inflammatoires (CGRP, substance P...). Le CGRP exercerait son action vasodilatatrice en agissant principalement sur les cellules musculaires lisses et endothéliales des vaisseaux sous-cutanées.

La rosacée est une dermatose inflammatoire commune chronique et progressive liée à une relaxation vasculaire. Elle affecte principalement la partie centrale du visage et se caractérise par un rougissement du visage ou des bouffées de chaleur, un érythème facial, des papules, des pustules, une télangiectasie et parfois des lésions oculaires appelées rosacée oculaire.

Par ailleurs, il s'associe à ces caractéristiques primaires une composante neurogène secondaire, c'est-à-dire une hyperréactivité cutanée de la peau du visage et du cou, caractérisée par l'apparition de rougeurs et de sensations subjectives du type démangeaisons ou prurit, de sensations de brûlures ou d'échauffement, de sensations de picotements, de fourmillements, d'inconforts, de tiraillements, etc.

La rosacée est ainsi classiquement traitée par voie orale ou par voie topique par des actifs tels que les antiséborrhéiques et les antiinfectieux, par exemple le peroxyde de benzoyle, l'acide rétinoïque, le métronidazole ou les cyclines.

Toutefois, ces traitements qui agissent sur l'infection et l'hyperséborrhée ne permettent pas de traiter et/ou prévenir efficacement l'ensemble des symptômes associés à la rosacée, en particulier de traiter la composante neurogène de cette affection, et notamment l'hyperréactivité de la peau et la rougeur.

Aussi, on connaît plus particulièrement le brevet EP0734729 qui divulgue l'utilisation d'un antagoniste de CGRP pour traiter les rougeurs cutanées d'origine neurogène, et notamment la rosacée et/ou l'érythème pudique.

Toutefois, le seul antagoniste décrit est le CGRP 8-37, un peptide anti-CGRP. Il doit diminuer la vasodilatation induite par la capsaïcine et/ou provoquer une inhibition de la libération de CGRP par les fibres nerveuses sensitives et/ou provoquer une inhibition de la contraction du muscle lisse du canal déférent induite par le CGRP.

Considérant ce qui précède, il existe donc un besoin de disposer d'actifs efficaces dans le traitement de maladies inflammatoires avec une composante neurogène telle que la la rosacée, qui soient utilisables pendant de longues périodes, avec des effets secondaires aussi limités que possible.

Un problème que se propose de résoudre la présente invention est de proposer un traitement permettant de réduire ou abolir totalement cette inflammation, en utilisant un antagoniste non peptidique du récepteur au CGRP afin de cibler l'inflammation neurogène, avec pour résultat un effet persistant à l'arrêt du traitement, tout en limitant les effets secondaires pour le patient, en particulier les risques de toxicité hépatique.

Le Demandeur a ainsi identifié de nouveaux composés hétérocycliques antagonistes du récepteur CGRP, sélectifs du récepteur humain, qui présentent un très bon potentiel sur le plan de l'activité biologique. De manière inattendue, ces nouveaux composés présentent une instabilité métabolique au niveau hépatique, ce qui devrait limiter le risque d'hépatotoxicité rencontré avec de nombreux antagonistes CGRP connus de l'art antérieur comme par exemple l'olcegepant, le telcagepant et MK-3207.

L'invention a pour objet un composé hétérocyclique choisi parmi les composés de formule générale (I) : 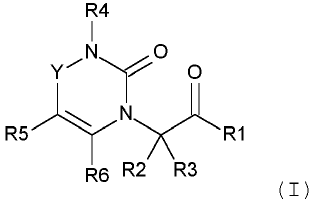 dans laquelle R1, R2, R3, R4, R5, R6 et Y sont tels que définis dans la description détaillée ci-dessous, ainsi que les sels pharmaceutiquement acceptables des composés de formule générale (I) et les énantiomères des composés de formule générale (I).

L'invention a également pour objet une composition pharmaceutique comprenant au moins un composé de formule générale (I) et un véhicule pharmaceutiquement acceptable, de préférence adapté pour une administration par voie topique.

Elle a encore pour objet l'utilisation cosmétique d'un composé de formule générale (I).

L'invention a aussi pour objet un composé de formule générale (I) pour son utilisation comme médicament.

Elle a en particulier pour objet un composé de formule générale (I) pour son utilisation dans la prévention et/ou le traitement de maladies inflammatoires avec une composante neurogène.

L'invention a en outre pour objet l'utilisation d'un composé de formule générale (I) pour préparer un médicament destiné à la prévention et/ou au traitement de maladies inflammatoires avec une composante neurogène.

L'invention et les avantages qui en découlent seront mieux compris à la lecture de la description et des modes de réalisation non limitatifs qui suivent, rédigés au regard des figures annexées, dans lesquelles :

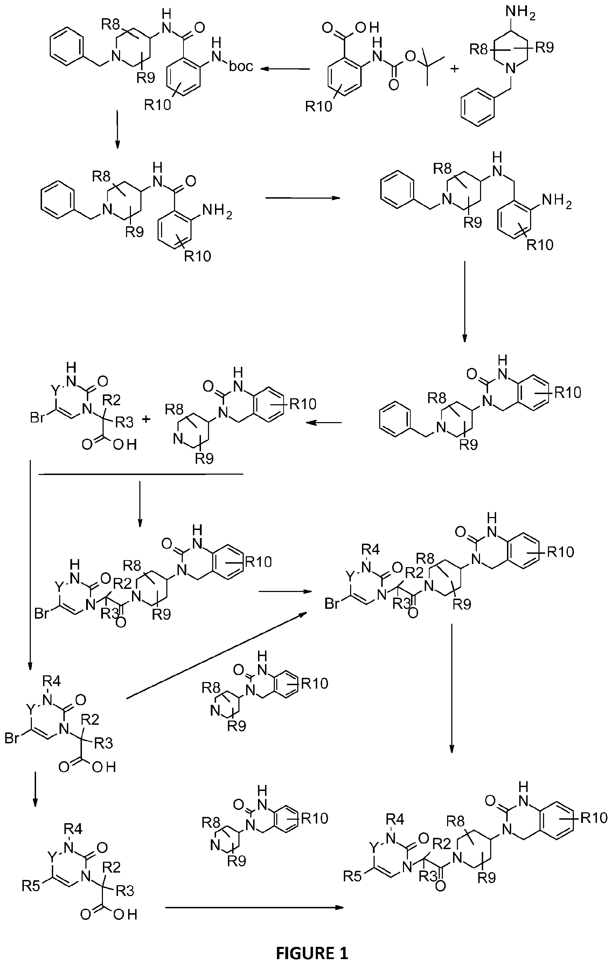

- la figure 1 représente une voie de synthèse générale dans des conditions classiques de composés selon l'invention selon le schéma réactionnel n°1 ;

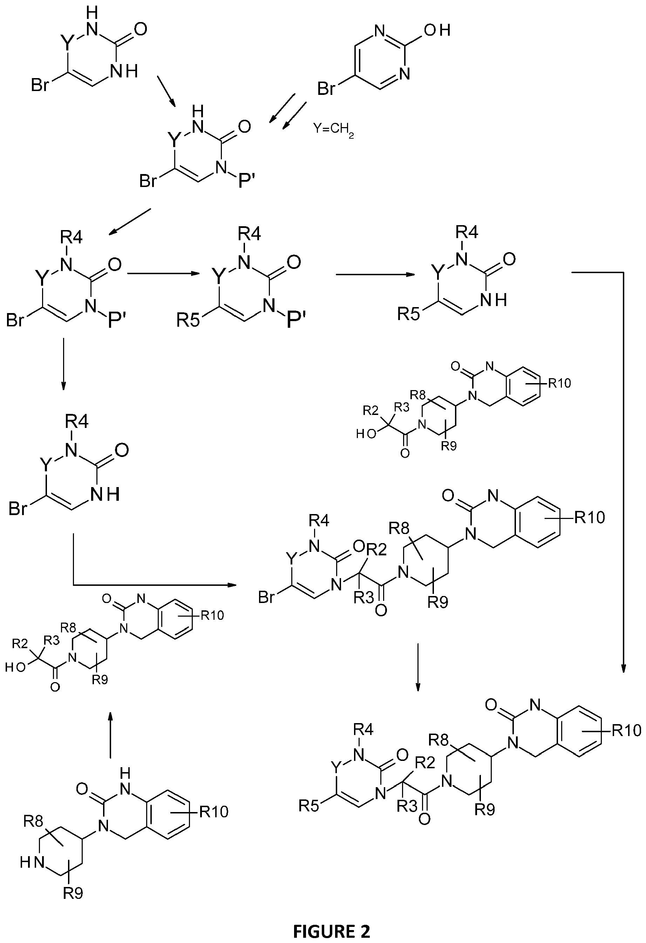

- la figure 2 représente une voie de synthèse générale dans des conditions classiques de composés selon l'invention selon le schéma réactionnel n°2, dans lequel P' désigne un groupement protecteur de type benzyle, 4-méthoxy-benzyle, benzhydrile, -COOtBu ou autre ;

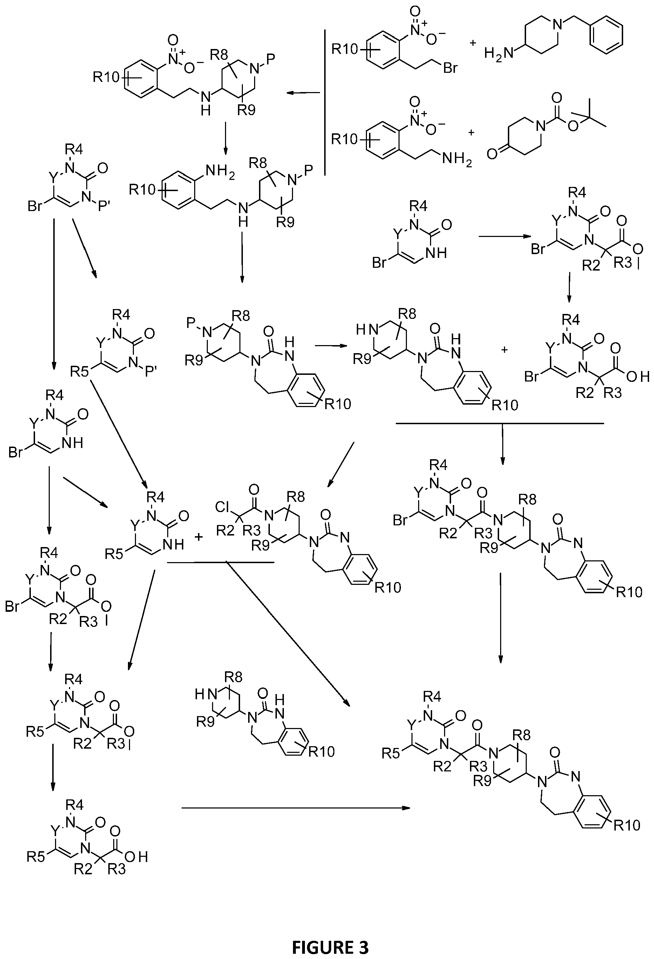

- la figure 3 représente une voie de synthèse générale dans des conditions classiques de composés selon l'invention selon le schéma réactionnel n°3, dans lequel P' désigne un groupement protecteur de type benzyle, 4-méthoxy-benzyle, benzhydrile, -COOtBu ou autre ; et

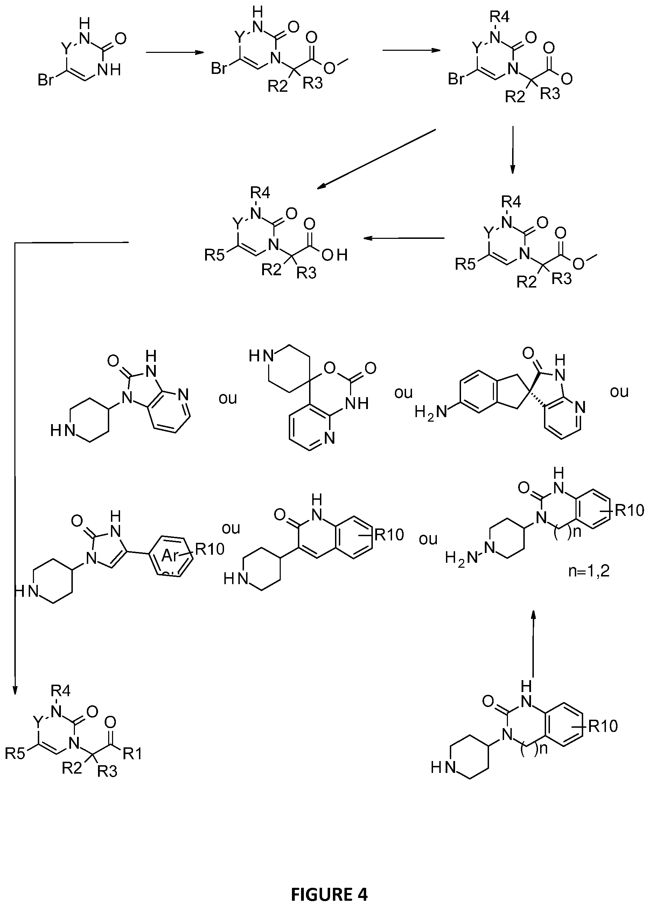

- la figure 4 représente une voie de synthèse générale dans des conditions classiques de composés selon l'invention selon le schéma réactionnel n°4.

Dans l'ensemble de la présente description, à moins qu'il ne soit spécifié autrement, il est entendu que, lorsque des intervalles de concentrations sont donnés, ils incluent les bornes supérieures et inférieures dudit intervalle.

Les composés selon l'invention sont de nouveaux composés hétérocycliques antagonistes du récepteur CGRP choisis parmi les composés de formule générale (I) : 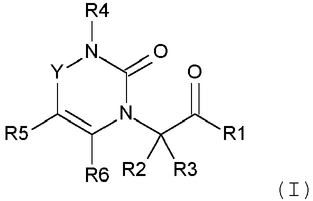 dans laquelle,
- Y est choisi parmi -CH₂, -C(O), -C(CH₃)₂, ou un spirocyclopropyle ;
- R1 est choisi parmi les groupements (1-1) à (1-12) suivants : 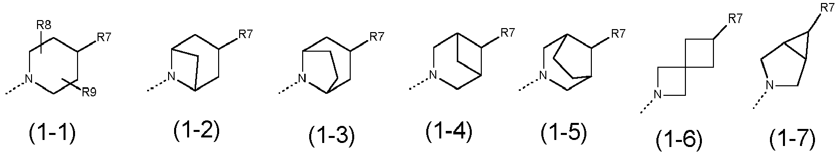 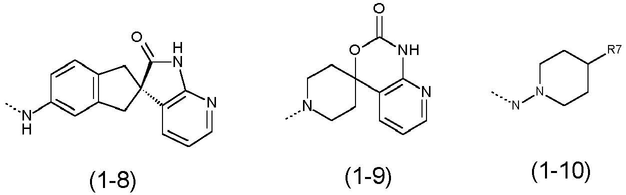 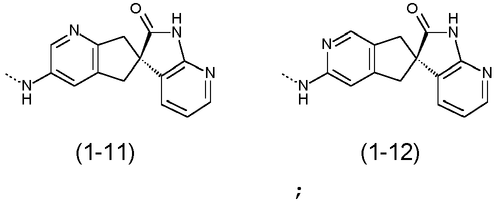
- R2, R3, R6 sont identiques ou différents, et choisis parmi un atome d'hydrogène, F ou -CH₃ ;
- R4 est choisi parmi un atome d'hydrogène, un alkyle, un alcène, un alcyne, un cycloalkyle, un cycloalcène, un aralkyle, un hétéroaralkyle, ou les groupements (4-1) à (4-55) suivants : 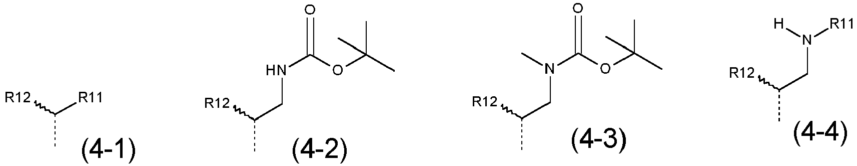 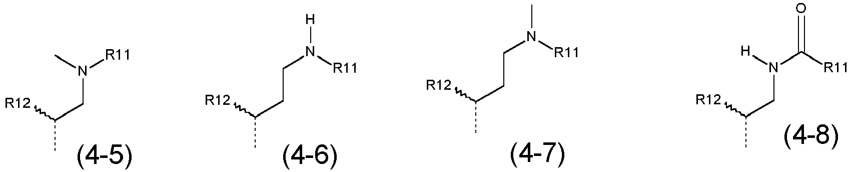 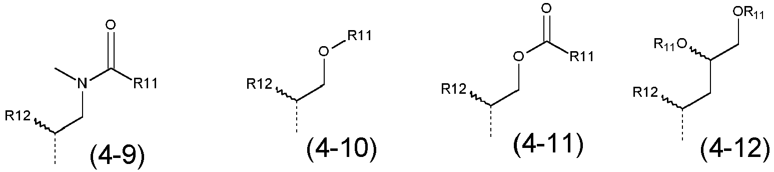 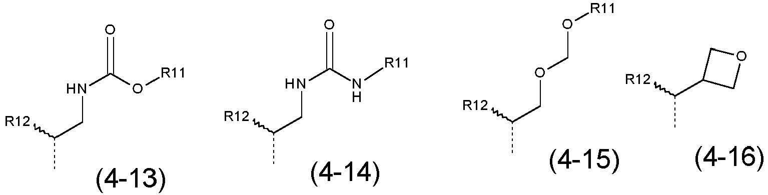 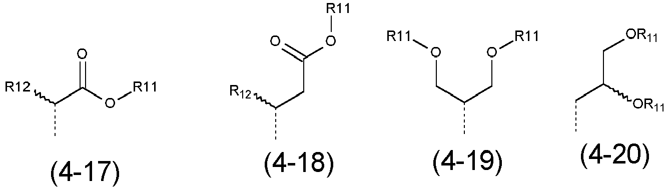 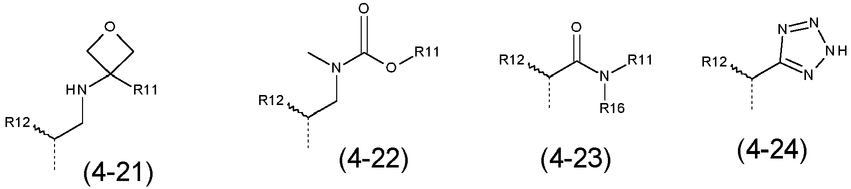 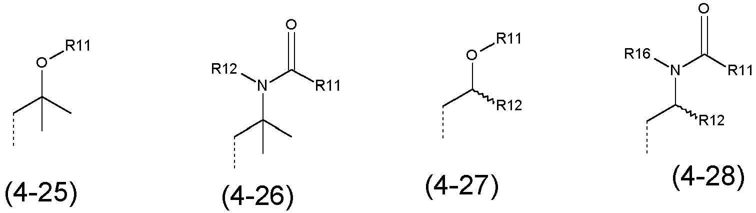 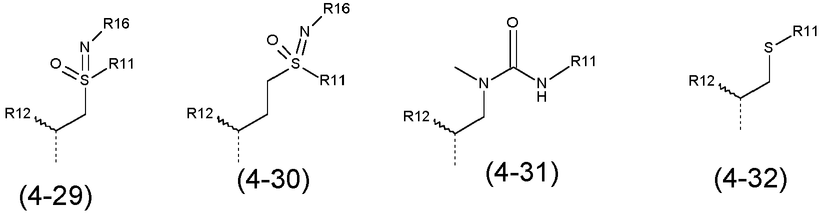 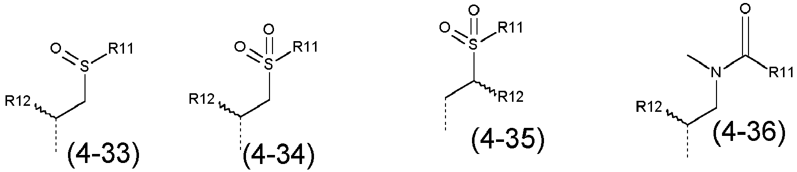 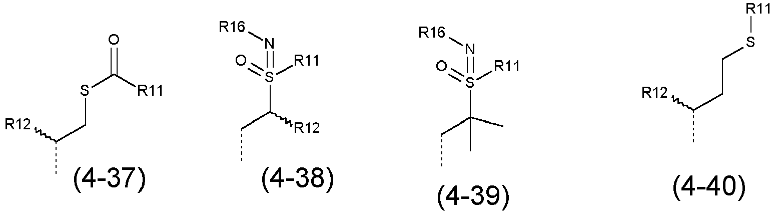 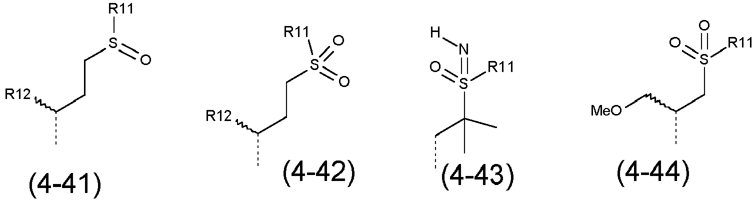 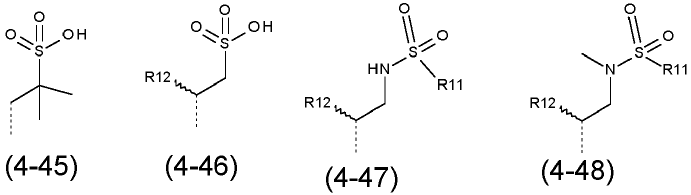 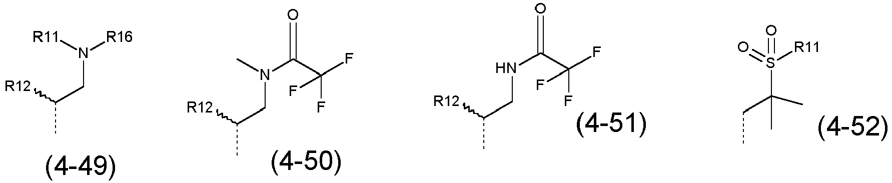 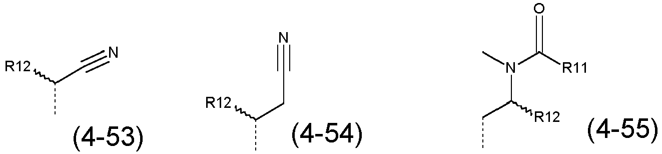
- R5 est choisi parmi un halogène, un alkyle, un cycloalkyle éventuellement substitué par R13, R14 et/ou R15, un alcène, un cycloalcène, un alcyne, un éther ou les groupements (5-1) à (5-5) suivants : 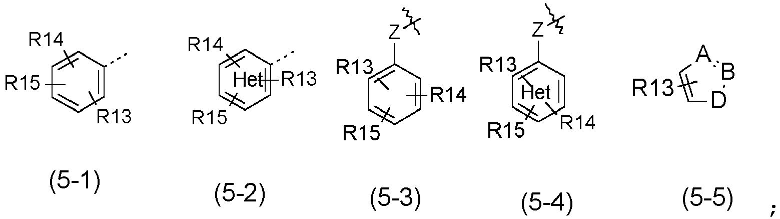 avec Het représentant 1 à 3 atomes d'azote parmi les 6 atomes du cycle aromatique, ces atomes d'azote pouvant, indépendamment les uns des autres, être substitués par un atome d'oxygène pour former un groupe N-oxyde ;
- R7 est choisi parmi les groupements (7-1) à (7-28) suivants : 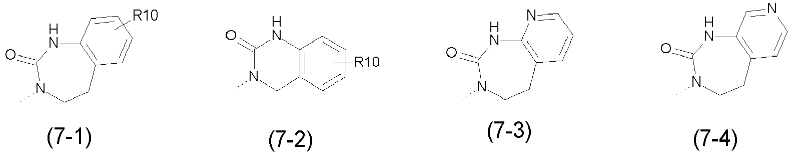 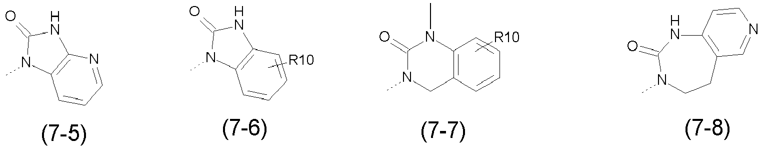 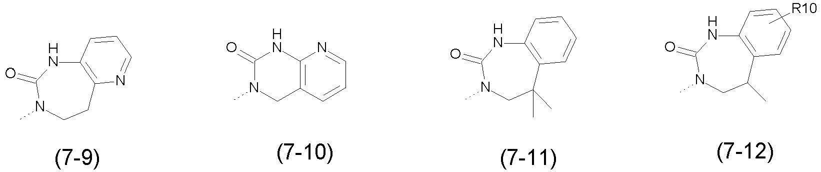 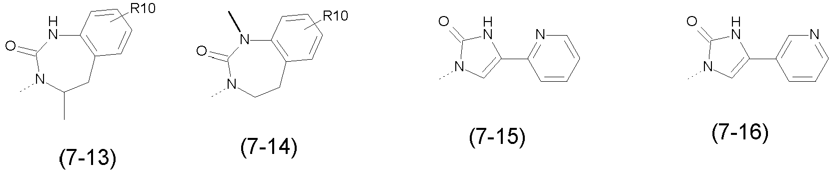 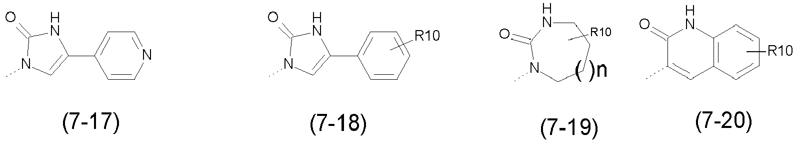 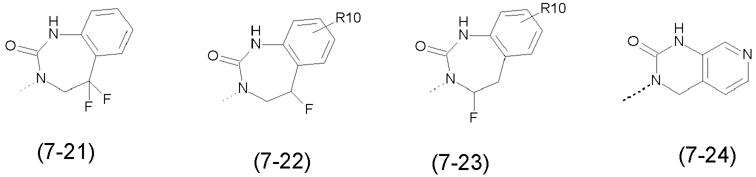 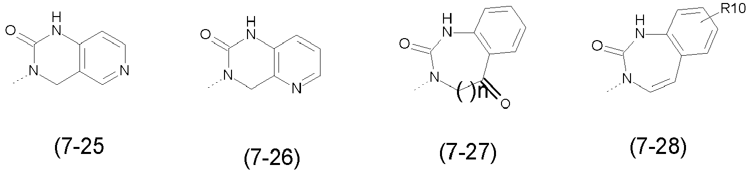 avec n = 0 ou 1 ;
- R8, R9 sont identiques ou différents, et choisis parmi un atome d'hydrogène, F ou -CH₃ ;
- R10 est choisi parmi un atome d'hydrogène, -OR11, -SR11, -NR11R12, -S(O)R11, -SO₂R11, -OC(O)R11, -CO₂R11, un halogène,-NO₂, -CN, -C(O)NR11R12, -CF₃ ou -OCF₃ ;
- R11, R12 sont identiques ou différents, et choisis parmi un atome d'hydrogène, un alkyle en C1-C6, CF₃ ou un éther;
- R13, R14, R15 sont identiques ou différents, et choisis parmi un atome d'hydrogène, un alkyle C1-C6, un cycloalkyle,-OR16, -NR16R17, un halogène, -OCF₃, -CF₃, -CN, -CO₂R16,-CONR16R17, -NO₂, -OCH₂OR16, -SR16, -S(O)R16, -SO₂R16 ou un éther;
- A, B, D sont identiques ou différents, et choisis parmi un atome C, N, O ou S ;
- R16 et R17 sont identiques ou différents, et choisis parmi un atome d'hydrogène, un alkyle C1-C6 ou -C(O)R18 ;
- Z est choisi parmi -CH₂, O ou -NR18 ; et
- R18 est choisi parmi un atome d'hydrogène, un alkyle C1-C3,
ainsi que leurs sels pharmaceutiquement acceptables, et leurs énantiomères
et dans laquelle
- un alkyle est une chaîne hydrocarbonée saturée, linéaire ou ramifiée, comprenant de 1 à 8 atomes de carbone, éventuellement substituée par un ou plusieurs atomes de fluor ;
- un alcène est une chaîne hydrocarbonée insaturée, linéaire ou ramifiée, comprenant de 2 à 8 atomes de carbone et comprenant une ou deux doubles liaisons, éventuellement substituée par un ou plusieurs atomes de fluor ;
- un alcyne est une chaîne hydrocarbonée insaturée, linéaire ou ramifiée, comprenant de 2 à 8 atomes de carbone et comprenant une ou deux triples liaisons, éventuellement substituée par un ou plusieurs atomes de fluor ;
- un cycloalkyle est une chaine hydrocarbonée, saturée, cyclique comprenant de 3 à 7 atomes de carbones, éventuellement substituée par un ou plusieurs atomes de fluor ;
- un cycloalcène est une chaine hydrocarbonée, insaturée, cyclique comprenant de 4 à 7 atomes de carbones et comprenant une ou deux doubles liaisons, éventuellement substituée par un ou plusieurs atomes de fluor ;
- un aryle est un cycle hydrocarboné aromatique ou deux cycles fusionnés hydrocarbonés aromatiques ;
- un aralkyle est un alkyle substitué par un aryle ;
- un hétéroaryle est un radical hétérocyclique aromatique comprenant un ou plusieurs hétéroatomes choisis parmi O, S et N, éventuellement substituée par un ou plusieurs groupes d'atomes choisis par exemple parmi un alkyle, un alkoxy, un aryle, un aryle substitué, un halogène, un hydroxy, un cyano, un trifluorométhyle, un sulfure, un pentafluorosulfure, un sulfoxyde, un sulfone, un acide carboxylique, un ester et un nitro ;
- un hétéroaralkyle est un alkyle substitué par un hétéroaryle ;
- un hétérocycle désigne, en particulier, une chaine hydrocarbonée cyclique ou bicylique, saturée ou insaturée, comprenant un ou plusieurs hétéroatomes choisis parmi O, S et N, éventuellement substitué par un ou plusieurs groupes choisis parmi un alkyle, un alkoxyle, un halogène, un hydroxyle, un cyano, un trifluorométhyle, un sulfure, un pentafluorosulfure, un sulfoxyde, un sulfone, un acide carboxylique, un ester et un nitro ;
- un ether est une chaîne hydrocarbonée saturée, linéaire ou ramifiée, comprenant de 1 à 8 atomes de carbone, et dans laquelle 1 à 3 atomes de carbone sont remplacés respectivement par 1 à 3 atomes d'oxygène, deux atomes d'oxygène étant toujours séparés par au moins un atome de carbone, ladite chaine étant éventuellement substituée par un ou plusieurs atomes de fluor.

« Sel pharmaceutiquement acceptable » désigne les sels d'un composé d'intérêt qui possèdent l'activité biologique souhaitée. Les sels pharmaceutiquement acceptables comprennent des sels de groupes acides ou basiques présents dans les composés spécifiés. Les sels d'addition acide pharmaceutiquement acceptables comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, ptoluènesulfonate et le pamoate (c'est-à-dire, 1,1'- méthylène-bis-(2-hydroxy-3-naphtoate)). Des sels de base adaptés comprennent, mais ne sont pas limités à, des sels d'aluminium, calcium, lithium, magnésium, potassium, sodium, zinc, et diéthanolamine. Une liste de sels pharmaceutiquement acceptables est notamment publiée dans la revue de Berge et al. (J. Pharm. Sci. 1977, 66(1), 1-19) .

Le terme énantiomère désigne les configurations R ou S des composés de formule générale (I) selon l'invention.

A moins qu'il n'en soit précisé autrement, les définitions données ci-après s'appliquent par défaut à l'ensemble des radicaux utilisés pour définir la structure des composés de formule générale (I).

Lorsqu'une caractéristique du substituant diffère de celle indiquée dans les définitions ci-dessous, cette différence est explicitement indiquée.

A titre d'exemples, un alkyle qui ne comprendrait que 3 à 7 atomes de carbone serait défini par alkyle C3-C7 ; un alcène qui ne comprendrait que 3 à 7 atomes de carbone et qui serait uniquement linéaire serait défini par alcène C3-C7 linéaire, toutes autres caractéristiques étant égales par ailleurs à celles définies par défaut pour chaque radical ci-dessous.

Selon la présente invention :
Un alkyle désigne, en particulier, une chaîne hydrocarbonée saturée, linéaire ou ramifiée, comprenant de 1 à 8 atomes de carbone, éventuellement substituée par un ou plusieurs atomes de fluor.

Un alcène désigne, en particulier, une chaîne hydrocarbonée insaturée, linéaire ou ramifiée, comprenant de 2 à 8 atomes de carbone et comprenant une ou deux doubles liaisons, éventuellement substituée par un ou plusieurs atomes de fluor.

Un alcyne désigne, en particulier, une chaîne hydrocarbonée insaturée, linéaire ou ramifiée, comprenant de 2 à 8 atomes de carbone et comprenant une ou deux triples liaisons, éventuellement substituée par un ou plusieurs atomes de fluor.

Un cycloalkyle désigne, en particulier, une chaine hydrocarbonée, saturée, cyclique comprenant de 3 à 7 atomes de carbones, éventuellement substituée par un ou plusieurs atomes de fluor.

Un cycloalcène désigne, en particulier, une chaine hydrocarbonée, insaturée, cyclique comprenant de 4 à 7 atomes de carbones et comprenant une ou deux doubles liaisons, éventuellement substituée par un ou plusieurs atomes de fluor.

Un aryle désigne, en particulier, un cycle hydrocarboné aromatique ou deux cycles fusionnés hydrocarbonés aromatiques. Les radicaux aryles préférés sont choisis parmi les radicaux phényle et naphtyle, éventuellement substitué par un ou plusieurs groupes d'atomes choisis parmi un alkyle, un alkoxyle, un aryle, un halogène, un hydroxyle, un cyano, un trifluorométhyle, un sulfure, un pentafluorosulfure, un sulfoxyde, une sulfone, un acide carboxylique, un ester et un nitro.

Un aralkyle désigne, en particulier, un alkyle substitué par un aryle.

Un hétéroaryle désigne, en particulier, un radical hétérocyclique aromatique c'est-à-dire une chaîne hydrocarbonée cyclique ou polycyclique, aromatique, comprenant un ou plusieurs hétéroatomes choisis parmi O, S et N, éventuellement substituée par un ou plusieurs groupes d'atomes choisis par exemple parmi un alkyle, un alkoxy, un aryle, un aryle substitué, un halogène, un hydroxy, un cyano, un trifluorométhyle, un sulfure, un pentafluorosulfure, un sulfoxyde, une sulfone, un acide carboxylique, un ester et un nitro.

Un hétéroaralkyle désigne, en particulier, un alkyle substitué par un hétéroaryle.

Un hétérocycle désigne, en particulier, une chaine hydrocarbonée cyclique ou bicylique, saturée ou insaturée, comprenant un ou plusieurs hétéroatomes choisis parmi O, S et N, éventuellement substitué par un ou plusieurs groupes choisis parmi un alkyle, un alkoxyle, un halogène, un hydroxyle, un cyano, un trifluorométhyle, un sulfure, un pentafluorosulfure, un sulfoxyde, une sulfone, un acide carboxylique, un ester et un nitro.

Un halogène désigne un atome de fluor, chlore ou brome.

Un alkoxyle désigne un atome d'oxygène substitué par un radical alkyle.

Un ether désigne, en particulier, une chaîne hydrocarbonée saturée, linéaire ou ramifiée, comprenant de 1 à 8 atomes de carbone, et dans laquelle 1 à 3 atomes de carbone sont remplacés respectivement par 1 à 3 atomes d'oxygène, deux atomes d'oxygène étant toujours séparés par au moins un atome de carbone, ladite chaine étant éventuellement substituée par un ou plusieurs atomes de fluor

Les composés selon l'invention sont préférentiellement choisis parmi les composés de formule générale (I) dans laquelle :
- Y est choisi parmi -CH₂ ou -C(O) ;
- R1 est choisi parmi les groupements suivants : 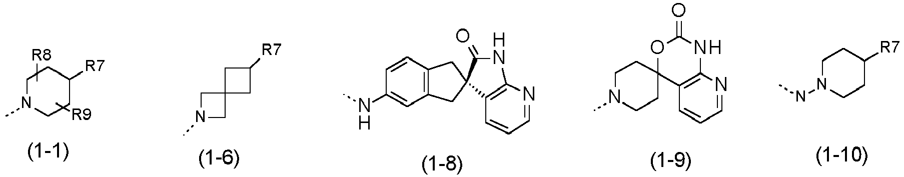
- R2, R3, R6 sont identiques ou différents, et choisis parmi un atome d'hydrogène, F ou -CH₃ ;
- R4 est choisi parmi un alkyle C1-C4, un aralkyle, un hétéroaralkyle, ou les groupements suivants : 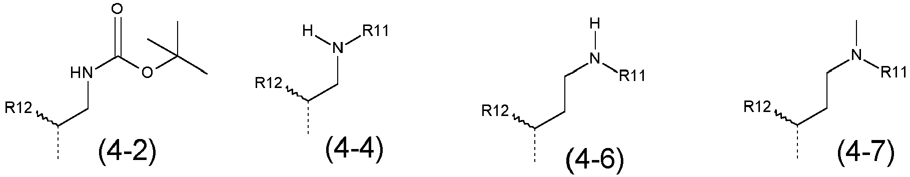 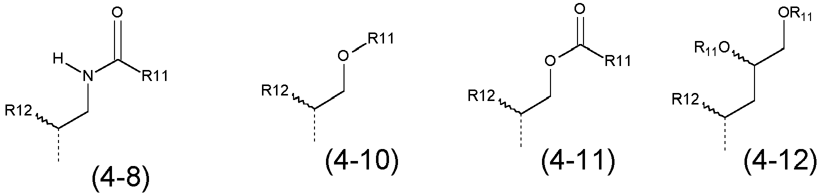 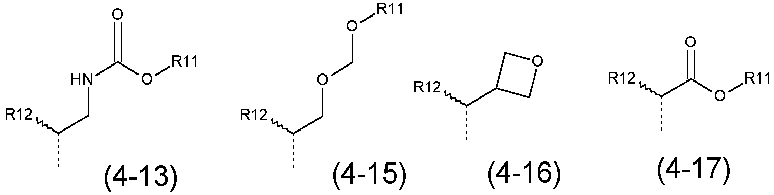 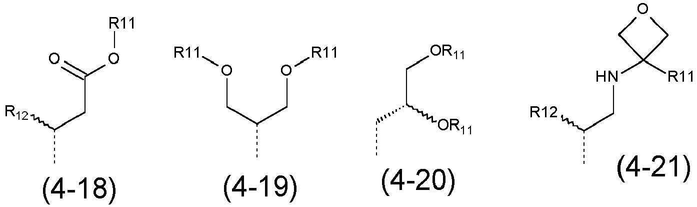 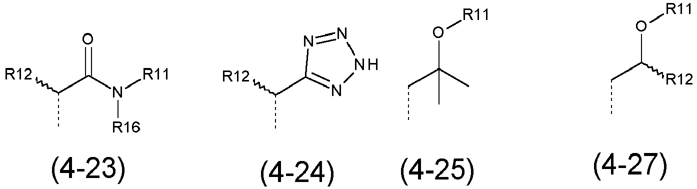 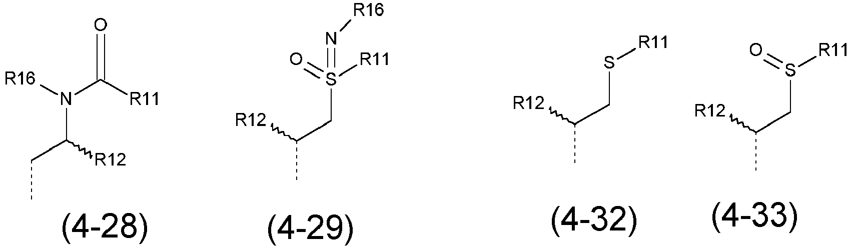 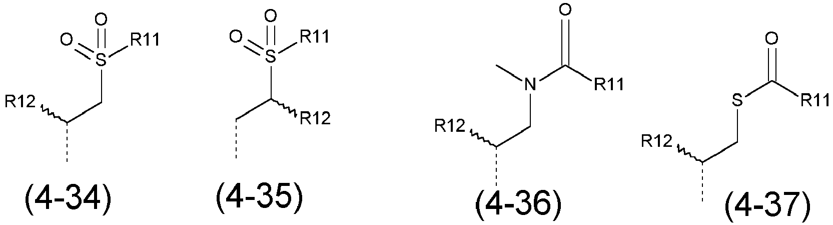 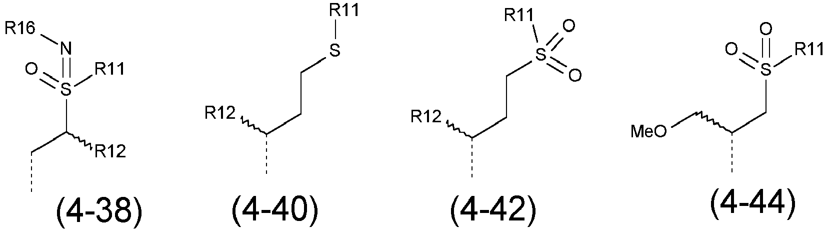 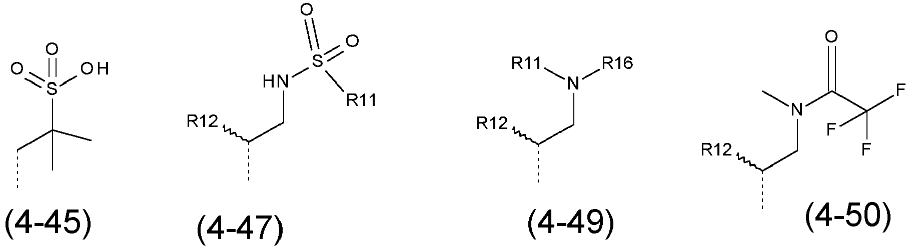 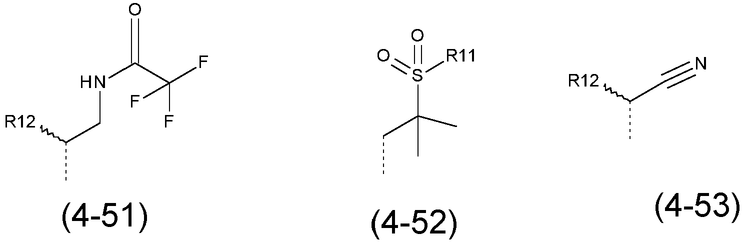
- R5 est choisi parmi Br, -CH₃, un cyclohexène ou les groupements suivants : 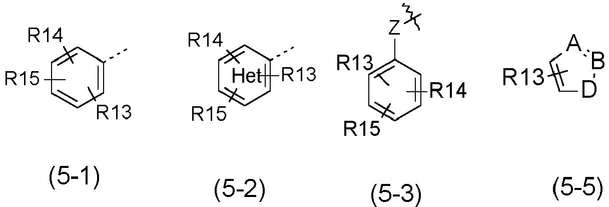 avec Het représentant 1 à 2 atomes de N parmi les 6 atomes du cycle aromatique, ces atomes d'azote pouvant, indépendamment les uns des autres, être substitués par un atome d'oxygène pour former un groupe N-oxyde ;
- R7 est choisi parmi les groupements suivants : 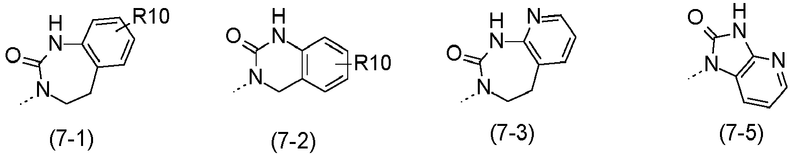 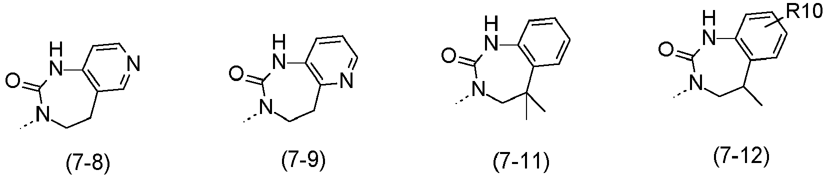 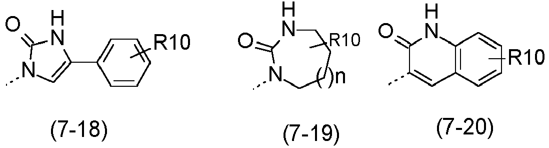 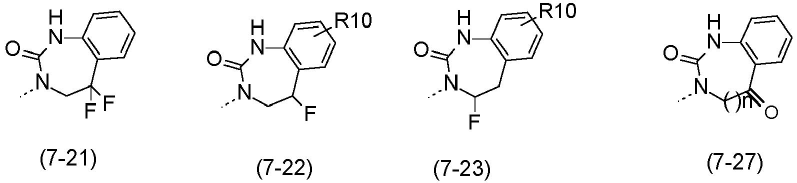 avec n = 1 ;
- R8, R9 sont identiques ou différents, et choisis parmi un atome d'hydrogène, F ou -CH₃ ;
- R10 est choisi parmi un atome d'hydrogène, -OR11, -SR11, -S(O)R11, -SO₂R11, -CO₂H ou un halogène choisi parmi Br ou F ;
- R11, R12 sont identiques ou différents, et choisis parmi un atome d'hydrogène, un alkyle en C1-C3 ou CF₃;
- R13, R14, R15 sont identiques ou différents, et choisis parmi un atome d'hydrogène, -CH₃, -OR16, un halogène, -OCF₃,-CN, -CO₂R16, -SR16, -S(O)R16, ou -SO₂R16 ;
- A, B, D sont identiques ou différents, et choisis parmi un atome C, N ou S ;
- R16 est choisi parmi un atome d'hydrogène ou un alkyle ; et
- Z est choisi parmi -CH₂ ou O.

A cet effet, les composés de formule générale (I) selon l'invention sont plus préférentiellement choisis parmi les composés suivants :
Composé 14 : 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl}-1H-pyrimidine-2,4-dione ;
Composé 16 : 5-(2,3-difluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 17 : 5-(2,3-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 18 : 5-(2-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 19 : 3-méthyl-1-{2-oxo-2-[4-(2-oxo-1,2,4,5-tétrahydro-benzo[d][1,3]diazépin-3-yl)-pipéridin-1-yl]-éthyl}-5-(2-trifluorométhoxy-phényl)-1H-pyrimidine-2,4-dione ;
Composé 22 : 5-(3-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 23 : 5-(3-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 24 : 5-(3-chloro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 28 : 5-(3-fluoro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 29 : 5-(3-chloro-2-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 30 : 5-(2,3-diméthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 31 : 5-(2-chloro-3-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 47 : 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 51 : 5-(2,3-diméthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 52 : 5-(3-chloro-2-méthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 53 : 5-(2,3-dichloro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 54 : 5-(2-chloro-3-fluoro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 72 : (5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-acetonitrile ;
Composé 73 : acide 3-(5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-propanoïque ;
Composé 83 : 5-(2,3-dichloro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 89 : 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 90 : 5-(2,3-dichloro-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 93 : 5-(2,3-diméthyl-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 95 : 5-(3-chloro-2-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 96 : 5-(2-chloro-3-fluoro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 97 : 5-(2-chloro-3-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 98 : 5-(2,3-diméthyl-phényl)-3-(2-hydroxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 100 : 5-(2,3-diméthyl-phényl)-3-(2-méthoxyméthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 101 : N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide ;
Composé 103 : (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle ;
Composé 104 : acide (5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-acétique ;
Composé 105 : 5-(2-chloro-3-fluoro-phényl)-3-oxétan-3-ylméthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 106 : 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 108 : 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 110 : 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 112 : 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 113 : 5-(2,3-difluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 114 : 3-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle ;
Composé 115 : N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 116 : N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 117 : N-[2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-éthyl]-acétamide ;
Composé 118 : N-[2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide ;
Composé 119 : 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 120 : N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-méthanesulfonamide ;
Composé 121 : 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-[(S)-1-méthyl-2-(3-méthyl-oxétan-3-ylamino)-éthyl]-1H-pyrimidine-2,4-dione ;
Composé 122 : 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 123 : (S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-acétate de propyle ;
Composé 124 : 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 126 : 5-(2,3-difluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 127 : 5-(2,3-dichloro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 128 : 5-(2,3-difluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 130 : 5-(2-chloro-3-méthoxy-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 131 : 5-(2-chloro-3-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 132 : 5-(2,3-dichloro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 134 : 5-(3-bromo-2-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 135 : 5-(2-chloro-3-fluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 136 : 5-(2,3-difluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 137 : 5-(2,3-dichloro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 138 : 5-(2,3-diméthyl-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 139 : 5-(2,3-diméthoxy-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 140 : 5-(2-chloro-3-méthoxy-phényl)-3-(2-methanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 142 : 5-(2-chloro-3-fluoro-phényl)-3-((S)-2-méthanesulfonyl-1-méthyl-éthyl)-1-{2-[4-(7-méthoxy-2-oxo-1,2, 4,5-tétrahydro-benzo[d][1,3]diazépin-3-yl)-pipéridin-1-yl]-2-oxo-éthyl}-1H-pyrimidine-2,4-dione ;
Composé 143 : 5-(2-chloro-3-fluoro-phényl)-3-((R)-2-méthanesulfonyl-1-méthyl-ethyl)-1-{2-[4-(7-méthoxy-2-oxo-1,2, 4,5-tétrahydro-benzo[d][1,3]diazépin-3-yl)-pipéridin-1-yl]-2-oxo-éthyl}-1H-pyrimidine-2,4-dione ;
Composé 144 : 5-(2,3-dichloro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 145 : 5-(2,3-dichloro-phényl)-3-((R)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 146 : 5-(2-chloro-3-fluoro-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 148 : 5-(2-chloro-3-méthoxy-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 152 : 3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-5-(3-méthoxy-2-trifluorométhoxy-phényl)-1H-pyrimidine-2,4-dione ;
Composé 155 : 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione ;
Composé 156 : 5-(2-chloro-3-fluoro-phényl)-3-(3-méthanesulfonyl-propyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 157 : 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione ;
Composé 158 : N-[(S)-2-(5-(2-chloro-3-fluoro-phényl)-3-{2-[4-(7-méthoxy-2-oxo-1,2,4,5-tétrahydro-benzo[d][1,3]diazepin-3-yl)-pipéridin-1-yl]-2-oxo-éthyl}-2,6-dioxo-3,6-dihydro-2H-pyrimidin-1-yl)-propyl]-propionamide ;
Composé 159 : 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-((S)-2-methoxy-1-methyl-ethyl)-1H-pyrimidine-2,4-dione ;
Composé 160 : 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-(2-methanesulfonyl-ethyl)-1H-pyrimidine-2,4-dione ;
Composé 161 : N-[(S)-2-(5-(2-Chloro-3-fluoro-phenyl)-3-{2-[4-(7-fluoro-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide ;
Composé 162 : N-[(S)-2-(5-(2,3-Dichloro-phenyl)-3-{2-[4-(7-fluoro-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide ;
Composé 165 : 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide ;
Composé 166 : 5-(2-chloro-3-fluoro-phényl)-3-isopropyl-pyrimidine-2,4-dione-1-[(2R)-2'-oxospiro[1,3-dihydroindene-2,3'-1H-pyrrolo[2,3-b]pyridine]-5-yl]-acetamide ;
Composé 170 : 5-(2,3-dichlorophenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide ;
Composé 178 : 5-(2,3-dichlorophenyl)-3-[(1S)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 184 : 5-(2-Chloro-3-fluoro-phényl)-3-(2-méthanesulfonyl-éthyl)-1-{2-[4-(7-méthanesulfonyl-2-oxo-1,2,4,5-tétrahydro-benzo[d][1,3]diazepin-3-yl)-piperidin-1-yl]-2-oxo-ethyl}-1H-pyrimidine-2,4-dione ;
Composé 185 : 5-(2-Chloro-3-fluoro-phenyl)-3-isopropyl-1-{2-[4-(7-methanesulfonyl-2-oxo-1,2,4,5-tetrahydro-benzo[d][1,3]diazepin-3-yl)-piperidin-1-yl]-2-oxo-ethyl}-1H-pyrimidine-2,4-dione ;
Composé 186 : 5-(2-chloro-3-fluoro-phényl)-3-(2-méthanesulfonyl-éthyl)-1-{2-[4-(7-méthylsulfanyl-2-oxo-1,2, 4,5-tetrahydro-benzo[d][1,3]diazepin-3-yl)-piperidin-1-yl]-2-oxo-éthyl}-1H-pyrimidine-2,4-dione ;
Composé 187 : 5-(2-Chloro-3-fluoro-phenyl)-3-((R)-2-methoxy-1-methyl-ethyl)-1-{2-[4-(7-methylsulfanyl-2-oxo-1,2, 4,5-tetrahydro-benzo[d][1,3]diazepin-3-yl)-piperidin-1-yl]-2-oxo-ethyl}-1H-pyrimidine-2,4-dione ;
Composé 188 : 5-(2-Chloro-3-fluoro-phenyl)-3-(2-methoxy-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione ;
Composé 189 : 5-(2-Chloro-3-fluoro-phenyl)-3-isopropyl-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione ;
Composé 192 : 3-(1-{2-[5-(2-chloro-3-fluoro-phenyl)-3-(2-methoxy-ethyl)-2-oxo-3,4-dihydro-2H-pyrimidin-1-yl]-acetyl}-piperidin-4-yl)-1,3,4,5-tetrahydro-benzo[d][1,3]diazepin-2-one ;
Composé 199 : 3-(1-{2-[5-(2,3-Dichloro-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 202 : N-[2-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide ;
Composé 203 : N-[(R)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 205 : N-[(S)-2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 206 : N-[(S)-2-(5-(2-chloro-3-méthoxy-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 207 : N-[2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide ;
Composé 208 : 5-(2,3-dichlorophenyl)-3-methyl-1-[2-oxo-2-[4-(2-oxo-5-phenyl-1H-imidazol-3-yl)-1-piperidyl]ethyl]pyrimidine-2,4-dione ;
Composé 210 : 5-(2,3-dichlorophenyl)-3-isopropyl-1-[2-oxo-2-[4-(2-oxo-4,5-dihydro-1H-1,3-benzodiazepin-3-yl)-1-piperidyl]ethyl]pyrimidine-2,4-dione ;
Composé 213 : 5-(2,3-dichlorophenyl)-3-methyl-1-[2-oxo-2-[4-(2-oxo-4,5-dihydro-1H-pyrido[2,3-d][1,3]diazepin-3-yl)-1-piperidyl]ethyl]pyrimidine-2,4-dione ;
Composé 215 : 5-(2,3-dichlorophenyl)-3-methyl-1-[2-oxo-2-[4-(2-oxo-1H-quinolin-3-yl)-1-piperidyl]ethyl]pyrimidine-2,4-dione ;
Composé 220 : 5-(3,4-dichlorophenyl)-3-methyl-1-[2-oxo-2-[4-(2-oxo-4,5-dihydro-1H-1,3-benzodiazepin-3-yl)-1-piperidyl]ethyl]pyrimidine-2,4-dione ;
Composé 221 : 1-[2-[4-(7-bromo-2-oxo-4,5-dihydro-1H-1,3-benzodiazepin-3-yl)-1-piperidyl]-2-oxo-ethyl]-5-(2-chloro-3-fluoro-phenyl)-3-isopropyl-pyrimidine-2,4-dione ;
Composé 225 : 5-(2-chloro-3-fluoro-phenyl)-3-isopropyl-1-[2-[4-(7-methylsulfinyl-2-oxo-4,5-dihydro-1H-1,3-benzodiazepin-3-yl)-1-piperidyl]-2-oxo-ethyl]pyrimidine-2,4-dione ;
Composé 226 : 2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]acetamide ;
Composé 227 : 5-(2-chloro-3-fluoro-phenyl)-3-(2-methylsulfinylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 228 : 5-(2-chloro-3-fluoro-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 229 : 5-(2-chloro-3-fluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 231 : 5-(2,3-difluorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 232 : 5-(2-chloro-3-fluoro-phenyl)-3-[2-méthoxy-1-(méthoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 233 : 5-(2-chloro-3-fluoro-phenyl)-3-[(1S)-2-methoxy-1-methyl-ethyl]-1-[2-[4-(7-methylsulfanyl-2-oxo-4,5-dihydro-1H-1,3-benzodiazepin-3-yl)-1-piperidyl]-2-oxo-ethyl]pyrimidine-2,4-dione ;
Composé 234 : 5-(2-chloro-3-fluoro-phenyl)-3-(2-hydroxy-2-methyl-propyl)-1-[2-oxo-2-[4-(2-oxo-4,5-dihydro-1H-1,3-benzodiazepin-3-yl)-1-piperidyl]ethyl]pyrimidine-2,4-dione ;
Composé 235 : 5-(2-chloro-3-fluoro-phenyl)-1-[2-oxo-2-[4-(2-oxo-4,5-dihydro-1H-1,3-benzodiazepin-3-yl)-1-piperidyl]ethyl]-3-(2H-tetrazol-5-ylmethyl)pyrimidine-2,4-dione ;
Composé 236 : 5-(2,3-difluorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 237 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1S)-1-methylpropyl]pyrimidine-2,4-dione ;
Composé 238 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1R)-1-methylpropyl]pyrimidine-2,4-dione ;
Composé 239 : 5-(2-chloro-3-fluoro-phenyl)-3-[1-(methoxymethyl)propyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 240 : 5-(2-chloro-3-fluoro-phenyl)-3-(2-methoxy-2-methyl-propyl)-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 241 : 5-(2-chloro-3-methoxy-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 242 : 5-(2,3-dichlorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 243 : 5-(2-chloro-3-methoxy-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 244 : 5-(2-chloro-3-fluoro-phenyl)-3-[(1S)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione ;
Composé 245 : 5-(2,3-dichlorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 246 : acide 3-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propanoique ;
Composé 247 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,3,4-oxadiazol-2-yl)ethyl]pyrimidine-2,4-dione ;
Composé 248 : acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]acetique ;
Composé 249 : 5-(2-chloro-3-fluoro-phenyl)-3-[(1R)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione ;
Composé 251 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,2,4-triazol-4-yl)ethyl]pyrimidine-2,4-dione ;
Composé 252 : 5-(2-chloro-3-fluoro-phenyl)-1-[2-[4-(7-fluoro-2-oxo-4,5-dihydro-1H-1,3-benzodiazepin-3-yl)-1-piperidyl]-2-oxo-ethyl]-3-[(1R)-1-methyl-2-methylsulfonyl-ethyl]pyrimidine-2,4-dione ;
Composé 253 : acide 3-[1-[2-[5-(2-chloro-3-fluoro-phenyl)-3-(2-methylsulfonylethyl)-2,4-dioxo-pyrimidin-1-yl]acetyl]-4-piperidyl]-2-oxo-4,5-dihydro-1H-1,3-benzodiazepine-7-carboxylique ;
Composé 254 : S-[2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethyl]ethanethioate ;
Composé 255 : N-[(2S)-2-[5-(2,3-dichlorophenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide ;
Composé 256 : N-[(2S)-2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide ;
Composé 257 : acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethanesulfonique ;
Composé 258 : 5-(2-chloro-3-fluoro-phenyl)-3-[(1S)-1-(methoxymethyl)-2-methylsulfonyl-ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 259 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-(2-methyl-2-methylsulfonyl-propyl)pyrimidine-2,4-dione ;

A cet effet, les composés selon l'invention sont encore plus préférentiellement choisis parmi les composés listés dans le tableau I ci-après et pour lesquels, l'activité antagoniste au récepteur CGRP, définie par le Kd apparent ou Kdapp, est inférieure à 10nM (indiquée classe A dans le tableau I)

Parmi les composés selon l'invention, lorsque Y est -C(O), les composés selon l'invention sont avantageusement des dérivés uraciles.

Selon un mode particulièrement préféré de l'invention, les composés sont choisis parmi les composés de formule générale (I) dans laquelle :
- Y est -C(O) ;
- R1 est choisi parmi les groupements suivants : 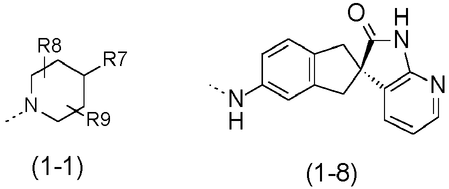
- R2, R3, R6 sont un atome d'hydrogène ;
- R4 est choisi parmi les groupements suivants : 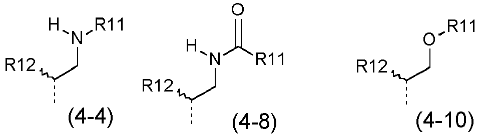 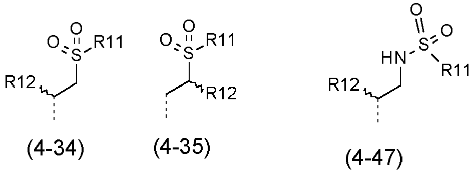
- R5 est 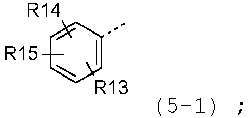
- R7 est 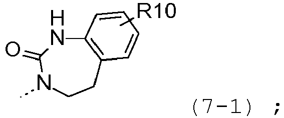
- R8, R9 sont un atome d'hydrogène ;
- R10 est choisi parmi un atome d'hydrogène, -OR11, -SR11 ou Br ;
- R11, R12 sont identiques ou différents, et choisis parmi un atome d'hydrogène ou un alkyle C1-C2 ;
- R13, R14, R15 sont identiques ou différents, et choisis parmi un atome d'hydrogène, -OR16, ou un halogène choisi parmi F et Cl ; et
- R16 est un alkyle C1-C3

Selon un mode encore plus particulièrement préféré de l'invention, les composés sont choisis parmi les composés de formule générale (I) dans laquelle :
- Y est -C(O) ;
- R1 est 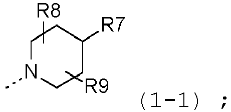
- R2, R3, R6 sont un atome d'hydrogène ;
- R4 est 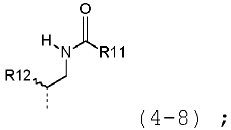
- R5 est 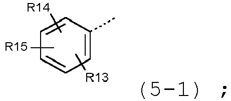
- R7 est 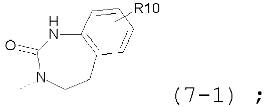
- R8, R9 sont un atome d'hydrogène ;
- R10 est -OR11 ;
- R11, R12 sont identiques ou différents, et choisis parmi -CH₃ ou -CH₂CH₃ ;
- R13, R14, R15 sont identiques ou différents, et choisis parmi un atome d'hydrogène, -OR16, ou un halogène choisi parmi F et Cl ; et
- R16 est un alkyle C1-C3.

Il va être décrit, à titre d'illustration et sans aucun caractère limitatif, divers exemples de préparation des composés selon l'invention.

Les composés correspondant à la présente invention peuvent être obtenus de manière non limitative en utilisant des conditions classiques en synthèse organique selon les voies réactionnelles générales décrites dans les schémas réactionnels n°1, 2, 3 et 4 des figures respectivement 1, 2, 3 et 4. Les conditions experimentales relatives à chaque schéma de synthèse sont divulguées dans les exemples ci-dessous qui renvoient à chaque fois au schéma suivi pour leur préparation. Ainsi, l'homme du métier saura apprécier si les conditions décrites dans ces schémas sont adaptées pour l'introduction des groupements fonctionnels voulus et éventuellement il adaptera la voie de synthèse par l'utilisation de groupements protecteurs adaptés, stables dans les conditions réactionnelles. Certains intermédiaires, permettant l'introduction de certains groupements R1 envisagés dans nos composés, sont obtenus au préalable en suivant des méthodes d'obtention déjà décrites dans la littérature ou décrites dans les exemples ci-après.

Plus particulièrement, les exemples ci-dessous décrivent de façon non limitative les modes opératoires suivis pour l'obtention des composés selon les schémas de synthèse n°1, 2, 3 ou 4. Chaque composé est principalement caractérisé par son spectre de RMN ¹H réalisé sur un spectromètre à résonance magnétique nucléaire Bruker 400MHz.

### Exemple 14: 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl}-1H-pyrimidine-2,4-dione (schéma réactionnel n°1, composé 14)

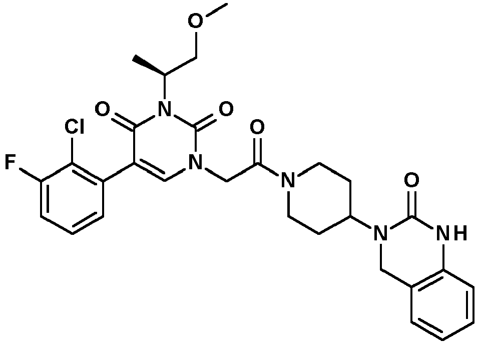

### 14.1: acétate de [5-bromo-3-((S)-2-méthoxy-1-méthyl-éthyl)-2,4-dioxo-3,4-dihydro-2H-pyrimidin-1-yl]-méthyle

De manière analogue à l'exemple 2.5, à partir de 1g (3,8 mmoles) de (5-bromo-2,4-dioxo-3,4-dihydro-2H-pyrimidin-1-yl)-acétate de méthyle de 0,4ml (4 mmoles) de (R)-1-méthoxy-propan-2-ol et après purification par chromatographie sur gel de silice élué par un mélange heptane / acétate d'éthyle 60/40, 1,2g (78%) d'acétate de [5-bromo-3-((S)-2-méthoxy-1-méthyl-éthyl)-2,4-dioxo-3,4-dihydro-2H-pyrimidin-1-yl]-méthyle sont obtenus sous la forme d'une huile incolore.

### 14.2: acide [5-(2-chloro-3-fluoro-phényl)-3-((S)-2-méthoxy-1-méthyl-éthyl)-2,4-dioxo-3,4-dihydro-2H-pyrimidin-1-yl]-acétique

De manière analogue à l'exemple 1.8, à partir de 250mg (1,4 mmoles) d'acide 2-chloro-3-fluorophénylboronique et de 400mg (1.2 mmoles) d' acétate de [5-bromo-3-((S)-2-méthoxy-1-méthyl-éthyl)-2,4-dioxo-3,4-dihydro-2H-pyrimidin-1-yl]-méthyle, 345mg (78 %) d'acide [5-(2-chloro-3-fluoro-phényl)-3-((S)-2-méthoxy-1-méthyl-éthyl)-2,4-dioxo-3,4-dihydro-2H-pyrimidin-1-yl]-acétique sont obtenus sous forme d'huile marron.

### 14.3: 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl}-1H-pyrimidine-2,4-dione (composé 14)

De manière analogue à l'exemple 13.4, à partir de 345mg (0,9 mmoles) d'acide [5-(2-chloro-3-fluoro-phényl)-3-((S)-2-méthoxy-1-méthyl-éthyl)-2,4-dioxo-3,4-dihydro-2H-pyrimidin-1-yl]-acétique, 170mg (30%) de 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide blanc.

RMN ¹H (δ, DMSO) : 1.34 (d, J = 7.0 Hz, 3H); 1.66 (m, 3H); 1.82-1.85 (m, 1H); 2.68-2.74 (m, 1H); 3.17 (t, J = 13.0 Hz, 1H); 3.24 (s, 3H); 3.53-3.57 (m, 1H); 3.90-3.98 (m, 2H); 4.29-4.30 (m, 2H); 4.42-4.48 (m, 2H); 4.76 (s, 2H); 5.14-5.16 (m, 1H); 6.77-6.79 (m, 1H); 6.84-6.88 (m, 1H); 7.09-7.15 (m, 2H); 7.21-7.23 (m, 1H); 7.44-7.47 (m, 2H); 7.81 (s, 1H); 9.24 (s, 1H).

### Exemple 16 : 5-(2,3-difluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 16)

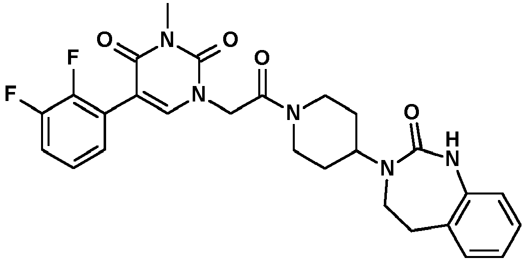

### 16.1: (1-benzyl-pipéridin-4-yl)-[2-(2-nitro-phényl)-éthyl]-amine

8ml (40 mmoles) de 1-benzyl-pipéridin-4-ylamine sont additionnés sur 4g (17,4 mmoles) de 1-(2-bromo-éthyl)-2-nitro-benzene et le mélange est chauffé à 100°C pendant 18 heures. Après refroidissement, de l'éther diéthylique est rajouté, le milieu est filtré et le filtrat est concentré. Le résidu brut est purifié par chromatographie sur gel de silice élué par un mélange acétate d'éthyle / heptane, 60/40 puis dichlorométhane / méthanol / ammoniaque, 95/3/2. 4,9g (82%) de (1-benzyl-pipéridin-4-yl)-[2-(2-nitro-phényl)-éthyl]-amine sont obtenus sous la forme d'une huile orangée.

### 16.2: [2-(2-amino-phényl)-éthyl]-(1-benzyl-pipéridin-4-yl)-amine

De manière analogue à l'exemple 1.5, à partir de 4,8g (14 mmoles) de (1-benzyl-pipéridin-4-yl)-[2-(2-nitro-phényl)-éthyl]-amine, 140mg d'oxyde de platine (10% molaire) et après purification du résidu par chromatographie sur gel de silice élué par un mélange dichlorométhane / méthanol / ammoniaque 90/8/2, 4,6g (100%) de [2-(2-amino-phényl)-éthyl]-(1-benzyl-pipéridin-4-yl)-amine sont obtenus sous la forme d'une huile brune.

### 16.3: 3-(1-benzyl-pipéridin-4-yl)-1,3,4,5-tétrahydro-benzo[d][1,3]diazépin-2-one

De manière analogue à l'exemple 1.4, à partir de 1g (6,5 mmoles) de [2-(2-amino-phényl)-éthyl]-(1-benzyl-pipéridin-4-yl)-amine, 3,9g (78%) de 3-(1-benzyl-pipéridin-4-yl)-1,3,4,5-tetrahydro-benzo[d] [1,3]diazépin-2-one sont obtenus sous la forme d'une poudre blanche.

### 16.4: 3-pipéridin-4-yl-1,3,4,5-tétrahydro-benzo[d][1,3]diazépin-2-one

De manière analogue à l'exemple 1.5, à partir de 3, 7g (11 mmoles) de 3-(1-benzyl-pipéridin-4-yl)-1,3,4,5-tetrahydro-benzo[d][1,3]diazépin-2-one, le milieu réactionnel étant placé sous 5bar de dihydrogène, 2,6g (96%) de 3-pipéridin-4-yl-1,3,4,5-tétrahydro-benzo[d][1,3]diazépin-2-one sont obtenus sous la forme d'une poudre blanche.

### 16.5: 1-benzhydryl-5-(2,3-difluoro-phényl)-3-méthyl-1H-pyrimidine-2,4-dione

De manière analogue à l'exemple 1.8, à partir de 2g (5,4 mmoles) de 1-benzhydryl-5-bromo-3-méthyl-1H-pyrimidine-2,4-dione (préparé comme décrit dans l'exemple 2.2) et 4,3g (27 mmoles) d'acide 2,3-difluorophénylboronique, et après purification par chromatographie sur gel de silice élué avec un mélange heptane / acétate d'éthyle 80/20, 1,6g (73%) de 1-benzhydryl-5-(2,3-difluoro-phényl)-3-méthyl-1H-pyrimidine-2,4-dione sont obtenus sous la forme d'un solide blanc.

### 16.6: 5-(2,3-difluoro-phényl)-3-méthyl-1H-pyrimidine-2,4-dione

De manière analogue à l'exemple 2.3, à partir de 1,6g (4 mmoles) de 1-benzhydryl-5-(2,3-difluoro-phényl)-3-méthyl-1H-pyrimidine-2,4-dione et après trituration du résidu brut dans l'éther diéthylique, filtration et séchage sous azote; 900mg (96%) de 5-(2,3-difluoro-phényl)-3-méthyl-1H-pyrimidine-2,4-dione sont obtenus sous la forme d'un solide beige.

### 16.7: [5-(2,3-difluoro-phényl)-3-méthyl-2,4-dioxo-3,4-dihydro-2H-pyrimidin-1-yl]-acétate de méthyle

De manière analogue à l'exemple 13.1, à partir de 450mg (1,9 mmoles) de 5-(2,3-difluoro-phényl)-3-méthyl-1H-pyrimidine-2,4-dione et 0,2ml (2,1 mmoles) de chloroacétate de méthyle, 475mg (81%) de [5-(2,3-difluoro-phényl)-3-méthyl-2,4-dioxo-3,4-dihydro-2H-pyrimidin-1-yl]-acétate de méthyle sont obtenus sous la forme d'un solide blanc.

### 16.8: acide [5-(2,3-difluoro-phényl)-3-méthyl-2,4-dioxo-3,4-dihydro-2H-pyrimidin-1-yl]-acétique

2,3ml (2,3 mmoles) d'une solution aqueuse d'hydroxyde de lithium 1N sont ajoutés à une solution de 475mg (1,5 mmoles) de [5-(2,3-difluoro-phényl)-3-méthyl-2,4-dioxo-3,4-dihydro-2H-pyrimidin-1-yl]-acétate de méthyle dans 15ml de tétrahydrofurane et 3mL d'eau. Le milieu réactionnel est agité à température ambiante pendant 2 heures, puis amené à pH6 par ajout de 4ml d'une solution aqueuse d'acide acétique 1N. Le produit est extrait à l'acétate d'éthyle. La phase organique est lavée par de l'eau puis par une solution aqueuse saturée de chlorure de sodium, séchée sur sulfate de magnésium, filtrée et concentrée sous vide. 400mg (88%) d'acide [5-(2,3-difluoro-phényl)-3-méthyl-2,4-dioxo-3,4-dihydro-2H-pyrimidin-1-yl]-acétique sont obtenus sous la forme d'un solide blanc.

### 16.9: 5-(2,3-difluoro-phényl)-3-méthyl-1-{2-oxo-2-[4-(2-oxo-1,2, 4,5-tétrahydro-benzo [d][1,3]diazépin-3-yl)-pipéridin-1-yl]-éthyl}-1H-pyrimidine-2,4-dione (composé 16)

De manière analogue à l'exemple 1.6, à partir de 100mg (0,3 mmoles) d'acide [5-(2,3-difluoro-phényl)-3-méthyl-2,4-dioxo-3,4-dihydro-2H-pyrimidin-1-yl]-acétique et 100mg (0,4 mmoles) de 3-pipéridin-4-yl-1,3,4,5-tétrahydro-benzo[d][1,3]diazépin-2-one (préparé comme décrit dans l'exemple 16.4) et après cristallisation dans un mélange heptane / acétate d'éthyle, 70/30; 120mg (67%) de 5-(2,3-difluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 160°C.

RMN ¹H (δ, DMSO) : 1.46 - 1.62 (m, 1H), 1.62 - 1.82 (m, 3H), 2.67 (t, J = 16 Hz, 1H), 2.89 (t, J = 4.9 Hz, 2H), 3.15 (t, J = 16 Hz, 1H), 3.25 (s, 3H), 3.33 - 3.41 (m, 2H), 3.95 (d, J = 13.5 Hz, 1H), 4.27 - 4.37 (m, 1H), 4.41 (d, J = 8 Hz, 1H), 4.79 (s, 2H), 6.73 - 6.85 (m, 1H), 6.96 - 7.10 (m, 3H), 7.16 - 7.34 (m, 2H), 7.38 - 7.55 (m, 1H), 7.93 (s, 1H), 8.54 (s, 1H).

### Exemple 17 : 5-(2,3-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 17)

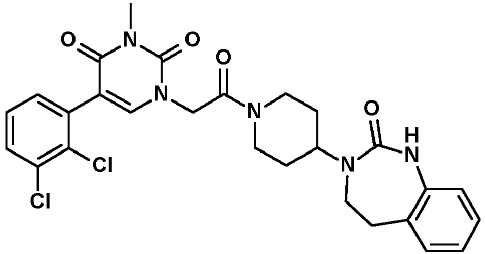

### 17.1: (5-bromo-3-méthyl-2,4-dioxo-3,4-dihydro-2H-pyrimidin-1-yl)-acétate de méthyle

De manière analogue à l'exemple 13.1, à partir de 1,5g (7,1 mmoles) de 5-bromo-3-méthyl-1H-pyrimidine-2,4-dione (préparé comme décrit dans l'exemple 2.3) et 0,7ml (7,8 mmoles) de chloro-acétate de méthyle, 1,6g (79%) de (5-bromo-3-méthyl-2,4-dioxo-3,4-dihydro-2H-pyrimidin-1-yl)-acétate de méthyle sont obtenus sous la forme d'un solide blanc.

### 17.2: acide (5-bromo-3-méthyl-2,4-dioxo-3,4-dihydro-2H-pyrimidin-1-yl)-acétique

De manière analogue à l'exemple 16.8, à partir de 1,6g (5,6 mmoles) de (5-bromo-3-méthyl-2,4-dioxo-3,4-dihydro-2H-pyrimidin-1-yl)-acétate de méthyle, 1,1g (76%) d'acide (5-bromo-3-méthyl-2,4-dioxo-3,4-dihydro-2H-pyrimidin-1-yl)-acétique sont obtenus sous la forme d'un solide blanc.

### 17.3: 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione

De manière analogue à l'exemple 1.6, à partir de 960mg (3,7 mmoles) d'acide (5-bromo-3-méthyl-2,4-dioxo-3,4-dihydro-2H-pyrimidin-1-yl)-acétique et 895mg (3,7 mmoles) de 3-pipéridin-4-yl-1,3,4,5-tetrahydro-benzo[d][1,3]diazépin-2-one (préparé comme décrit dans l'exemple 16.4), 1,7g (95%) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.

### 17.4: 5-(2,3-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 17)

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione et 58mg (0,3 mmoles) d'acide 2,3-dichlorophénylboronique, et après purification par chromatographie sur gel de silice élué par un mélange dichlorométhane / méthanol 99/1, 40mg (35%) de 5-(2,3-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 170°C.

RMN ¹H (δ, DMSO) : 1.39 - 1.53 (m, 1H), 1.55 - 1.72 (m, 3H), 2.61 (t, J = 12.4 Hz, 1H), 2.77 - 2.88 (m, 2H), 3.08 (t, J = 12.7 Hz, 1H), 3.18 (s, 3H), 3.27 - 3.34 (m, 2H), 3.87 (d, J = 13.6 Hz, 1H), 4.24 - 4.34 (m, 1H), 4.35 (d, J = 12.9 Hz, 1H), 4.70 (d, J = 3.4 Hz, 2H), 6.66 - 6.80 (m, 1H), 6.89 - 7.04 (m, 3H), 7.26 (dd, J = 7.7, 1.6 Hz, 1H), 7.36 (t, J = 7.8 Hz, 1H), 7.62 (dd, J = 8.1, 1.6 Hz, 1H), 7.76 (s, 1H), 8.48 (s, 1H).

### Exemple 18 : 5-(2-chloro-phényl)-3-méthyl-1-{2-oxo-2-[4-(2-oxo-1,2,4,5-tétrahydro benzo[d][1,3]diazépin-3-yl)-pipéridin-1-yl]-éthyl}-1H-pyrimidine-2,4-dione (schéma réactionnel n°3, composé 18)

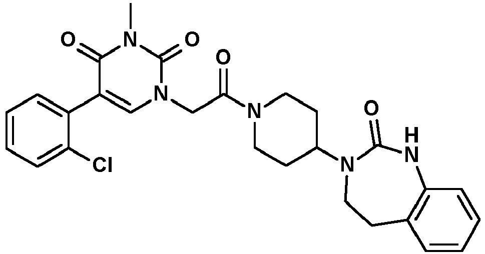

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 48mg (0,3 mmoles) d'acide 2-chlorophénylboronique, 40mg (38%) de 5-(2-chloro-phényl)-3-méthyl-1-{2-oxo-2-[4-(2-oxo-1,2,4,5 tétrahydrobenzo[d] [1,3]diazépin-3-yl)-pipéridin-1-yl]-éthyl}-1H-pyrimidine-2,4-dione sont obtenus sous la forme d'un solide beige de point de fusion 175°C.

RMN ¹H (δ, DMSO) : 1.40-1.55 (m, 1H), 1.58-1.70 (m, 3H), 2.55-2.68 (m, 1H), 2.82 (t, J = 4.7 Hz, 2H), 3.05-3.12 (m, 1H), 3.18 (s, 3H), 3.30 (t, J = 4.4 Hz, 2H), 3.88 (m, 1H), 4.20-4.30 (m, 1H), 4.38 (m, 1H), 4.70 (d, J = 4.2 Hz, 2H), 6.72-6.75 (m, 1H), 6.95-6.98 (m, 3H), 7.26-7.28 (m, 1H), 7.31-7.36 (m, 2H), 7.46-7.48 (m, 1H), 7.71 (s, 1H), 8.47 (s, 1H).

### Exemple 19 : 3-méthyl-1-{2-oxo-2-[4-(2-oxo-1,2,4,5-tétrahydro-benzo[d][1,3]diazépin-3-yl)-pipéridin-1-yl]-éthyl}-5-(2-trifluorométhoxy-phényl)-1H-pyrimidine-2,4-dione (schéma réactionnel n°3, composé 19)

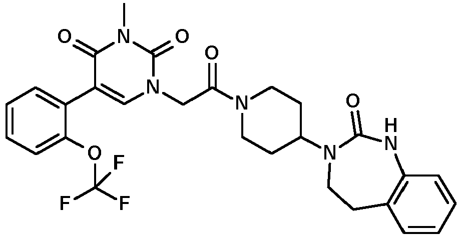

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 63mg (0,3 mmoles) d'acide 2-(trifluorométhoxy)benzène boronique, 40mg (34%) de 3-méthyl-1-{2-oxo-2-[4-(2-oxo-1,2,4,5-tétrahydro-benzo[d][1,3]diazépin-3-yl)-pipéridin-1-yl]-éthyl}-5-(2-trifluorométhoxy-phényl)-1H-pyrimidine-2,4-dione sont obtenus sous la forme d'un solide rose de point de fusion 235°C.

RMN ¹H (δ, DMSO) : 1.40-1.55 (m, 1H), 1.58-1.72 (m, 3H), 2.55-2.68 (m, 1H), 2.82 (t, J = 4.5 Hz, 2H), 3.05-3.15 (m, 1H), 3.18 (s, 3H), 3.30 (t, J = 4.5 Hz, 2H), 3.90 (m, 1H), 4.20-4.30 (m, 1H), 4.35 (m, 1H), 4.71 (s, 2H), 6.71-6.75 (m, 1H), 6.95-6.98 (m, 3H), 7.33-7.40 (m, 3H), 7.44-7.48 (m, 1H), 7.74 (s, 1H), 8.47 (s, 1H).

### Exemple 22 : 5-(3-chloro-phényl)-3-méthyl-1-{2-oxo-2-[4-(2-oxo-1,2, 4,5-tétrahydro-benzo[d][1,3]diazépin-3-yl)-pipéridin-1-yl]-éthyl}-1H-pyrimidine-2,4-dione (schéma réactionnel n°3, composé 22)

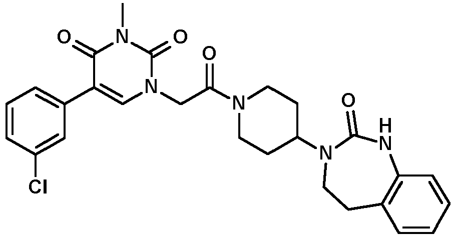

De manière analogue à l'exemple 1.8, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 48mg (0,3 mmoles) d'acide 3-chlorophénylboronique, 55mg (52%) de 5-(3-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 220°C.

RMN ¹H (δ, DMSO) : 1.50-1.60 (m, 1H), 1.62-1.70 (m, 3H), 2.69 (m, 1H), 2.90 (t, J = 4.8 Hz, 2H), 3.26 (m, 1H), 3.33 (s, 3H), 3.38 (t, J = 4.6 Hz, 2H), 3.95 (m, 1H), 4.30-4.40 (m, 1H), 4.45 (m, 1H), 4.81 (s, 2H), 6.79-6.82 (m, 1H), 7.03-7.05 (m, 3H), 7.38-7.41 (m, 1H), 7.45 (t, J = 7.9 Hz, 1H), 7.55-7.65 (m, 1H), 7.65 (d, J = 1.8 Hz, 1H), 8.05 (s, 1H), 8.55 (s, 1H).

### Exemple 23 : 5-(3-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 23)

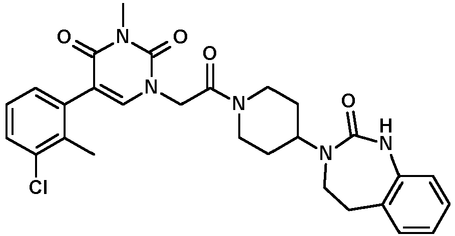

De manière analogue à l'exemple 9, à partir de 150mg (0,3 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 78mg (0,5 mmoles) d'acide 3-chloro-2-méthylphénylboronique, 75mg (46%) de 5-(3-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide gris de point de fusion 190°C.

RMN ¹H (δ, DMSO) : 1.45-1.60 (m, 1H), 1.62-1.80 (m, 3H), 2.20 (s, 3H), 2.70 (m, 1H), 2.89 (t, J = 4.5 Hz, 2H), 3.15 (m, 1H), 3.25 (s, 3H), 3.37 (t, J = 4.8 Hz, 2H), 3.95 (m, 1H), 4.35 (m, 1H), 4.45 (m, 1H), 4.76 (d, J = 2.6 Hz, 2H), 6.78-6.85 (m, 1H), 7.02-7.05 (m, 3H), 7.13 (d, J = 6.8 Hz, 1H), 7.26 (t, J = 7.8 Hz, 1H), 7.47 (d, J = 7.2 Hz, 1H), 7.72 (s, 1H), 8.54 (s, 1H).

### Exemple 24 : 5-(3-chloro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 24)

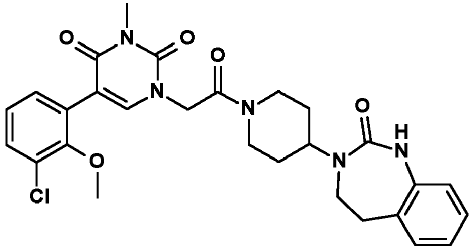

De manière analogue à l'exemple 9, à partir de 150mg (0,3 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 86mg (0,5 mmoles) d'acide 3-chloro-2-méthoxyphényl boronique, 60mg (36%) de 5-(3-chloro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 260°C.

RMN ¹H (δ, DMSO) : 1.50-1.60 (m, 1H), 1.62-1.80 (m, 3H), 2.68 (m, 1H), 2.89 (t, J = 4.4 Hz, 2H), 3.15 (m, 1H), 3.26 (s, 3H), 3.37 (t, J = 4.5 Hz, 2H), 3.65 (s, 3H), 3.95 (m, 1H), 4.30 (m, 1H), 4.45 (m, 1H), 4.78 (d, J = 2.44 Hz, 2H), 6.78-6.85 (m, 1H), 7.02-7.05 (m, 3H), 7.18 (t, J = 7.8 Hz, 1H), 7.27 (dd, J = 1.7-7.7 Hz, 1H), 7.49 (dd, J = 1.7-8.0 Hz, 1H), 7.80 (s, 1H), 8.54 (s, 1H).

### Exemple 28 : 5-(3-fluoro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 28)

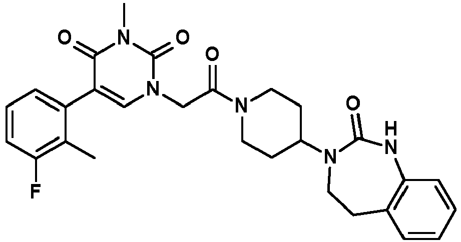

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 47mg (0,3 mmoles) d'acide 3-fluoro-2-méthyl-phényl boronique, 50mg (47%) de 5-(3-fluoro-2-méthyl-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide gris de point de fusion 160°C.

RMN ¹H (δ, DMSO) : 1.50-1.60 (m, 1H), 1.55-1.74 (m, 3H), 2.08 (d, J = 2 Hz, 3H), 2.55-2.70 (m, 1H), 2.89 (t, J = 4.5 Hz, 2H), 3.10-3.20 (m, 1H), 3.25 (s, 3H), 3.37 (d, J = 4.4 Hz, 2H), 3.95 (d, J = 13.7 Hz, 1H), 4.30-4.40 (m, 1H), 4.42 (d, J = 12.5 Hz, 1H), 4.77 (d, J = 2 Hz, 2H), 6.78-6.82 (m, 1H), 7.00-7.05 (m, 4H), 7.18 (t, J = 8.8 Hz, 1H), 7.24-7.27 (m, 1H), 7.71 (s, 1H), 8.54 (s, 1H).

### Exemple 29 : 5-(3-chloro-2-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 29)

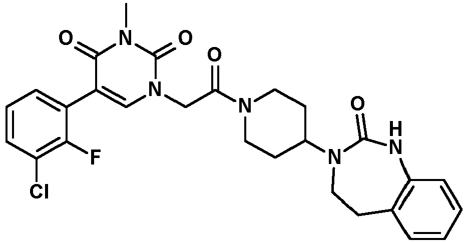

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 53mg (0,3 mmoles) d'acide 3-chloro-2-fluoro-phényl boronique, 65mg (57%) de 5-(3-chloro-2-fluoro-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide gris de point de fusion 206°C.

RMN ¹H (δ, DMSO) : 1.50-1.60 (m, 1H), 1.65-1.80 (m, 3H), 2.69 (t, J = 12 Hz, 1H), 2.90 (m, 2H), 3.16 (t, J = 10 Hz, 1H), 3.26 (s, 3H), 3.37 (m, 2H), 3.95 (d, J = 12.6 Hz, 1H), 4.30-4.40 (m, 1H), 4.42 (d, J = 11.7 Hz, 1H), 4.80 (s, 2H), 6.81 (d, J = 3.7 Hz, 1H), 7.03-7.04 (m, 3H), 7.29 (t, J = 7.8 Hz, 1H), 7.37 (t, J = 6.5 Hz, 1H), 7.61 (t, J = 7.2 Hz, 1H), 7.93 (s, 1H), 8.54 (s, 1H).

### Exemple 30 : 5-(2,3-diméthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 30)

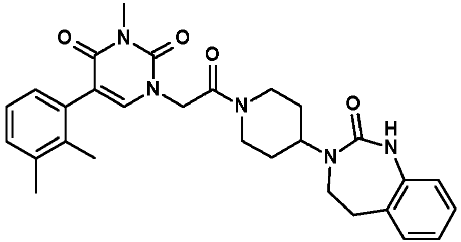

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 46mg (0,3 mmoles) d'acide 2,3-diméthyl-phényl boronique, 50mg (48%) de 5-(2,3-diméthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc cassé de point de fusion 253°C.

RMN ¹H (δ, DMSO) : 1.50-1.60 (m, 1H), 1.55-1.80 (m, 3H), 2.06 (s, 3H), 2.28 (s, 3H), 2.68 (t, J = 12 Hz, 1H), 2.90 (t, J = 4 Hz, 2H), 3.15 (t, J = 12 Hz, 1H), 3.25 (s, 3H), 3.39 (t, J = 4 Hz, 2H), 3.95 (d, J = 12 Hz, 1H), 4.30-4.40 (m, 1H), 4.44 (d, J = 12 Hz, 1H), 4.77 (d, J = 4 Hz, 2H), 6.79-6.83 (m, 1H), 6.96 (d, J = 8 Hz, 1H), 7.02-7.05 (m, 3H), 7.11 (t, J = 8 Hz, 1H), 7.18 (d, J = 8 Hz, 1H), 7.60 (s, 1H), 8.52 (s, 1H).

### Exemple 31 : 5-(2-chloro-3-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 31)

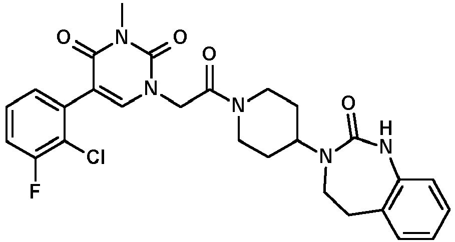

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 53mg (0,3 mmoles) d'acide 2-chloro-3-fluoro-phényl-phénylboronique, 75mg (68%) de 5-(2-chloro-3-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide gris de point de fusion 162°C.

RMN ¹H (δ, DMSO) : 1.50-1.60 (m, 1H), 1.65-1.75 (m, 3H), 2.68 (t, J = 12 Hz, 1H), 2.89 (t, J = 4.4 Hz, 2H), 3.15 (t, J = 12 Hz, 1H), 3.25 (s, 3H), 3.37 (t, J = 4.3 Hz, 2H), 3.94 (d, J = 12 Hz, 1H), 4.31-4.34 (m, 1H), 4.42 (d, J = 12 Hz, 1H), 4.78 (d, J = 1.96 Hz, 2H), 6.78-6.82 (m, 1H), 7.02-7.05 (m, 3H), 7.20-7.22 (m, 1H), 7.43-7.47 (m, 2H), 7.84 (s, 1H), 8.54 (s, 1H) .

### Exemple 47 : 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 47)

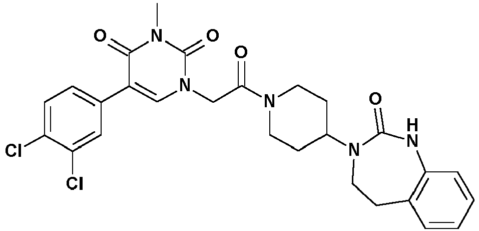

### 47.1: acide [5-(3,4-dichloro-phényl)-3-méthyl-2,4-dioxo-3,4-dihydro-2H-pyrimidin-1-yl]-acétique

De manière analogue à l'exemple 44.1, à partir de 450mg (1,6 mmoles) de (5-bromo-3-méthyl-2,4-dioxo-3,4-dihydro-2H-pyrimidin-1-yl)-acétate de méthyle (préparé comme décrit dans l'exemple 17.1) et 620mg (3,3 mmoles) d'acide 3,4-dichlorophénylboronique, et après ajout de 5ml d'une solution de soude aqueuse 1N et chauffage à 100°C pendant 1 heure, 520mg (97%) d' acide [5-(3,4-dichloro-phényl)-3-méthyl-2,4-dioxo-3,4-dihydro-2H-pyrimidin-1-yl]-acétique sont obtenus sous la forme d'un solide beige.

### 47.2: 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 47)

De manière analogue à l'exemple 1.6, à partir de 400mg (1,2 mmoles) d' acide [5-(3,4-dichloro-phényl)-3-méthyl-2,4-dioxo-3,4-dihydro-2H-pyrimidin-1-yl]-acétique et 358mg (1,5 mmoles) de 3-pipéridin-4-yl-1,3,4,5-tétrahydro-benzo[d][1,3]diazépin-2-one (préparé comme décrit dans l'exemple 16.4), 320mg (47%) de 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.

RMN ¹H (δ, DMSO) : 1.52-1.61 (m, 4 H). 2.63 (t, J = 10.6 Hz, 1 H); 2.84 (m, 2 H); 3.10 (m, 1 H); 3.19 (s, 3 H), 3.30-3.33 (m, 2 H); 3.90 (d, J = 13.6 Hz, 1 H); 4.26 (m, 1 H); 4.35 (d, J = 12.5 Hz, 1 H); 4.74 (s, 2 H); 6.73-6.74 (m, 1 H); 6.96-6.97 (m,

3 H); 7.55 (dd, J = 8.5, 2.1 Hz, 1 H); 7.62 (d, J = 8.5 Hz, 1 H); 7.81 (d, J = 2.1 Hz, 1 H); 8.05 (s, 1 H); 8.47 (s, 1 H).

### Exemple 51 : 5-(2,3-diméthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 51)

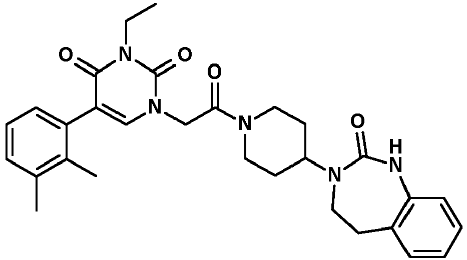

### 51.1: (5-bromo-3-éthyl-2,4-dioxo-3,4-dihydro-2H-pyrimidin-1-yl)-acétate de méthyle

2,4g (17 mmoles) de carbonate de potassium et 1,4ml (17 mmoles) de iodoéthane sont ajoutés à une solution de 3g (11 mmoles) de (5-bromo-2,4-dioxo-3,4-dihydro-2H-pyrimidin-1-yl)-acétate de méthyle (préparé comme décrit dans l'exemple 13.1) dans 60ml de diméthylformamide. Le milieu réactionnel est chauffé à 50°C pendant 2 heures puis hydrolysé et extrait par de l'acétate d'éthyle. La phase organique est lavée une fois par de l'eau et une fois par une solution aqueuse saturée de chlorure de sodium, puis séchée sur sulfate de magnésium, filtrée et concentrée sous vide. Le résidu brut est repris dans de l'éther diéthylique puis filtré et séché sous azote. 1,7g (50%) de (5-bromo-3-éthyl-2,4-dioxo-3,4-dihydro-2H-pyrimidin-1-yl)-acétate de méthyle sont obtenus sous la forme d'un solide crème.

### 51.2: [5-(2,3-diméthyl-phényl)-3-éthyl-2,4-dioxo-3,4-dihydro-2H-pyrimidin-1-yl]-acétate de méthyle

De manière analogue à l'exemple 44.1, à partir de 350mg (1,2 mmoles) de (5-bromo-3-éthyl-2,4-dioxo-3,4-dihydro-2H-pyrimidin-1-yl)-acétate de méthyle et 289mg (1,9 mmoles) d'acide 2,3-diméthylphénylboronique, et après purification par chromatographie sur gel de silice élué avec un mélange heptane / acétate d'éthyle 70/30, 255mg (67%) de [5-(2,3-diméthyl-phényl)-3-éthyl-2,4-dioxo-3,4-dihydro-2H-pyrimidin-1-yl]-acétate de méthyle sont obtenus sous la forme d'un solide blanc.

### 51.3: acide [5-(2,3-diméthyl-phényl)-3-éthyl-2,4-dioxo-3,4-dihydro-2H-pyrimidin-1-yl]-acétique

De manière analogue à l'exemple 16.8, à partir de 255mg (0,8 mmoles) de [5-(2,3-diméthyl-phényl)-3-éthyl-2,4-dioxo-3,4-dihydro-2H-pyrimidin-1-yl]-acétate de méthyle, 220mg (90%) d' acide [5-(2,3-diméthyl-phényl)-3-éthyl-2,4-dioxo-3,4-dihydro-2H-pyrimidin-1-yl]-acétique sont obtenus sous la forme d'un solide blanc.

### 51.4: 5-(2,3-diméthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 51)

De manière analogue à l'exemple 1.6, à partir de 220mg (0,7 mmoles) d' acide [5-(2,3-diméthyl-phényl)-3-éthyl-2,4-dioxo-3,4-dihydro-2H-pyrimidin-1-yl]-acétique, 196mg (0,8 mmoles) de 3-pipéridin-4-yl-1,3,4,5-tétrahydro-benzo[d] [1,3]diazépin-2-one (préparé comme décrit dans l'exemple 16.4) et 0,1ml (0,9 mmoles) de triéthylamine et après purification par chromatographie sur gel de silice élué avec un mélange dichlorométhane / méthanol 97/3 suivie d'une cristallisation dans un mélange heptane / acétate d'éthyle 70/30, 280mg (72%) de 5-(2,3-diméthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 170°C.

RMN ¹H (δ, DMSO) : 1.13 (t, J = 7 Hz, 3H), 1.48-1.60 (m, 1H), 1.62-1.80 (m, 3H), 2.05 (t, 3H), 2.30 (t, 3H), 2.68 (t, J = 11.7 Hz, 1H), 2.89 (t, J = 4.6 Hz, 2H), 3.15 (t, J = 11.7 Hz, 1H), 3.37 (t, J = 4.6 Hz, 2H), 3.88-3.95 (m, 3H), 4.28-4.36 (m, 1H), 4.43 (d, J = 12.7 Hz, 1H), 4.75 (d, J = 6.4 Hz, 2H), 6.77-6.83 (m, 1H), 6.96 (d, J = 7 Hz, 1H), 7.02-7.06 (m, 3H), 7.10 (t, J = 7.5 Hz, 1H), 7.17 (d, J = 7.3 Hz, 1H), 7.58 (s, 1H), 8.52 (s, 1H) .

### Exemple 52 : 5-(3-chloro-2-méthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 52)

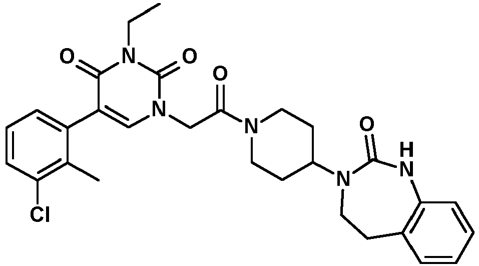

### 52.1: acide [5-(3-chloro-2-méthyl-phényl)-3-éthyl-2,4-dioxo-3,4-dihydro-2H-pyrimidin-1-yl]-acétique

De manière analogue à l'exemple 13.3, à partir de 350mg (1,2 mmoles) de (5-bromo-3-éthyl-2,4-dioxo-3,4-dihydro-2H-pyrimidin-1-yl)-acétate de méthyle (préparé comme décrit dans l'exemple 51.1), et 328mg (1,9 mmoles) d'acide 3-chloro-2-méthyl-phényl boronique, 330mg (85%) d'acide [5-(3-chloro-2-méthyl-phényl)-3-éthyl-2,4-dioxo-3,4-dihydro-2H-pyrimidin-1-yl]-acétique sont obtenus sous la forme d'un solide beige.

### 52.2: 5-(3-chloro-2-méthyl-phényl)-3-éthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 52)

De manière analogue à l'exemple 51.4, à partir de 330mg (1 mmole) d' acide [5-(3-chloro-2-méthyl-phényl)-3-éthyl-2,4-dioxo-3,4-dihydro-2H-pyrimidin-1-yl]-acétique et 276mg (1,1 mmoles) de 3-pipéridin-4-yl-1,3,4,5-tétrahydro-benzo[d] [1,3]diazépin-2-one (préparé comme décrit dans l'exemple 16.4), 290mg (51%) de 5-(3-chloro-2-méthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 247°C.

RMN ¹H (δ, DMSO) : 1.13 (t, J = 7 Hz, 3H), 1.50-1.62 (m, 1H), 1.52-1.80 (m, 3H), 2.19 (s, 3H), 2.68 (t, J = 12.2 Hz, 1H), 2.89 (t, J = 4.1 Hz, 2H), 3.15 (t, J = 12.2 Hz, 1H), 3.37 (t, J = 4.1 Hz, 2H), 3.88-3.96 (m, 3H), 4.29-4.35 (m, 1H), 4.43 (d, J = 12.7 Hz, 1H), 4.76 (d, J = 4.2 Hz, 2H), 6.78-6.83 (m, 1H), 7.02-7.05 (m, 3H), 7.14 (d, J = 6.8 Hz, 1H), 7.26 (t, J = 7.8 Hz, 1H), 7.46 (d, J = 7.2 Hz, 1H), 7.70 (s, 1H), 8.52 (s, 1H).

### Exemple 53 : 5-(2,3-dichloro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 53)

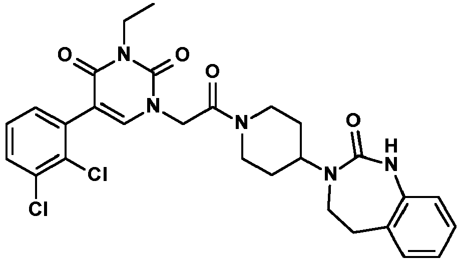

### 53.1: acide [5-(2,3-dichloro-phényl)-3-éthyl-2,4-dioxo-3,4-dihydro-2H-pyrimidin-1-yl]-acétique

De manière analogue à l'exemple 13.3, à partir de 350mg (1,2 mmoles) de (5-bromo-3-éthyl-2,4-dioxo-3,4-dihydro-2H-pyrimidin-1-yl)-acétate de méthyle (préparé comme décrit dans l'exemple 51.1), et 367mg (1,9 mmoles) d'acide 2,3-dichlorophénylboronique, 120mg (29%) d'acide [5-(2,3-dichloro-phényl)-3-éthyl-2,4-dioxo-3,4-dihydro-2H-pyrimidin-1-yl]-acétique sont obtenus sous la forme d'un solide beige.

### 53.2: 5-(2,3-dichloro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 53)

De manière analogue à l'exemple 51.4, à partir de 120mg (0,4 mmoles) d' acide [5-(2,3-dichloro-phényl)-3-éthyl-2,4-dioxo-3,4-dihydro-2H-pyrimidin-1-yl]-acétique et 94mg (0,4 mmoles) 3-pipéridin-4-yl-1,3,4,5-tétrahydro-benzo[d][1,3]diazépin-2-one (préparé comme décrit dans l'exemple 16.4), 130mg (63%) de 5-(2,3-dichloro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 170°C.

RMN ¹H (δ, DMSO) : 1.13 (t, J = 6.9 Hz, 3H), 1.48-1.60 (m, 1H), 1.62-1.80 (m, 3H), 2.68 (t, J = 11 Hz, 1H), 2.88 (t, J = 4.4 Hz, 2H), 3.15 (t, J = 11 Hz, 1H), 3.37 (t, J = 4.0 Hz, 2H), 3.89-3.91 (m, 2H), 3.95 (d, J = 13 Hz, 1H), 4.28-4.38 (m, 1H), 4.45 (d, J = 13 Hz, 1H), 4.76 (d, J = 5.2 Hz, 2H), 6.75-6.83 (m, 1H), 7.02-7.04 (m, 3H), 7.33 (dd, J = 1.6-7.6 Hz, 1H), 7.42 (t, J = 7.8 Hz, 1H), 7.68 (dd, J = 1.6-8.1 Hz, 1H), 7.80 (s, 1H), 8.54 (s, 1H).

### Exemple 54 : 5-(2-chloro-3-fluoro-phényl)-3-éthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 54)

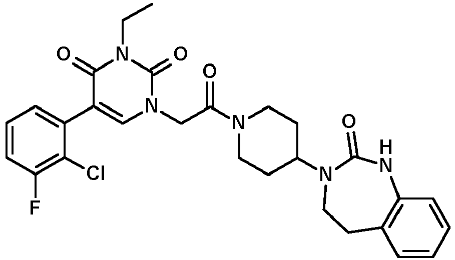

### 54.1: acide [5-(2-chloro-3-fluoro-phényl)-3-éthyl-2,4-dioxo-3,4-dihydro-2H-pyrimidin-1-yl]-acétique

De manière analogue à l'exemple 13.3, à partir de 300mg (1 mmole) de (5-bromo-3-éthyl-2,4-dioxo-3,4-dihydro-2H-pyrimidin-1-yl)-acétate de méthyle (préparé comme décrit dans l'exemple 51.1), et 270mg (1,6 mmoles) d'acide 2-chloro-3-fluoro-phényl boronique, 255mg (76%) d'acide [5-(2-chloro-3-fluoro -phényl)-3-éthyl-2,4-dioxo-3,4-dihydro-2H-pyrimidin-1-yl]-acétique sont obtenus sous la forme d'un solide marron.

### 54.2: 5-(2-chloro-3-fluoro-phényl)-3-éthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 54)

De manière analogue à l'exemple 51.4, à partir de 255mg (0,8 mmoles) d' acide [5-(2-chloro-3-fluoro-phényl)-3-éthyl-2,4-dioxo-3,4-dihydro-2H-pyrimidin-1-yl]-acétique et 211mg (0,9 mmoles) de 3-pipéridin-4-yl-1,3,4,5-tétrahydro-benzo[d] [1,3]diazépin-2-one (préparé comme décrit dans l'exemple 16.4), 190mg (44%) de 5-(2-chloro-3-fluoro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 248°C.

RMN ¹H (δ, DMSO) : 1.13 (t, J = 6.9 Hz, 3H), 1.45-1.60 (m, 1H), 1.62-1.80 (m, 3H), 2.68 (t, J = 12.1 Hz, 1H), 2.89 (t, J = 4.2 Hz, 2H), 3.15 (t, J = 10.3 Hz, 1H), 3.37 (t, J = 4.2 Hz, 2H), 3.88-3.95 (m, 3H), 4.3-4.35 (m, 1H), 4.43 (d, J = 12.6 Hz, 1H), 4.77 (d, J = 4.4 Hz, 2H), 6.78-6.82 (m, 1H), 7.02-7.05 (m, 3H), 7.21-7.23 (m, 1H), 7.43-7.48 (m, 2H), 7.82 (s, 1H), 8.54 (s, 1H).

### composé 72: (5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-acetonitrile

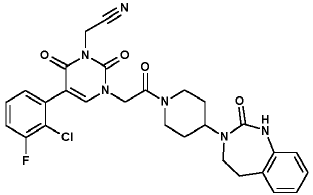

RMN ¹H (400 MHz, DMSO): δ (ppm) 1.56 (d, J = 13.8 Hz, 1H); 1.65-1.77 (m, 3H); 2.70 (t, J = 12.5 Hz, 1H); 2.90 (t, J = 4.9 Hz, 2H); 3.17 (t, J= 12.6 Hz, 1H); 3.38 (s, 2H); 3.93-3.96 (m, 1H); 4.33 (t, J = 11.4 Hz, 1H); 4.43 (d, J = 12.9 Hz, 1H); 4.85 (s, 2H); 4.94 (s, 2H); 6.79-6.83 (m, 1H); 7.04 (t, J = 4.5 Hz, 3H); 7.24-7.29 (m, 4H); 7.44-7.49 (m, 2H); 7.98 (s, 1H); 8.53 (s, 1H).

### composé 73: Acide 3-(5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-propanoïque

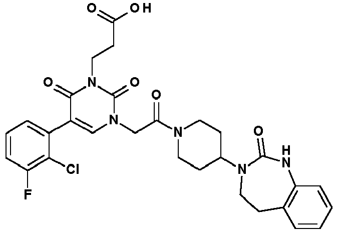

RMN ¹H (400 MHz, DMSO): δ (ppm) 1.55 (d, J= 12.9 Hz, 1H); 1.72 (t, J = 21.2 Hz, 3H); 2.50 (m, 2H); 2.69 (t, J = 12.4 Hz, 1H); 2.90 (m, 2H); 3.16 (m, 1H); 3.38 (m, 2H); 3.95 (d, J = 13.6 Hz, 1H); 4.09 (t, J = 7.7 Hz, 2H); 4.33 (s, 1H); 4.43 (d, J = 12.8 Hz, 1H); 4.78 (s, 2H); 6.81 (dt, J = 7.8, 4.2 Hz, 1H); 7.04 (t, J = 4.0 Hz, 3H); 7.22 (dd, J = 6.3, 2.8 Hz, 1H); 7.43-7.46 (m, 2H); 7.84 (s, 1H); 8.53 (s, 1H).

### Exemple 83 : 5-(2,3-dichloro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 83)

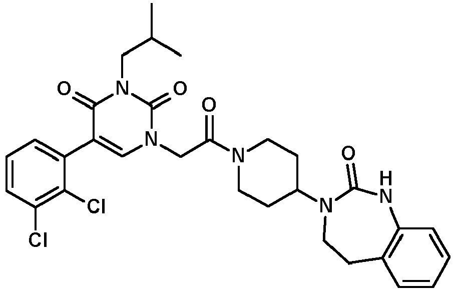

### 83.1 : (5-bromo-3-isobutyl-2,4-dioxo-3,4-dihydro-2H-pyrimidin-1-yl)-acétate de méthyle

De manière analogue à l'exemple 1.7, à partir de 2g (8 mmoles) de (5-bromo-2,4-dioxo-3,4-dihydro-2H-pyrimidin-1-yl)-acétate de méthyle (préparé comme décrit dans l'exemple 13.1), et de 1,2ml (11 mmoles) de 1-bromo-2-méthyl propane, et après chauffage du mélange à 50°C pendant 4 heures, 1,8g (74%) de (5-bromo-3-isobutyl-2,4-dioxo-3,4-dihydro-2H-pyrimidin-1-yl)-acétate de méthyle sont obtenus sous la forme d'un solide blanc.

### 83.2 : [5-(2,3-dichloro-phényl)-3-isobutyl-2,4-dioxo-3,4-dihydro-2H-pyrimidin-1-yl]-acétate de méthyle

De manière analogue à l'exemple 44.1, à partir de 400mg (1,3 mmoles) de (5-bromo-3-isobutyl-2,4-dioxo-3,4-dihydro-2H-pyrimidin-1-yl)-acétate de méthyle et de 717mg (3,8 mmoles) d'acide 2,3-dichlorophénylboronique et après purification par chromatographie sur gel de silice élué par un mélange heptane / acétate d'éthyle 80/20, 260mg (54%) de [5-(2,3-dichloro-phényl)-3-isobutyl-2,4-dioxo-3,4-dihydro-2H-pyrimidin-1-yl]-acétate de méthyle sont obtenus sous la forme d'un solide jaune pâle.

### 83.3 : acide [5-(2,3-dichloro-phényl)-3-isobutyl-2,4-dioxo-3,4-dihydro-2H-pyrimidin-1-yl]-acétique

De manière analogue à l'exemple 16.8, à partir de 260mg (0,7 mmoles) de de [5-(2,3-dichloro-phényl)-3-isobutyl-2,4-dioxo-3,4-dihydro-2H-pyrimidin-1-yl]-acétate de méthyle, 250mg (100%) d'acide [5-(2,3-dichloro-phényl)-3-isobutyl-2,4-dioxo-3,4-dihydro-2H-pyrimidin-1-yl]-acétique sont obtenus sous la forme d'un solide beige.

### 83.4 : 5-(2,3-dichloro-phényl)-3-isobutyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 83)

De manière analogue à l'exemple 13.4, à partir de 250mg (0,7 mmoles) d'acide [5-(2,3-dichloro-phényl)-3-isopropyl-2,4-dioxo-3,4-dihydro-2H-pyrimidin-1-yl]-acétique et de 192mg (0,7 mmoles) de 3-pipéridin-4-yl-1,3,4,5-tétrahydro-benzo[d] [1,3]diazépin-2-one (préparé comme décrit dans l'exemple 16.4), et après cristallisation dans l'acétate d'éthyle, 290mg (71%) de 5-(2,3-dichloro-phényl)-3-isobutyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide beige de point de fusion 248°C.

RMN ¹H (δ, DMSO) : 0.86 (d, J = 6.7 Hz, 6H), 1.48-1.60 (m, 1H), 1.62-1.80 (m, 3H), 2.03 (m, 1H), 2.67 (t, J = 12 Hz, 1H), 2.89 (t, J = 4.5 Hz, 2H), 3.14 (t, J = 12 Hz, 1H), 3.36 (t, J = 4.5 Hz, 2H), 3.71 (d, J = 7.3 Hz, 2H), 3.92 (d, J = 13 Hz, 1H), 4.28-4.38 (m, 1H), 4.42 (d, J = 13 Hz, 1H), 4.76 (d, J = 2.2 Hz, 2H), 6.75-6.82 (m, 1H), 7.01-7.04 (m, 3H), 7.32 (dd, J = 1.5-7.6 Hz, 1H), 7.42 (t, J = 7.8 Hz, 1H), 7.68 (dd, J = 1.48 Hz, 1H), 7.81 (s, 1H), 8.53 (s, 1H).

### Exemple 89 : 3-(2-amino-éthyl)-5-(2,3-dichloro-phényl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 89)

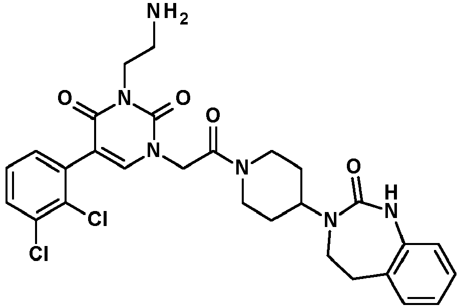

### 89.1 : acide [3-(2-tert-butoxycarbonylamino-éthyl)-5-(2,3-dichloro-phényl)-2,4-dioxo-3,4-dihydro-2H-pyrimidin-1-yl]-acétique

De manière analogue à l'exemple 44.1, à partir de 700mg (1,7 mmoles) de [5-bromo-3-(2-tert-butoxycarbonylamino-éthyl)-2,4-dioxo-3,4-dihydro-2H-pyrimidin-1-yl]-acétate de méthyle (préparé comme décrit dans l'exemple 87.1) et 526mg (2,8 mmoles) d'acide 2,3-dichlorophénylboronique, 210mg (27%) d'acide [3-(2-tert-butoxycarbonylamino-éthyl)-5-(2,3-dichloro-phényl)-2,4-dioxo-3,4-dihydro-2H-pyrimidin-1-yl]-acétique sont obtenus sous la forme d'une huile orange.

### 89.2 : [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert-butyle

De manière analogue à l'exemple 13.4, à partir de 210mg (0,5 mmoles) d'acide [3-(2-tert-butoxycarbonylamino-éthyl)-5-(2,3-dichloro-phényl)-2,4-dioxo-3,4-dihydro-2H-pyrimidin-1-yl]-acétique et de 112mg (0,5 mmoles) de 3-pipéridin-4-yl-1,3,4,5-tétrahydro-benzo[d][1,3]diazépin-2-one (préparé comme décrit dans l'exemple 16.4), 190mg (60%) de [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert butyle sont obtenus sous la forme d'un solide blanc.

### 89.3 : 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 89)

De manière analogue à l'exemple 88, à partir de 190mg (0,3 mmoles) de [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert butyle, 145mg (88%) de 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 250°C.

RMN ¹H (δ, DMSO) : 1.48-1.85 (m, 4H); 2.65-2.73 (m, 3H); 2.89 (m, 2H); 3.11-3.16 (m, 1H); 3.35-3.50 (m, 2H); 3.89-3.93 (m, 3H); 4.32 (m, 1H); 4.42 (d, J = 12.7 Hz, 1H); 4.79 (s, 2H); 6.79-6.81 (m, 1H); 7.02-7.04 (m, 3H); 7.34 (dd, J = 7.6, 1.6 Hz, 1H); 7.43 (t, J = 7.9 Hz, 1H); 7.69 (dd, J = 8.0, 1.6 Hz, 1H); 7.85 (s, 1H); 8.55 (s, 1H).

### Exemple 90 : 5-(2,3-dichloro-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 90)

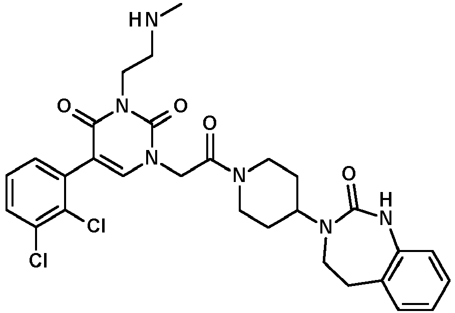

### 90.1 : [3-(2-tert-butoxycarbonylamino-éthyl)-5-(2,3-dichloro-phényl)-2,4-dioxo-3,4-dihydro-2H-pyrimidin-1-yl]-acétate de méthyle

De manière analogue à l'exemple 44.1, à partir de 700mg (1,7 mmoles) de [5-bromo-3-(2-tert-butoxycarbonylamino-éthyl)-2,4-dioxo-3,4-dihydro-2H-pyrimidin-1-yl]-acétate de méthyle (préparé comme décrit dans l'exemple 87.1) et 526mg (2,8 mmoles) d'acide 2,3-dichlorophénylboronique, 130mg (16%) de [3-(2-tert-butoxycarbonylamino-éthyl)-5-(2,3-dichloro-phényl)-2,4-dioxo-3,4-dihydro-2H-pyrimidin-1-yl]-acétate de méthyle sont obtenus sous la forme d'un solide beige.

### 90.2 : acide[3-[2-(tert-butoxycarbonyl-méthyl-amino)-éthyl]-5-(2,3-dichloro-phényl)-2,4-dioxo-3,4-dihydro-2H-pyrimidin-1-yl]-acétique

De manière analogue à l'exemple 13.2, à partir de 130mg (0,3 mmoles) de [3-(2-tert-butoxycarbonylamino-éthyl)-5-(2,3-dichloro-phényl)-2,4-dioxo-3,4-dihydro-2H-pyrimidin-1-yl]-acétate de méthyle et 26µl (0,4 mmoles) de iodométhane, 100mg (77%) d'acide[3-[2-(tert-butoxycarbonyl-méthyl-amino)-éthyl]-5-(2,3-dichloro-phényl)-2,4-dioxo-3,4-dihydro-2H-pyrimidin-1-yl]-acétique sont obtenus sous la forme d'un solide jaune pâle.

### 90.3 : [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tert-butyle

De manière analogue à l'exemple 13.4, à partir de 100mg (0,2 mmoles) d'acide [3-[2-(tert-butoxycarbonyl-méthyl-amino)-éthyl]-5-(2,3-dichloro-phényl)-2,4-dioxo-3,4-dihydro-2H-pyrimidin-1-yl]-acétique et de 52mg (0,2 mmoles) de 3-pipéridin-4-yl-1,3,4,5-tétrahydro-benzo[d][1,3]diazépin-2-one (préparé comme décrit dans l'exemple 16.4), 95mg (64%) de [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tert-butyle sont obtenus sous la forme d'un solide blanc.

### 90.4 : 5-(2,3-dichloro-phényl)-3-(2-méthylamino-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 90)

De manière analogue à l'exemple 88, à partir de 95mg (0,1 mmoles) [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tert-butyle, et après recristallisation dans l'éthanol, 55mg (66%) de 5-(2,3-dichloro-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 225°C.

RMN ¹H (δ, DMSO) : 1.45-1.69 (m, 4H); 2.29 (s, 3H); 2.65-2.71 (m, 3H); 2.90 (m, 2H); 3.14-3.20 (m, 1H); 3.38 (m, 2H); 3.95 (t, J = 7.0 Hz, 2H); 4.33 (m, 1H); 4.43 (d, J = 12.9 Hz, 1H); 4.77 (d, J = 2.9 Hz, 2H); 6.80-6.81 (m, 1H); 7.03-7.05 (m, 3H); 7.34 (dd, J = 7.7, 1.6 Hz, 1H); 7.43 (t, J = 7.9 Hz, 1H); 7.69 (dd, J = 8.0, 1.6 Hz, 1H); 7.81 (s, 1H); 8.53 (s, 1H).

### Exemple 93 : 5-(2,3-diméthyl-phényl)-3-(2-méthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 93)

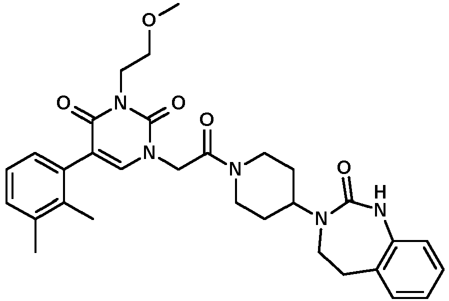

### 93.1 : [5-bromo-3-(2-méthoxy-éthyl)-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,8g (5,7 mmoles) de 2-bromoéthyl méthyl éther, 0,8g (62%) de [5-bromo-3-(2-méthoxy-éthyl)-2,4-dioxo-3,4-dihydro-2H-pyrimidin-1-yl]-acétate de méthyle sont obtenus sous la forme d'un solide blanc.

### 93.2 : acide [5-(2,3-diméthyl-phényl)-3-(2-méthoxy-éthyl)-2,4-dioxo-3,4-dihydro-2H-pyrimidin-1-yl]-acétique

De manière analogue à l'exemple 13.3, à partir de 400mg (1,3 mmoles) de [5-bromo-3-(2-méthoxy-éthyl)-2,4-dioxo-3,4-dihydro-2H-pyrimidin-1-yl]-acétate de méthyle et 280mg (1,9 mmoles) d'acide 2,3-diméthylphénylboronique, 410mg (99%) d'acide [5-(2,3-diméthyl-phényl)-3-(2-méthoxy-éthyl)-2,4-dioxo-3,4-dihydro-2H-pyrimidin-1-yl]-acétique sont obtenus sous la forme d'une huile orange.

### 93.3 : 5-(2,3-diméthyl-phényl)-3-(2-méthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 93)

De manière analogue à l'exemple 13.4, à partir de 410mg (1,2 mmoles) d'acide [5-(2,3-diméthyl-phényl)-3-(2-méthoxy-éthyl)-2,4-dioxo-3,4-dihydro-2H-pyrimidin-1-yl]-acétique et 363mg (1,5 mmoles) de 3-pipéridin-4-yl-1,3,4,5-tétrahydro-benzo[d] [1,3]diazépin-2-one (préparé comme décrit dans l'exemple 16.4), et après recristallisation dans l'éthanol, 400mg (57%) de 5-(2,3-diméthyl-phényl)-3-(2-méthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 227°C.

RMN ¹H (δ, DMSO) : 1.50-1.80 (m, 4H); 2.05 (s, 3H); 2.28 (s, 3H); 2.68 (t, J = 12.6 Hz, 1H); 2.86-2.91 (m, 2H); 3.15 (t, J = 12.5 Hz, 1H); 3.26 (s, 3H); 3.36-3.39 (m, 2H); 3.52 (t, J = 6.1 Hz, 2H); 3.94 (d, J = 13.6 Hz, 1H); 4.07 (t, J = 6.1 Hz, 2H); 4.33 (m, 1H); 4.43 (d, J = 12.9 Hz, 1H); 4.76 (d, J = 4.9 Hz, 2H); 6.80-6.81 (m, 1H); 6.97 (d, J = 7.5 Hz, 1H); 7.04-7.06 (m, 3H); 7.11 (t, J = 7.5 Hz, 1H); 7.18 (d, J = 7.5 Hz, 1H); 7.60 (s, 1H); 8.53 (s, 1H).

### Exemple 95 : 5-(3-chloro-2-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 95)

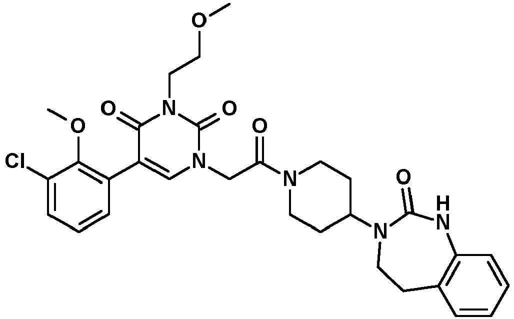

### 95.1 : acide [5-(3-chloro-2-méthoxy-phényl)-3-(2-méthoxy-éthyl)-2,4-dioxo-3,4-dihydro-2H-pyrimidin-1-yl]-acétique

De manière analogue à l'exemple 13.3, à partir de 300mg (0,9 mmoles) de [5-bromo-3-(2-méthoxy-éthyl)-2,4-dioxo-3,4-dihydro-2H-pyrimidin-1-yl]-acétate de méthyle (préparé comme décrit dans l'exemple 93.1) et 261mg (1,4 mmoles) d'acide 3-chloro-2-méthoxyphénylboronique, 340mg (98%) d' acide [5-(3-chloro-2-méthoxy-phényl)-3-(2-méthoxy-éthyl)-2,4-dioxo-3,4-dihydro-2H-pyrimidin-1-yl]-acétique sont obtenus sous la forme d'une huile marron.

### 95.2 : 5-(3-chloro-2-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 95)

De manière analogue à l'exemple 13.4, à partir de 340mg (0,9 mmoles) d' acide [5-(3-chloro-2-méthoxy-phényl)-3-(2-méthoxy-éthyl)-2,4-dioxo-3,4-dihydro-2H-pyrimidin-1-yl]-acétique et 271mg (1,1 mmoles) de 3-pipéridin-4-yl-1,3,4,5-tétrahydro-benzo[d][1,3]diazépin-2-one (préparé comme décrit dans l'exemple 16.4), et après recristallisation dans l'éthanol, 360mg (63%) de 5-(3-chloro-2-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 206°C.

RMN ¹H (δ, DMSO) : 1.61-1.69 (m, 4H); 2.68 (t, J = 12.6 Hz, 1H); 2.90 (m, 2H); 3.15 (t, J = 12.6 Hz, 1H); 3.26 (s, 3H); 3.36-3.39 (m, 2H); 3.53 (t, J = 6.1 Hz, 2H); 3.67 (s, 3H); 3.95 (d, J = 13.6 Hz, 1H); 4.08 (t, J = 6.1 Hz, 2H); 4.29-4.36 (m, 1H); 4.43 (d, J = 12.9 Hz, 1H); 4.79 (s, 2H); 6.80-6.81 (m, 1H); 7.03-7.05 (m, 3H); 7.19 (t, J = 7.8 Hz, 1H); 7.27 (dd, J = 7.7, 1.7 Hz, 1H); 7.50 (dd, J = 7.9, 1.7 Hz, 1H); 7.78 (s, 1H); 8.53 (s, 1H).

### Exemple 96 : 5-(2-chloro-3-fluoro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 96)

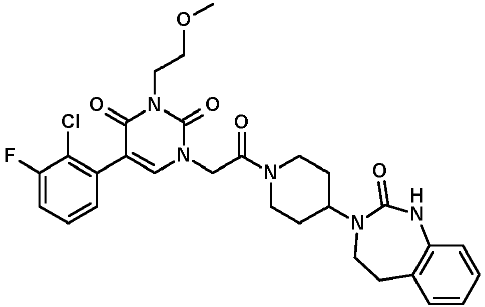

### 96.1 : acide [5-(2-chloro-3-fluoro-phényl)-3-(2-méthoxy-éthyl)-2,4-dioxo-3,4-dihydro-2H-pyrimidin-1-yl]-acétique

De manière analogue à l'exemple 13.3, à partir de 300mg (0,9 mmoles) de [5-bromo-3-(2-méthoxy-éthyl)-2,4-dioxo-3,4-dihydro-2H-pyrimidin-1-yl]-acétate de méthyle (préparé comme décrit dans l'exemple 93.1) et 244mg (1,4 mmoles) d'acide 2-chloro-3-fluorophénylboronique, 300mg (90%) d'acide [5-(2-chloro-3-fluoro-phényl)-3-(2-méthoxy-éthyl)-2,4-dioxo-3,4-dihydro-2H-pyrimidin-1-yl]-acétique sont obtenus sous la forme d'une huile marron.

### 96.2 : 5-(2-chloro-3-fluoro-phényl)-3-(2-méthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 96)

De manière analogue à l'exemple 13.4, à partir de 300mg (0,8 mmoles) d'acide [5-(2-chloro-3-fluoro-phényl)-3-(2-méthoxy-éthyl)-2,4-dioxo-3,4-dihydro-2H-pyrimidin-1-yl]-acétique et 248mg (1 mmole) de 3-pipéridin-4-yl-1,3,4,5-tétrahydro-benzo[d] [1,3]diazépin-2-one (préparé comme décrit dans l'exemple 16.4), et après recristallisation dans un mélange n-butanol / éthanol, 140mg (27%) de 5-(2-chloro-3-fluoro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 232°C.

RMN ¹H (δ, DMSO) : 1.59-1.71 (m, 4H); 2.69 (t, J = 12.6 Hz, 1H); 2.90 (m, 2H); 3.15 (t, J = 12.3 Hz, 1H); 3.26 (s, 3H); 3.36-3.39 (m, 2H); 3.53 (t, J = 6.0 Hz, 2H); 3.95 (d, J = 13.6 Hz, 1H); 4.07 (t, J = 6.1 Hz, 2H); 4.26-4.37 (m, 1H); 4.43 (d, J = 13.0 Hz, 1H); 4.78 (d, J = 2.7 Hz, 2H); 6.80-6.81 (m, 1H); 7.03-7.05 (m, 3H); 7.21-7.22 (m, 1H); 7.45-7.46 (m, 2H); 7.84 (s, 1H); 8.54 (s, 1H).

### Exemple 97 : 5-(2-chloro-3-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 97)

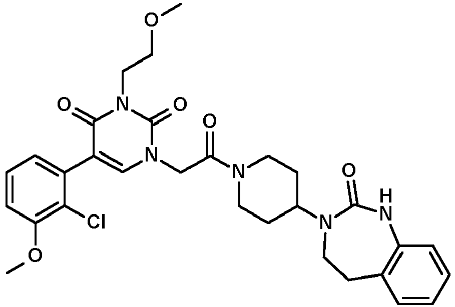

### 97.1 : acide [5-(2-chloro-3-méthoxy-phényl)-3-(2-méthoxy-éthyl)-2,4-dioxo-3,4-dihydro-2H-pyrimidin-1-yl]-acétique

De manière analogue à l'exemple 13.3, à partir de 200mg (0,6 mmoles) de [5-bromo-3-(2-méthoxy-éthyl)-2,4-dioxo-3,4-dihydro-2H-pyrimidin-1-yl]-acétate de méthyle (préparé comme décrit dans l'exemple 93.1) et 232mg (1,3 mmoles) d'acide 2-chloro-3-méthoxyphénylboronique, 158mg (69%) d' acide [5-(2-chloro-3-méthoxy-phényl)-3-(2-méthoxy-éthyl)-2,4-dioxo-3,4-dihydro-2H-pyrimidin-1-yl]-acétique sont obtenus sous la forme d'une huile jaune.

### 97.2 : 5-(2-chloro-3-méthoxy-phényl)-3-(2-méthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 97)

De manière analogue à l'exemple 13.4, à partir de 150mg (0,4 mmoles) d' acide [5-(2-chloro-3-méthoxy-phényl)-3-(2-méthoxy-éthyl)-2,4-dioxo-3,4-dihydro-2H-pyrimidin-1-yl]-acétique et 120mg (0,5 mmoles) de 3-pipéridin-4-yl-1,3,4,5-tétrahydro-benzo[d] [1,3]diazépin-2-one (préparé comme décrit dans l'exemple 16.4), 180mg (72%) de 5-(2-chloro-3-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.

RMN ¹H (δ, DMSO) : 1.55 (m, 1 H); 1.71 (m, 3 H); 2.68-2.70 (m, 1 H); 2.90 (m, 2 H); 3.13-3.17 (m, 1 H); 3.26 (s, 3 H); 3.38 (m, 2 H); 3.51 (t, J = 6.1 Hz, 2 H); 3.89 (s, 3 H); 3.94 (d, J = 14.0 Hz, 1 H); 4.06 (t, J = 6.1 Hz, 2 H); 4.31-4.33 (m, 1 H); 4.43 (d, J = 12.8 Hz, 1 H); 4.76-4.78 (m, 2 H); 6.80-6.81 (m, 1 H); 6.92 (dd, J = 7.6, 1.4 Hz, 1 H); 7.04-7.06 (m, 3 H); 7.19 (dd, J = 8.4, 1.4 Hz, 1 H); 7.35 (t, J = 8.0 Hz, 1 H); 7.73 (s, 1 H); 8.54 (s, 1 H).

### Exemple 98 : 5-(2,3-diméthyl-phényl)-3-(2-hydroxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 98)

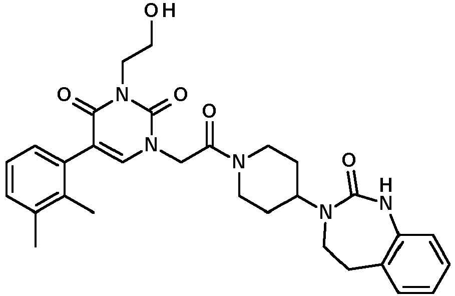

### 98.1 : [3-(2-benzyloxy-éthyl)-5-bromo-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,7ml (4,2 mmoles) de benzyl-2-bromoéthyl éther, 0,9g (60%) de [3-(2-benzyloxy-éthyl)-5-bromo-2,4-dioxo-3,4-dihydro-2H-pyrimidin-1-yl]-acétate de méthyle sont obtenus sous la forme d'un solide beige.

### 98.2 : acide [3-(2-benzyloxy-éthyl)-5-(2,3-diméthyl-phényl)-2,4-dioxo-3,4-dihydro-2H-pyrimidin-1-yl]-acétique

De manière analogue à l'exemple 13.3, à partir de 400mg (1 mmole) de [3-(2-benzyloxy-éthyl)-5-bromo-2,4-dioxo-3,4-dihydro-2H-pyrimidin-1-yl]-acétate de méthyle et 242mg (1,6 mmoles) d'acide 2,3-diméthylphénylboronique, 410mg (100%) d' acide [3-(2-benzyloxy-éthyl)-5-(2,3-diméthyl-phényl)-2,4-dioxo-3,4-dihydro-2H-pyrimidin-1-yl]-acétique sont obtenus sous la forme d'une huile orange.

### 98.3 : 3-(2-benzyloxy-éthyl)-5-(2,3-diméthyl-phényl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione

De manière analogue à l'exemple 13.4, à partir de 410mg (1 mmole) d' acide [3-(2-benzyloxy-éthyl)-5-(2,3-diméthyl-phényl)-2,4-dioxo-3,4-dihydro-2H-pyrimidin-1-yl]-acétique et 296mg (1,2 mmoles) de 3-pipéridin-4-yl-1,3,4,5-tétrahydro-benzo[d] [1,3]diazépin-2-one (préparé comme décrit dans l'exemple 16.4), 320mg (50%) de 3-(2-benzyloxy-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide rose pâle.

### 98.4 : 5-(2,3-diméthyl-phényl)-3-(2-hydroxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 98)

De manière analogue à l'exemple 1.5, à partir de 320mg (0,5 mmoles) de 3-(2-benzyloxy-éthyl)-5-(2,3-diméthyl-phényl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione et 256mg (0,1 mmoles) de palladium sur charbon à 10%, le milieu réactionnel étant placé sous 3bar de dihydrogène pendant 24 heures, 180mg (66%) de 5-(2,3-diméthyl-phényl)-3-(2-hydroxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 237°C.

RMN ¹H (δ, DMSO) : 1.58-1.70 (m, 4H); 2.05 (s, 3H); 2.28 (s, 3H); 2.68 (t, J = 12.5 Hz, 1H); 2.89 (m, 2H); 3.15 (t, J = 12.3 Hz, 1H); 3.36-3.39 (m, 2H); 3.53 (q, J = 6.4 Hz, 2H); 3.94-3.97 (m, 3H); 4.33-4.34 (m, 1H); 4.43 (d, J = 12.9 Hz, 1H); 4.70-4.85 (m, 3H); 6.80-6.81 (m, 1H); 6.97 (d, J = 7.5 Hz, 1H); 7.04-7.06 (m, 3H); 7.11 (t, J = 7.5 Hz, 1H); 7.18 (d, J = 7.5 Hz, 1H); 7.59 (s, 1H); 8.53 (s, 1H).

### Exemple 100 : 5-(2,3-diméthyl-phényl)-3-(2-méthoxyméthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 100)

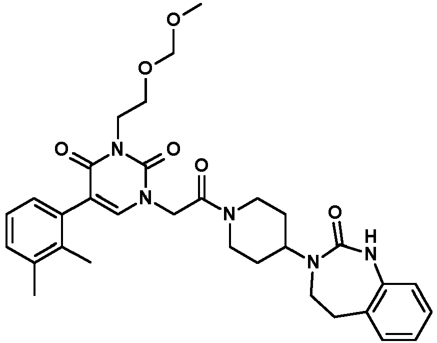

### 100.1 : [5-bromo-3-(2-méthoxyméthoxy-éthyl)-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), 0et 0,5ml (4,6 mmoles) de 1-bromo-2-méthoxyméthoxy-éthane, 0,6g (43%) de [5-bromo-3-(2-méthoxyméthoxy-éthyl)-2,4-dioxo-3,4-dihydro-2H-pyrimidin-1-yl]-acétate de méthyle sont obtenus sous la forme d'un solide blanc.

### 100.2 : acide [5-(2,3-diméthyl-phényl)-3-(2-méthoxyméthoxy-éthyl)-2,4-dioxo-3,4-dihydro-2H-pyrimidin-1-yl]-acétique

De manière analogue à l'exemple 13.3, à partir de 300mg (0,9 mmoles) de [5-bromo-3-(2-méthoxyméthoxy-éthyl)-2,4-dioxo-3,4-dihydro-2H-pyrimidin-1-yl]-acétate de méthyle et 205mg (1,4 mmoles) d'acide 2,3-diméthylphénylboronique, 250mg (81%) d'acide [5-(2,3-diméthyl-phényl)-3-(2-méthoxyméthoxy-éthyl)-2,4-dioxo-3,4-dihydro-2H-pyrimidin-1-yl]-acétique sont obtenus sous la forme d'une huile brune.

### 100.3 : 5-(2,3-diméthyl-phényl)-3-(2-méthoxyméthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 100)

De manière analogue à l'exemple 13.4, à partir de 250mg (0,7 mmoles) d'acide [5-(2,3-diméthyl-phényl)-3-(2-méthoxyméthoxy-éthyl)-2,4-dioxo-3,4-dihydro-2H-pyrimidin-1-yl]-acétique et 203mg (0,8 mmoles) de 3-pipéridin-4-yl-1,3,4,5-tétrahydro-benzo[d][1,3]diazépin-2-one (préparé comme décrit dans l'exemple 16.4), et après recrsitallisation dans un mélange heptane / acétone 50/50; 200mg (49%) de 5-(2,3-diméthyl-phényl)-3-(2-méthoxyméthoxy-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 160°C.

RMN ¹H (δ, DMSO) : 1.50-1.80 (m, 4H); 2.05 (s, 3H); 2.28 (s, 3H); 2.68 (m, 1H); 2.89 (m, 2H); 3.16 (m, 1H); 3.23 (s, 3H); 3.38 (m, 2H); 3.65 (t, J = 6.1 Hz, 2H); 4.95 (d, J=12.9 Hz, 1H); 4.10 (t, J = 6.2 Hz, 2H); 4.33 (m, 1H); 4.43 (d, J = 12.9 Hz, 1H); 4.54 (s, 2H); 4.76 (d, J = 3.8 Hz, 2H); 6.80-6.81 (m, 1H); 6.96 (d, J = 7.5 Hz, 1H); 7.03-7.05 (m, 3H); 7.11 (t, J = 7.5 Hz, 1H); 7.18 (d, J = 7.5 Hz, 1H); 7.61 (s, 1H); 8.53 (s, 1H).

### Exemple 101 : N-[2-(5-(2,3-diméthyl-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-éthyl]-acétamide (schéma réactionnel n°3, composé 101)

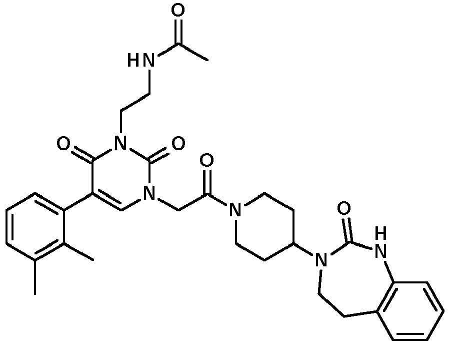

### 101.1 : [3-(2-acétylamino-éthyl)-5-bromo-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,4ml (3,8 mmoles) de N-(2-chloro-éthyl)-acétamide, 170mg (13%) de [3-(2-acétylamino-éthyl)-5-bromo-2,4-dioxo-3,4-dihydro-2H-pyrimidin-1-yl]-acétate de méthyle sont obtenus sous la forme d'un solide jaune pâle.

### 101.2 : acide [3-(2-acétylamino-éthyl)-5-(2,3-diméthyl-phényl)-2,4-dioxo-3,4-dihydro-2H-pyrimidin-1-yl]-acétique

De manière analogue à l'exemple 44.1, à partir de 170mg (0,5 mmoles) de [3-(2-acétylamino-éthyl)-5-bromo-2,4-dioxo-3,4-dihydro-2H-pyrimidin-1-yl]-acétate de méthyle et 117mg (0,8 mmoles) d'acide 2,3-diméthylphénylboronique, 35mg (20%) d'acide [3-(2-acétylamino-éthyl)-5-(2,3-diméthyl-phényl)-2,4-dioxo-3,4-dihydro-2H-pyrimidin-1-yl]-acétique sont obtenus sous la forme d'un solide rose.

### 101.3 : N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide (composé 101)

De manière analogue à l'exemple 13.4, à partir de 35mg (0,1 mmoles) d' acide [3-(2-acétylamino-éthyl)-5-(2,3-diméthyl-phényl)-2,4-dioxo-3,4-dihydro-2H-pyrimidin-1-yl]-acétique et 29mg (0,1 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 8mg (14%) de N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide sont obtenus sous la forme d'un solide blanc de point de fusion 267°C.

RMN ¹H (δ, DMSO) : 1.56-1.68 (m, 4 H), 1.76 (s, 3 H), 2.07 (s, 3 H), 2.28 (s, 3 H), 2.68-2.72 (m, 1 H), 2.90 (m, 2 H), 3.14 (t, 1 H), 3.25-3.28 (m, 2 H), 3.36-3.38 (m, 2 H), 3.95 (m, 3 H), 4.32 (m, 1 H), 4.44 (m, 1 H), 4.76 (s, 2 H), 6.81 (m, 1 H), 6.96 (d, J=7.5 Hz, 1 H), 7.05 (m, 3 H), 7.11 (t, J=7.5 Hz, 1 H), 7.18 (d, J=7.5 Hz, 1 H), 7.58 (s, 1 H), 7.92 (t, J=5.9 Hz, 1 H), 8.52 (s, 1 H).

### Exemple 103 : (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle (schéma réactionnel n°3, composé 103)

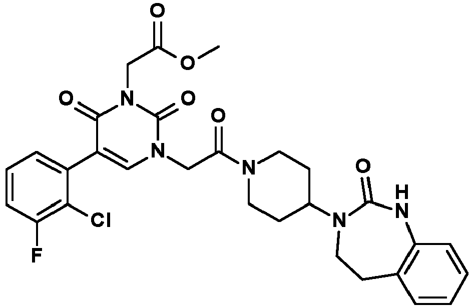

### 103.1 : (3-benzhydryl-5-bromo-2,6-dioxo-3,6-dihydro-2H-pyrimidin-1-yl)-acétate de méthyle

De manière analogue à l'exemple 2.2, à partir de 1g (2,8 mmoles) de 1-benzhydryl-5-bromo-1H-pyrimidine-2,4-dione (préparé comme décrit dans l'exemple 2.1) et 346µl (3,6 mmoles) de bromoacétate de méthyle, 960mg (80%) de (3-benzhydryl-5-bromo-2,6-dioxo-3,6-dihydro-2H-pyrimidin-1-yl)-acétate de méthyle sont obtenus sous la forme d'une pâte blanche.

### 103.2 : [3-benzhydryl-5-(2-chloro-3-fluoro-phényl)-2,6-dioxo-3,6-dihydro-2H-pyrimidin-1-yl]-acétate de méthyle

De manière analogue à l'exemple 44.1, à partir de 500mg (1,2 mmoles) de (3-benzhydryl-5-bromo-2,6-dioxo-3,6-dihydro-2H-pyrimidin-1-yl)-acétate de méthyle et 305mg (1,8 mmoles) d'acide 2-chloro-3-fluorophénylboronique, et après purification par chromatographie sur gel de silice élué par un mélange heptane / acétate d'éthyle 80/20, 208mg (37%) de [3-benzhydryl-5-(2-chloro-3-fluoro-phényl)-2,6-dioxo-3,6-dihydro-2H-pyrimidin-1-yl]-acétate de méthyle sont obtenus sous la forme d'un solide beige.

### 103.3 : [5-(2-chloro-3-fluoro-phényl)-2,6-dioxo-3,6-dihydro-2H-pyrimidin-1-yl]-acétate de méthyle

De manière analogue à l'exemple 2.3, à partir de 200mg (0,4 mmoles) de [3-benzhydryl-5-(2-chloro-3-fluoro-phényl)-2,6-dioxo-3,6-dihydro-2H-pyrimidin-1-yl]-acétate de méthyle et après 10 jours d'agitation à température ambiante, 75mg (57%) de [5-(2-chloro-3-fluoro-phényl)-2,6-dioxo-3,6-dihydro-2H-pyrimidin-1-yl]-acétate de méthyle sont obtenus sous la forme d'une huile brune.

### 103.4 : (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle (composé 103)

De manière analogue à l'exemple 39.4, à partir de 74mg (0,2 mmoles) de 3-[1-(2-chloro-acétyl)-pipéridin-4-yl]-1,3,4,5-tétrahydro-benzo[d] [1,3]diazépin-2-one (préparé comme décrit dans l'exemple 39.1) et 65mg (0,2 mmoles) de [5-(2-chloro-3-fluoro-phényl)-2,6-dioxo-3,6-dihydro-2H-pyrimidin-1-yl]-acétate de méthyle, 72mg (57%) de (5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-acétate de méthyle sont obtenus sous la forme d'un solide blanc

RMN ¹H (δ, DMSO) : 1.51-1.59 (m, 1 H); 1.65-1.75 (m, J = 22.0 Hz, 3 H); 2.69 (t, J = 12.6 Hz, 1 H); 2.88-2.90 (m, 2 H); 3.15 (t, J = 12.0 Hz, 1 H); 3.36-3.38 (m, 2 H); 3.69 (s, 3 H); 3.95 (d, J = 13.5 Hz, 1 H); 4.30-4.33 (m, 1 H); 4.43 (d, J = 12.9 Hz, 1 H); 4.66 (s, 2 H); 4.82 (s, 2 H); 6.81 (dt, J = 7.7, 4.2 Hz, 1 H); 7.06-7.04 (m, 3 H); 7.24 (dd, J = 6.5, 2.7 Hz, 1 H); 7.48-7.46 (m, 2 H); 7.93 (s, 1 H); 8.53 (s, 1 H).

### Exemple 104 : acide (5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-acétique (schéma réactionnel n°3, composé 104)

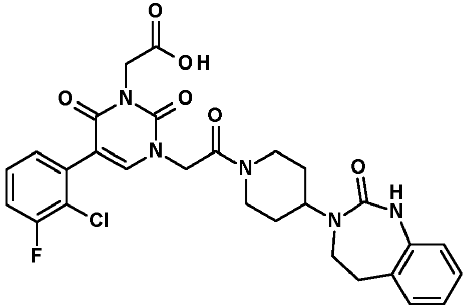

De manière analogue à l'exemple 16.8, à partir de 50mg (80 µmoles) de (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle, et après purification par chromatographie sur gel de silice élué par un mélange dichlorométhane / méthanol 98/2, 10mg (20%) d'acide (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétique sont obtenus sous la forme d'un solide blanc.

RMN ¹H (δ, DMSO) : 1.51-1.59 (m, 1 H); 1.65-1.75 (m, J = 22.0 Hz, 3 H); 2.69 (t, J = 12.6 Hz, 1 H); 2.88-2.90 (m, 2 H); 3.15 (t, J = 12.0 Hz, 1 H); 3.36-3.38 (m, 2 H); 3.95 (d, J = 13.5 Hz, 1 H); 4.30-4.33 (m, 1 H); 4.43 (d, J = 12.9 Hz, 1 H); 4.66 (s, 2 H); 4.82 (s, 2 H); 6.81 (dt, J = 7.7, 4.2 Hz, 1 H); 7.06-7.04 (m, 3 H); 7.24 (dd, J = 6.5, 2.7 Hz, 1 H); 7.48-7.46 (m, 2 H); 7.93 (s, 1 H); 8.53 (s, 1 H).

### Exemple 105 : 5-(2-chloro-3-fluoro-phényl)-3-oxétan-3-ylméthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 105)

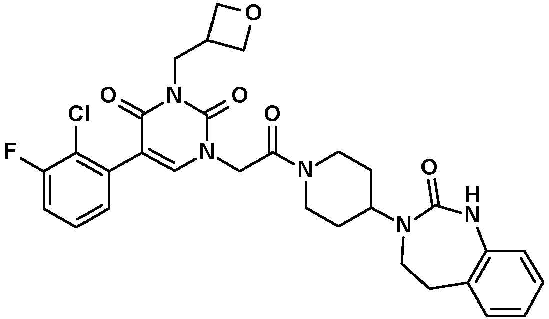

### 105.1 : (5-bromo-3-oxétan-3-ylméthyl-2,4-dioxo-3,4-dihydro-2H-pyrimidin-1-yl)-acétate de méthyle

De manière analogue à l'exemple 13.2, à partir de 500mg (1,9 mmoles) de (5-bromo-2,4-dioxo-3,4-dihydro-2H-pyrimidin-1-yl)-acétate de méthyle (préparé comme décrit dans l'exemple 13.1), et 0,15 ml (2,9 mmoles) de 3-bromométhyl-oxétane, 310mg (49%) de (5-bromo-3-oxétan-3-ylméthyl-2,4-dioxo-3,4-dihydro-2H-pyrimidin-1-yl)-acétate de méthyle sont obtenus sous la forme d'un solide blanc.

### 105.2 : acide [5-(2-chloro-3-fluoro-phényl)-3-oxétan-3-ylméthyl-2,4-dioxo-3,4-dihydro-2H-pyrimidin-1-yl]-acétique

De manière analogue à l'exemple 44.1, à partir de 310mg (0,9 mmoles) de (5-bromo-3-oxétan-3-ylméthyl-2,4-dioxo-3,4-dihydro-2H-pyrimidin-1-yl)-acétate de méthyle et 243mg (1,4 mmoles) d'acide 2-chloro-3-fluorophénylboronique, 80mg (23%) d'acide [5-(2-chloro-3-fluoro-phényl)-3-oxétan-3-ylméthyl-2,4-dioxo-3,4-dihydro-2H-pyrimidin-1-yl]-acétique sont obtenus sous la forme d'une huile marron.

### 105.3 : 5-(2-chloro-3-fluoro-phényl)-3-oxétan-3-ylméthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 105)

De manière analogue à l'exemple 13.4, à partir de 80mg (0,2 mmoles) d'acide [5-(2-chloro-3-fluoro-phényl)-3-oxétan-3-ylméthyl-2,4-dioxo-3,4-dihydro-2H-pyrimidin-1-yl]-acétique et 59mg (0,2 mmoles) de 3-pipéridin-4-yl-1,3,4,5-tétrahydro-benzo[d] [1,3]diazépin-2-one (préparé comme décrit dans l'exemple 16.4), 30mg (22%) de 5-(2-chloro-3-fluoro-phényl)-3-oxétan-3-ylméthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 244°C.

RMN ¹H (δ, DMSO) : 1.53-1.56 (m, 1 H); 1.66-1.72 (m, 3 H); 2.69 (t, J = 12.3 Hz, 1 H); 2.90 (m, 2 H); 3.15 (t, J = 12.2 Hz, 1 H); 3.25-3.30 (m, 1 H); 3.37-3.39 (m, 2 H); 3.92-3.95 (m, 1 H); 4.19 (d, J = 6.7 Hz, 2 H); 4.33 (m, 1 H); 4.40-4.43 (m, 3 H); 4. 60 (dd, J = 7.9, 6.0 Hz, 2 H); 4.78 (s, 2 H); 6.79-6.83 (m, 1 H); 7.02-7.05 (m, 3 H); 7.21-7.23 (m, 1 H); 7.44-7.48 (m, 2 H); 7.85 (s, 1 H); 8.53 (s, 1H).

### Exemple 106 : 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 106)

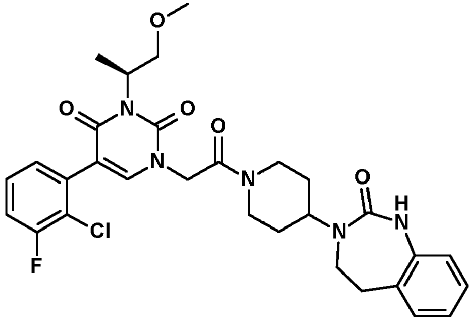

### 106.1 : [5-bromo-3-((S)-2-méthoxy-1-méthyl-éthyl)-2,4-dioxo-3,4-dihydro-2H-pyrimidin-1-yl]-acétate de méthyle

De manière analogue à l'exemple 2.5, à partir de 1g (3,8 mmoles) de (5-bromo-2,4-dioxo-3,4-dihydro-2H-pyrimidin-1-yl)-acétate de méthyle (préparé comme décrit dans l'exemple 13.1), 0,8ml (7,8 mmoles) de (R)-1-méthoxy-propan-2-ol et 1,2ml (7,6 mmoles) de diéthyl azodicarboxylate rajoutés à 0°C, et après purification par chromatographie sur gel de silice élué par un mélange heptane / acétate d'éthyle 60/40, 1,2g (78%) de [5-bromo-3-((S)-2-méthoxy-1-méthyl-éthyl)-2,4-dioxo-3,4-dihydro-2H-pyrimidin-1-yl]-acétate de méthyle sont obtenus sous la forme d'une huile incolore.

### 106.2 : [5-(2-chloro-3-fluoro-phényl)-3-((S)-2-méthoxy-1-méthyl-éthyl)-2,4-dioxo-3,4-dihydro-2H-pyrimidin-1-yl]-acétate de méthyle

De manière analogue à l'exemple 44.1, à partir de 300mg (0,9 mmoles) de [5-bromo-3-((S)-2-méthoxy-1-méthyl-éthyl)-2,4-dioxo-3,4-dihydro-2H-pyrimidin-1-yl]-acétate de méthyle, 234mg (1,3 mmoles) d'acide 2-chloro-3-fluoro-phénylboronique, et 1,3ml (2,7 mmoles) d'une solution aqueuse de carbonate de potassium 2M, dans 8ml de toluène, 130mg (38%) de [5-(2-chloro-3-fluoro-phényl)-3-((S)-2-méthoxy-1-méthyl-éthyl)-2,4-dioxo-3,4-dihydro-2H-pyrimidin-1-yl]-acétate de méthyle sont obtenus sous la forme d'une huile incolore.

### 106.3 : acide [5-(2-chloro-3-fluoro-phényl)-3-((S)-2-méthoxy-1-méthyl-éthyl)-2,4-dioxo-3,4-dihydro-2H-pyrimidin-1-yl]-acétique

De manière analogue à l'exemple 16.8, à partir de 130mg (0,3 mmoles) [5-(2-chloro-3-fluoro-phényl)-3-((S)-2-méthoxy-1-méthyl-éthyl)-2,4-dioxo-3,4-dihydro-2H-pyrimidin-1-yl]-acétate de méthyle, 125mg (100%) d' acide [5-(2-chloro-3-fluoro-phényl)-3-((S)-2-méthoxy-1-méthyl-éthyl)-2,4-dioxo-3,4-dihydro-2H-pyrimidin-1-yl]-acétique sont obtenus sous la forme d'une huile incolore.

### 106.4 : 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 106)

De manière analogue à l'exemple 13.4, à partir de 125mg (0,3 mmoles) d' acide [5-(2-chloro-3-fluoro-phényl)-3-((S)-2-méthoxy-1-méthyl-éthyl)-2,4-dioxo-3,4-dihydro-2H-pyrimidin-1-yl]-acétique et 99mg (0,4 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 150mg (73%) de 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 228°C.

RMN ¹H (δ, DMSO) : 1.34 (d, J = 7.0 Hz, 3H); 1.50-1.80 (m, 4H); 2.68 (t, J = 12.5 Hz, 1H); 2.90 (m, 2H); 3.15 (m, 1H); 3.33 (s, 3H); 3.38 (m, 2H); 3.54 (dd, J = 9.9, 5.9 Hz, 1H); 3.90-3.95 (m, 2H); 4.33 (m, 1H); 4.43 (d, J = 12.9 Hz, 1H); 4.75 (m, 2H); 5.15 (m, 1H); 6.80-6.81 (m, 1H); 7.04-7.06 (m, 3H); 7.20-7.21 (m, 1H); 7.44-7.45 (m, 2H); 7.80 (s, 1H); 8.53 (s, 1H).

### Exemple 108 : 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 108)

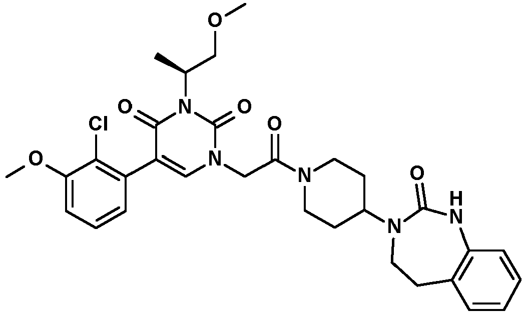

### 108.1 : acide [5-(2-chloro-3-méthoxy-phényl)-3-((S)-2-méthoxy-1-méthyl-éthyl)-2,4-dioxo-3,4-dihydro-2H-pyrimidin-1-yl]-acétique

De manière analogue à l'exemple 13.3, à partir de 800mg (2,4 mmoles) de [5-bromo-3-((S)-2-méthoxy-1-méthyl-éthyl)-2,4-dioxo-3,4-dihydro-2H-pyrimidin-1-yl]-acétate de méthyle (préparé comme décrit dans l'exemple 106.1) et 534mg (2,9 mmoles) de 2-chloro-3-méthoxy-phénylboronique, 912mg (100%) d'acide [5-(2-chloro-3-méthoxy-phényl)-3-((S)-2-méthoxy-1-méthyl-éthyl)-2,4-dioxo-3,4-dihydro-2H-pyrimidin-1-yl]-acétique sont obtenus sous la forme d'une huile marron.

### 108.2 : 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 108)

De manière analogue à l'exemple 13.4, à partir de 450mg (1,2 mmoles) d'acide [5-(2-chloro-3-méthoxy-phényl)-3-((S)-2-méthoxy-1-méthyl-éthyl)-2,4-dioxo-3,4-dihydro-2H-pyrimidin-1-yl]-acétique et 346mg (1,4 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 250mg (35%) de 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 274°C.

RMN ¹H (δ, DMSO) : 1.33 (d, J = 6.9 Hz, 3H); 1.53-1.56 (m, 1H); 1.65-1.70 (m, 3H); 2.68 (t, J = 12.4 Hz, 1H); 2.88-2.90 (m, 2H); 3.14 (t, J = 12.5 Hz, 1H); 3.23 (s, 3H); 3.37-3.39 (m, 2H); 3.54 (dd, J = 9.9, 5.9 Hz, 1H); 3.89 (s, 3H); 3.91-3.95 (m, 2H); 4.26-4.36 (m, 1H); 4.42-4.45 (m, 1H); 4.73-4.77 (m, 2H); 5.09-5.19 (m, 1H); 6.79-6.83 (m, 1H); 6.91 (dd, J = 7.6, 1.4 Hz, 1H); 7.02-7.05 (m, 3H); 7.18 (dd, J = 8.4, 1.4 Hz, 1H); 7.33-7.37 (m, 1H); 7.69 (s, 1H); 8.53 (s, 1H).

### Exemple 110 : 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 110)

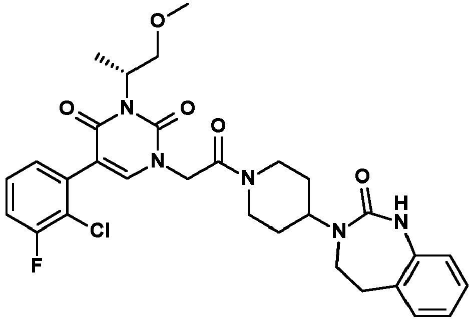

### 110.1 : [5-bromo-3-((R)-2-méthoxy-1-méthyl-éthyl)-2,4-dioxo-3,4-dihydro-2H-pyrimidin-1-yl]-acétate de méthyle

De manière analogue à l'exemple 106.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,4ml (4,2 mmoles) de (S)-1-méthoxy-propan-2-ol, 0,8g (63%) de [5-bromo-3-((R)-2-méthoxy-1-méthyl-éthyl)-2,4-dioxo-3,4-dihydro-2H-pyrimidin-1-yl]-acétate de méthyle sont obtenus sous la forme d'une huile incolore.

### 110.2 : acide [5-(2-chloro-3-fluoro-phényl)-3-((R)-2-méthoxy-1-méthyl-éthyl)-2,4-dioxo-3,4-dihydro-2H-pyrimidin-1-yl]-acétique

De manière analogue à l'exemple 13.3, à partir de 300mg (0,9 mmoles) de [5-bromo-3-((R)-2-méthoxy-1-méthyl-éthyl)-2,4-dioxo-3,4-dihydro-2H-pyrimidin-1-yl]-acétate de méthyle et 156mg (0,9 mmoles) d'acide 2-chloro-3-fluoro-phénylboronique, 180mg (54%) d' acide [5-(2-chloro-3-fluoro-phényl)-3-((R)-2-méthoxy-1-méthyl-éthyl)-2,4-dioxo-3,4-dihydro-2H-pyrimidin-1-yl]-acétique sont obtenus sous la forme d'une huile brune.

### 110.3 : 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 110)

De manière analogue à l'exemple 13.4, à partir de 180mg (0,5 mmoles) d'acide [5-(2-chloro-3-fluoro-phényl)-3-((R)-2-méthoxy-1-méthyl-éthyl)-2,4-dioxo-3,4-dihydro-2H-pyrimidin-1-yl]-acétique et 143mg (0,6 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 105mg (36%) de 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 225°C.

RMN ¹H (δ, DMSO) : 1.34 (d, J = 7.0 Hz, 3H); 1.50-1.80 (m, 4H); 2.68 (t, J = 12.5 Hz, 1H); 2.90 (m, 2H); 3.15 (m, 1H); 3.33 (s, 3H); 3.38 (m, 2H); 3.54 (dd, J = 9.9, 5.9 Hz, 1H); 3.90-3.95 (m, 2H); 4.33 (m, 1H); 4.43 (d, J = 12.9 Hz, 1H); 4.75 (m, 2H); 5.15 (m, 1H); 6.80-6.81 (m, 1H); 7.04-7.06 (m, 3H); 7.20-7.21 (m, 1H); 7.44-7.45 (m, 2H); 7.80 (s, 1H); 8.53 (s, 1H).

### Exemple 112 : 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 112)

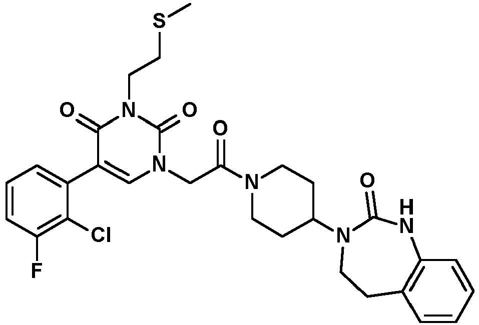

### 112.1 : [5-bromo-3-(2-méthylsulfanyl-éthyl)-2,4-dioxo-3,4-dihydro-2H-pyrimidin-1-yl]-acétate de méthyle

De manière analogue à l'exemple 1.7, à partir de 0,5g (1,9 mmoles) de 5-bromo-2,4-dioxo-3,4-dihydro-2H-pyrimidin-1-yl)-acétate de méthyle (préparé comme dans l'exemple 13.1) et de 420mg (3,8 mmoles) de 2-chloroéthyl méthyl sulfide, et après chauffage du mélange à 50 °C pendant 5 heures, 520mg (81%) de [5-bromo-3-(2-méthylsulfanyl-éthyl)-2,4-dioxo-3,4-dihydro-2H-pyrimidin-1-yl]-acétate de méthyle sont obtenus sous la forme d'un solide blanc.

### 112.2 : acide [5-(2-chloro-3-fluoro-phényl)-3-(2-méthylsulfanyl-éthyl)-2,4-dioxo-3,4-dihydro-2H-pyrimidin-1-yl]-acétique

De manière analogue à l'exemple 13.3, à partir de 520mg (1,5 mmoles) de [5-bromo-3-(2-méthylsulfanyl-éthyl)-2,4-dioxo-3,4-dihydro-2H-pyrimidin-1-yl]-acétate de méthyle et 296mg (1,7 mmoles) d'acide 2-chloro-3-fluorophénylboronique, 490mg (85%) d'acide [5-(2-chloro-3-fluoro-phényl)-3-(2-méthylsulfanyl-éthyl)-2,4-dioxo-3,4-dihydro-2H-pyrimidin-1-yl]-acétique sont obtenus sous la forme d'un solide beige.

### 112.3 : 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 112)

De manière analogue à l'exemple 13.4, à partir de 490mg (1,3 mmoles) d'acide [5-(2-chloro-3-fluoro-phényl)-3-(2-méthylsulfanyl-éthyl)-2,4-dioxo-3,4-dihydro-2H-pyrimidin-1-yl]-acétique et 387mg (1,6 mmoles) de 3-pipéridin-4-yl-1,3,4,5-tétrahydro-benzo[d][1,3]diazépin-2-one (préparé comme décrit dans l'exemple 16.4), et après recristallisation dans le n-butanol, 470mg (58%) de 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 207°C.

RMN ¹H (δ, DMSO): 1.50-1.80 (m, 4H), 2.12 (s, 3H), 2.69 (t, 3H, J = 7.19 Hz), 2.90 (m, 2H), 3.16 (m, 1H), 3.38 (m, 2H), 3.95 (d, 1H, J = 13.66 Hz), 4.08 (t, 2H, J = 7.21 Hz), 4.33 (m, 1H), 4.43 (d, 1H, J = 12.91 Hz), 4.79 (s, 2H), 6.79-6.83 (m, 1H), 7.02-7.05 (m, 3H), 7.21-7.23 (m, 1H), 7.44-7.48 (m, 2H), 7.85 (s, 1H), 8.52 (s, 1H).

### Exemple 113 : 5-(2,3-difluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 113)

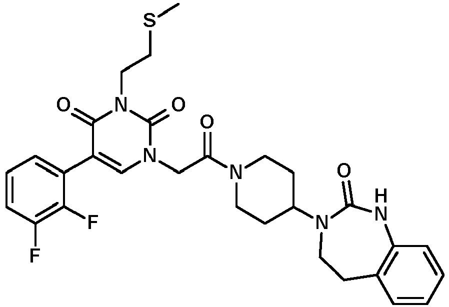

### 113.1 : (5-bromo-2,4-dioxo-3,4-dihydro-2H-pyrimidin-1-yl)-acétate de méthyle

23ml (0,2 mmoles) de 1,8-diazabicyclo[5.4.0]undec-7-ène sont additionnés sur une solution contenant 30g (0.2 mmoles) de 5-bromouracile dans 294ml d'acétonitrile. Le milieu passe en solution puis rechute après 5 min. Il est alors refroidi à -5°C avec un bain glace / acétone puis 16ml (0.2 mmoles) de bromoacétate de méthyle sont ajoutés. Le milieu est agité pendant 45 minutes à température ambiante, traité en coulant 150ml d'eau, puis filtré.Le précipité blanc est rincé avec un peu de méthyl tert-butyl éther et séché sous vide à 40°C. 25,5g (63%) de (5-bromo-2,4-dioxo-3,4-dihydro-2H-pyrimidin-1-yl)-acétate de méthyle sont obtenus sous la forme d'un solide blanc.

### 113.2 : [5-bromo-3-(2-méthylsulfanyl-éthyl)-2,4-dioxo-3,4-dihydro-2H-pyrimidin-1-yl]-acétate de méthyle

De manière analogue à l'exemple 1.7, à partir de 1,2g (4,6 mmoles) de (5-bromo-2,4-dioxo-3,4-dihydro-2H-pyrimidin-1-yl)-acétate de méthyle et de 1,4ml (13,7 mmoles) de 2-chloroéthyl méthyl sulfide, et après chauffage du mélange à 50 °C pendant toute une nuit, 850mg (55%) de [5-bromo-3-(2-méthylsulfanyl-éthyl)-2,4-dioxo-3,4-dihydro-2H-pyrimidin-1-yl]-acétate de méthyle sont obtenus sous la forme d'une huile incolore qui cristallise.

### 113.3 : [5-(2,3-difluoro-phényl)-3-(2-méthylsulfanyl-éthyl)-2,4-dioxo-3,4-dihydro-2H-pyrimidin-1-yl]-acétate de méthyle

De manière analogue à l'exemple 44.1, à partir de 550mg (1,6 mmoles) de [5-bromo-3-(2-méthylsulfanyl-éthyl)-2,4-dioxo-3,4-dihydro-2H-pyrimidin-1-yl]-acétate de méthyle et 1,1g (6,5 mmoles) d'acide 2,3-difluorophénylboronique, 450mg (75%) de [5-(2,3-difluoro-phényl)-3-(2-méthylsulfanyl-éthyl)-2,4-dioxo-3,4-dihydro-2H-pyrimidin-1-yl]-acétate de méthyle sont obtenus sous la forme d'un solide blanc.

### 113.4 : acide [5-(2,3-difluoro-phényl)-3-(2-méthylsulfanyl-éthyl)-2,4-dioxo-3,4-dihydro-2H-pyrimidin-1-yl]-acétique

De manière analogue à l'exemple 16.8, à partir de 450mg (1,2 mmoles) de [5-(2,3-difluoro-phényl)-3-(2-méthylsulfanyl-éthyl)-2,4-dioxo-3,4-dihydro-2H-pyrimidin-1-yl]-acétate de méthyle, 430mg (99%) d' acide [5-(2,3-difluoro-phényl)-3-(2-méthylsulfanyl-éthyl)-2,4-dioxo-3,4-dihydro-2H-pyrimidin-1-yl]-acétique sont obtenus sous la forme d'un solide beige.

### 113.5 : 5-(2,3-difluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 113)

De manière analogue à l'exemple 13.4, à partir de 200mg (0,6 mmoles) d'acide [5-(2,3-difluoro-phényl)-3-(2-méthylsulfanyl-éthyl)-2,4-dioxo-3,4-dihydro-2H-pyrimidin-1-yl]-acétique et 165mg (0,7 mmoles) de 3-pipéridin-4-yl-1,3,4,5-tétrahydro-benzo[d][1,3]diazépin-2-one, et après cristallisation dans un mélange heptane/acétate d'éthyle 50/50, 230mg (65%) de 5-(2,3-difluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 220°C.

RMN ¹H (400 MHz, DMSO-d6) δ: 1.50-1.63 (m, 1H), 1.69 (m, 3H), 2.12 (s, 3H), 2.69 (m, 3H), 2.82-2.97 (m, 2H), 3.16 (m, 1H), 3.35-3.48 (m, 2H), 3.95 (m, 1H), 4.03-4.15 (m, 2H), 4.28-4.38 (m, 1H), 4.43 (m, 1H), 4.80 (s, 2H), 6.73-6.88 (m, 1H), 6.96-7.10 (m, 3H), 7.17-7.35 (m, 2H), 7.46 (m, 1H), 7.94 (s, 1H), 8.53 (s, 1H).

### Exemple 114: 3-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle (schéma réactionnel n°3, composé 114)

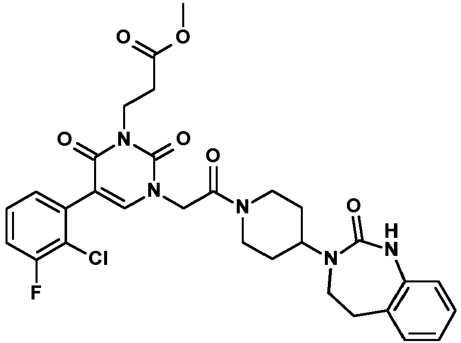

### 114.1 : 3-(3-benzhydryl-5-bromo-2,6-dioxo-3,6-dihydro-2H-pyrimidin-1-yl)-propanoate de méthyle

De manière analogue à l'exemple 2.2, à partir de 1g (2,8 mmoles) de 1-benzhydryl-5-bromo-1H-pyrimidine-2,4-dione (préparé comme dans l'exemple 2.1) et 608mg (3,6 mmoles) de 3-bromopropionate de méthyle, 1,1g (85%) de 3-(3-benzhydryl-5-bromo-2,6-dioxo-3,6-dihydro-2H-pyrimidin-1-yl)-propanoate de méthyle sont obtenus sous la forme d'un solide blanc.

### 114.2 : 3-[3-benzhydryl-5-(2-chloro-3-fluoro-phényl)-2,6-dioxo-3,6-dihydro-2H-pyrimidin-1-yl]-propanoate de méthyle

De manière analogue à l'exemple 44.1, à partir de 1,1g (2,4 mmoles) de 3-(3-benzhydryl-5-bromo-2,6-dioxo-3,6-dihydro-2H-pyrimidin-1-yl)-propanoate de méthyle et 620mg (3,6 mmoles) d'acide 2-chloro-3-fluorophénylboronique, et après purification par chromatographie sur gel de silice élué avec un mélange heptane / acétate d'éthyle 80/20, 660mg (57%) de 3-[3-benzhydryl-5-(2-chloro-3-fluoro-phényl)-2,6-dioxo-3,6-dihydro-2H-pyrimidin-1-yl]-propanoate de méthyle sont obtenus sous la forme d'un solide jaune.

### 114.3 : 3-[5-(2-chloro-3-fluoro-phényl)-2,6-dioxo-3,6-dihydro-2H-pyrimidin-1-yl]-propanoate de méthyle

De manière analogue à l'exemple 2.3, à partir de 500mg (1,0 mmoles) de 3-[3-benzhydryl-5-(2-chloro-3-fluoro-phényl)-2,6-dioxo-3,6-dihydro-2H-pyrimidin-1-yl]-propanoate de méthyle, 310mg (94%) de 3-[5-(2-chloro-3-fluoro-phényl)-2,6-dioxo-3,6-dihydro-2H-pyrimidin-1-yl]-propanoate de méthyle sont obtenus sous la forme d'un solide jaunâtre.

### 114.4 : 3-[1-(2-chloro-acétyl)-pipéridin-4-yl]-1,3,4,5-tétrahydro-benzo[d][1,3]diazépin-2-one

De manière analogue à l'exemple 2.4, à partir de 6g (24,5 mmoles) de 3-pipéridin-4-yl-1,3,4,5-tétrahydro-benzo[d][1,3]diazépin-2-one (préparé comme dans l'exemple 16.4) et 5g (53,8 mmoles) d'acide chloroacétique, 6,4g (81%) de 3-[1-(2-chloro-acétyl)-pipéridin-4-yl]-1,3,4,5-tétrahydro-benzo[d] [1,3]diazépin-2-one sont obtenus sous la forme d'un solide blanc.

### 114.5 : 3-(5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-propanoate de méthyle (composé 114)

De manière analogue à l'exemple 111.6, à partir de 150mg (0,5 mmoles) de 3-[5-(2-chloro-3-fluoro-phényl)-2,6-dioxo-3,6-dihydro-2H-pyrimidin-1-yl]-propanoate de méthyle et 163mg (0,5 mmoles) de 3-[1-(2-chloro-acétyl)-pipéridin-4-yl]-1,3,4,5-tétrahydro-benzo[d][1,3]diazépin-2-one, et après cristallisation dans l'acétate d'éthyle puis l'heptane, 230mg (80%) de 3-(5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-propanoate de méthyle sont obtenus sous la forme d'un solide blanc cassé.

RMN ¹H (δ, DMSO): 1.55 (d, J = 13.8 Hz, 1H); 1.69 (d, J = 23.0 Hz, 3H); 2.59 (t, J = 7.4 Hz, 2H); 2.69 (t, 1H); 2.90 (m, 2H); 3.16 (br s, 1H); 3.38 (m, 2H); 3.60 (s, 3H); 3.95 (d, J = 13.5 Hz, 1H); 4.13 (t, J = 7.4 Hz, 2H); 4.33 (br s, 1H); 4.43 (d, J = 12.9 Hz, 1H); 4.78 (s, 2H); 6.81 (dt, J = 7.8, 4.3 Hz, 1H); 7.04 (t, J = 3.8 Hz, 3H); 7.21-7.23 (m, 1H); 7.44-7.47 (m, 2H); 7.85 (s, 1H); 8.52 (s, 1H).

Autre composé synthétisé selon un mode opératoire analogue en utilisant les réactifs appropriés commerciaux ou préalablement préparé :

### composé 202: N-[2-(5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-éthyl]-acétamide

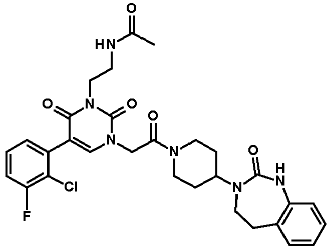

RMN ¹H (δ, DMSO): 1.47-1.83 (m, 4H), 1.76 (s, 3H), 2.62 -2.77 (m, 1H), 2.81-2.97 (m, 2H), 3.07 - 3.20 (m, 1H), 3.26 (q, J = 6.43 Hz, 2H), 3.34 - 3.44 (m, 2H), 3.84 - 4.04 (m, 3H), 4.25 - 4.38 (m, 1H), 4.43 (d, J = 13.11 Hz, 1H), 4.78 (s, 2H), 6.68 - 6.87 (m, 1H), 6.90 - 7.10 (m, 3H), 7.14 - 7.31 (m, 1H), 7.34 - 7.56 (m, 2H), 7.82 (s, 1H), 7.92 (t, J = 5.91 Hz, 1H), 8.52 (s, 1H).

### Exemple 115: N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide (schéma réactionnel 3 ou 4, composé 115)

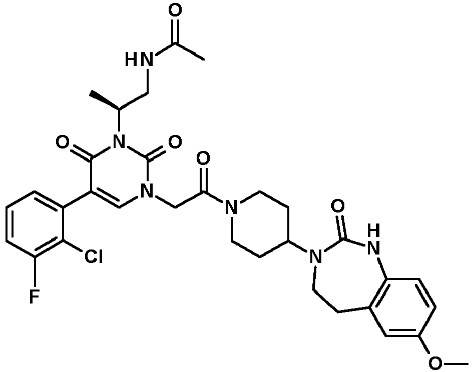

### 115.1 : (5-méthoxy-2-nitro-phényl)-acétonitrile

Une solution contenant 30,5g (199 mmoles) de 4-nitroanisole et 40g (239 mmoles) de (4-chloro-phénoxy)-acétonitrile dans 305ml de N,N-diméthylformamide sont rajoutés à -20°C sous azote à une solution contenant 53,6g (478 mmoles) de tert-butylate de potassium dans 610ml de N,N-diméthylformamide. Le milieu réactionnel est laissé remonté jusqu'à 0°C et maintenu à cette température. Après 1 heure, le milieu réactionnel est placé à -50°C et hydrolysé lentement avec 1000ml d'une solution aqueuse glacée d'acide chlorhydrique 6N, puis 1000ml d'acétate d'éthyle et 500ml d'eau sont additionnés. La phase aqueuse est extraite avec de l''acétate d'éthyle. La phase organique est lavée par une solution aqueuse saturée de chlorure d'ammonium puis par de l'eau, et ensuite séchée sur sulfate de sodium anhydre, filtrée et concentrée. Le résidu obtenu est repris dans du toluène et évaporé. L'huile ainsi obtenue est reprise sous agitation dans 250ml d'un mélange acétate d'éthyle / heptane (50/50) pendant une nuit puis le mélange est refroidi dans un bain de glace et filtré. Après rinçage à l'heptane, 26,7g (68%) de (5-méthoxy-2-nitro-phényl)-acétonitrile sont obtenus sous la forme d'un solide orange.

### 115.2 : 2-(5-méthoxy-2-nitro-phényl)-éthylamine

200ml (200 mmoles) d'une solution complexée de boranetétrahydrofurane sont rajoutés à une solution contenant 26g (136 mmoles) de (5-méthoxy-2-nitro-phényl)-acétonitrile dans 130ml de 2-méthyltétrahydrofuranne à 60°C. Après 3 heures, le chauffage est arrêté et 35ml (863 mmoles) de méthanol sont rajoutés lentement ainsi que 200ml d'une solution aqueuse d'hydroxyde de sodium 1N. La phase aqueuse est ensuite extraite avec 250ml de méthyl-tétrahydrofurane. La phase organique est lavée par de l'eau, séchée sur sulfate de sodium anhydre, filtrée et concentrée partiellement. 400ml d'éther tertbutylique et 10ml (175 mmoles) d'acide acétique sont rajoutés. Le mélange est refroidi, laissé sous agitation toute une nuit puis concentré et coévaporé au toluène. 40,6g (100%) d' acétate de 2-(5-méthoxy-2-nitro-phényl)-éthylamine sont obtenus sous la forme d'une huile brune.

### 115.3 : 4-[2-(5-méthoxy-2-nitro-phényl)-éthylamino]-pipéridine-1-carboxylate de méthyle

44,5g (210 mmoles) de triacétoxyhydroborate de sodium sont rajoutés à une solution sous azote contenant 40,6g (136 mmoles) d'acide 2-(5-méthoxy-2-nitro-phényl)-éthylamine acétique, 35,9g (180 mmoles) de N-(tert-butoxycarbonyl)-4-pipéridone dans 360ml de 2-méthyltétrahydrofuranne. Après 2 heures, le milieu réactionnel est hydrolysé avec une solution aqueuse d'hydroxyde de sodium 2N. La phase aqueuse est extraite avec du méthyltétrahydrofurane. La phase organique est lavée par de l'eau pour être amenée à pH=6, séchée sur sulfate de sodium, filtrée et évaporée. 69,2g (100%) de 4-[2-(5-méthoxy-2-nitro-phényl)-éthylamino]-pipéridine-1-carboxylate de méthyle sont obtenus sous la forme d'une huile foncée.

### 115.4 : 4-[2-(2-amino-5-méthoxy-phényl)-éthylamino]-pipéridine-1-carboxylate de tert-butyle

3,7g (70 mmoles) de chlorure d'ammonium et 31,3g (560 mmoles) de fer sont rajoutés à une solution contenant 69,2g (137 mmoles) de 4-[2-(5-méthoxy-2-nitro-phényl)-éthylamino]-pipéridine-1-carboxylate de méthyle dans 130ml de 2-méthyltétrahydrofuranne,130ml de méthanol et 130ml d'eau. Le milieu réactionnel est chauffé à reflux pendant 4 heures puis laissé revenir à température ambiante. Au bout de 16 heures, le milieu réactionnel est filtré sur célite et le filtrat est concentré jusqu'à évaporation du méthanol. Du méthyltétrahydrofurane est rajouté ainsi qu'une solution à 10 % d'acide éthylènediaminetetraacétique. La phase organique est lavée par de l'eau puis séchée sur sulfate de sodium anhydre, filtrée et concentrée. 53,2g (100%) de 4-[2-(2-amino-5-méthoxy-phényl)-éthylamino]-pipéridine-1-carboxylate de tert-butyle sont obtenus sous la forme d'une huile orange.

### 115.5 : 4-(7-méthoxy-2-oxo-1,2,4,5-tétrahydro-benzo[d][1,3]diazépin-3-yl)-pipéridine-1-carboxylate de tert-butyle

27,2g (168 mmoles) de N,N'-carbonyldiimidazole sont rajoutés par portion à une solution contenant 53,2g (137 mmoles) de 4-[2-(2-amino-5-méthoxy-phényl)-éthylamino]-pipéridine-1-carboxylate de tert-butyle à 90% dans du 1000ml de toluène. Après 1 heure, le précipité formé est filtré et le filtrat est concentré partiellement. Le résidu est lavé deux fois par une solution aqueuse de chlorure d'ammonium. La phase organique est séchée sur sulfate de sodium anhydre, filtrée et concentrée. 58g d'une huile orange sont obtenus. Après purification par chromatographie sur gel de silice élué par un mélange d'acétate d'éthyle dans l'heptane, suivant un gradient de polarité (de 20% à 70% d'acétate d'éthyle dans l'heptane), 35g d'un solide jaune sont obtenus et repris dans 200ml d'éther isopropylique au reflux, 100ml d'éther isopropylique, 100ml de tétrahydrofurane, 80ml d'éthanol et 300ml de méthanol. Le mélange est filtré puis concentré. Après purification par chromatographie sur gel de silice élué par un mélange d'acétate d'éthyle dans l'heptane, suivant un gradient de polarité (de 20% à 100% d'acétate d'éthyle dans l'heptane), 21,1g (34%) de 4-(7-méthoxy-2-oxo-1,2,4,5-tétrahydro-benzo[d][1,3]diazépin-3-yl)-pipéridine-1-carboxylate de tert-butyle sont obtenus sous la forme d'un solide beige.

### 115.6 : 7-méthoxy-3-pipéridin-4-yl-1,3,4,5-tétrahydro-benzo[d] [1,3]diazépin-2-one

35ml (380 mmoles) d'acide trifluoroacétique sont rajoutés à 0°C à une solution contenant 83% de 4-(7-méthoxy-2-oxo-1,2,4,5-tétrahydro-benzo[d] [1,3]diazépin-3-yl)-pipéridine-1-carboxylate de tert-butyle dans 300ml de dichlorométhane. Après 15 heures, le milieu réactionnel est concentré lentement à l'évaporateur rotatif. Le résidu est coévaporé au toluène,repris dans 250ml de dichlorométhane puis le mélange est amené à pH=10 avec une solution aqueuse d'hydroxyde de sodium 1N. La phase aqueuse est extraite par du dichlorométhane, la phase organique est séchée sur sulfate de sodium anhydre, filtrée et concentrée. 12,7g (96%) de 7-méthoxy-3-pipéridin-4-yl-1,3,4,5-tétrahydro-benzo[d] [1,3]diazépin-2-one sont obtenus sous la forme d'un solide beige.

### 115.7 : [5-bromo-3-((S)-2-tert-butoxycarbonylamino-1-méthyl-éthyl)-2,4-dioxo-3,4-dihydro-2H-pyrimidin-1-yl]-acétate de méthyle

De manière analogue à l'exemple 2.5, à partir de 3,3 g (12,6 mmoles) de (5-bromo-2,4-dioxo-3,4-dihydro-2H-pyrimidin-1-yl)-acétate de méthyle (préparé comme dans l'exemple 13.1) et 2g (11,4 mmoles) de ((R)-2-hydroxy-propyl)-carbamate de tert-butyle, et après purification par chromatographie sur gel de silice élué par un mélange d'acétate d'éthyle dans l'heptane, suivant un gradient de polarité (de 20% à 50% d'acétate d'éthyle dans l'heptane), 4,1g (85%) de [5-bromo-3-((S)-2-tert-butoxycarbonylamino-1-méthyl-éthyl)-2,4-dioxo-3,4-dihydro-2H-pyrimidin-1-yl]-acétate de méthyle sont obtenus sous la forme d'une huile visqueuse.

### 115.8 : [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétate de méthyle

De manière analogue à l'exemple 44.1, à partir de 1,9g (11,1 mmoles) d'acide 2-chloro-3-fluorophénylboronique et 3,1g (7,4 mmoles) de [5-bromo-3-((S)-2-tert-butoxycarbonylamino-1-méthyl-éthyl)-2,4-dioxo-3,4-dihydro-2H-pyrimidin-1-yl]-acétate de méthyle, 2,4g (68%) de [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétate de méthyle sont obtenus sous la forme d'une meringue.

### 115.9 : acide [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique

De manière analogue à l'exemple 16.8, à partir de 1g (2,1 mmoles) de [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétate de méthyle, 983mg (101%) d' acide [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique sont obtenus sous la forme d'un solide beige.

### 115.10 : [(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]- carbamate de tert-butyle

490mg (2,6 mmoles) de chlorhydrate de 1-éthyl-3-(3-diméthylaminopropyl)carbodiimide et 284mg (2,6 mmoles) de 1-oxy-pyridin-2-ol sont additionnés à une solution contenant 971mg (2,1 mmoles) d'acide [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique dans 5ml de N,N-diméthylformamide. Le milieu réactionnel est agité pendant 5 minutes puis 645mg (2,3 mmoles) de 7-méthoxy-3-pipéridin-4-yl-1,3,4,5-tétrahydro-benzo[d] [1,3]diazépin-2-one et 0,4ml (2,6 mmoles) de triéthylamine sont additionnés. Le milieu réactionnel est agité à température ambiante pendant 12 heures puis dilué par de l'acétate d'éthyle et de l'eau. La phase aqueuse est extraite trois fois par de l'acétate d'éthyle, la phase organique est lavée par une solution saturée de carbonate de sodium puis par une solution aqueuse d'acide chlorhydrique 1N. Après séchage sur sulfate de magnésium, la solution est filtrée et concentrée sous vide. Après purification par chromatographie sur gel de silice élué par un mélange de méthanol dans le dichlorométhane, suivant un gradient de polarité (de 0% à 5% de méthanol dans le dichlorométhane), 1,2g (79%) de [(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-carbamate de tert-butyle sont obtenus sous la forme d'un solide beige.

### 115.11 : 3-((S)-2-amino-1-méthyl-éthyl)-5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione

0,8ml (7,8 mmoles) d'acide trifluoroacétique sont ajoutés à une solution contenant 800mg (1,1 mmoles) de [(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-carbamate de tert-butyle dans 7ml de dichlorométhane. Le mélange est agité à température ambiante pendant deux heures puis deux équivalents d'acide trifluoroacétique sont ajoutés . Le mélange est concentré sous vide et repris dans le dichlorométhane et traité avec une solution aqueuse d'hydroxyde de sodium 1N. La phase aqueuse est extraite trois fois par du dichlorométhane, la phase organique est lavée par de l'eau puis par de la saumure. La phase organique est séchée sur sulfate de sodium anhydre, filtrée et concentrée à sec. 646mg (94%) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide blanc cassé.

### 115.12 : N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide (composé 115)

0,1ml (0,8 mmoles) de triéthylamine et 68mg (0,7 mmoles) d'anhydride acétique sont ajoutés à -10°C à une solution contenant 391mg (0,6 mmoles) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione dans 3,2ml de tétrahydrofuranne. Le mélange est hydrolysé après 5 minutes à -10°C par de l'eau et dilué à l'acétate d'éthyle. La phase aqueuse est extraite trois fois par de l'acétate d'éthyle, la phase organique est lavée par de la soude 1N puis par de la saumure. Après séchage sur sulfate de magnésium, la solution est filtrée puis concentrée. Le précipité est repris dans l'acétate d'éthyle et agité pendant 24 heures à température ambiante. 190mg (44%) de N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide sont obtenus sous la forme d'un solide blanc de point de fusion 200°C.

RMN ¹H (δ, DMSO): 1.34 (d, J = 6.5 Hz, 3H), 1.42-1.75 (m, 4H), 1.77 (s, 3H), 2.60-2.75 (m, 1H), 2.82-2.93 (m, 2H), 3.14 (t, J = 12.6 Hz, 1H), 3.32-3.42 (m, 2H), 3.40-3.61 (m, 2H),3.67 (s, 3H), 3.94 (bt d, J = 13.3 Hz, 1H), 4.18-4.38 (m, 1H), 4.43 (d, J = 12.7 Hz, 1H), 4.58-4.90 (m, 2H), 4.99 (m, 1H), 6.62 (d, J = 2.9 Hz, 1H), 6.66 (dd, J = 2.9, 8.8Hz, 1H), 6.98 (d, J = 8.8 Hz, 1H), 7.13 - 7.31 (m, 1H), 7.35-7.57 (m, 2H), 7.8 (s, 1H), 7.88-8.02 (m, 1H), 8.32 (s, 1H).

Les composés suivants ont été synthétisés selon un mode opératoire analogue en utilisant les réactifs appropriés commerciaux ou préalablement préparés:

### composé 203: N-[(R)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide

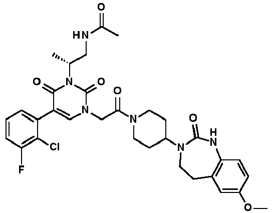

RMN ¹H (δ, DMSO) : 1.34 (dd, J = 6.8, 1.4, 3H), 1.45-1.75 (m, 4H), 1.67-1.87 (m, 3H), 2.62-2.78 (m, 1H), 2.81-2.94 (m, 2H), 3.14 (t, J = 12.5 Hz, 1H), 3.32-3.43 (m, 2H), 3.39-3.62 (m, 2H), 3.67 (s, 3H), 3.87-3.99 (m, 1H), 4.17-4.35 (m, 1H), 4.44 (d, J = 12.6 Hz, 1H), 4.60-4.86 (m, 2H), 4.99 (q, J = 7.2 Hz, 1H), 6.44-6.76 (m, 2H), 6.98 (d, J = 8.8 Hz, 1H), 7.21 (dd, J = 5.7, 3.3 Hz, 1H), 7.39-7.58 (m, 2H), 7.79 (s, 1H), 7.93 (t, J=6.1, 1H), 8.32 (s, 1H).

### composé 205: N-[(S)-2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide

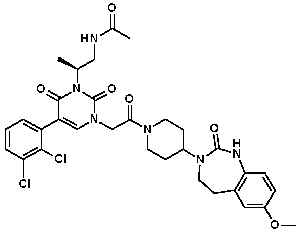

RMN ¹H (δ, DMSO) : 1.34 (d, J = 6.9 Hz, 3H); 1.56-1.71 (m, 4H); 1.77 (s, 3H); 2.68 (t, J = 12.3 Hz, 1H); 2.88 (m, 2H); 3.15 (m, 1H); 3.36 (m, 2H); 3.46-3.58 (m, 2H); 3.67 (s, 3H); 3.95 (d, J = 13.6 Hz, 1H); 4.28 (m, 1H); 4.44 (d, J = 12.8 Hz, 1H); 4.67-4.81 (m, 2H); 4.99 (m, 1H); 6.63-6.68 (m, 2H); 6.98 (d, J = 8.8 Hz, 1H); 7.33 (d, J = 7.7 Hz, 1H); 7.43 (t, J = 7.9 Hz, 1H); 7.68 (dd, J = 8.0, 1.6 Hz, 1H); 7.77 (s, 1H); 7.93 (t, J = 5.9 Hz, 1H); 8.32 (s, 1H).

### composé 206: N-[(S)-2-(5-(2-chloro-3-méthoxy-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide

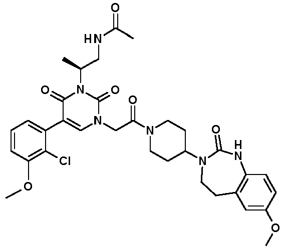

RMN ¹H (δ, DMSO) : 1.33 (d; J = 6.91 Hz; 3 H); 1.56 (m; 1 H); 1.68 (m; J = 20.83 Hz; 3 H); 1.77 (s; 3 H); 2.68 (t; J = 12.42 Hz; 1 H); 2.88 (d; J = 6.51 Hz; 2 H); 3.14 (t; J = 12.32 Hz; 1 H); 3.36 (d; J = 6.38 Hz; 2 H); 3.46-3.41 (m; 1 H); 3.59-3.54 (m; 1 H); 3.67 (s; 3 H); 3.89 (s; 3 H); 3.94 (d; J = 13.73 Hz; 1 H); 4.28 (m; 1 H); 4. 44 (d; J = 12.77 Hz; 1 H); 4.80-4.66 (m; 2 H); 4.99-4.97 (m; 1 H); 6.69-6.63 (m; 2 H); 6.91 (d; J = 7.64 Hz; 1 H); 6.98 (d; J = 8.79 Hz; 1 H); 7.18 (dd; J = 8.37; 1.40 Hz; 1 H); 7.34 (t; J = 7.96 Hz; 1 H); 7.68 (s; 1 H); 7.92 (t; J = 5.78 Hz; 1 H); 8.32 (s; 1 H).

### Exemple 116: N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide (schéma réactionnel n°4, composé 116)

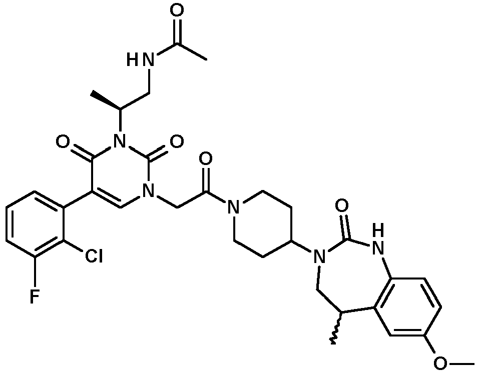

116.1: 2-(5-méthoxy-2-nitro-phényl)-propionitrile Une solution contenant 1g (5,2 mmoles) de (5-méthoxy-2-nitro-phényl)-acétonitrile (préparé comme dans l'exemple 115.1) et 0,6ml (8,9 mmoles) d'iodométhane dans 5ml de N,N-diméthylformamide est ajoutée à 0°C à une solution contenant 208mg (5,2 mmoles) d'hydrure de sodium dans 7ml (91 mmoles) de N,N-diméthylformamide. Après 1 heure, le mélange est dilué par de l'acétate d'éthyle puis par de l'eau. La phase aqueuse est extraite trois fois à l'acétate d'éthyle, la phase organique est lavée à l'eau puis brine. Après séchage sur sulfate de magnésium, la solution est filtrée puis concentrée sous vide. Après purification par chromatographie sur gel de silice, 663mg (62%) de 2-(5-méthoxy-2-nitro-phényl)-propionitrile sont obtenus sous la forme d'une huile claire.

### 116.2: 2-(5-méthoxy-2-nitro-phényl)-propylamine

De manière analogue à l'exmple 115.2, à partir de 663mg (3,2 mmoles) de 2-(5-méthoxy-2-nitro-phényl)-propionitrile, 900mg (104%) de 2-(5-méthoxy-2-nitro-phényl)-propylamine sont obtenus sous la forme d'une huile.

### 116.3: 4-[2-(5-méthoxy-2-nitro-phényl)-propylamino]-pipéridine-1-carboxylate de tert-butyle

De manière analogue à l'exemple 115.3, à partir de 677mg (3,2 mmoles) de 2-(5-méthoxy-2-nitro-phényl)-propylamine, 1,2g (95%) de 4-[2-(5-méthoxy-2-nitro-phényl)-propylamino]-pipéridine-1-carboxylate de tert-butyle sont obtenus sous la forme d'une huile orange.

### 116.4: 4-[2-(2-amino-5-méthoxy-phényl)-propylamino]-pipéridine-1-carboxylate de tert-butyle

De manière analogue à l'exemple 115.4, à partir de 1,2g (3,2 mmoles) de 4-[2-(5-méthoxy-2-nitro-phényl)-propylamino]-pipéridine-1-carboxylate de tert-butyle, 1,2g (103%) de 4-[2-(2-amino-5-méthoxy-phényl)-propylamino]-pipéridine-1-carboxylate de tert-butyle sont obtenus sous la forme d'un solide orange.

### 116.5: 4-(7-méthoxy-5-méthyl-2-oxo-1,2,4,5-tétrahydro-benzo[d][1,3]diazépin-3-yl)-pipéridine-1-carboxylate de tert-butyle

De manière analogue à l'exemple 115.5, à partir de 1,2g (3,2 mmoles) de 4-[2-(2-amino-5-méthoxy-phényl)-propylamino]-pipéridine-1-carboxylate de tert-butyle, 455mg (36%) de 4-(7-méthoxy-5-méthyl-2-oxo-1,2,4,5-tétrahydro-benzo[d][1,3]diazépin-3-yl)-pipéridine-1-carboxylate de tert-butyle sont obtenus sous la forme d'un solide beige.

### 116.6: 7-méthoxy-5-méthyl-3-pipéridin-4-yl-1,3,4,5-tétrahydro-benzo[d][1,3]diazépin-2-one

De manière analogue à l'exemple 115.6, à partir de 455mg (1,2 mmoles) de 4-(7-méthoxy-5-méthyl-2-oxo-1,2,4,5-tétrahydro-benzo[d][1,3]diazépin-3-yl)-pipéridine-1-carboxylate de tert-butyle, 335mg (99%) de 7-méthoxy-5-méthyl-3-pipéridin-4-yl-1,3,4,5-tétrahydro-benzo[d][1,3]diazépin-2-one sont obtenus sou la forme d'un solide beige.

### 116.7: Acide [(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle

De manière analogue à l'exemple 115.10, à partir de 549mg (1,2 mmoles) d'acide [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique (préparé comme dans l'exemple 115.9) et 332 mg (1,2 mmoles) de 7-méthoxy-5-méthyl-3-pipéridin-4-yl-1,3,4,5-tétrahydro-benzo[d][1,3]diazépin-2-one, 832mg (100%) d'acide [(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle sont obtenus.

### 116.8: 3-((S)-2-amino-1-méthyl-éthyl)-5-(2-chloro-3-fluoro-phényl)-1-{2-[4-(7-méthoxy-5-méthyl-2-oxo-1,2,4,5-tétrahydro-benzo[d][1,3]diazépin-3-yl)-pipéridin-1-yl]-2-oxo-éthyl}-1H-pyrimidine-2,4-dione

De manière analogue à l'exemple 115.11, à partir de 836mg (1,2 mmoles) d'acide [(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]- ]-carbamate de tert-butyle, et après ajout de 412mg (1,2 mmoles) d'acide(+)-o,o-dibenzoyl-D-tartrique dans le méthanol, chauffage et purification par chromatographie sur gel de silice, 492mg (68%) de 3-((S)-2-amino-1-méthyl-éthyl)-5-(2-chloro-3-fluoro-phényl)-1-{2-[4-(7-méthoxy-5-méthyl-2-oxo-1,2,4,5-tétrahydro-benzo[d][1,3]diazépin-3-yl)-pipéridin-1-yl]-2-oxo-éthyl}-1H-pyrimidine-2,4-dione sont obtenus.

### 116.9: N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide (composé 116)

De manière analogue à l'exemple 115.12, à partir de 100mg (0,2 mmoles) de 3-((S)-2-amino-1-méthyl-éthyl)-5-(2-chloro-3-fluoro-phényl)-1-{2-[4-(7-méthoxy-5-méthyl-2-oxo-1,2,4,5-tétrahydro-benzo[d] [1,3]diazépin-3-yl)-pipéridin-1-yl]-2-oxo-éthyl}-1H-pyrimidine-2,4-dione, 7mg (6%) de N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide sont obtenus sous la forme d'un solide beige.

RMN ¹H (400 MHz, DMSO-d6) δ: 1.18 (m, 3H), 1.34 (dd, J = 7.0, 1.8 Hz, 3H), 1.68 (m, 4H), 1.76 (s, 3H), 2.59-2.73 (m, 1H), 3.04 (m, 1H), 3.13 (m, 1H), 3.30 (m, 2H), 3.51 (m, 2H), 3.68 (s, 3H), 3.95 (m, 1H), 4.25 (m, 1H), 4.44 (m, 1H), 4.57-4.86 (m, 2H), 4.98 (m, 1H), 6.57-6.73 (m, 2H), 6.97 (d, J = 8.6 Hz, 1H), 7.21 (m, 1H), 7.36-7.52 (m, 2H), 7.79 (s, 1H), 7.95 (t, J = 6.1 Hz, 1H), 8.36 (s, 1H).

### Exemple 117: N-[2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-éthyl]-acétamide (schéma réactionnel n°3, composé 117)

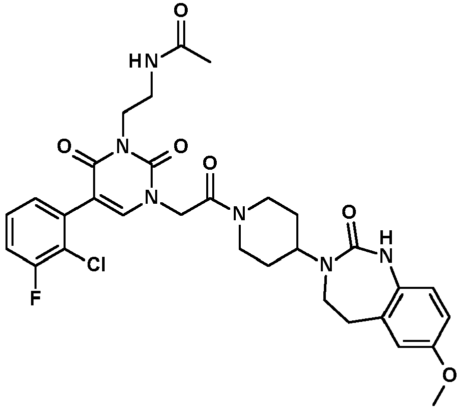

### 117.1: [5-bromo-3-(2-tert-butoxycarbonylamino-éthyl)-2,4-dioxo-3,4-dihydro-2H-pyrimidin-1-yl]-acétate de méthyle

2,4g (17,1 mmoles) de carbonate de potassium et 3,1g (13,7 mmoles) de N-(2-bromoéthyl)carbamate de tert-butyle sont additionnés sur une solution contenant 3g (11,4 mmoles) de (5-bromo-2,4-dioxo-3,4-dihydro-2H-pyrimidin-1-yl)-acétate de méthyle (préparé comme dans l'exemple 13.1) dans 60ml de N,N-diméthylformamide. Le milieu réactionnel est chauffé à 50°C pendant 2 heures puis est hydrolysé et dilué par de l'acétate d'éthyle. Le produit est extrait à l'acétate d'éthyle, la phase organiqus est lavée deux fois par de l'eau et une fois par une solution aqueuse saturée de chlorure de sodium, puis séchée sur sulfate de magnésium, filtrée et concentrée sous vide. Le produit est précipité dans 100ml d'ether diéthylique puis filtré et séché sous vide pendant 2 heures. 3,5g (76%) de [5-bromo-3-(2-tert-butoxycarbonylamino-éthyl)-2,4-dioxo-3,4-dihydro-2H-pyrimidin-1-yl]-acétate de méthyle sont obtenus sous la forme d'un solide blanc.

### 117.2: [3-(2-tert-butoxycarbonylamino-éthyl)-5-(2-chloro-3-fluoro-phényl)-2,4-dioxo-3,4-dihydro-2H-pyrimidin-1-yl]-acétate de méthyle

De manière analogue à l'exemple 115.8, à partir de 429mg (2,5 mmoles) d'acide 2-chloro-3-fluorophénylboronique et 500mg (1,2 mmoles) de [5-bromo-3-(2-tert-butoxycarbonylamino-éthyl)-2,4-dioxo-3,4-dihydro-2H-pyrimidin-1-yl]-acétate de méthyle, et après purification par chromatographie sur gel de silice élué par un mélange d'acétate d'éthyle dans l'heptane, suivant un gradient de polarité (de 40% à 80% d'acétate d'éthyle dans l'heptane), 370mg (66%) de [3-(2-tert-butoxycarbonylamino-éthyl)-5-(2-chloro-3-fluoro-phényl)-2,4-dioxo-3,4-dihydro-2H-pyrimidin-1-yl]-acétate de méthyle sont obtenus sous la forme d'un solide beige.

### 117.3: Acide [3-(2-tert-butoxycarbonylamino-éthyl)-5-(2-chloro-3-fluoro-phényl)-2,4-dioxo-3,4-dihydro-2H-pyrimidin-1-yl]-acétique

De manière analogue à l'exemple 115.9, à partir de 370mg (0,8 mmoles) de [3-(2-tert-butoxycarbonylamino-éthyl)-5-(2-chloro-3-fluoro-phényl)-2,4-dioxo-3,4-dihydro-2H-pyrimidin-1-yl]-acétate de méthyle, 353mg (98%) d'acide [3-(2-tert-butoxycarbonylamino-éthyl)-5-(2-chloro-3-fluoro-phényl)-2,4-dioxo-3,4-dihydro-2H-pyrimidin-1-yl]-acétique sont obtenus sous la forme d'un solide jaune.

### 117.4 [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-carbamate de tert-butyle

De manière analogue à l'exemple 115.10, à partir de 353mg (0,8 mmoles) d'acide [3-(2-tert-butoxycarbonylamino-éthyl)-5-(2-chloro-3-fluoro-phényl)-2,4-dioxo-3,4-dihydro-2H-pyrimidin-1-yl]-acétique et 242mg (0,9 mmoles) de 7-méthoxy-3-pipéridin-4-yl-1,3,4,5-tétrahydro-benzo[d][1,3]diazépin-2-one (préparé comme dans l'exemple 115.6), 270mg (48%) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-carbamate de tert-butyle sont obtenus sous la forme d'une pâte incolore.

### 117.5: 3-(2-amino-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

De manière analogue à l'exemple 115.11, à partir de 270mg (13,1 mmoles) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-carbamate de tert-butyle, 240mg (104%) de 3-(2-amino-éthyl)-5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione sont obtenus sous la forme d'un solide beige.

### 117.6: N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide (composé 117)

De manière analogue à l'exemple 115.12, à partir de 240mg (0,4 mmoles) de 3-(2-amino-éthyl)-5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione et 2,5mg (0,02 mmoles) de 4-diméthylaminopyridine, 202mg (76%) de N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide sont obtenus sous la forme d'un solide blanc de point de fusion 266°C.

RMN ¹H (δ, DMSO): 1.56-1.51 (1 H, m), 1.73-1.65 (3 H, m), 1.76 (3 H, s), 2.68 (1 H, t, J = 12.29 Hz), 2.88 (2 H, m), 3.15 (1 H, t, J = 11.94 Hz), 3.28-3.23 (2 H, m), 3.40-3.30 (2 H, m), 3.67 (3 H, s), 3.95 (3 H, t, J = 6.79 Hz), 4.28 (1 H, m), 4.43 (1 H, d, J = 12.86 Hz), 4.78 (2 H, s), 6.68-6.62 (2 H, m), 6.98 (1 H, d, J = 8.78 Hz), 7.22-7.20 (1 H, m), 7.47-7.44 (2 H, m), 7.82 (1 H, s), 7.93 (1 H, t, J = 5.87 Hz), 8.33 (1 H, s).

Autre composé synthétisé selon un mode opératoire analogue en utilisant les réactifs appropriés ou préalablement préparés: composé 207: N-[2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide

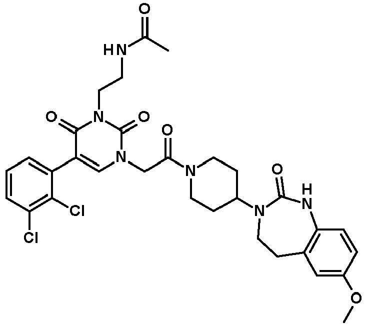

Masse = 657

### Exemple 118 : N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide (schéma réactionnel n°3, composé 118)

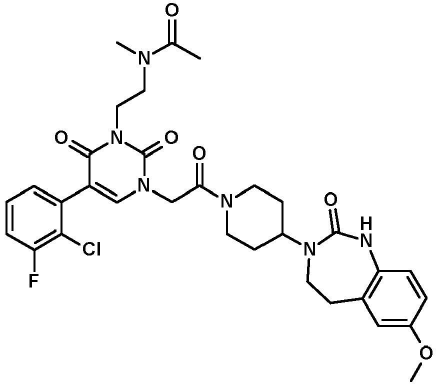

### 118.1 : (2-chloro-éthyl)-méthyl-carbamate de tert-butyle

1,2g (5,6 mmoles) de dicarbonate de di-tert-butyle sont additionnés sur une solution contenant 0,5g (5,3 mmoles) de (2-chloro-éthyl)-méthyl-amine dans 10ml de dichlorométhane. Le mélange est agité à température ambainte pendant deux heures, de l'eau est rajoutée puis le produit est extrait avec du dichlorométhane La phase organique est séchée sur sulfate de sodium anhydre puis filtrée et concentrée à sec. 1g (97%) de (2-chloro-éthyl)-méthyl-carbamate de tert-butyle sont obtenus sous la forme d'une huile incolore.

### 118.2: [5-bromo-3-[2-(tert-butoxycarbonyl-méthyl-amino)-éthyl]-2,4-dioxo-3,4-dihydro-2H-pyrimidin-1-yl]-acétate de méthyle

0,6g (4,2 mmoles) de carbonate de potassium et 0,8g (4,2 mmoles) de (2-chloro-éthyl)-méthyl-carbamate de tert-butyle sont additionnés sur une solution contenant 1g (3,8 mmoles) de (5-bromo-2,4-dioxo-3,4-dihydro-2H-pyrimidin-1-yl)-acétate de méthyle (préparé comme dans l'exemple 13.1) dans 13ml de N,N-diméthylformamide. Le mélange est chauffé à 70°C pendant trois heures puis de l'eau est rajoutée et le mélange est extrait deux fois par de l'acétate d'éthyle. La phase organique est lavée à l'eau, séchée sur sulfate de magnésium, filtrée et évaporée. Après purification par chromatographie sur gel de silice, 300mg (19%) de [5-bromo-3-[2-(tert-butoxycarbonyl-méthyl-amino)-éthyl]-2,4-dioxo-3,4-dihydro-2H-pyrimidin-1-yl]-acétate de méthyle sont obtenus sous la forme d'une huile incolore.

### 118.3 : [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétate de méthyle

De manière analogue à l'exemple 115.8, à partir de 300mg (0,7 mmoles) de [5-bromo-3-[2-(tert-butoxycarbonyl-méthyl-amino)-éthyl]-2,4-dioxo-3,4-dihydro-2H-pyrimidin-1-yl]-acétate de méthyle et 249mg (1,4 mmoles) d'acide 2-chloro-3-fluorophénylboronique, et après purification par chromatographie sur gel de silice élué par un mélange d'acétate d'éthyle dans l'heptane, suivant un gradient de polarité (de 40% à 70% d'acétate d'éthyle dans l'heptane), 140mg (42%) de [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétate de méthyle sont obtenus sous la forme d'un solide beige.

### 118.4 : Acide [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétique

De manière analogue à l'exemple 115.9, à partir de 140mg (0,3 mmoles) de [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétate de méthyle, 160mg (118%) d' acide [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétique ont obtenus sous la forme d'une huile incolore.

### 118.5 : [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-méthyl-carbamate de tert-butyle

De manière analogue à l'exemple 115.10, à partir de 160mg (0,4 mmoles) d'acide [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétique et 106mg (0,4 mmoles) de 7-méthoxy-3-pipéridin-4-yl-1,3,4,5-tétrahydro-benzo[d][1,3]diazépin-2-one (préparé comme dans l'exemple 115.6), 120mg (48%) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-méthyl-carbamate de tert-butyle sont obtenus sous la forme d'une huile incolore.

### 118.6 : 5-(2-Chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylamino-éthyl)-1H-pyrimidine-2,4-dione

De manière analogue à l'exemple 115.11, à partir de 120mg (0,2 mmoles) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-méthyl-carbamate de tert-butyle, 120mg (116%) de 5-(2-Chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-3-(2-méthylamino-éthyl)-1H-pyrimidine-2,4-dione sont obtenus sous la forme d'un solide blanc.

### 118.7 : N-[2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide (composé 118)

0,03ml (0,2 mmoles) de triéthylamine et 1mg (0,01 mmoles) de 4-diméthylaminopyridine sont additionnés à une solution contenant 120mg (0,2 mmoles) de 5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-3-(2-méthylamino-éthyl)-1H-pyrimidine-2,4-dione dans 6ml de tétrahydrofuranne. Le mélange est refroidi à -10°C, puis 0,01ml (0,2 mmoles) de chlorure d'acétyle sont rajoutés. Après 30 minutes d'agitation à -10°C, le mélange est versé sur une solution aqueuse de carbonate de sodium saturée, puis extrait deux fois à l'acétate d'éthyle. La phase organique est séchée sur sulfate de sodium anhydre puis filtrée et concentrée à sec. Le solide obtenu est purifié par chromatographie sur gel de silice élué par un mélange de méthanol dans le dichlorométhane, suivant un gradient de polarité (de 0% à 5% de méthanol dans le dichlorométhane) . Après reprise dans du dichlorométhane et de l'heptane, précipitation et concentration à sec, 58mg (45%) de N-[2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide sont obtenus sous la forme d'un solide blanc.

RMN ¹H (400 MHz, DMSO-d6) δ: 1.46-1.61 (m, 1H), 1.61-1.82 (m, 3H), 1.89 (s, 1.5H), 1.95 (s, 1.5H), 2.67 (m, 1H), 2.87 (m, 2H), 2.82 (s, 1.5H), 2.95 (s, 1.5H), 3.14 (m, 1H), 3.33-3.42 (m, 2H), 3.52 (m, 2H), 3.67 (s, 3H), 3.95 (m, 1H), 4.01-4.18 (m, 2H), 4.28 (m, 1H), 4.42 (d, J = 12.8 Hz, 1H), 4.78 (d, J = 10.3 Hz, 2H), 6.98 (d, J = 8.7 Hz, 1H), 7.19 (m, 1H), 7.45 (m, 2H), 7.84 (d, J = 28 Hz, 1H), 8.32 (s, 1H).

### Exemple 119 : 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 119)

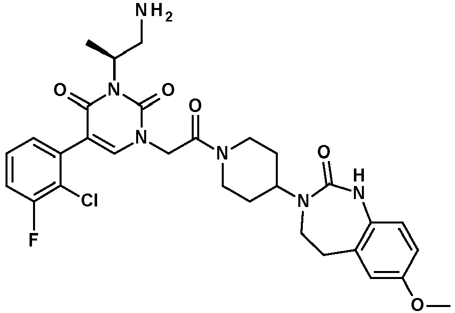

De manière analogue à l'exemple 115.10, à partir de 280mg (0,5 mmoles) de [(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle (préparé comme dans l'exemple 115.10), et après purification par chromatographie sur gel de silice, 265 mg (95%) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide blanc cassé de point de fusion 206°C.

RMN ¹H (δ, DMSO): 1.34 (d, J = 6.9 Hz, 3H), 1.38 - 1.58 (m, 3H), 1.67 (m, 3H), 2.68 (m, 1H), 2.80-2.94 (m, 3H), 3.04 (m, 1H), 3.14 (m, 1H), 3.34-3.43 (m, 2H), 3.67 (s, 3H), 3.93 (d, J = 13.5 Hz, 1H), 4.19-4.35 (m, 1H), 4.43 (d, J = 13.0 Hz, 1H), 4.74 (m, 2H), 4.82 (m, 1H), 6.62 (d, J = 3.0 Hz, 1H), 6.67 (dd, J = 8.8, 3.0 Hz, 1H), 6.98 (d, J = 8.8 Hz, 1H), 7.22 (m, 1H), 7.36-7.53 (m, 2H), 7.78 (s, 1H), 8.32 (s, 1H).

### Exemple 120 : N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-méthanesulfonamide (schéma réactionnel n°3 ou 4, composé 120)

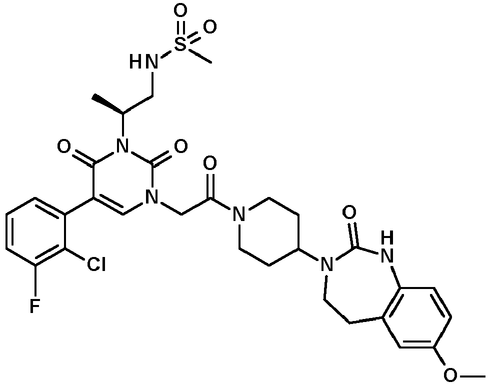

0,3ml (3,7 mmoles) de chlorure de méthanesulfonyle et 0,5ml (3,7 mmoles) de triéthylamine sont ajoutés à 0°C à une solution contenant 1,5g (2,5 mmoles) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (préparé comme dans l'exemple 119) dans 15ml de dichlorométhane. Après 30 minutes, du dichlorométhane est rajouté et le milieu réactionnel est hydrolysé avec une solution aqueuse d'acide chlorhydrique 1N. La phase aqueuse est réextraite au dichlorométhane. La phase organique est séchée sur sulfate de sodium anhydre, filtrée et concentrée. Après purification par chromatographie, reprise dans le pentane et est filtration, 0,8g (45%) de N-[(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-méthanesulfonamide sont obtenus sous la forme d'un solide blanc.

RMN ¹H (δ, DMSO): 1.36 (dd, J = 2.1, 7.0 Hz, 3H), 1.43 - 1.80 (m, 4H), 2.59 - 2.72 (m, 1H), 2.86 (br s, 3+2H), 3.13 (t, J = 12.4 Hz, 1H), 3.34 - 3.40 (m, 2H), 3.46 - 3.63 (m, 2H), 3.66 (s, 3H), 3.94 (d, J = 13.5 Hz, 1H), 4.18 - 4.35 (m, 1H), 4.42 (d, J = 12.9 Hz, 1H), 4.66 - 4.82 (m, 2H), 4.88 - 5.16 (m, 1H), 6.61 (d, J = 3.0 Hz, 1H), 6.66 (dd, J = 3.0, 8.7 Hz, 1H), 6.97 (d, J = 8.8 Hz, 1H), 7.15 - 7.30 (m, 2H), 7.38 - 7.51 (m, 2H), 7.80 (s, 1H), 8.34 (s, 1H).

### Exemple 121 : 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-[(S)-1-méthyl-2-(3-méthyl-oxétan-3-ylamino)-éthyl]-1H-pyrimidine-2,4-dione (schéma réactionnel n°3, composé 121)

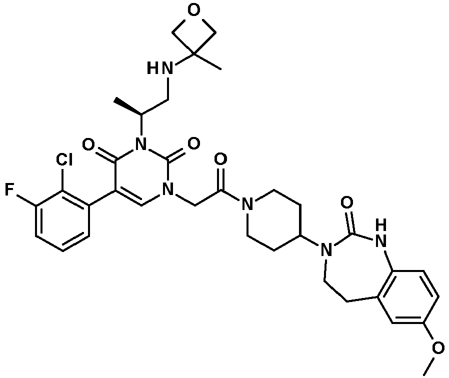

### 121.1: [5-bromo-3-((S)-2-chloro-1-méthyl-éthyl)-2,4-dioxo-3,4-dihydro-2H-pyrimidin-1-yl]-acétate de méthyle

1,3g (5,7 mmoles) d'azodicarboxylate de di-tert-butyle sont additionnés lentement sur une solution préalablement refroidie à 0°C contenant 1g (3,8 mmoles) de (5-bromo-2,4-dioxo-3,4-dihydro-2H-pyrimidin-1-yl)-acétate de méthyle (préparé comme dans l'exemple 13.1), 0,4ml (4,6 mmoles) de (R)-1-chloro-propan-2-ol et 1,5g (5,7 mmoles) de triphénylphosphine dans 20ml de tétrahydrofuranne. Après agitation à température ambiante durant 4 heures, le tétrahydrofurane est éliminé sous vide puis le résidu est repris dans l'acétate d'éthyle. La phase organique est lavée une fois par une solution aqueuse saturée d'hydrogénocarbonate de sodium, une fois par de l'eau puis par une solution aqueuse saturée de chlorure de sodium, séchée sur sulfate de magnésium, filtrée et concentrée sous vide. Après purification par chromatographie sur gel de silice élué par un mélange d'acétate d'éthyle dans l'heptane, suivant un gradient de polarité (de 2% à 50% d'acétate d'éthyle dans l'heptane), 1,2g (93%) de [5-bromo-3-((S)-2-chloro-1-méthyl-éthyl)-2,4-dioxo-3,4-dihydro-2H-pyrimidin-1-yl]-acétate de méthyle sont obtenus sous la forme d'une huile incolore.

### 121.2: [5-bromo-3-((S)-2-hydroxy-1-méthyl-éthyl)-2,4-dioxo-3,4-dihydro-2H-pyrimidin-1-yl]-acétate de méthyle

1ml (7,1 mmoles) de triéthylamine et 3,6ml de méthanol sont additionnés sur une solution contenant 1,2g (3,5 mmoles) de [5-bromo-3-((S)-2-chloro-1-méthyl-éthyl)-2,4-dioxo-3,4-dihydro-2H-pyrimidin-1-yl]-acétate de méthyle dans 12ml de tétrahydrofuranne et 2,7ml (151 mmoles) d'eau. Le mélange réactionnel est chauffé à 70°C pendant 22 heures puis les solvants sont concentrés sous vide. 440mg (39%) de [5-bromo-3-((S)-2-hydroxy-1-méthyl-éthyl)-2,4-dioxo-3,4-dihydro-2H-pyrimidin-1-yl]-acétate de méthyle sont obtenus sous la forme d'une huile claire.

### 121.3: [5-Bromo-3-((S)-1-méthyl-2-oxo-éthyl)-2,4-dioxo-3,4-dihydro-2H-pyrimidin-1-yl]-acétate de méthyle

697mg (1,6 mmoles) de périodinane de Dess-Martin sont additionnés à température ambiante sur une solution contenant 440mg (1,4 mmoles) de [5-bromo-3-((S)-2-hydroxy-1-méthyléthyl)-2,4-dioxo-3,4-dihydro-2H-pyrimidin-1-yl]-acétate de méthyle dans 13ml de dichlorométhane. Après 20 heures, de l'eau et du dichlorométhane sont rajoutés. La phase organique est récupérée puis concentrée sous vide. 440mg de [5-Bromo-3-((S)-1-méthyl-2-oxo-éthyl)-2,4-dioxo-3,4-dihydro-2H-pyrimidin-1-yl]-acétate de méthyle sont obtenus.

### 121.4: [5-bromo-3-[(S)-1-méthyl-2-(3-méthyl-oxétan-3-ylamino)-éthyl]-2,4-dioxo-3,4-dihydro-2H-pyrimidin-1-yl]-acétate de méthyle

440mg (1,4 mmoles) de [5-bromo-3-((S)-1-méthyl-2-oxo-éthyl)-2,4-dioxo-3,4-dihydro-2H-pyrimidin-1-yl]-acétate de méthyle, 0,1ml (1,4 mmoles) d'acide acétique et 0,1ml (1,4 mmoles) de 3-méthyloxétan-3-amine sont dilués dans 8,8ml de dichlorométhane. 409mg (1,9 mmoles) de triacétoxyborohydrure de sodium et 87mg (1,4 mmoles) de cyanoborohydrure de sodium sont ensuite rajoutés. L'agitation à température ambiante est maintenue pendant 20 heures. Le mélange est ensuite versé sur une solution aqueuse d'hydroxyde de sodium 1N puis extrait par du dichlorométhane.La phase organique est séchée sur sulfate de sodium anhydre puis filtrée et concentrée à sec. 220mg (41%) de [5-bromo-3-[(S)-1-méthyl-2-(3-méthyl-oxétan-3-ylamino)-éthyl]-2,4-dioxo-3,4-dihydro-2H-pyrimidin-1-yl}-acétate de méthyle sont obtenus sous la forme d'une huile incolore.

### 121.5: [5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétate de méthyle

De manière analogue à l'exemple 115.8, à partir d'une solution contenant 220mg (0,6 mmoles) de [5-bromo-3-[(S)-1-méthyl-2-(3-méthyl-oxétan-3-ylamino)-éthyl]-2,4-dioxo-3,4-dihydro-2H-pyrimidin-1-yl}-acétate de méthyle dans 2,2ml de toluène et 1,4ml de carbonate de potassium et après purification par chromatographie sur gel de silice élué par un mélange de méthanol dans le dichlorométhane, suivant un gradient de polarité (de 0% à 5% de méthanol dans le dichlorométhane), 30mg (12%) de [5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétate de méthyle sont obtenus sous la forme d'une huile jaune.

### 121.6: Acide[5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl}-acétique

De manière analogue à l'exemple 115.9, à partir de 30mg (0,1 mmoles) de [5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétate de méthyle, 29mg (100%) d'acide[5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl}-acétique sont obtenus sous la forme d'une huile marron.

### 121.7: 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-[(S)-1-méthyl-2-(3-méthyl-oxétan-3-ylamino)-éthyl]-1H-pyrimidine-2,4-dione (composé 121)

10mg (0,1 mmoles) de 1-oxy-pyridin-2-ol et 21mg (0,1 mmoles) de 7-méthoxy-3-pipéridin-4-yl-1,3,4,5-tétrahydrobenzo[d][1,3]diazépin-2-one (préparé comme dans l'exemple 115.6) sont additionnés sur une solution contenant 30mg (0,1 mmoles) d'acide[5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétique dans 0,6ml de N,N-diméthylformamide. 17,6mg (0,1 mmoles) de chlorhydrate de 1-éthyl-3-(3-diméthylaminopropyl) carbodiimide sont rajoutés puis l'agitation est maintenue pendant 20 heures à température ambiante. Le mélange est versé sur de l'eau puis extrait par du dichlorométhane. La phase organique est lavée avec une solution aqueuse d'hydrogénocarbonate de sodium saturée puis rincée à l'eau, séchée sur sulfate de sodium anhydre puis filtrée et concentrée à sec. Après purification par chromatographie sur gel de silice élué par un mélange de méthanol dans le dichlorométhane, suivant un gradient de polarité (de 0% à 10% de méthanol dans le dichlorométhane), 18mg (37%) de 5-(2-chloro-3-fluoro-phényl)-1-[2-[4-(7-méthoxy-2-oxo-1,2,4,5-tétrahydro-benzo[d] [1,3]diazépin-3-yl)-pipéridin-1-yl]-2-oxo-éthyl]-3-[(S)-1-méthyl-2-(3-méthyl-oxétan-3-ylamino)-éthyl]-1H-pyrimidine-2,4-dione sont obtenus sous la forme d'un solide blanc.

RMN ¹H (δ, DMSO): 1.33 (s; 3 H); 1.36-1.39 (m; 4 H); 1.53-1.56 (m; 1 H); 1.65-1.75 (m; 3 H); 2.68 (m; 1 H); 2.87-2.92 (m; 3 H); 3.02-3.07 (m; 1 H); 3.12-3.22 (m; 1 H); 3.36 (m ; 2H); 3.67 (s; 3 H); 3.94 (d; J = 13.50 Hz; 1 H); 4.16 (t; J = 5.37 Hz; 2 H); 4.28 (m; 1 H); 4.39 (d; J = 5.31 Hz; 2 H); 4.43 (d; J = 12.35 Hz; 1 H); 4.74 (m; 2 H); 4.89 (m; 1 H); 6.62 (d; J = 2.75 Hz; 1 H); 6.67 (dd; J = 8.79; 2.92 Hz; 1 H); 6.98 (d; J = 8.79 Hz; 1 H); 7.20-7.22 (m; 1 H); 7.43-7.46 (m; 2 H); 7.78 (s; 1 H); 8.32 (s; 1 H).

### Exemple 122: 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3 ou 4, composé 122)

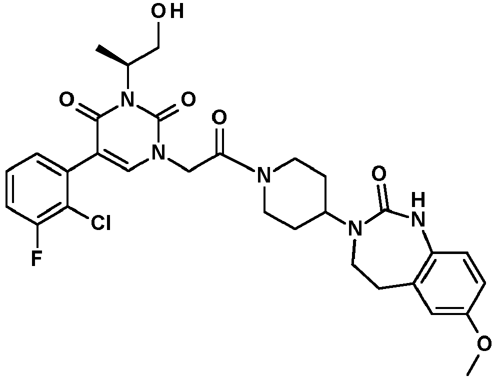

### 122.1: Acide [5-(2-chloro-3-fluoro-phényl)-3-((S)-2-hydroxy-1-méthyl-éthyl)-2,4-dioxo-3,4-dihydro-2H-pyrimidin-1-yl]-acétique

De manière analogue à l'exemple 115.8, à partir de 366mg (2,1 mmoles) d'acide 2-chloro-3-fluorophénylboronique et 450mg (1,4 mmoles) de [5-bromo-3-((S)-2-hydroxy-1-méthyl-éthyl)-2,4-dioxo-3,4-dihydro-2H-pyrimidin-1-yl]-acétate de méthyle (préparé comme dans l'exemple 121.2), 500mg (100%) d' acide [5-(2-chloro-3-fluoro-phényl)-3-((S)-2-hydroxy-1-méthyl-éthyl)-2,4-dioxo-3,4-dihydro-2H-pyrimidin-1-yl]-acétique sont obtenus.

### 122.2: 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (composé 122)

De manière analogue à l'exemple 115.10, à partir de 110mg (0,3 mmoles) d'acide [5-(2-chloro-3-fluoro-phényl)-3-((S)-2-hydroxy-1-méthyl-éthyl)-2,4-dioxo-3,4-dihydro-2H-pyrimidin-1-yl]-acétique et 93mg (0,3 mmoles) de 7-méthoxy-3-pipéridin-4-yl-1,3,4,5-tétrahydro-benzo[d][1,3]diazépin-2-one (préparé comme dans l'exemple 115.6), 150mg (78%) de 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide blanc de point de fusion 138°C.

RMN ¹H (δ, DMSO): 1.32 (dd, J = 7.1, 2.1 Hz, 3H), 1.55 (m, 1H), 1.61-1.83 (m, 3H), 2.68 (m, 1H), 2.82 - 2.92 (m, 2H), 3.14 (m, 1H), 3.34-3.41 (m, 2H), 3.67 (s, 3H), 3.69 (m, 1H), 3.83 (m, 1H), 3.94 (d, J = 13.7 Hz, 1H), 4.27 (m, 1H), 4.43 (d, J = 13.0 Hz, 1H), 4.74 (m, 2H), 4.79 (t, J = 5.7 Hz, 1H), 4.96 (h, J = 6.9 Hz, 1H), 6.62 (d, J = 3.0 Hz, 1H), 6.67 (dd, J = 8.9, 3.0 Hz, 1H), 6.98 (d, J = 8.8 Hz, 1H), 7.21 (m, 1H), 7.35-7.52 (m, 2H), 7.78 (s, 1H), 8.32 (s, 1H).

### Exemple 123 : (S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-acétate de propyle (schéma réactionnel n°3, composé 123)

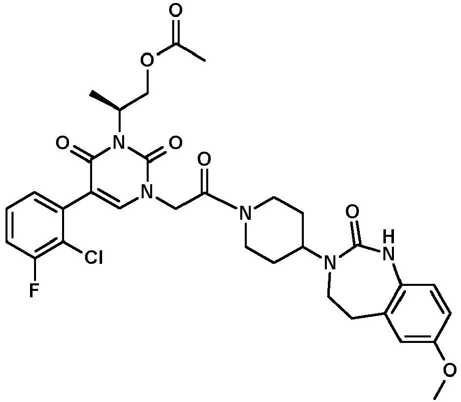

De manière analogue à l'exemple 115.12, à partir de 80mg (0,1 mmoles) de 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (préparé comme dans l'exemple 122.2), 62mg (71%) de (S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-acétate de propyle sont obtenus sous la forme d'un solide blanc de point de fusion 193°C.

RMN ¹H (δ, DMSO) : 1.39 (d, J = 6.9 Hz, 3H), 1.55 (m, 1H), 1.70 (m, 3H), 1.97 (s, 3H), 2.68 (m, 1H), 2.78-2.93 (m, 2H), 3.14 (m, 1H), 3.34-3.43 (m, 2H), 3.67 (s, 3H), 3.93 (d, J = 13.7 Hz, 1H), 4.18 - 4.38 (m, 2H), 4.46 (m, 2H), 4.66-4.85 (m, 2H), 5.18 (q, J = 7.2 Hz, 1H), 6.62 (d, J = 3.0 Hz, 1H), 6.67 (dd, J = 8.7, 2.9 Hz, 1H), 6.98 (d, J = 8.8 Hz, 1H), 7.22 (m, 1H), 7.37-7.55 (m, 2H), 7.82 (s, 1H), 8.32 (s, 1H).

### Exemple 124 : 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 124)

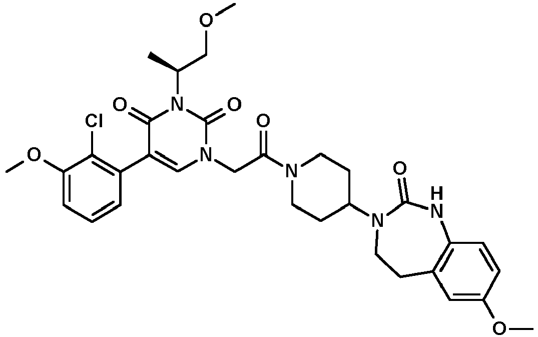

### 124.1 : [5-bromo-3-((S)-2-méthoxy-1-méthyl-éthyl)-2,4-dioxo-3,4-dihydro-2H-pyrimidin-1-yl]-acétate de méthyle

De manière analogue à l'exemple 2.5, à partir de 3g (11,4 mmoles) de (5-bromo-2,4-dioxo-3,4-dihydro-2H-pyrimidin-1-yl)-acétate de méthyle ( préparé comme dans l'exemple 13.1) et 1,2ml (12,6 mmoles) de (R)-1-méthoxy-propan-2-ol , et après purification par chromatographie sur gel de silice élué par un mélange d'acétate d'éthyle dans l'heptane, suivant un gradient de polarité (de 30% à 50% d'acétate d'éthyle dans l'heptane), 2,6g (67%) de [5-bromo-3-((S)-2-méthoxy-1-méthyl-éthyl)-2,4-dioxo-3,4-dihydro-2H-pyrimidin-1-yl]-acétate de méthyle sont obtenus sous la forme d'une huile incolore.

### 124.2 : Acide [5-(2-chloro-3-méthoxy-phényl)-3-((S)-2-méthoxy-1-méthyl-éthyl)-2,4-dioxo-3,4-dihydro-2H-pyrimidin-1-yl]-acétique

759mg (7,2 mmoles) de carbonate de sodium et 979mg (5,3 mmoles) d'acide 2-chloro-3-méthoxyphénylboronique sont additionnés sur une solution contenant 800mg (2,4 mmoles) de [5-bromo-3-((S)-2-méthoxy-1-méthyl-éthyl)-2,4-dioxo-3,4-dihydro-2H-pyrimidin-1-yl]-acétate de méthyle dans 80ml de 1,4-dioxanne et 8ml d'eau. Le milieu est dégazé à l'azote pendant 15 minutes, puis 194mg (0,2 mmoles) de complexe de dichlorométhane de 1,1'-bis-(diphénylphosphino)ferrocène-dichloropalladium(II) sont rajoutés et le milieu est chauffé à 100°C pendant 2 heures. Après refroidissement, 3,6ml d'hydroxyde de lithium monohydraté et 16ml d'eau sont rajoutés au millieu réactionnel qui est laissé toute une nuit. De l'eau et de l'acétate d'éthyle sont ajoutés au milieu réactionnel. La phase aqueuse est acidifiée à pH4 avec une solution aqueuse d'acide chlorhydrique 1N. Cette phase est extraite deux fois avec de l'acétate d'éthyle. La phase organique est lavée par de l'eau puis par une solution aqueuse de chlorure de sodium saturée et séchée sur sulfate de sodium anhydre, filtrée et concentrée à sec. 912mg (100%) d'acide[5-(2-chloro-3-méthoxy-phényl)-3-((S)-2-méthoxy-1-méthyl-éthyl)-2,4-dioxo-3,4-dihydro-2H-pyrimidin-1-yl]-acétique sont obtenus sous la forme d'une huile marron.

### 124.3 : 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (composé 124)

De manière analogue à l'exemple 115.10, à partir de 415mg (1,1 mmoles) d'acide [5-(2-chloro-3-méthoxy-phényl)-3-((S)-2-méthoxy-1-méthyl-éthyl)-2,4-dioxo-3,4-dihydro-2H-pyrimidin-1-yl]-acétique et 328mg (1,2 mmoles) de 7-méthoxy-3-pipéridin-4-yl-1,3,4,5-tétrahydro-benzo[d][1,3]diazépin-2-one (préparé comme dans l'exemple 115.6), 180mg (26%) de 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide blanc de point de fusion 269°C.

RMN ¹H (δ, DMSO): 1.33 (d, J = 6.9 Hz, 3H); 1.55-1.69 (m, 4H); 2.67-2.68 (m, 1H); 2.87 (m, 2H); 3.13 (m, 1H); 3.23 (s, 3H); 3.35-3.37 (m, 2H); 3.52-3.56 (m, 1H); 3.67 (s, 3H); 3.89 (s, 3H), 3.91 (m, 2H); 4.28 (m, 1H); 4.41-4.44 (m, 1H); 4.73 (m, 2H); 5.14 (m, 1H); 6.62 (d, J = 2.9 Hz, 1H); 6.67 (dd, J = 8.8, 2.9 Hz, 1H); 6.91 (dd, J = 7.6, 1.4 Hz, 1H); 6.98 (d, J = 8.8 Hz, 1H); 7.18 (dd, J = 8.4, 1.4 Hz, 1H); 7.35 (m, 1H); 7.69 (s, 1H); 8.33 (s, 1H).

Les composés suivants ont été synthétisés selon un mode opératoire analogue, à partir des réactifs appropriés commerciaux ou préalablement préparés:

### Composé 126: 5-(2,3-difluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

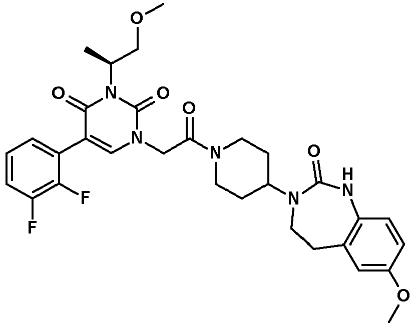

RMN ¹H (δ, DMSO) : 1.34 (d, J = 7.0 Hz, 3H); 1.52-1.70 (m, 4H); 2.68 (t, J = 12.5 Hz, 1H); 2.88 (m, 2H); 3.15 (t, J = 12.4 Hz, 1H); 3.23 (s, 3H); 3.36-3.38 (m, 2H); 3.54 (dd, J = 9.9, 5.9 Hz, 1H); 3.67 (s, 3H); 3.90-3.95 (m, 2H); 4.28 (m, 1H); 4.43 (d, J = 12.9 Hz, 1H); 4.77 (s, 2H); 5.16 (m, 1H); 6.63-6.69 (m, 2H); 6.98 (d, J = 8.8 Hz, 1H); 7.19-7.29 (m, 2H); 7.42-7.49 (m, 1H); 7.88 (s, 1H); 8.33 (s, 1H).

### Composé 127: 5-(2,3-dichloro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

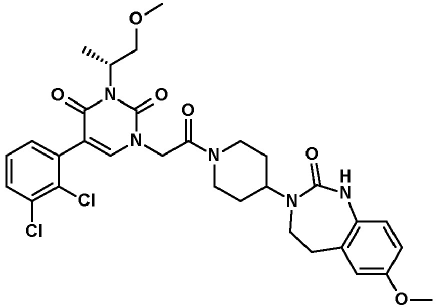

RMN ¹H (δ, DMSO) : 1.33 (d, J = 7.0 Hz, 3H); 1.54 (d, J = 14.1 Hz, 1H); 1.67 (d, J = 21.3 Hz, 3H); 2.68 (t, J = 12.4 Hz, 1H); 2.87 (m, 2H); 3.15 (d, J = 13.4 Hz, 1H); 3.23 (s, 3H); 3.36 (d, J = 6.2 Hz, 2H); 3.54 (dd, J = 9.9, 5.9 Hz, 1H); 3.67 (s, 3H); 3.90-3.95 (m, 2H); 4.28 (m, 1H); 4.43 (d, J = 12.8 Hz, 1H); 4.74 (d, J = 4.4 Hz, 2H); 5.14 (m, 1H); 6.62 (d, J = 2.9 Hz, 1H); 6.67 (dd, J = 8.8, 2.9 Hz, 1H); 6.98 (d, J = 8.8 Hz, 1H); 7.33 (dd, J = 7.6, 1.6 Hz, 1H); 7.42 (t, J = 7.9 Hz, 1H); 7.69 (dd, J = 8.0, 1.6 Hz, 1H); 7.79 (s, 1H); 8.34 (s, 1H).

### Composé 128: 5-(2,3-difluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

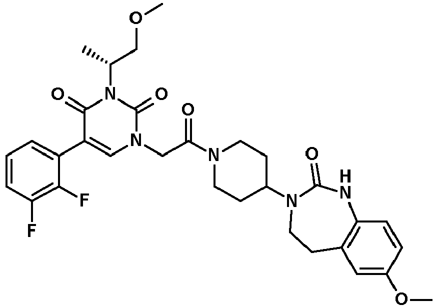

RMN ¹H (δ, DMSO) : 1.34 (d, J = 6.9 Hz, 3H); 1.53-1.56 (m, 1H); 1.65-1.71 (m, 3H); 2.68 (t, J = 12.5 Hz, 1H); 2.88 (d, J = 6.5 Hz, 2H); 3.14 (t, J = 10.2 Hz, 1H); 3.23 (s, 3H); 3.37 (m, 2H); 3.54 (dd, J = 9.9, 5.9 Hz, 1H); 3.67 (s, 3H); 3.94 (d, J = 11.6 Hz, 2H); 4.28 (m, 1H); 4.43 (d, J = 12.8 Hz, 1H); 4.77 (s, 2H); 5.17 (m, 1H); 6.63-6.68 (m, 2H); 6.98 (d, J = 8.8 Hz, 1H); 7.19-7.29 (m, 2H); 7.46 (q, J = 8.9 Hz, 1H); 7.89 (s, 1H); 8.33 (s, 1H).

### Composé 130: 5-(2-chloro-3-méthoxy-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

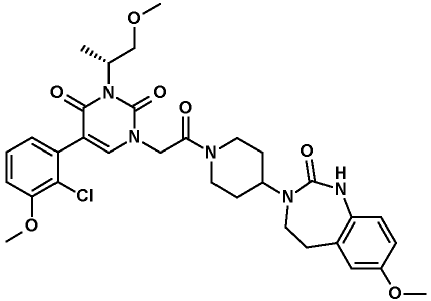

RMN ¹H (δ, DMSO) : 1.33 (d, J = 6.9 Hz, 3H); 1.55 (m, 1H); 1.70 (m, 3H); 2.67 (t, J = 11.9 Hz, 1H); 2.88 (d, J = 6.9 Hz, 2H); 3.13 (m, 1H); 3.23 (s, 3H); 3.36 (d, J = 6.6 Hz, 2H); 3.54 (dd, J = 9.9, 5.9 Hz, 1H); 3.67 (s, 3H); 3.89 (s, 3H); 3.93 (m, 2H); 4.28 (m, 1H); 4.43 (d, J = 12.8 Hz, 1H); 4.73 (m, 2H); 5.14 (m, 1H); 6.62-6.68 (m, 2H); 6.91 (dd, J = 7.6, 1.4 Hz, 1H); 6.98 (d, J = 8.8 Hz, 1H); 7.18 (dd, J = 8.4, 1.4 Hz, 1H); 7.35 (t, J = 8.0 Hz, 1H); 7.69 (s, 1H); 8.33 (s, 1H).

### Composé 131: 5-(2-chloro-3-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

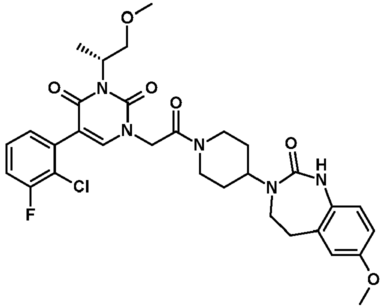

RMN ¹H (δ, DMSO) : 1.34 (d, J = 6.9 Hz, 3H); 1.53-1.56 (m, 1H); 1.65-1.70 (m, 3H); 2.68 (t, J = 12.5 Hz, 1H); 2.88 (m, 2H); 3.14 (m, 1H); 3.24 (s, 3H); 3.36 (d, J = 6.5 Hz, 2H); 3.54 (dd, J = 9.9, 5.9 Hz, 1H); 3.67 (s, 3H); 3.92 (t, J = 10.3 Hz, 2H); 4.28 (m, 1H); 4.43 (d, J = 12.8 Hz, 1H); 4.75 (s, 2H); 5.15 (m, 1H); 6.62-6.68 (m, 2H); 6.98 (d, J = 8.8 Hz, 1H); 7.20-7.22 (m, 1H); 7.44-7.47 (m, 2H); 7.80 (s, 1H); 8.33 (s, 1H).

### Composé 132: 5-(2,3-dichloro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

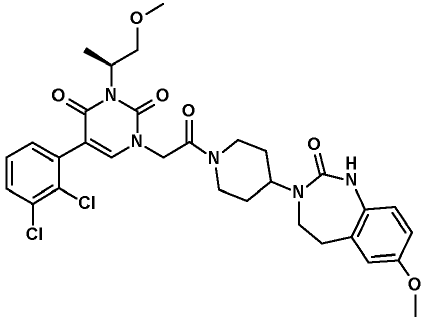

RMN ¹H (δ, DMSO) : 1.32 (d, J = 6.9 Hz, 3H); 1.49-1.58 (m, 1H); 1.63-1.74 (m, 3H); 2.64-2.70 (m, 1H); 2.85-2.87 (m, 2H); 3.10-3.16 (m, 1H); 3.22 (s, 3H); 3.34-3.36 (m, 2H); 3.53 (dd, J = 9.9, 5.9 Hz, 1H); 3.66 (s, 3H); 3.89-3.95 (m, 2H); 4.22-4.29 (m, 1H); 4.42 (d, J = 12.8 Hz, 1H); 4.72-4.73 (m, 2H); 5.08-5.15 (m, 1H); 6.61 (d, J = 2.9 Hz, 1H); 6.66 (dd, J = 8.8, 2.9 Hz, 1H); 6.97 (d, J = 8.8 Hz, 1H); 7.32 (dd, J = 7.7, 1.6 Hz, 1H); 7.41 (t, J = 7.9 Hz, 1H); 7.68 (dd, J = 8.0, 1.6 Hz, 1H); 7.77 (s, 1H); 8.32 (s, 1H).

### Composé 134: 5-(3-bromo-2-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

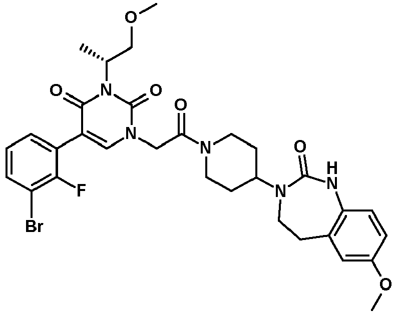

RMN ¹H (δ, DMSO) : 1.21-1.46 (m, 3H), 1.48-1.81 (m, 4H), 2.63-2.74 (m, 1H), 2.83-2.95 (m, 2H), 3.14 (t, J = 11.8 Hz, 1H), 3.23 (s, 3H), 3.34-3.47 (m, 2H), 3.54 (dd, J = 9.9, 5.9 Hz, 1H), 3.67 (s, 3H), 3.85-3.99 (m, 2H), 4.20-4.34 (m, 1H), 4.43 (d, J = 12.9, 1H), 4.76 (s, 2H), 5.07-5.27 (m, 1H), 6.53-6.77 (m, 2H), 6.98 (d, J = 8.8 Hz, 1H), 7.22 (td, J = 7.8, 0.8 Hz, 1H), 7.40 (ddd, J=8.2, 6.7, 1.7 Hz, 1H), 7.72 (ddd, J=8.2, 6.7, 1.7 Hz, 1H), 7.87 (s, 1H), 8.32 (s, 1H).

### Exemple 135 : 5-(2-chloro-3-fluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 135)

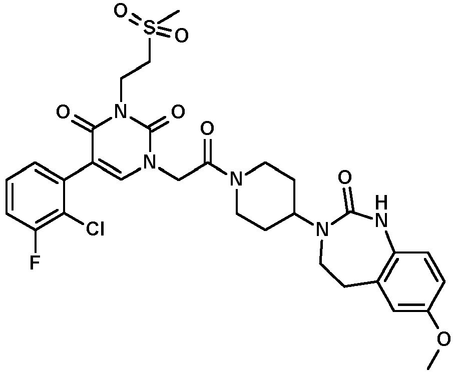

### 135.1 : Acide [5-(2-chloro-3-fluoro-phényl)-3-(2-méthylsulfanyl-éthyl)-2,4-dioxo-3,4-dihydro-2H-pyrimidin-1-yl]-acétique

De manière analogue à l'exemple 115.8, à partir de 300mg (0,9 mmoles) de [5-bromo-3-(2-méthylsulfanyl-éthyl)-2,4-dioxo-3,4-dihydro-2H-pyrimidin-1-yl]-acétate de méthyle (préparé comme dans l'exemple 112.1) et 171mg (1,0 mmoles) d'acide 2-chloro-3-fluorophénylboronique, 320mg (96%) d' acide [5-(2-chloro-3-fluoro-phényl)-3-(2-méthylsulfanyl-éthyl)-2,4-dioxo-3,4-dihydro-2H-pyrimidin-1-yl]-acétique sont obtenus sous la forme d'un solide marron.

### 135.2 : 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione

De manière analogue à l'exemple 115.10, à partir de 284mg (1,0 mmoles) de 7-méthoxy-3-pipéridin-4-yl-1,3,4,5-tétrahydro-benzo[d][1,3]diazépin-2-one (préparé comme dans l'exemple 115.6) et 320mg (0,9 mmoles) d' acide [5-(2-chloro-3-fluoro-phényl)-3-(2-méthylsulfanyl-éthyl)-2,4-dioxo-3,4-dihydro-2H-pyrimidin-1-yl]-acétique, et après purification par chromatographie sur gel de silice élué par un mélange dichlorométhane/méthanol 97/3, 325mg (60%) de 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione sont obtenus sous la forme d'un solide blanc.

### 135.3: 5-(2-chloro-3-fluoro-phényl)-3-(2-méthanesulfonyl-éthyl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (composé 135)

Un mélange contenant 71mg (0,06 mmoles) de molybdate d'ammonium tétrahydraté dans 311mg (9,2 mmoles) de péroxyde d'hydrogène est additionné sur une solution contenant 240mg (0,4 mmoles) de 5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-3-(2-méthylsulfanyl-éthyl)-1H-pyrimidine-2,4-dione dans 4,8ml d'éthanol. Après agitation à température ambiante pendant 46 heures, 5ml de dichlorométhane sont additionnés et le milieu réactionnel est agité à température ambiante pendant 48 heures de plus. Il est ensuite hydrolysé par une solution aqueuse saturée d'hydrogénocarbonate de sodium et le produit est extrait au dichlorométhane. La phase organique est lavée deux fois par une solution aqueuse à 10% de thiosulfate de sodium et une fois par de l'eau, séchée sur sulfate de magnésium, filtrée et concentrée sous vide. Le produit brut est chromatographié sur gel de silice élué par un mélange dichlorométhane /méthanol 97/3, le solide blanc obtenu est repris dans 2ml d'acétate d'éthyle et lentement agité à température ambiante pendant 16 heures.La suspension obtenue est filtrée et rincée une fois avec 2ml d'ether diéthylique, puis séchée sous vide. 150mg (59%) de 5-(2-chloro-3-fluoro-phényl)-3-(2-méthanesulfonyl-éthyl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione sont obtenus sous la forme d'un solide blanc de point de fusion 212°C.

RMN ¹H (δ, DMSO): 1.45-1.61 (m, 1H), 1.70 (m, 3H), 2.68 (m, 1H), 2.80-2.93 (m, 2H), 3.08 (s, 3H), 3.14 (m, 1H), 3.36 (m, 2H), 3.41 (t, J = 7.3 Hz, 2H), 3.67 (s, 3H), 3.95 (d, J = 13.8 Hz, 1H), 4.31 (m, 3H), 4.42 (d, J = 12.9 Hz, 1H), 4.80 (m, 2H), 6.62 (d, J = 3.0 Hz, 1H), 6.67 (dd, J = 8.7, 3.0 Hz, 1H), 6.98 (d, J = 8.8 Hz, 1H), 7.15-7.28 (m, 1H), 7.37-7.54 (m, 2H), 7.88 (s, 1H), 8.35 (s, 1H).

Les composés suivants ont été synthétisés selon un mode opératoire analogue, en utilisant les réactifs appropriés commerciaux ou préalablement préparés:

### composé 136: 5-(2,3-difluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

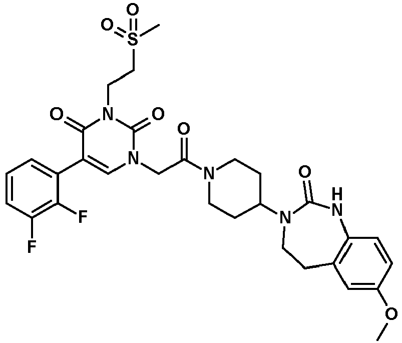

RMN ¹H (δ, DMSO) : 1.51-1.70 (m, 4H); 2.68 (t, J = 12.4 Hz, 1H); 2.88 (m, 2H); 3.09 (s, 3H); 3.15 (t, J = 12.5 Hz, 1H); 3.36-3.44 (m, 4H); 3.67 (s, 3H); 3.96 (d, J = 13.5 Hz, 1H); 4.31 (m, 3H); 4.42 (d, J = 12.7 Hz, 1H); 4.81 (s, 2H); 6.63-6.69 (m, 2H); 6.98 (d, J = 8.8 Hz, 1H); 7.22-7.31 (m, 2H); 7.44-7.51 (m, 1H); 7.97 (s, 1H); 8.35 (s, 1H).

### Composé 137: 5-(2,3-dichlorophényl)-1-[2-[4-(7-méthoxy-2-oxo-4,5-dihydro-1H-1,3-benzodiazépin-3-yl)-1-pipéridyl]-2-oxo-éthyl]-3-(2-méthylsulfonyléthyl)pyrimidine-2,4-dione

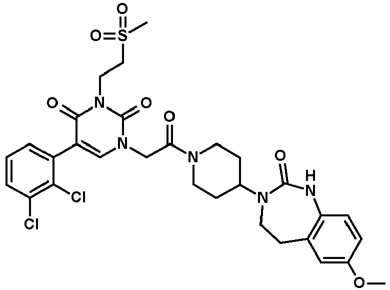

RMN ¹H (δ, DMSO) : 1.52-1.70 (m, 4H); 2.68 (t, J = 12.2 Hz, 1H); 2.88 (m, 2H); 3.08 (s, 3H); 3.15 (m, 1H); 3.36 (m, 2H); 3.41 (t, J = 7.2 Hz, 2H); 3.67 (s, 3H); 3.95 (d, J = 13.4 Hz, 1H); 4.29-4.33 (m, 3H); 4.43 (d, J = 12.7 Hz, 1H); 4.79 (s, 2H); 6.62-6.68 (m, 2H); 6.98 (d, J = 8.8 Hz, 1H); 7.33 (dd, J = 7.7, 1.6 Hz, 1H); 7.44 (t, J = 7.9 Hz, 1H); 7.70 (dd, J = 8.1, 1.6 Hz, 1H); 7.87 (s, 1H); 8.33 (s, 1H).

### Composé 138: 5-(2,3-diméthyl-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

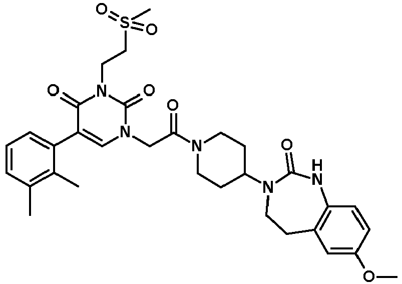

RMN ¹H (δ, DMSO) : 1.56 (m, 1H), 1.70 (m, 3H), 2.07 (s, 3H), 2.28 (s, 3H), 2.71 (m, 1H), 2.87 (m, 2H), 3.08 (s, 3H), 3.14 (m, 1H), 3.34 - 3.38 (m, 2H), 3.41 (t, J = 7.1 Hz, 2H), 3.67 (s, 3H), 3.95 (d, J = 13.7 Hz, 1H), 4.29 (m, 3H), 4.43 (d, J = 13.0 Hz, 1H), 4.77 (m, 2H), 6.62 (d, J = 2.9 Hz, 1H), 6.67 (dd, J = 8.7, 2.9 Hz, 1H), 6.97 (m, 2H), 7.11 (t, J = 7.5 Hz, 1H), 7.19 (d, J = 7.4 Hz, 1H), 7.63 (s, 1H), 8.32 (s, 1H).

### Composé 139: 5-(2,3-diméthoxy-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

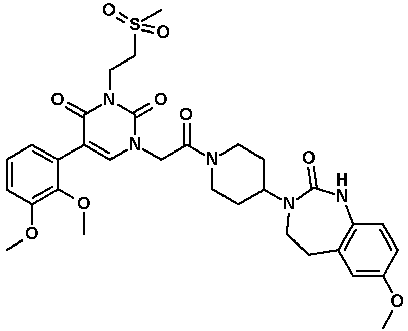

RMN ¹H (δ, DMSO) : 1.50-1.80 (m, 4H); 2.68 (m, 1H); 2.88 (m, 2H); 3.08 (s, 3H); 3.14 (t, J = 13.0 Hz, 1H); 3.36 (m, 2H); 3.41 (t, J = 7.2 Hz, 2H); 3.68 (d, J = 3.5 Hz, 6H); 3.84 (s, 3H); 3.95 (d, J = 13.4 Hz, 1H); 4.29-4.33 (m, 3H); 4.43 (d, J = 12.7 Hz, 1H); 4.78 (s, 2H); 6.62 (d, J = 2.9 Hz, 1H); 6.67 (dd, J = 8.8, 2.9 Hz, 1H); 6.81 (dd, J = 5.3, 3.9 Hz, 1H); 6.98 (d, J = 8.8 Hz, 1H); 7.08-7.09 (m, 2H); 7.67 (s, 1H); 8.32 (s, 1H).

### Composé 140: 5-(2-chloro-3-méthoxy-phényl)-3-(2-methanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

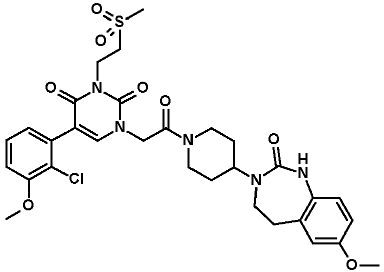

RMN ¹H (δ, DMSO) : 1.55 (1 H, m), 1.68 (3 H, m), 2.68 (1 H, t, J = 12.29 Hz), 2.88 (2 H, m), 3.08 (3 H, s), 3.15 (1 H, t, J = 12Hz), 3.42-3.33 (4 H, m), 3.67 (3 H, s), 3.90 (3 H, s), 3.95 (1 H, d, J = 14.37 Hz), 4.30 (3 H, t, J = 7.38 Hz), 4.43 (1 H, d, J = 12.88 Hz), 4.78 (2 H, s), 6. 68-6. 62 (2 H, m), 6.93-6.91 (1 H, m), 6.98 (1 H, d, J = 8.78 Hz), 7.20 (1 H, d, J = 8.34 Hz), 7.36 (1 H, t, J = 7.98 Hz), 7.77 (1 H, s), 8.33 (1 H, s).

### Exemple 142: 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 142)

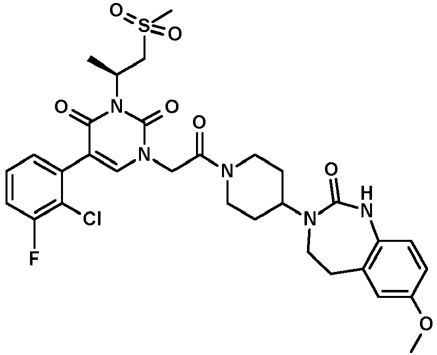

### 142.1: (R)-1-méthylsulfanyl-propan-2-ol

4,8ml (69 mmoles) de (R)-(+)-propylène-1,2 oxyde sont rajoutés goutte à goutte sur une solution contenant 7,2g (103,3 mmoles) de méthanethiolate de sodium dans 32ml d'acétonitrile. Le mélange est agité en tube scellé pendant 4 heures à 80°C puis dilué par de l'eau et par du dichlorométhane. La phase aqueuse est extraite trois fois au dichlorométhane, la phase organique est lavée par l'eau puis à la saumure. Après séchage sur sulfate de magnésium, la solution est filtrée puis concentrée sous vide. 3,5g (48%) de (R)-1-méthylsulfanyl-propan-2-ol sont obtenus sous la forme d'une huile ambrée.

### 142.2: [5-bromo-3-((S)-1-méthyl-2-méthylsulfanyl-éthyl)-2,4-dioxo-3,4-dihydro-2H-pyrimidin-1-yl]-acétate de méthyle

De manière analogue à l'exemple 121.1, à partir de 3,7g (14,1 mmoles) de (5-bromo-2,4-dioxo-3,4-dihydro-2H-pyrimidin-1-yl)-acétate de méthyle (préparé comme dans l'exemple 13.1) et 1,5g (14,1 mmoles) de (R)-1-méthylsulfanyl-propan-2-ol, 1,6g (32%) de [5-bromo-3-((S)-1-méthyl-2-méthylsulfanyl-éthyl)-2,4-dioxo-3,4-dihydro-2H-pyrimidin-1-yl]-acétate de méthyle sont obtenus sous la forme d'une huile visqueuse.

### 142.3: [5-(2-chloro-3-fluoro-phényl)-3-((S)-1-méthyl-2-méthylsulfanyl-éthyl)-2,4-dioxo-3,4-dihydro-2H-pyrimidin-1-yl]-acétate de méthyle

De manière analogue à l'exemple 115.8, à partir de 1,6g (4,6 mmoles) de [5-bromo-3-((S)-1-méthyl-2-méthylsulfanyl-éthyl)-2,4-dioxo-3,4-dihydro-2H-pyrimidin-1-yl]-acétate de méthyle et 1,6g (9,1 mmoles) d'acide 2-chloro-3-fluorophénylboronique, 1,7g (93%) de [5-(2-chloro-3-fluoro-phényl)-3-((S)-1-méthyl-2-méthylsulfanyl-éthyl)-2,4-dioxo-3,4-dihydro-2H-pyrimidin-1-yl]-acétate de méthyle sont obtenus sous la forme d'un solide marron.

### 142.4: [5-(2-chloro-3-fluoro-phényl)-3-((S)-2-méthanesulfonyl-1-méthyl-éthyl)-2,4-dioxo-3,4-dihydro-2H-pyrimidin-1-yl]-acétate de méthyle

1,7g (4,2 mmoles) de 5-(2-chloro-3-fluoro-phényl)-3-((S)-1-méthyl-2-méthylsulfanyl-éthyl)-2,4-dioxo-3,4-dihydro-2H-pyrimidin-1-yl]-acétate de méthyle sont additionnés sur un mélange contenant 262mg (0,2 mmoles) de molybdate d'ammonium tétrahydraté dans 4,3ml (42,4 mmoles) de solution aqueuse de péroxyde d'hydrogène à 30% à 0°C. Après 2 heures d'agitation à température ambiante, le mélange est hydrolysé avec 30ml d'eau puis extrait par de l'acétate d'éthyle. La phase organique est lavée par une solution aqueuse de sulfate de sodium, par de l'eau puis brine, séchée sur sulfate de magnésium anhydre et concentrée sous vide. 1,8g (96%) de [5-(2-chloro-3-fluoro-phényl)-3-((S)-2-méthanesulfonyl-1-méthyl-éthyl)-2,4-dioxo-3,4-dihydro-2H-pyrimidin-1-yl]-acétate de méthyle sont obtenus sous la forme d'un solide.

### 142.5: Acide 5-(2-chloro-3-fluoro-phényl)-3-((S)-2-méthanesulfonyl-1-méthyl-éthyl)-2,4-dioxo-3,4-dihydro-2H-pyrimidin-1-yl]-acétique

1,8g (4,2 mmoles) de [5-(2-chloro-3-fluoro-phényl)-3-((R)-2-méthanesulfonyl-1-méthyl-éthyl)-2,4-dioxo-3,4-dihydro-2H-pyrimidin-1-yl]-acétate de méthyle sont ajoutés à 20ml de tétrahydrofuranne et 2,8ml (5,5 mmoles) de solution aqueuse d'hydroxyde de sodium 1N. Après 2 heures, le mélange est dilué par de l'acétate d'éthyle et par de l'eau, la phase aqueuse est portée à pH 3 avec une solution aqueuse d'acide chlorhydrique 1N. Cette phase est ensuite extraite trois fois par de l'acétate d'éthyle, la phase organique est lavée à l'eau puis brine. Après séchage sur sulfate de magnésium anhydre, la solution est filtrée puis concentrée sous vide. 1,4g (80%) d'acide 5-(2-chloro-3-fluoro-phényl)-3-((S)-2-méthanesulfonyl-1-méthyl-éthyl)-2,4-dioxo-3,4-dihydro-2H-pyrimidin-1-yl]-acétique sont obtenus sous la forme d'une huile.

### 142.6: 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (composé 142)

De manière analogue à l'exemple 115.10, à partir de 1,4g (3,3 mmoles) d'acide [5-(2-chloro-3-fluoro-phényl)-3-((S)-2-méthanesulfonyl-1-méthyl-éthyl)-2,4-dioxo-3,4-dihydro-2H-pyrimidin-1-yl]-acétique et 1g (3,7 mmoles) de 7-méthoxy-3-pipéridin-4-yl-1,3,4,5-tétrahydro-benzo[d] [1,3]diazépin-2-one (préparé comme dans l'exemple 115.6), 720mg (31%) de 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide blanc.

RMN ¹H (δ, DMSO): 1.23 (d, J = 6.9 Hz, 3H), 1.44 - 1.83 (m, 4H), 2.67 (dd, J = 3.5, 1.8 Hz, 1H), 2.87 (d, J = 7.6 Hz, 2H), 3.05 (s, 3H), 3.09-3.21 (m, 1H), 3.32 - 3.40 (m, 2H), 3.52 (br s, 1H), 3.66 (s, 3H), 3.93 (d, J = 13.2 Hz, 1H), 4.12-4.35 (m, 3H), 4.42 (d, J = 13.1 Hz, 1H), 4.79 (s, 2H), 6.54 - 6.73 (m, 2H), 6.97 (d, J = 8.8 Hz, 1H), 7.15-7.26 (m, 1H), 7.41-7.51 (m, 2H), 7.89 (s, 1H), 8.31 (s, 1H).

Les composés suivants ont été synthétisés selon un mode opératoire analogue, en utilisant les réactifs appropriés commerciaux ou préalablement préparés:

### composé 143: 5-(2-chloro-3-fluoro-phényl)-3-((R)-2-méthanesulfonyl-1-méthyl-ethyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

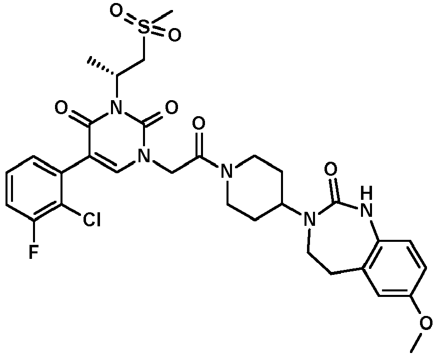

RMN ¹H (δ, DMSO) : 1.24 (d, J = 7.0 Hz, 3 H); 1.55 (m, 1 H); 1.80-1.60 (m, 3 H); 2.68 (t, J = 12.5 Hz, 1 H); 2.88 (m, 2 H); 3.06 (s, 3 H); 3.15 (t, J = 12.5 Hz, 1 H); 3.40-3.32 (m, 2 H); 3.53 (m, 1 H); 3.67 (s, 3 H); 3.94 (d, J = 13.5 Hz, 1 H); 4.28-4.22 (m, 3 H); 4.43 (d, J = 12.8 Hz, 1 H); 4.80 (s, 2 H); 6.68-6.62 (m, 2 H); 6.98 (d, J = 8.8 Hz, 1 H); 7.23 (dd, J = 6.3, 2.8 Hz, 1 H); 7.48-7.45 (m, 2 H); 7.90 (s, 1 H); 8.32 (s, 1 H).

### Composé 144: 5-(2,3-dichloro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

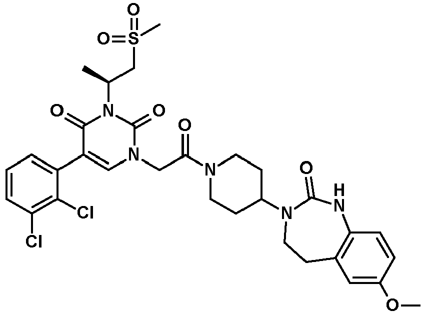

RMN ¹H (δ, DMSO) : 1.24 (d, J = 7.02 Hz, 3H), 1.42 - 1.83 (m, 4H), 2.62 - 2.77 (m, 1H), 2.83 - 2.96 (m, 2H), 3.06 (s, 3H), 3.10 - 3.23 (m, 1H), 3.33 - 3.39 (m, 2H), 3.52 (dt, J = 7.17, 11.03 Hz, 1H), 3.67 (s, 3H), 3.94 (d, J = 13.53 Hz, 1H), 4.13 - 4.38 (m, 3H), 4.43 (d, J = 12.91 Hz, 1H), 4.79 (s, 2H), 6.62 (d, J = 2.91 Hz, 1H), 6.67 (dd, J = 2.97, 8.78 Hz, 1H), 6.98 (d, J = 8.78 Hz, 1H), 7.35 (dd, J = 1.59, 7.68 Hz, 1H), 7.44 (t, J = 7.85 Hz, 1H), 7.71 (dd, J = 1.61, 8.03 Hz, 1H), 7.89 (s, 1H), 8.33 (s, 1H).

### Composé 145: 5-(2,3-dichloro-phényl)-3-((R)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

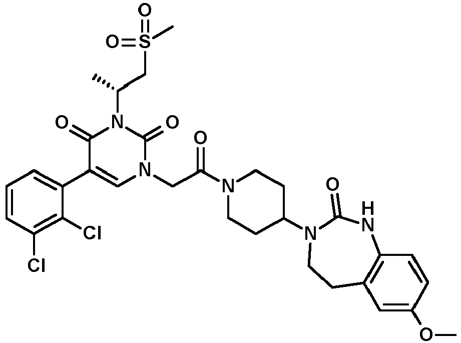

RMN ¹H (δ, DMSO) : 1.23 (d, J = 7.02 Hz, 3H), 1.40 - 1.81 (m, 4H), 2.61 - 2.75 (m, 1H), 2.77 - 2.96 (m, 2H), 3.05 (s, 3H), 3.09 - 3.28 (m, 1H), 3.32 - 3.41 (m, 2H), 3.45 - 3.61 (m, 1H), 3.67 (s, 3H), 3.94 (d, J = 13.4 Hz, 1H), 4.12 - 4.35 (m, 3H), 4.42 (d, J = 12.9 Hz, 1H), 4.79 (br s, 2H), 6.42 - 6.78 (m, 2H), 6.97 (d, J = 8.8 Hz, 1H), 7.34 (dd, J = 1.6, 7.6 Hz, 1H), 7.44 (t, J = 7.9 Hz, 1H), 7.70 (dd, J = 1.57, 8.1 Hz, 1H), 7.88 (s, 1H), 8.32 (s, 1H).

### Exemple 146: 5-(2-chloro-3-fluoro-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 146)

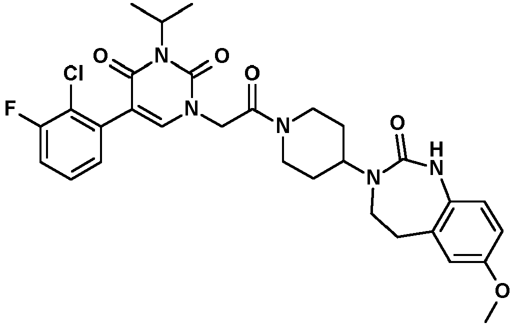

### 146.1: (5-bromo-3-isopropyl-2,4-dioxo-3,4-dihydro-2H-pyrimidin-1-yl)-acétate de méthyle

1,2g (29,7 mmoles) d'hydrure de sodium sont ajoutés par portion à une solution préalablement refroidie à 0°C de 6,5g (24,7 mmoles) de (5-bromo-2,4-dioxo-3,4-dihydro-2H-pyrimidin-1-yl)-acétate de méthyle ( préparé comme dans l'exemple 13.1) dans 130ml de N,N-diméthylformamide. Le milieu réactionnel est agité pendant 5 minutes puis 6,3ml (66,7 mmoles) de 2-bromopropane sont additionnés. Le milieu réactionnel est chauffé à 50°C pendant 6 heures puis agité à température ambiante toute une nuit. Le mélange est hydrolysé puis dilué par de l'acétate d'éthyle. Le produit est extrait à l'acétate d'éthyle, la phase organique est lavée une fois par de l'eau et une fois par une solution aqueuse saturée de chlorure de sodium, puis séchée sur sulfate de magnésium, filtrée et concentrée sous vide. Après purification par chromatographie sur gel de silice élué par un mélange heptane/acétate d'éthyle 50/50, 3,5g (46%) de (5-bromo-3-isopropyl-2,4-dioxo-3,4-dihydro-2H-pyrimidin-1-yl)-acétate de méthyle sont obtenus sous la forme d'un solide blanc cassé.

### 146.2: [5-(2-chloro-3-fluoro-phényl)-3-isopropyl-2,4-dioxo-3,4-dihydro-2H-pyrimidin-1-yl]-acétate de méthyle

De manière analogue à l'exemple 115.8, à partir de 3,4g (19,7 mmoles) d'acide 2-chloro-3-fluorophénylboronique et 3,0g (9,8 mmoles) de 5-bromo-3-isopropyl-2,4-dioxo-3,4-dihydro-2H-pyrimidin-1-yl)-acétate de méthyle, 1,4g (34%) de [5-(2-chloro-3-fluoro-phényl)-3-isopropyl-2,4-dioxo-3,4-dihydro-2H-pyrimidin-1-yl]-acétate de méthyle sont obtenus sous la forme d'un solide beige.

### 146.3: Acide [5-(2-chloro-3-fluoro-phényl)-3-isopropyl-2,4-dioxo-3,4-dihydro-2H-pyrimidin-1-yl]-acétique

De manière analogue à l'exemple 142.5, à partir de 500mg (1,4 mmoles) de [5-(2-chloro-3-fluoro-phényl)-3-isopropyl-2,4-dioxo-3,4-dihydro-2H-pyrimidin-1-yl]-acétate de méthyle, 470mg (98%) d'acide [5-(2-chloro-3-fluoro-phényl)-3-isopropyl-2,4-dioxo-3,4-dihydro-2H-pyrimidin-1-yl]-acétique sont obtenus sous la forme d'une huile.

### 146.4: 5-(2-chloro-3-fluoro-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (composé 146)

De manière analogue à l'exemple 115.10, à partir de 427mg (1,6 mmoles) de 7-méthoxy-3-pipéridin-4-yl-1,3,4,5-tétrahydro-benzo[d] [1,3]diazépin-2-one (préparé comme dans l'exemple 115.6) et 480mg (1,4 mmoles) d'acide [5-(2-chloro-3-fluoro-phényl)-3-isopropyl-2,4-dioxo-3,4-dihydro-2H-pyrimidin-1-yl]-acétique, 684mg (81%) de 5-(2-chloro-3-fluoro-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione sont obtenus sous la forme d'un solide blanc de point de fusion 230°C.

RMN ¹H (δ, DMSO): 1.39 (d, J = 6.9 Hz, 6 H); 1.51-1.69 (m, 4 H); 2.66 (m, 1 H); 2.85 (d, J = 6.7 Hz, 2 H); 3.12 (t, J = 11.9 Hz, 1 H); 3.36-3.33 (m, 2 H); 3.66 (s, 3 H); 3.89-3.93 (m, 1 H); 4.24-4.30 (m, 1 H); 4.41 (d, J = 12.8 Hz, 1 H); 4.72 (d, J = 6.4 Hz, 2 H); 5.05-5.12 (m, 1 H); 6.60 (d, J = 2.9 Hz, 1 H); 6.63-6.66 (m, 1 H); 6.96 (d, J = 8.8 Hz, 1 H); 7.20 (d, J = 2.6 Hz, 1 H); 7.41-7.45 (m, 2 H); 7.76 (s, 1 H); 8.33 (s, 1 H).

Les composés suivants ont été synthétisés selon un mode opératoire analogue, en utilisant les réactifs appropriés commerciaux ou préalablement préparés:

### Composé 148: 5-(2-chloro-3-méthoxy-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

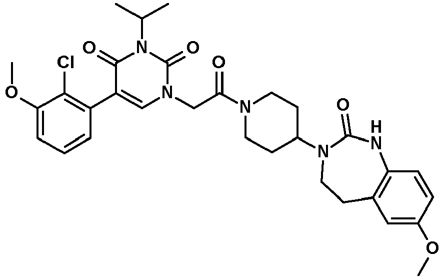

RMN ¹H (δ, DMSO) : 1.38 (6 H, d, J = 6.89 Hz); 1.55-1.68 (4 H, m); 2.69-2.63 (1 H, m); 2.85 (2 H, s); 3.12 (1 H, br t, J = 12.3 Hz); 3.35-3.33 (2 H, m); 3.65 (3 H, s); 3.87 (3 H, s); 3.92 (1 H, m); 4.30-4.22 (1 H, m); 4.41 (1 H, d, J = 12.9 Hz); 4.76-4.66 (2 H, m); 5.11-5.04 (1 H, m); 6.66-6.60 (2 H, m); 6.89 (1 H, dd, J = 7.6,1.4 Hz); 6.96 (1 H, d, J = 8.8 Hz); 7.16 (1 H, dd, J = 8.4, 1.4 Hz); 7.32 (1 H, t, J = 8.0 Hz); 7.65 (1 H, s);8.30 (1 H, s).

### Composé 152: 3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-5-(3-méthoxy-2-trifluorométhoxy-phényl)-1H-pyrimidine-2,4-dione

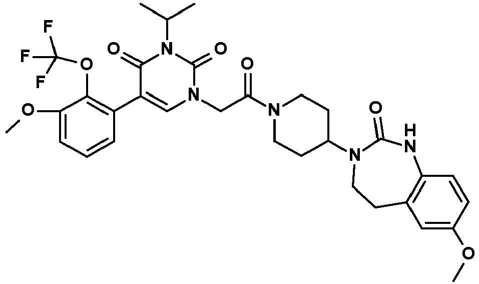

RMN ¹H (δ, DMSO) : 1.37 (d, J = 6.9 Hz, 6 H); 1.49-1.72 (m, 4 H); 2.62-2.68 (m, 1 H); 2.85-2.87 (m, 2 H); 3.06-3.14 (m, 1 H); 3.33-3.35 (m, 2 H); 3.65 (s, 3 H); 3.86 (s, 3 H); 3.92 (d, J = 13.3 Hz, 1 H); 4.24 (d, J = 11.7 Hz, 1 H); 4.41 (d, J = 13.0 Hz, 1 H); 4.71 (d, J = 3.3 Hz, 2 H); 5.04-5.11 (m, 1 H); 6.60-6.66 (m, 2 H); 6.89-6.96 (m, 2 H); 7.24 (dd, J = 8.4, 1.5 Hz, 1 H); 7.37 (t, J = 8.0 Hz, 1 H); 7.71 (s, 1 H); 8.31 (s, 1 H).

### Exemple 155: 5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-3-(2-méthylsulfanyl-éthyl)-1H-pyrimidine-2,4-dione (schéma réactionnel n°3, composé 155)

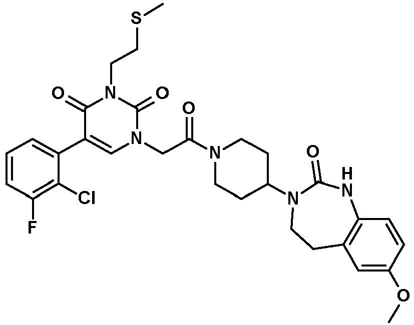

De manière analogue à l'exemple 115.10, à partir de 284mg (1 mmole) de 7-méthoxy-3-pipéridin-4-yl-1,3,4,5-tétrahydro-benzo[d][1,3]diazépin-2-one (préparé comme dans l'exemple 115.6) et 320mg (0,9 mmoles) d'acide [5-(2-chloro-3-fluoro-phényl)-3-(2-méthylsulfanyl-éthyl)-2,4-dioxo-3,4-dihydro-2H-pyrimidin-1-yl]-acétique (préparé comme dans l'exemple 125.1), 325mg (60%) de 5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-3-(2-méthylsulfanyl-éthyl)-1H-pyrimidine-2,4-dione sont obtenus sous la forme d'un solide blanc de point de fusion 230°C.

RMN ¹H (δ, DMSO) : 1.50-1.80 (m, 4H); 2.12 (s, 3H); 2.65-2.71 (m, 3H); 2.88 (m, 2H); 3.15 (t, J = 12.1 Hz, 1H); 3.35-3.37 (m, 2H); 3.67 (s, 3H); 3.94 (d, J = 13.6 Hz, 1H); 4.08 (t, J = 7.2 Hz, 2H); 4.22-4.32 (m, 1H); 4.43 (d, J = 12.8 Hz, 1H); 4.79 (s, 2H); 6.62 (d, J = 2.9 Hz, 1H); 6.67 (dd, J = 8.8, 2.9 Hz, 1H); 6.98 (d, J = 8.8 Hz, 1H); 7.21-7.23 (m, 1H); 7.40-7.50 (m, 2H); 7.85 (s, 1H); 8.33 (s, 1H).

### Exemple 156: 5-(2-chloro-3-fluoro-phényl)-3-(3-méthanesulfonyl-propyl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (schéma réactionnel n°3, composé 156)

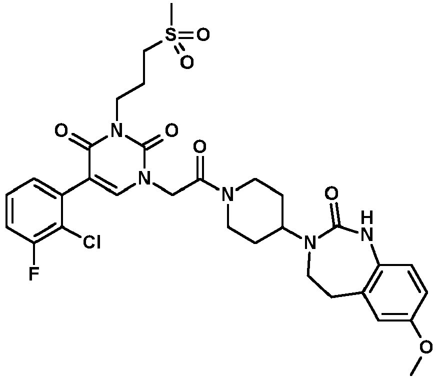

### 156.1: [5-bromo-3-(3-méthylsulfanyl-propyl)-2,4-dioxo-3,4-dihydro-2H-pyrimidin-1-yl]-acétate de méthyle

De manière analogue à l'exemple 121.1, à partir de 3g (11,4 mmoles) de (5-bromo-2,4-dioxo-3,4-dihydro-2H-pyrimidin-1-yl)-acétate de méthyle (préparé comme dans l'exemple 13.1) et 1,4ml (13,7 mmoles) de 3-méthylthiopropanol, 3,5g (87%) de [5-bromo-3-(3-méthylsulfanyl-propyl)-2,4-dioxo-3,4-dihydro-2H-pyrimidin-1-yl]-acétate de méthyle sont obtenus sous la forme d'un solide blanc.

### 156.2: [5-(2-chloro-3-fluoro-phényl)-3-(3-méthylsulfanyl-propyl)-2,4-dioxo-3,4-dihydro-2H-pyrimidin-1-yl]-acétate de méthyle

De manière analogue à l'exemple 115.8, à partir de 695mg (4 mmoles) d'acide 2-chloro-3-fluorophénylboronique et 1g (2,9 mmoles) de [5-bromo-3-(3-méthylsulfanyl-propyl)-2,4-dioxo-3,4-dihydro-2H-pyrimidin-1-yl]-acétate de méthyle, 750mg (66%) de [5-(2-chloro-3-fluoro-phényl)-3-(3-méthylsulfanyl-propyl)-2,4-dioxo-3,4-dihydro-2H-pyrimidin-1-yl]-acétate de méthyle sont obtenus sous la forme d'une huile incolore.

### 156.3: [5-(2-chloro-3-fluoro-phényl)-3-(3-méthanesulfonyl-propyl)-2,4-dioxo-3,4-dihydro-2H-pyrimidin-1-yl]-acétate de méthyle

De manière analogue à l'exemple 125.3, à partir de 750mg (1,9 mmoles) de [5-(2-chloro-3-fluoro-phényl)-3-(3-méthylsulfanyl-propyl)-2,4-dioxo-3,4-dihydro-2H-pyrimidin-1-yl]-acétate de méthyle, 810mg (100%) de [5-(2-chloro-3-fluoro-phényl)-3-(3-méthanesulfonyl-propyl)-2,4-dioxo-3,4-dihydro-2H-pyrimidin-1-yl]- acétate de méthyle sont obtenus sous la forme d'une meringue beige.

### 156.4: Acide [5-(2-chloro-3-fluoro-phényl)-3-(3-méthanesulfonyl-propyl)-2,4-dioxo-3,4-dihydro-2H-pyrimidin-1-yl]-acétique

De manière analogue à l'exemple 115.9, à partir de 810mg (1,9 mmoles) de [5-(2-chloro-3-fluoro-phényl)-3-(3-méthanesulfonyl-propyl)-2,4-dioxo-3,4-dihydro-2H-pyrimidin-1-yl]- acétate de méthyle , 750mg (96%) d'acide [5-(2-chloro-3-fluoro-phényl)-3-(3-méthanesulfonyl-propyl)-2,4-dioxo-3,4-dihydro-2H-pyrimidin-1-yl]-acétique sont obtenus sous la forme d'une meringue beige.

### 156.5: 5-(2-chloro-3-fluoro-phényl)-3-(3-méthanesulfonyl-propyl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (composé 156)

De manière analogue à l'exemple 115.10, à partir de 226mg (0,9 mmoles) de 7-méthoxy-3-pipéridin-4-yl-1,3,4,5-tétrahydro-benzo[d] [1,3]diazépin-2-one (préparé comme dans l'exemple 115.6) et 343mg (0,8 mmoles) d'acide [5-(2-chloro-3-fluoro-phényl)-3-(3-méthanesulfonyl-propyl)-2,4-dioxo-3,4-dihydro-2H-pyrimidin-1-yl]-acétique, 400mg (72%) de 5-(2-chloro-3-fluoro-phényl)-3-(3-méthanesulfonyl-propyl)-1-{2-[4-(7-méthoxy-2-oxo-1,2,4,5-tétrahydro-benzo[d][1,3]diazépin-3-yl)-piperidin-1-yl]-2-oxo-ethyl}-1H-pyrimidine-2,4-dione sont obtenus sous la forme d'un solide blanc de point de fusion 189°C.

RMN ¹H (δ, DMSO): 1.53-1.70 (m, 4H); 1.96-2.04 (m, 2H); 2.68 (t, J = 12.4 Hz, 1H); 2.88 (m, 2H); 2.98 (s, 3H); 3.15-3.19 (m, 3H); 3.35-3.37 (m, 2H); 3.67 (s, 3H); 3.94 (d, J = 13.7 Hz, 1H); 4.01 (t, J = 7.1 Hz, 2H); 4.28 (m, 1H); 4.43 (d, J = 12.9 Hz, 1H); 4.79 (s, 2H); 6.63 (d, J = 2.9 Hz, 1H); 6.67 (dd, J = 8.8, 3.0 Hz, 1H); 6.98 (d, J = 8.8 Hz, 1H); 7.23-7.25 (m, 1H); 7.44-7.47 (m, 2H); 7.86 (s, 1H); 8.33 (s, 1H).

### Exemple 157: 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione (schéma réactionnel n°3, composé 157)

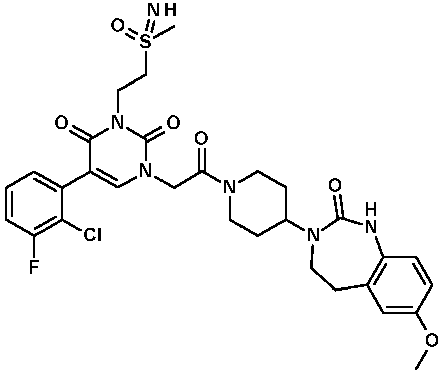

### 157.1: [5-(2-chloro-3-fluoro-phényl)-3-(2-méthylsulfanyl-éthyl)-2,4-dioxo-3,4-dihydro-2H-pyrimidin-1-yl]-acétate de méthyle

De manière analogue à l'exemple 115.8, à partir de 1,9g (11,1 mmoles) d'acide 2-chloro-3-fluorophénylboronique et 2,5g (7,4 mmoles) de [5-bromo-3-(2-méthylsulfanyl-éthyl)-2,4-dioxo-3,4-dihydro-2H-pyrimidin-1-yl]-acétate de méthyle (préparé comme dans l'exemple 112.1), 2,2g (77%) de [5-(2-chloro-3-fluoro-phényl)-3-(2-méthylsulfanyl-éthyl)-2,4-dioxo-3,4-dihydro-2H-pyrimidin-1-yl]- acétate de méthyle sont obtenus sous la forme d'un solide blanc-cassé.

### 157.2: [5-(2-chloro-3-fluoro-phényl)-3-(2-méthanesulfinyl-éthyl)-2,4-dioxo-3,4-dihydro-2H-pyrimidin-1-yl]-acétate de méthyle

0,9g (4,1 mmoles) d'acide 3-chloropéroxybenzoïque sont additionnés à une solution refroidie à 0°C contenant 1,5g (3,9 mmoles) de [5-(2-chloro-3-fluoro-phényl)-3-(2-méthylsulfanyl-éthyl)-2,4-dioxo-3,4-dihydro-2H-pyrimidin-1-yl]-acétate de méthyle dans 30ml de dichlorométhane. Le milieu est ensuite laissé revenir lentement à température ambiante. 20ml d'une solution aqueuse d'hydroxyde de sodium 1N est ajoutée goutte à goutte à 10°C avec 20ml d'eau puis le milieu réactionnel est décanté. La phase aqueuse est extraite deux fois au dichlorométahne. La phase organique est lavée avec une solution aqueuse de thiosulfate de sodium , séchée sur sulfate de magnésium, filtrée et évaporée. L'huile obtenue est précipitée avec du dichlorométhane et de l'heptane. Le solide est filtré, rincé à l'heptane et séché. 1,4g (90%) de [5-(2-chloro-3-fluoro-phényl)-3-(2-méthanesulfinyl-éthyl)-2,4-dioxo-3,4-dihydro-2H-pyrimidin-1-yl]-acétate de méthyle sont obtenus sous la forme d'un solide blanc.

### 157.3: 2-[5-(2-chloro-3-fluoro-phényl)-3-[2-(méthylsulfonimidoyl)éthyl]-2,4-dioxo-pyrimidin-1-yl]acétate de méthyle

1,7g (5,2 mmoles) de diacétate d'iodobenzène sont additionnés sur une solution contenant 1,4g (3,5 mmoles) de [5-(2-chloro-3-fluoro-phényl)-3-(2-méthanesulfinyl-éthyl)-2,4-dioxo-3,4-dihydro-2H-pyrimidin-1-yl]-acétate de méthyle, 786mg (7 mmoles) de 2,2,2-trifluoroacétamide, 560mg (13,9 mmoles) d'oxyde de magnésium et 184mg (0,4 mmoles) de dirhodium tétraacétate dans 42ml de dichlorométhane. Le milieu est agité à température ambiante pendant 4 heures puis filtré et évaporé. Le résidu est repris par 42ml de méthanol puis 2,4g (17,4 mmoles) de carbonate de potassium sont ajoutés. Le mélange est agité à température ambiante pendant 30 minutes puis le méthanol est évaporé et le résidu est repris par de l'acétate d'éthyle puis par de l'eau.La phase aqueuse est extraite deux fois par de l'acétate d'éthyle. La phase organique est lavée à l'eau, séchée sur sulfate de magnésium, filtrée et évaporée. Après purification par chromatographie sur gel de silice élué par de l'acétate d'éthyle puis avec 10% de méthanol, 460mg (32%) de 2-[5-(2-chloro-3-fluoro-phényl)-3-[2-(méthylsulfonimidoyl)éthyl]-2,4-dioxo-pyrimidin-1-yl]acétate de méthyle sont obtenus sous la forme d'un solide jaune pâle.

### 157.4: Acide 2-[5-(2-chloro-3-fluoro-phényl)-3-[2-(méthylsulfonimidoyl)éthyl]-2,4-dioxo-pyrimidin-1-yl]acétique

De manière analogue à l'exemple 115.9, à partir de 180mg (0,4 mmoles) de 2-[5-(2-chloro-3-fluoro-phényl)-3-[2-(méthylsulfonimidoyl)éthyl]-2,4-dioxo-pyrimidin-1-yl]acétate de méthyle, une certaine quantité d'acide 2-[5-(2-chloro-3-fluoro-phényl)-3-[2-(méthylsulfonimidoyl)éthyl]-2,4-dioxo-pyrimidin-1-yl]acétique est obtenue sous la forme d'un solide jaune pâle.

### 157.5: 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione (composé 157)

De manière analogue à l'exemple 115.10, à partir de 113mg (0,4 mmoles) de 7-méthoxy-3-pipéridin-4-yl-1,3,4,5-tétrahydro-benzo[d][1,3]diazépin-2-one (préparé comme dans l'exemple 115.6) et 150mg (0,4 mmoles) d'acide 2-[5-(2-chloro-3-fluoro-phényl)-3-[2-(méthylsulfonimidoyl)éthyl]-2,4-dioxo-pyrimidin-1-yl]acétique, et après purification par chromatographie sur gel de silice élué, 23mg (9 %) de 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione sont obtenus sous la forme d'un solide rose pâle de point de fusion 197°C.

RMN ¹H (δ, DMSO): 1.55 (m, 1H), 1.61-1.83 (m, 3H), 2.65 (m, 1H), 2.88 (m, 2H), 2.98 (s, 3H), 3.07-3.22 (m, 1H), 3.67 (s, 3H), 3.84 (s, 1H), 3.95 (d, J = 13.7 Hz, 1H), 4.28 (m, 3H), 4.43 (d, J = 13.0 Hz, 1H), 4.79 (m, 2H), 6.62 (d, J = 2.9 Hz, 1H), 6.67 (dd, J = 8.6, 3.0 Hz, 1H), 6.98 (d, J = 8.9 Hz, 1H), 7.22 (m, 1H), 7.37-7.56 (m, 2H), 7.87 (s, 1H), 8.32 (s, 1H). Les 4 protons manquants sont sous le pic de l'eau et/ou du DMSO.

### Exemple 158: N-[(S)-2-(5-(2-chloro-3-fluoro-phényl)-3-{2-[4-(7-méthoxy-2-oxo-1,2,4,5-tétrahydro-benzo[d] [1,3]diazepin-3-yl)-pipéridin-1-yl]-2-oxo-éthyl}-2,6-dioxo-3,6-dihydro-2H-pyrimidin-1-yl)-propyl]-propionamide (schéma réactionnel n°3, composé 158)

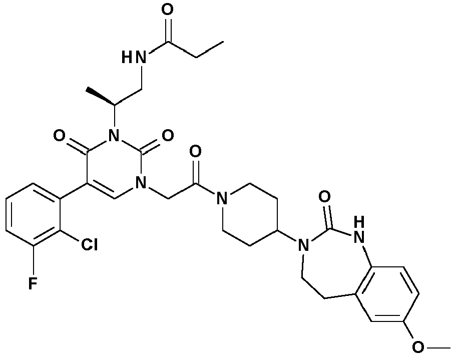

156µL (1,8 mmoles) de chlorure de propanoyle sont ajoutés à une solution de 1g (1,6 mmoles) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazepin-3-yl)-piperidin-1-yl]-2-oxo-éthyl}-1H-pyrimidine-2,4-dione préparé comme décrit dans l'exemple 119 et 0,25mL (1,8 mmoles) de triéthylamine dans 20mL de dichlorométhane. Le milieu réactionnel est agité à température ambiante pendant 1h, hydrolysé et extrait avec du dichlorométhane. Les phases organiques sont rassemblées, lavées une fois par de l'eau puis séchées sur sulfate de magnésium, filtrées et concentrées sous vide. Le produit brut obtenu est purifié par chromatographie sur gel de silice élué avec un mélange dichlorométhane/méthanol 96/4. 0,7g (64%) de N-[(S)-2-(5-(2-chloro-3-fluoro-phényl)-3-{2-[4-(7-méthoxy-2-oxo-1,2,4,5-tétrahydro-benzo[d][1,3]diazepin-3-yl)-pipéridin-1-yl]-2-oxo-éthyl}-2,6-dioxo-3,6-dihydro-2H-pyrimidin-1-yl)-propyl]-propionamide est obtenu sous la forme d'un solide blanc.

RMN ¹H (δ, DMSO): 0.96 (td, J = 7.6, 1.7 Hz, 3H), 1.24-1.41 (d, J = 8.0 Hz, 3H), 1.55 (m, 1H), 1.60-1.84 (m, 3H), 2.03 (qd, J = 9.0, 8.3, 2.6 Hz, 2H), 2.60-2.76 (m, 1H), 2.80-2.96 (m, 2H), 3.15 (m, 1H), 3.36 (m, 2H), 3.52 (m, 2H), 3.67 (s, 3H), 3.95 (d, J = 13.4 Hz, 1H), 4.29 (m, 1H), 4.44 (d, J = 12.8 Hz, 1H), 4.57-4.88 (m, 2H), 5.00 (m, 1H), 6.60 - 6.70 (m, 2H), 6.98 (d, J = 8.8 Hz, 1H), 7.20 (m, 1H), 7.36-7.54 (m, 2H), 7.79 (s, 1H), 7.85 (m, 1H), 8.34 (s, 1H).

### Exemple 159: 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-((S)-2-methoxy-1-methyl-ethyl)-1H-pyrimidine-2,4-dione (schéma réactionnel n°3, composé 159)

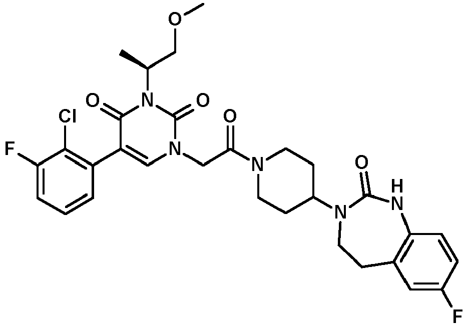

### 159.1 : 7-Fluoro-3-piperidin-4-yl-1,3,4,5-tetrahydro-benzo[d] [1,3]diazepin-2-one

De manière analogue à la séquence décrite dans l'exemple 115 (115.1 à 115.9), à partir de 1g (5,5 mmoles) de 2-(5-fluoro-2-nitrophenyl)acétonitrile, 315mg de 7-Fluoro-3-pipéridin-4-yl-1,3,4,5-tetrahydro-benzo[d][1,3]diazepin-2-one sont obtenus.

### 159.2 : N-[(S)-2-(5-(2-chloro-3-fluoro-phényl)-3-{2-[4-(7-méthoxy-2-oxo-1,2,4,5-tétrahydro-benzo[d] [1,3]diazepin-3-yl)-pipéridin-1-yl]-2-oxo-éthyl}-2,6-dioxo-3,6-dihydro-2H-pyrimidin-1-yl)-propyl]-propionamide

De manière analogue à l'exemple 115.10, à partir de 150mg (0,6 mmole) de 7-Fluoro-3-pipéridin-4-yl-1,3,4,5-tetrahydro-benzo[d] [1,3]diazepin-2-one et de 176mg (0,5 mmole) de l'acide [5-(2-Chloro-3-fluoro-phenyl)-3-((S)-2-methoxy-1-methyl-ethyl)-2,4-dioxo-3,4-dihydro-2H-pyrimidin-1-yl]-acétique (préparé comme décrit dans l'exemple 106.3), 60mg (21%) de N-[(S)-2-(5-(2-chloro-3-fluoro-phényl)-3-{2-[4-(7-méthoxy-2-oxo-1,2,4,5-tétrahydro-benzo[d][1,3]diazepin-3-yl)-pipéridin-1-yl]-2-oxo-éthyl}-2,6-dioxo-3,6-dihydro-2H-pyrimidin-1-yl)-propyl]-propionamide

Les composés suivants ont été synthétisés selon un mode opératoire analogue, en utilisant les réactifs appropriés commerciaux ou préalablement préparés:

### composé 160: 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-(2-methanesulfonyl-ethyl)-1H-pyrimidine-2,4-dione

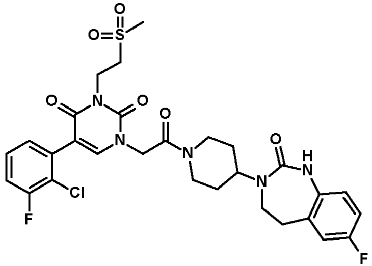

RMN ¹H (δ, DMSO) : 1.56 (m, 1H), 1.68 (m, 3H), 2.69 (m, 1H), 2.84 - 2.94 (m, 2H), 3.08 (s, 3H), 3.15 (m, 1H), 3.35-3.47 (m, 4H), 3.96 (d, J = 13.3 Hz, 1H), 4.31 (m, 3H), 4.43 (d, J = 13.1 Hz, 1H), 4.80 (m, 2H), 6.92 (m, 2H), 7.03 - 7.13 (m, 1H), 7.16-7.29 (m, 1H), 7.37-7.55 (m, 2H), 7.87 (s, 1H), 8.56 (s, 1H).

### Composé 161: N-[(S)-2-(5-(2-Chloro-3-fluoro-phenyl)-3-{2-[4-(7-fluoro-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide

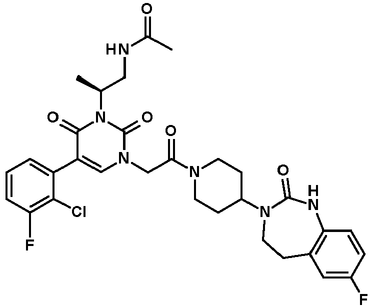

RMN ¹H (δ, DMSO) : 1.34 (d, J = 6.8 Hz, 3H) , 1.56 (m, 1H), 1.68 (m, 3H), 1.77 (s, 3H), 2.68 (m, 1H), 2.84 - 2.94 (m, 2H), 3.15 (m, 1H), 3.38 (m, 2H), 3.41-3.63 (m, 2H), 3.95 (d, J = 13.6 Hz, 1H), 4.29 (m, 1H), 4.44 (d, J = 13.0 Hz, 1H), 4.58-4.87 (m, 2H), 4.91-5.12 (m, 1H), 6.81-6.99 (m, 2H), 7.07 (m, 1H), 7.21 (m, 1H), 7.34-7.51 (m, 2H), 7.78 (s, 1H), 7.93 (t, J = 5.9 Hz, 1H), 8.55 (s, 1H).

### composé 162: N-[(S)-2-(5-(2,3-dichloro-phenyl)-3-{2-[4-(7-fluoro-2-oxo-1,2,4,5-tetrahydro benzo[d][1,3]diazepin-3-yl)-piperidin-1-yl]-2-oxo-ethyl}-2,6-dioxo-3,6-dihydro-2H-pyrimidin-1-yl)-propyl]-acetamide

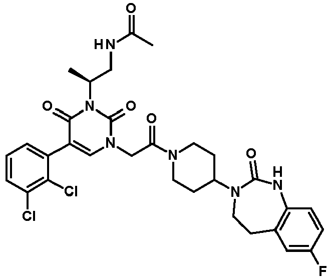

RMN ¹H (δ, DMSO) : 1.34 (d, J = 6.9 Hz, 3H), 1.55 (m, 1H), 1.71 (m, 3H), 1.77 (s, 3H), 2.68 (m, 1H), 2.82-2.97 (m, 2H), 3.14 (m, 1H), 3.37 (m, 2H), 3.51 (m, 2H), 3.95 (d, J = 13.6 Hz, 1H), 4.29 (m, 1H), 4.44 (d, J = 13.0 Hz, 1H), 4.61-4.86 (m, 2H), 5.06 - 4.89 (m, 1H), 6.81-7.00 (m, 2H), 7.07 (m, 1H), 7.33 (m, 1H), 7.42 (t, J = 7.8 Hz, 1H), 7.68 (dd, J = 8.0, 1.6 Hz, 1H), 7.77 (s, 1H), 7.92 (t, J = 5.9 Hz, 1H), 8.55 (s, 1H).

### Exemple 165 : 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide (schéma réactionnel 4, composé 165)

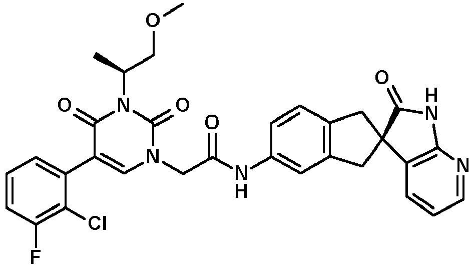

### 165.1: 5-Bromo-2,4-dioxo-3,4-dihydro-2H-pyrimidin-1-yl-acétate de méthyle

21,3g (150 mmoles) de carbonate de potassium et 14,5mL (150 mmoles) de bromoacétate de méthyle sont additionnés sur une solution de 30g (150mmoles) de 5-bromouracile dans 294mL de N,N-diméthylformamide. Le milieu réactionnel est agité à température ambiante pendant 50min puis hydrolysé (250mL d'eau et 50mL de NaOHaq 1N) et extrait par du dichlorométhane (300mL) . Les phases aqueuses sont rassemblées et lavées une fois par 100mL de dichlorométhane. La phase aqueuse contient le produit attendu. Le pH de la phase aqueuse est amené à pH=6-7 par 300mL d'une solution aqueuse d'acide chlorhydrique C=1N et 8mL d'acide chlorhydrique concentré (pour éviter d'avoir un trop grand volume d'eau). Le produit précipite. La suspension est agitée pendant 2h puis filtrée. Le solide obtenu est rincé par de l'eau et de l'éther diéthylique puis séché sous vide à 50°C pendant 24h pour donner 22g (54%) (5-bromo-2,4-dioxo-3,4-dihydro-2H-pyrimidin-1-yl)-acétate de méthyle sous la forme d'un solide blanc.

### 165.2: [5-bromo-3-((S)-2-méthoxy-1-méthyl-éthyl)-2,4-dioxo-3,4-dihydro-2H-pyrimidin-1-yl]-acétate de méthyle

2,7mL (11,4 mmoles) de diéthylazodicarboxylate est additionné goutte à goutte sur une solution refroidie à 0°C de 1g (3,8 mmoles) de 5-bromo-2,4-dioxo-3,4-dihydro-2H-pyrimidin-1-yl)-acétate de méthyle, 0,8mL (8 mmoles) de (R)-1-méthoxy-propan-2-ol et 2g (7,6 mmoles) de triphénylphosphine dans 20mL de tétrahydrofurane. Le milieu réactionnel (suspension blanche) est agité à température ambiante pendant 48 heures. Le tétrahydrofuranne est éliminé sous vide puis le résidu est repris dans l'acétate d'éthyle. La phase organique est lavée une fois par une solution aqueuse saturée d'hydrogénocarbonate de sodium, une fois par de l'eau puis par une solution aqueuse saturée de chlorure de sodium, séchée sur sulfate de magnésium, filtrée et concentrée sous vide. Le produit brut est purifié par chromatographie sur gel de silice élué par un mélange Heptane/Acétate d'éthyle 60/40. 1,2g (78%) de [5-bromo-3-((S)-2-méthoxy-1-méthyl-éthyl)-2,4-dioxo-3,4-dihydro-2H-pyrimidin-1-yl]-acétate de méthyle est obtenu sous la forme d'une huile incolore.

### 165.3: acide 5-(2-chloro-3-fluoro-phenyl)-3-((S)-2-méthoxy-1-méthyl-éthyl)-2,4-dioxo-3,4-dihydro-2H-pyrimidin-1-yl]-acétique

54mg (70µmoles) de 1,1'-bis(diphénylphosphino)ferrocène palladium (II) dichlorure complexé au dichlorométhane sont additionnés à une solution de 400mg (1,3 mmoles) de [5-bromo-3-((S)-2-méthoxy-1-méthyl-éthyl)-2,4-dioxo-3,4-dihydro-2H-pyrimidin-1-yl]-acétate de méthyle, de 417mg (4 mmoles) de carbonate de sodium et 571mg (3,3 mmoles) d'acide 2-chloro-3-fluoro-phényle boronique dans 44mL de 1,4-dioxanne et 4mL d'eau, préalablement dégazée pendant 5min. Le milieu réactionnel est ensuite chauffé à 100°C pendant 2h. 2mL d'une solution aqueuse 1M d'hydroxyde de lithium monohydrate et 2,2mL d'eau sont alors additionnés et le mélange réactionnel est agité pendant 1h. Le milieu réactionnel est hydrolysé puis dilué par de l'acétate d'éthyle. La première phase organique est écartée (impuretés) et la phase aqueuse est amenée à pH acide par une solution aqueuse d'acide chlorhydrique de concentration 1N. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le produit brut est purifié par chromatographie sur gel de silice élué par un mélange dichlorométhane/méthanol 95/5 + 0.1% d'acide acétique. 240mg (49%) d'acide [5-(2-Chloro-3-fluoro-phényl)-3-((S)-2-méthoxy-1-méthyl-éthyl)-2,4-dioxo-3,4-dihydro-2H-pyrimidin-1-yl]-acétique sont obtenus sous la forme d'une huile jaune.

### 165.4: 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide (composé 165)

162mg (0,65 mmoles) de (R)-5-amino-1,3-dihydrospiro[indene-2,3'-pyrrolo[2,3-b)pyridin]-2'(1'H)-one préparé comme décrit dans le brevet WO2011/005731 est additionné sur une solution préalablement agitée pendant 5min, de 240mg (0,65mmoles) d'acide [5-(2-chloro-3-fluoro-phenyl)-3-((S)-2-méthoxy-1-méthyl-éthyl)-2,4-dioxo-3,4-dihydro-2H-pyrimidin-1-yl]-acétique, 114mg (0,8 mmole) de 1-hydroxybenzotriazole et 161mg (0,8 mmole) de chlorhydrate de 1-éthyl-3-(3-diméthylaminopropyl)-carbodiimide dans 5mL de N,N-DIMETHYLFORMAMIDE. Le milieu réactionnel est agité à température ambiante pendant 18h puis dilué par de l'acétate d'éthyle. La phase organique est lavée une fois par une solution aqueuse saturée d'hydrogénocarbonate de sodium, une fois par de l'eau et une fois par une solution aqueuse saturée de chlorure de sodium, séchée sur sulfate de magnésium puis filtrée et concentrée sous vide.Le produit brut est purifié par chromatographie sur gel de silice (HP15) élué par un mélange dichlorométhane/méthanol 96/4. 155mg (39%) de 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide sont obtenus sous la forme d'un solide blanc.

RMN ¹H (δ, DMSO): 1.34 (d, J = 6.9 Hz, 3H); 3.04-3.11 (m, 2H); 3.23 (s, 3H); 3.33-3.37 (m, 2H); 3.54 (dd, J = 9.9, 5.9 Hz, 1H); 3.92 (dd, J = 9.9, 8.2 Hz, 1H); 4.65 (s, 2H); 5.13-5.15 (m, 1H); 6.87 (dd, J = 7.3, 5.3 Hz, 1H); 7.18 (dd, J = 7.3, 1.6 Hz, 1H); 7.21-7.24 (m, 2H); 7.38 (d, J = 8.3 Hz, 1H); 7.43-7.46 (m, 2H); 7.62 (s, 1H); 7.93 (s, 1H); 8.07 (dd, J = 5.3, 1.6 Hz, 1H); 10.35 (s, 1H); 11.09 (s, 1H).

Les composés suivants ont été synthétisés selon un mode opératoire analogue à partir des réactifs appropriés commerciaux ou préalablement préparés:

### composé 166: 5-(2-Chloro-3-fluoro-phenyl)-3-isopropyl-pyrimidine-2,4-dione-1-[(2R)-2'-oxospiro[1,3-dihydroindene-2,3'-1H-pyrrolo[2,3-b]pyridine]-5-yl]-acetamide

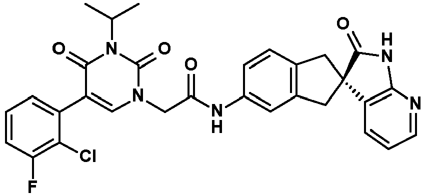

RMN ¹H (δ, DMSO) : 1.41 (d, J = 6.9 Hz, 6H); 3.08 (m, 2H); 3.33 (m, 2H); 4.65 (m, 2H); 5.10-5.11 (m, 1H); 6.87 (dd, J = 7.3, 5.3 Hz, 1H); 7.18 (m, 1H), 7.23 (m, 2H); 7.38 (m, 1H), 7.45 (m, 2H), 7.63 (m, 1H); 7.91 (s, 1H); 8.07 (dd, J = 5.3, 1.6 Hz, 1H); 10.35 (s, 1H); 11.09 (s, 1H).

### Composé 170: 5-(2,3-dichlorophenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide

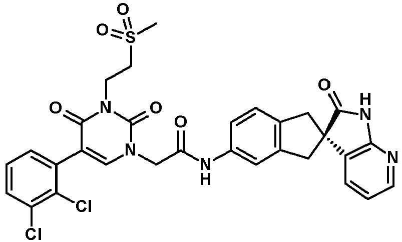

RMN ¹H (δ, DMSO) : 3.03-3.15 (m, 5H), 3.33-3.46 (m, 4H), 4.31 (dd, J = 8.4, 6.1 Hz, 2H), 4.69 (s, 2H), 6.87 (dd, J = 7.3, 5.3 Hz, 1H), 7.18 (dd, J = 7.3, 1.6 Hz, 1H), 7.24 (d, J = 8.1 Hz, 1H), 7.35 (dd, J = 7.7, 1.6 Hz, 1H), 7.38 (dd, J = 8.1, 2.0 Hz, 1H), 7.44 (t, J = 7.9 Hz, 1H), 7.61 (d, J = 2.0 Hz, 1H), 7.70 (dd, J = 8.0, 1.6 Hz, 1H), 8.00 (s, 1H), 8.07 (dd, J = 5.2, 1.6 Hz, 1H), 10.36 (s, 1H), 11.08 (br-s, 1H).

### composé 178: 5-(2,3-dichlorophenyl)-3-[(1S)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide

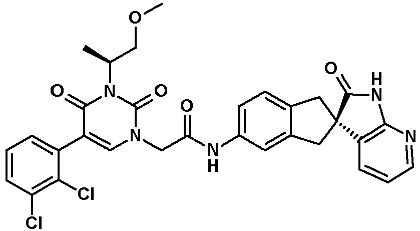

RMN ¹H (δ, DMSO) : 1.33 (d, J = 6.9 Hz, 3H), 3.01 - 3.14 (m, 2H), 3.23 (s, 3H), 3.40 (m, 2 H), 3.54 (m, 1H), 3.92 (m, 1H), 4.64 (s, 2H), 5.14 (m, 1H), 6.87 m, 1H), 7.18 (dd, J = 1.7, 7.3 Hz, 1H), 7.24 (d, J = 8.2 Hz, 1H), 7.30 - 7.47 (m, 3H), 7.62 (m, 1H), 7.69 (dd, J = 1.6, 8 Hz, 1H), 7.92 (s, 1H), 8.07 (dd, J = 1.6, 5.3 Hz, 1H), 10.34 (s, 1H), 11.08 (s, 1H).

### Exemple 184: 5-(2-Chloro-3-fluoro-phényl)-3-(2-méthanesulfonyl-éthyl)-1-{2-[4-(7-méthanesulfonyl-2-oxo-1,2,4,5-tétrahydro-benzo[d][1,3]diazepin-3-yl)-piperidin-1-yl]-2-oxo-ethyl}-1H-pyrimidine-2,4-dione (schéma réactionnel n°4, composé 184)

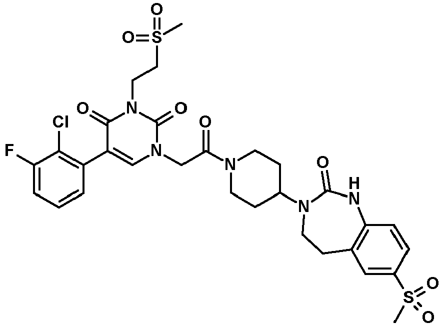

### 184.1 : 2-(5-méthylsulfanyl-2-nitro-phényl)acetonitrile

Une solution de 6g (47mmoles) de (4-chloro-phénoxy)-acétonitrile et 6g (40mmoles) de 1-méthylsulfanyl-4-nitrobenzène dans 60mL de N,N-diméthylformamide est ajouté goutte à goutte à une solution préalablement refroidie à -10°C de 10,6g (94mmoles) de potassium tert-butoxyde dans 120mL de N,N-diméthylformamide. La réaction est agitée à -10°C pendant 45min avant d'être hydrolysée par une solution glaciale d'acide chlorhydrique concentré puis extraite par de l'acétate d'éthyle. La phase organique est lavée à l'eau puis avec une solution saturée de chlorure de sodium, séchée sur sulfate de magnésium, filtrée puis concentrée sous vide. Le brut réactionnel est repris dans une solution 1:1 Heptane:AcOEt et après trituration pendant 1 heure, le mélange est filtré et le solide lavé avec une solution 1:1 Hept:AcOEt pour obtenir 2,9g de 2-(5-méthylsulfanyl-2-nitro-phényl)acétonitrile (58%) sous la forme d'un solide.

### 184.2 : 2-(5-méthylsulfanyl-2-nitro-phényl)éthanamine

28mL d'une solution 1M de boranne complexé au tétrahydrofuranne dans le tétrahydrofuranne est ajoutée goutte à goutte à température ambiante à une solution de 2,9g (14 mmoles) de 2-(5-méthylsulfanyl-2-nitro-phényl)acétonitrile dans 18mL de tétrahydrofuranne.Le mélange est ensuite chauffé à reflux pendant 3h. Le mélange est ensuite refroidi à température ambiante et 6mL de méthanol sont ajoutés lentement. Après 10min d'agitation et de méthanolyse du borane, le mélange est concentré sous vide. Le résidu est repris dans du 2-méthyl-tétrahydrofuranne et la phase organique lavée avec une solution aqueuse d'hydroxyde de sodium 1N. La phase aqueuse est extraite par trois fois au 2-méthyl-tétrahydrofuranne et les phases organiques sont rassemblées et concentrées partiellement. 1mL d'acide acétique est ajouté doucement et le mélange est laissé sous lente agitation pour revenir à température ambiante. Le milieu est donc concentré sous vide 3g (79%) de 2-(5-méthylsulfanyl-2-nitro-phényl)éthanamine est obtenu sous la forme d'une huile.

### 184.3 : 4-[2-(5-méthylsulfanyl-2-nitro-phenyl)-éthylamino]-piperidine-1-carboxylate de tert-butyle

3g (14 mmoles) 2-(5-méthylsulfanyl-2-nitro-phényl)éthanamine et 3,1g (15,4 mmoles) de 1-Boc-4-pipéridone sont ajoutés dans 40mL de 2-méthyltétrahydrofuranne. Le milieu réctionnel est peu soluble mais 4,5g (21 mmoles) de sodium triacétoxyborohydrure sont ajoutés lentement par portions. Le milieu est agité à température ambiante pendant 12h puis hydrolysé avec 15mL d'une solution aqueuse d'hydroxyde de sodium de concentration 2N et extrait avec du 2-méthyl-tétrahydrofuranne. La phase organique est relavée à l'eau jusqu'à pH=6, séchée sur sulfate de magnésium puis concentrée. 6,2g (100%) de 4-[2-(5-méthylsulfanyl-2-nitro-phényl)-éthylamino]-pipéridine-1-carboxylate de tert-butyle sont obtenus sous la forme d'une huile orange.

### 184.4 : 4-[2-(2-amino-5-méthylsulfanyl-phényl)-éthylamino]-pipéridine-1-carboxylate de tert-butyle

3,1g (56 mmoles) de fer et 0,4g (7 mmoles) de chlorure d'ammonium sont ajoutés à une solution de 5,5g (14 mmoles) de 4-[2-(5-méthylsulfanyl-2-nitro-phényl)-éthylamino]-pipéridine-1-carboxylate de tert-butyle dans 14mL de méthanol, 14mL de 2-méthyl-tétrahydrofuranne (14.00 ml) et 14mL d'eau. Le milieu réactionnel non homogène est chauffé au reflux pendant 3h. Une fois la reaction terminée, le milieu est filtré sur célite et le précipité est rincé avec du méthanol .Le filtrat est concentré jusqu'à l'élimination totale du méthanol puis repris dans du 2-méthyl-tétrahydrofuranne et hydrolysé avec 10mL d'une solution d'EDTA à 10%. La phase organique obtenue est lavée avec de l'eau, séchée sur sulfate de magnésium, filtrée et évaporée. 6,1g (100%) de résidu brut de 4-[2-(2-amino-5-méthylsulfanyl-phényl)-éthylamino]-pipéridine-1-carboxylate de tert-butyle sont obtenus sous la forme d'un solide orange.

### 184.5 : 4-(7-méthylsulfanyl-2-oxo-1,2,4,5-tétrahydrobenzo[d][1,3]diazepin-3-yl)-pipéridine-1-carboxylate de tert-butyle

2,5g (20 mmoles) de N,N'-carbonuldiimidazole sont additionnés par portions sur une solution de 5,1g (14 mmoles) de 4-[2-(2-amino-5-méthylsulfanyl-phényl)-éthylamino]-pipéridine-1-carboxylate de tert-butyle dans 77mL d'acétonitrile. Le milieu réactionnel est agité à température ambiante pendant 18 heures puis dilué par de l'acétate d'éthyle et hydrolysé par une solution aqueuse saturée de chlorure d'ammonium. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le résidu brut est purifié par chromatographie sur gel de silice élué par un mélange heptane / acétate d'éthyle en augmentant la polarité de 7/3 à 5/5. 2,4g (44%) de 4-[2-(2-amino-5-méthylsulfanyl-phényl)-éthylamino]-pipéridine-1-carboxylate de tert-butyle sont obtenus.

### 184.5 : 7-méthylsulfanyl-3-pipéridin-4-yl-1,3,4,5-tetrahydro-benzo[d][1,3]diazepin-2-one

0,8mL (8 mmoles) d'acide trifluoroacétique sont ajoutés à une solution de 1,2g (3 mmoles) de 4-(7-méthylsulfanyl-2-oxo-1,2,4,5-tétrahydro-benzo[d][1,3]diazepin-3-yl)-pipéridine-1-carboxylate de tert-butyle dans 7mL de dichlorométhane. Le mélange est agité 12 heures à température ambiante puis concentré sous vide. Le brut réactionnel est repris dans du toluène et co-evaporé plusieurs fois. 0,8g (90%) de 7-méthylsulfanyl-3-pipéridin-4-yl-1,3,4,5-tetrahydro-benzo[d] [1,3]diazepin-2-one sont obtenus et engagés dans l'étape 184.9 sans purification supplémentaire.

### 184.6 : [5-bromo-3-(2-méthylsulfanyl-éthyl)-2,4-dioxo-3,4-dihydro-2H-pyrimidin-1-yl]-acétate de méthyle

11,8g (85,5 mmoles) de carbonate de potassium puis 10,7mL (109 mmoles) de 2-chloroéthylméthyl sulfide sont additionnés sur une solution de 15g (57 mmoles) de 2-(5-bromo-2,4-dioxo-3,4-dihydro-2H-pyrimidin-1-yl)-acétate de méthyle dans 250mL de N,N-diméthylformamide. Le milieu réactionnel est chauffé à 60°C pendant 9h puis hydrolysé et dilué par de l'acétate d'éthyle. Le produit est extrait à l'acétate d'éthyle. Les phases organiques sont rassemblées, lavées deux fois par de l'eau et une fois par une solution aqueuse saturée de chlorure de sodium, séchées sur sulfate de magnésium, filtrées et concentrées sous vide. Le produit brut est purifié par chromatographie sur gel de silice élué par un mélange heptane/acétate d'éthyle 60/40. 13,3g (69%) de [5-bromo-3-(2-méthylsulfanyl-éthyl)-2,4-dioxo-3,4-dihydro-2H-pyrimidin-1-yl]-acétate de méthyle sont obtenus sous la forme d'un solide blanc.

### 184.7 : [5-(2-chloro-3-fluoro-phényl)-3-(2-méthylsulfanyl-éthyl)-2,4-dioxo-3,4-dihydro-2H-pyrimidin-1-yl]-acétate de méthyle

267mg (0,3 mmoles) de dichlorure de 1,1'-bis(diphénylphosphino)ferrocènepalladium (II) complexé au dichlorométhane sont additionnés à une solution de 2,2g (6,5 mmoles) de [5-bromo-3-(2-méthylsulfanyl-éthyl)-2,4-dioxo-3,4-dihydro-2H-pyrimidin-1-yl]-acétate de méthyle, de 2,1g (19,6 mmoles) de carbonate de sodium et de 2,5g (8,5 mmoles) d'acide 2-chloro-3-fluoro phénylboronique. dans 220mL de 1,4-dioxanne et 22mL d'eau, préalablement dégazée sous vide/azote pendant 5min. Le milieu réactionnel est ensuite chauffé à 100°C pendant 1h30 puis hydrolysé et dilué par de l'acétate d'éthyle. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide.

Le produit brut est purifié par chromatographie sur gel de silice HP 15 élué par un mélange heptane/acétate d'éthyle 70/30. 1,4g (55%) de [5-(2-chloro-3-fluoro-phényl)-3-(2-méthylsulfanyl-éthyl)-2,4-dioxo-3,4-dihydro-2H-pyrimidin-1-yl]-acétate de méthyle sont obtenus sous la forme d'un solide crème.

### 184.8 : acide [5-(2-chloro-3-fluoro-phenyl)-3-(2-methylsulfanyl-ethyl)-2,4-dioxo-3,4-dihydro-2H-pyrimidin-1-yl]-acétique

5,4mL (5,4 mmoles) d'hydroxyde de lithium monohydrate et 2,8mL d'eau sont additionnés sur une solution de 1,4g (3,6 mmoles) de [5-(2-chloro-3-fluoro-phényl)-3-(2-méthylsulfanyl-éthyl)-2,4-dioxo-3,4-dihydro-2H-pyrimidin-1-yl]-acétate de méthyle dans 28mL de tétrahydrofuranne. Le milieu réactionnel est agité à température ambiante pendant 2h puis hydrolysé et dilué par de l'acétate d'éthyle. Le pH de la phase aqueuse est amené à pH acide par une solution aqueuse d'acide chlorhydrique de concentration 1N. Le produit est extrait à l'acétate d'éthyle, les phases organiques sont rassemblées, lavées une fois par de l'eau et une fois par une solution aqueuse saturée de chlorure de sodium, séchées sur sulfate de magnésium, filtrées puis concentrées sous vide. 1,3g (98%) d'acide [5-(2-chloro-3-fluoro-phényl)-3-(2-méthylsulfanyl-éthyl)-2,4-dioxo-3,4-dihydro-2H-pyrimidin-1-yl]-acétique sont obtenus sous la forme d'un solide crème.

### 184.9 : 5-(2-chloro-3-fluoro-phényl)-3-(2-méthylsulfanyl-éthyl)-1-{2-[4-(7-méthylsulfanyl-2-oxo-1,2,4,5-tetrahydro-benzo[d] [1,3]diazepin-3-yl)-pipéridin-1-yl]-2-oxo-éthyl}-1H-pyrimidine-2,4-dione

185mg (1 mmole) de chlorhydrate de 1-éthyl-3-(3-diméthylaminopropyl)carbodiimide et 107mg (1 mmole) de 1-oxypyridin-2-ol sont additionnés à 0,3g (0,8 mmoles) d'acide [5-(2-chloro-3-fluoro-phényl)-3-(2-méthylsulfanyl-éthyl)-2,4-dioxo-3,4-dihydro-2H-pyrimidin-1-yl]-acétique dans 2mL de N,N-diméthylformamide. Le milieu réactionnel est agité pendant 5min puis 281mg (1 mmole) de 7-méthylsulfanyl-3-pipéridin-4-yl-1,3,4,5-tetrahydro-benzo[d] [1,3]diazepin-2-one (préparé comme décrit en 184.4) et 0,15mL (1 mmole) de triéthylamine sont ajoutés. Le milieu réactionnel est agité à température ambiante pendant 12 heures puis dilué par du dichlorométhane et de l'eau. La phase aqueuse est extraite trois fois au dichlorométhane, les phases organiques sont combinées et lavées avec une solution aqueuse saturée d'hydrogénocarbonate de sodium puis avec une solution aqueuse saturée de chlorure de sodium. Après séchage sur sulfate de magnésium, la solution est filtrée puis concentrée sous vide. Le résidu brut obtenu est purifié par chromatographie sur gel de silice élué avec un mélange heptane / acétate d'éthyle 7/3. 520mg (100%) de 5-(2-chloro-3-fluoro-phényl)-3-(2-méthylsulfanyl-éthyl)-1-{2-[4-(7-méthylsulfanyl-2-oxo-1,2,4,5-tetrahydro-benzo[d] [1,3]diazepin-3-yl)-pipéridin-1-yl]-2-oxo-éthyl}-1H-pyrimidine-2,4-dione sont obtenus sous forme d'un solide blanc.

### 184.10 : 5-(2-chloro-3-fluoro-phényl)-3-(2-méthanesulfonyl-éthyl)-1-{2-[4-(7-méthanesulfonyl-2-oxo-1,2,4,5-tétrahydro-benzo[d] [1,3]diazepin-3-yl)-piperidin-1-yl]-2-oxo-éthyl}-1H-pyrimidine-2,4-dione (composé 184)

50mg (0,04 mmole) d'ammonium molybdate tétrahydrate et 146µL (24 mmoles) d'une solution aqueuse de peroxyde d'hydrogène 30% sont ajoutés à 517mg (0,8 mmole) de 5-(2-chloro-3-fluoro-phényl)-3-(2-méthylsulfanyl-éthyl)-1-{2-[4-(7-méthylsulfanyl-2-oxo-1,2,4,5-tétrahydro-benzo[d][1,3]diazepin-3-yl)-piperidin-1-yl]-2-oxo-ethyl}-1H-pyrimidine-2,4-dione dans 3mL d'éthanol préalablement refroidie à 0°C. La réaction est agitée à température ambiante pendant 2 heures puis hydrolysée avec de l'eau (30mL) et extraite au dichlorométhane (3x30mL). Les phases organiques sont rassemblées, lavées avec une solution aqueuse saturée de thiosulfate de sodium (30 mL × 4), de l'eau (20 mL × 2), et une solution aqueuse saturée de chlorure de sodium (20 mL × 2), puis séchée sur sulfate de magnésium, filtrées et concentrées sous vide. Après trituration du résidu brut dans l'acétate d'éthyle pendant 12 heures et filtration, 210mg (35%) de 5-(2-chloro-3-fluoro-phényl)-3-(2-méthanesulfonyl-éthyl)-1-{2-[4-(7-méthanesulfonyl-2-oxo-1,2,4,5-tétrahydro-benzo[d][1,3]diazepin-3-yl)-piperidin-1-yl]-2-oxo-éthyl}-1H-pyrimidine-2,4-dione sont obtenus.

RMN ¹H (400 MHz, DMSO): δ (ppm) 1.52-1.75 (m, 4 H); 2.68 (t, J = 11.7 Hz, 1 H); 2.98 (br t, 2 H); 3.06 (s, 3 H); 3.10 (s, 3 H); 3.14-3.18 (br m, 1 H); 3.40 (br m, 4 H); 3.95 (d, J = 13.5 Hz, 1 H); 4.27-4.43 (m, 4 H); 4.79 (s, 2 H); 7.18-7.23 (m, 2 H); 7.42-7.45 (m, 2 H); 7.54-7.58 (m, 2 H); 7.86 (s, 1 H); 9.13 (s, 1H).

Autre composé synthétisé selon un mode opératoire analogue en utilisant les réactifs appropriés ou préalablement préparés:

### composé 185: 5-(2-Chloro-3-fluoro-phenyl)-3-isopropyl-1-{2-[4-(7-methanesulfonyl-2-oxo-1,2,4,5-tetrahydro-benzo[d][1,3]diazepin-3-yl)-piperidin-1-yl]-2-oxo-ethyl}-1H-pyrimidine-2,4-dione

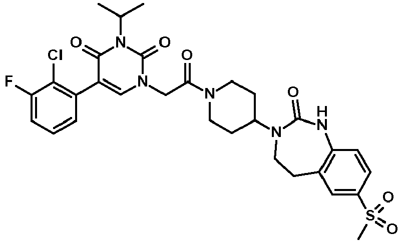

RMN ¹H (δ, DMSO) : 1.41 (d, J = 6.9 Hz, 6H), 1.57 (m, 1H), 1.70 (m, 3H), 2.64-2.77 (m, 1H), 2.93-3.05 (m, 2H), 3.12 (s, 3H), 3.17 (m, 1H), 3.37-3.48 (m, 2H), 3.95 (m, 1H), 4.28-4.50 (m, 2H), 4.64 - 4.84 (m, 2H), 5.10 (h, J = 6.8 Hz, 1H), 7.18-7.27 (m, 2H), 7.40 - 7.49 (m, 2H), 7.54-7.62 (m, 2H), 7.77 (s, 1H), 9.15 (s, 1H).

### Exemple 186: 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d][1,3]diazepin-3-yl)-piperidin-1-yl]-2-oxo-éthyl}-1H-pyrimidine-2,4-dione (schéma réactionnel n°4, composé 186)

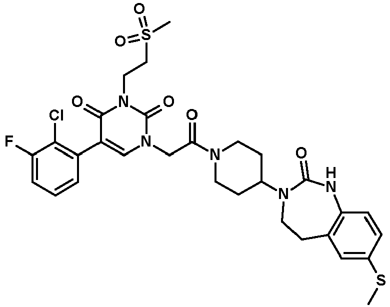

### 186.1 : acide [5-(2-chloro-3-fluoro-phényl)-3-(2-méthanesulfonyl-éthyl)-2,4-dioxo-3,4-dihydro-2H-pyrimidin-1-yl]-acétique

Une solution de 50mg (0,04 mmole) d'ammonium molybdate tétrahydrate et 146µL (24 mmoles) d'une solution aqueuse de peroxyde d'hydrogène 30% sont ajoutés à une solution d'acide [5-(2-chloro-3-fluoro-phenyl)-3-(2-methylsulfanyl-ethyl)-2,4-dioxo-3,4-dihydro-2H-pyrimidin-1-yl]-acétique (préparé comme décrit en 184.8). La réaction est agitée à température ambiante pendant 2 heures, hydrolysée avec de l'eau (30mL) et extraite avec de l'acétate d'éthyle (3x30mL). Les phases organiques sont rassemblées, lavées avec une solution aqueuse saturée de thiosulfate de sodium, de l'eau puis une solution aqueuse saturée de chlorure de sodium. Après séchage sur sulfate de sodium et filtration sous vide, 320mg (98%) d' acide [5-(2-Chloro-3-fluoro-phenyl)-3-(2-methanesulfonyl-ethyl)-2,4-dioxo-3,4-dihydro-2H-pyrimidin-1-yl]-acétique sont obtenus et utilisés dans l'étape suivante sans purification.

### 186.2 : 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d] [1,3]diazepin-3-yl)-piperidin-1-yl]-2-oxo-éthyl}-1H-pyrimidine-2,4-dione (composé 186)

182mg (1 mmole) de chlorhydrate de 1-éthyl-3-(3-diméthylaminopropyl)carbodiimide et 105mg (1 mmole) de 1-oxy-pyridin-2-ol sont ajoutés à une solution de 320mg (0,8 mmole) d'acide [5-(2-chloro-3-fluoro-phényl)-3-(2-méthanesulfonyl-éthyl)-2,4-dioxo-3,4-dihydro-2H-pyrimidin-1-yl]-acétique dans 2mL N,N-diméthylformamide. Le milieu réactionnel est agité pendant 5min puis 253mg (0,9 mmole) de 7-méthylsulfanyl-3-piperidin-4-yl-1,3,4,5-tetrahydro-benzo[d] [1,3]diazepin-2-one et de 0,15mL (1 mmole) de triéthylamine sont ajoutés. Le milieu réactionnel est agité à température ambiante pendant 12 heures, puis dilué par du dichlorométhane et de l'eau. La phase aqueuse est extraite trois fois au dichlorométhane, les phases organiques sont combinées et lavées avec une solution saturée d'hydrogénocarbonate de sodium, à l'eau puis avec une solution aqueuse saturée de chlorure de sodium. Après séchage sur sulfate de magnésium, la solution est filtrée puis concentrée sous vide. Après trituration du résidu brut dans l'acétate d'éthyle pendant 12 heures et filtration, 210mg (36%) de 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d] [1,3]diazepin-3-yl)-piperidin-1-yl]-2-oxo-éthyl}-1H-pyrimidine-2,4-dione sont obtenus.

RMN ¹H (400 MHz, DMSO): δ (ppm) 1.50-1.73 (m, 4 H); 2.38 (s, 3 H); 2.67 (t, J = 12.4 Hz, 1 H); 2.86 (d, J = 6.8 Hz, 2 H); 3.06 (s, 3 H); 3.13 (t, J = 12.4 Hz, 1 H); 3.33-3.41 (m, 4 H); 3.94 (d, J = 13.6 Hz, 1 H); 4.29 (t, J = 7.4 Hz, 3 H); 4.41 (d, J = 12.9 Hz, 1 H); 4.78 (s, 2 H); 6.99 (d, J = 10.8 Hz, 3 H); 7.19-7.21 (m, 1 H); 7.43-7.46 (m, 2 H); 7.86 (s, 1 H); 8.55 (s, 1 H).

Les composés suivants ont été synthétisés selon un mode opératoire analogue, en utilisant les réactifs appropriés commerciaux ou préalablement préparés:

### composé 187: 5-(2-Chloro-3-fluoro-phenyl)-3-((R)-2-methoxy-1-methyl-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione

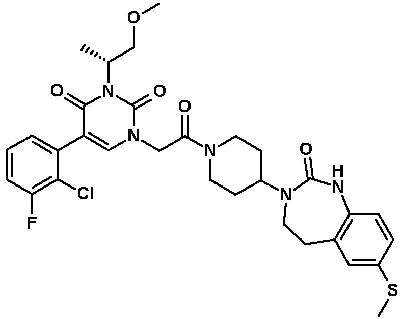

RMN ¹H (δ, DMSO) : 1.33 (d, J = 7.0 Hz, 3H), 1.56 (m, 1H), 1.67 (m, 3H), 2.40 (s, 3H), 2.68 (m, 1H), 2.88 (m, 2H), 3.14 (m, 1H), 3.23 (s, 3H), 3.34-3.42 (m, 2H), 3.54 (m, 1H), 3.83-4.04 (m, 2H), 4.33 (m, 1H), 4.43 (d, J = 12.8 Hz, 1H), 4.75 (m, 2H), 5.15 (m, 1H), 7.01 (m, 3H), 7.21 (m, 1H), 7.45 (m, 2H), 7.79 (s, 1H), 8.57 (s, 1H).

### composé 188: 5-(2-Chloro-3-fluoro-phenyl)-3-(2-methoxy-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione

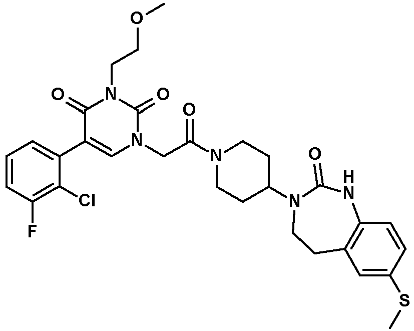

RMN ¹H (δ, DMSO) : 1.45-1.61 (m, 1H), 1.70 (m, 3H), 2.40 (s, 3H), 2.61-2.75 (m, 1H), 2.82-2.93 (m, 2H), 3.09-3.21 (m, 1H), 3.26 (s, 3H), 3.34-3.42 (m, 2H), 3.52 (t, J = 6.1 Hz, 2H), 3.94 (d, J = 13.5 Hz, 1H), 4.07 (m, 2H), 4.32 (m, 1H), 4.43 (d, J = 13.0 Hz, 1H), 4.78 (m, 2H), 6.91-7.09 (m, 3H), 7.17-7.29 (m, 1H), 7.36-7.54 (m, 2H), 7.83 (s, 1H), 8.57 (s, 1H).

### composé 189: 5-(2-Chloro-3-fluoro-phenyl)-3-isopropyl-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione

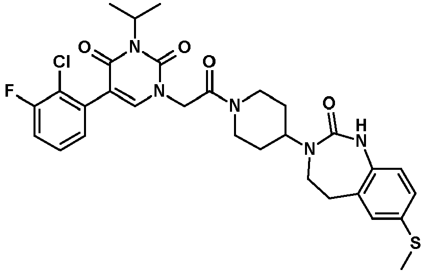

RMN ¹H (δ, DMSO) : 1.41 (d, J = 7.0 Hz, 6H), 1.56 (m, 1H), 1.63-1.86 (m, 3H), 2.40 (s, 3H), 2.68 (m, 1H), 2.78-2.95 (m, 2H), 3.15 (m, 1H), 3.33-3.45 (m, 2H), 3.94 (d, J = 13.6 Hz, 1H), 4.31 (m, 1H), 4.43 (d, J = 12.9 Hz, 1H), 4.74 (m, 2H), 5.11 (p, J = 6.9 Hz, 1H), 7.01 (m, 3H), 7.15-7.27 (m, 1H), 7.35-7.52 (m, 2H), 7.77 (s, 1H), 8.56 (s, 1H).

### composé 192: 3-(1-{2-[5-(2-chloro-3-fluoro-phenyl)-3-(2-methoxy-ethyl)-2-oxo-3,4-dihydro-2H-pyrimidin-1-yl]-acetyl}-piperidin-4-yl)-1,3,4,5-tetrahydro-benzo[d][1,3]diazepin-2-one

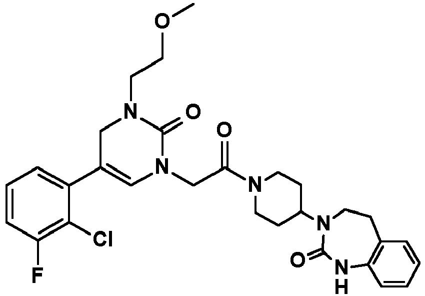

RMN ¹H (δ, DMSO) : 1.53 (m, 1 H); 1.67 (m, 3 H); 2.59-2.64 (m, 1 H); 2.89 (m, 2 H); 3.08 (m, 1 H); 3.27 (s, 3 H); 3.35-3.38 (m, 2 H); 3.46-3.50 (m, 4 H); 3.91 (d, J = 13.5 Hz, 1 H); 4.35 (m, 5 H); 4.44 (d, J = 13.0 Hz, 1 H); 6.46 (s, 1 H); 6.80-6.82 (m, 1 H); 7.04-7.06 (m, 3 H); 7.18 (m, 1 H); 7.35-7.36 (m, 2 H); 8.52 (s, 1 H).

### composé 199: 3-(1-{2-[5-(2,3-Dichloro-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-one

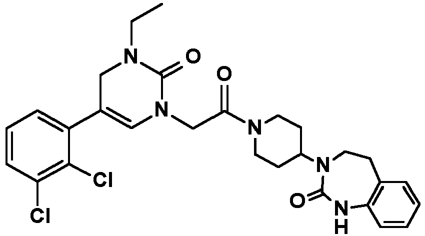

RMN ¹H (δ, DMSO) : 1.08 (t, J = 7.1 Hz, 3H), 1.52 (m, 1H), 1.64 (m, 3H), 2.61 (m, 1H), 2.88 (m, 2H), 3.08 (m, 1H), 3.36 (m, 4H), 3.90 (d, J = 13.4 Hz, 1H), 4.12-4.56 (m, 6H), 6.40 (s, 1H), 6.80 (m, 1H), 7.03 (m, 3H), 7.22-7.45 (m, 2H), 7.58 (dd, J = 7.7, 1.8 Hz, 1H), 8.52 (s, 1H).

Les composés décrits ci-après sont également préparés selon des modes opératoires analogues à ceux décrits précédemment dans l'ensemble de la partie expérimentale en utilisant les réactifs appropriés commerciaux ou préalablement préparés selon des méthodes classiques en synthèse organique :

### composé 208: 5-(2,3-dichlorophenyl)-3-methyl-1-[2-oxo-2-[4-(2-oxo-5-phenyl-1H-imidazol-3-yl)-1-piperidyl]ethyl]pyrimidine-2,4-dione

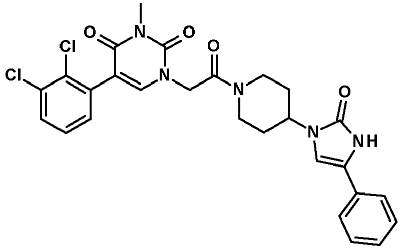

RMN ¹H (δ, DMSO) : 1.54 - 1.94 (m, 4H), 2.69-2.84 (m, 1H), 3.21 (m, 1H), 3.25 (s, 3H), 4.01 (d, J = 13.0 Hz, 1H), 4.09-4.26 (m, 1H), 4.46 (d, J = 12.7 Hz, 1H), 4.66-4.95 (m, 2H), 7.11 - 7.22 (m, 2H), 7.33 (m, 3H), 7.43 (t, J = 7.8 Hz, 1H), 7.47-7.54 (m, 2H), 7.69 (dd, J = 8.0, 1.6 Hz, 1H), 7.84 (s, 1H), 10.74 (m, 1H).

### composé 210: 5-(2,3-dichlorophenyl)-3-isopropyl-1-[2-oxo-2-[4-(2-oxo-4,5-dihydro-1H-1,3-benzodiazepin-3-yl)-1-piperidyl]ethyl]pyrimidine-2,4-dione

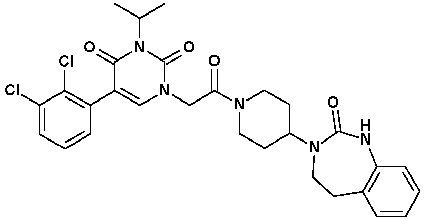

RMN ¹H (δ, DMSO) : 1.40 (d, J = 6.9 Hz, 6H), 1.48-1.60 (m, 1H), 1.64-1.80 (m, 3H), 2.68 (m, 1H), 2.89 (m, 2H), 3.14 (m, 1H), 3.37 (m, 2H), 3.91 (d, J = 12.8 Hz, 1H), 4.25-4.38 (m, 1H), 4.43 (d, J = 12.8 Hz, 1H), 4.73 (m, 2H), 5.10 (m, 1H), 6.78-6.82 (m, 1H), 7.01-7.04 (m, 3H), 7.32 (dd, J = 1.6-7.7 Hz, 1H), 7.41 (t, J = 7.8 Hz, 1H), 7.68 (dd, J = 1.6-8.0 Hz, 1H), 7.76 (s, 1H), 8.54 (s, 1H).

### composé 213: 5-(2,3-dichlorophenyl)-3-methyl-1-[2-oxo-2-[4-(2-oxo-4,5-dihydro-1H-pyrido[2,3-d][1,3]diazepin-3-yl)-1-piperidyl]ethyl]pyrimidine-2,4-dione

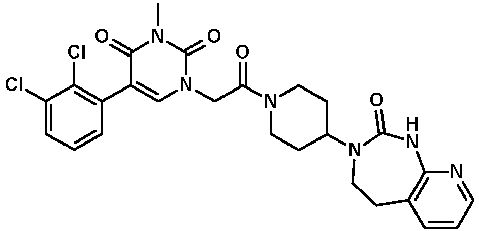

RMN ¹H (δ, DMSO) : 1.56 (m, 1H), 1.71 (m, 3H), 2.69 (m, 1H), 2.84-2.93 (m, 2H), 3.16 (m, 1H), 3.25 (s, 3H), 3.39-3.46 (m, 2H), 3.95 (d, J = 14.2 Hz, 1H), 4.33 (m, 1H), 4.43 (d, J = 12.4 Hz, 1H), 4.78 (s, 2H), 7.06 (m, 1H), 7.32 (dd, J = 7.6, 1.6 Hz, 1H), 7.43 (t, J = 7.9 Hz, 1H), 7.50 (dd, J = 7.6, 1.7 Hz, 1H), 7.68 (m, 1H), 7.82 (s, 1H), 8.18 (dd, J = 5.2, 1.7 Hz, 1H), 8.91 (s, 1H).

### composé 215: 5-(2,3-dichlorophenyl)-3-methyl-1-[2-oxo-2-[4-(2-oxo-1H-quinolin-3-yl)-1-piperidyl]ethyl]pyrimidine-2,4-dione

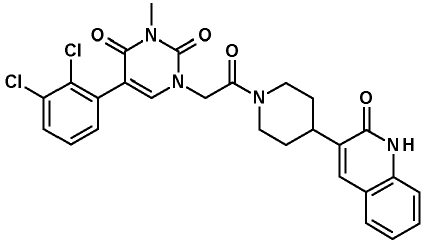

RMN ¹H (δ, DMSO) : 1.36 - 1.66 (m, 2H), 1.91 (m, 2H), 2.66-2.82 (m, 1H), 3.06 (m, 1H), 3.15-3.22 (m, 1H), 3.24 (s, 3H), 4.00 (d, J = 13.1 Hz, 1H), 4.49 (d, J = 13.0 Hz, 1H), 4.79 (s, 2H), 7.10 - 7.19 (m, 1H), 7.28 (d, J = 8.1 Hz, 1H), 7.32 (dd, J = 7.7, 1.6 Hz, 1H), 7.41 (d, J = 7.9 Hz, 1H), 7.43 - 7.48 (m, 1H), 7.63 (dd, J = 8.0, 1.4 Hz, 1H), 7.66 - 7.73 (m, 2H), 7.86 (s, 1H), 11.80 (s, 1H).

### composé 220: 5-(3,4-dichlorophenyl)-3-methyl-1-[2-oxo-2-[4-(2-oxo-4,5-dihydro-1H-1,3-benzodiazepin-3-yl)-1-piperidyl]ethyl]pyrimidine-2,4-dione

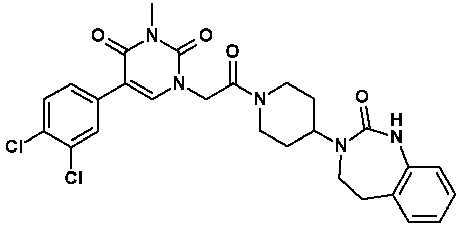

RMN ¹H (δ, DMSO) : 1.39-1.56 (m, 1H), 1.56-1.75 (m, 3H), 2.62 (m, 1H), 2.79-2.88 (m, 2H), 3.03-3.16 (m, 1H), 3.19 (s, 3H), 3.31 (m, 2H), 3.90 (m, 1H), 4.17-4.32 (m, 1H), 4.35 (d, J = 13.6 Hz, 1H), 4.74 (m, 2H), 6.67-6.83 (m, 1H), 6.90-7.06 (m, 3H), 7.54 (dd, J = 8.5, 2.0 Hz, 1H), 7.62 (d, J = 8.8 Hz, 1H), 7.81 (d, J = 2.0 Hz, 1H), 8.05 (s, 1H), 8.46 (s, 1H).

### composé 221: 1-[2-[4-(7-bromo-2-oxo-4,5-dihydro-1H-1,3-benzodiazepin-3-yl)-1-piperidyl]-2-oxo-ethyl]-5-(2-chloro-3-fluoro-phenyl)-3-isopropyl-pyrimidine-2,4-dione

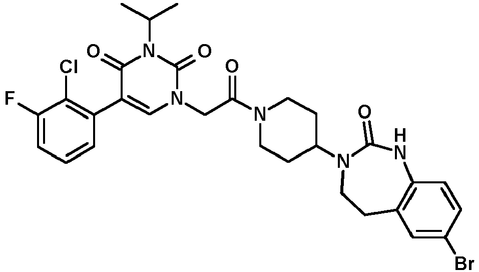

RMN ¹H (δ, DMSO) : 1.41 (d, J = 6.8 Hz, 6H), 1.55 (m, 1H), 1.61-1.82 (m, 3H), 2.65 (m, 1H), 2.84 - 2.94 (m, 2H), 3.15 (m, 1H), 3.34-3.42 (m, 2H), 3.84-4.03 (m, 1H), 4.32 (m, 1H), 4.38-4.51 (m, 1H), 4.64 - 4.84 (m, 2H), 5.11 (hept, J = 7.0 Hz, 1H), 7.01 (d, J = 8.7 Hz, 1H), 7.16-7.30 (m, 3H), 7.35-7.52 (m, 2H), 7.77 (s, 1H), 8.71 (s, 1H).

### composé 225: 5-(2-chloro-3-fluoro-phenyl)-3-isopropyl-1-[2-[4-(7-methylsulfinyl-2-oxo-4,5-dihydro-1H-1,3-benzodiazepin-3-yl)-1-piperidyl]-2-oxo-ethyl]pyrimidine-2,4-dione

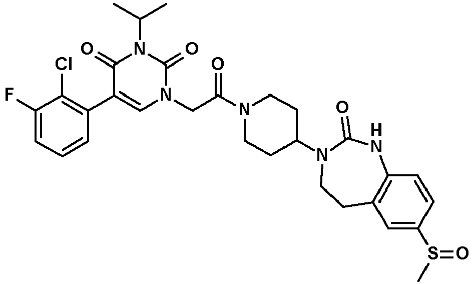

RMN ¹H (δ, DMSO) : 1.41 (d, J = 6.9 Hz, 6H), 1.48-1.63 (m, 1H), 1.63-1.86 (m, 3H), 2.67 (m, 4H), 2.97 (m, 2H), 3.15 (m, 1H), 3.36-3.49 (m, 2H), 3.94 (d, J = 13.5 Hz, 1H), 4.35 (m, 1H), 4.44 (d, J = 13.5 Hz, 1H), 4.75 (m, 2H), 5.11 (hept, J = 6.8 Hz, 1H), 7.18-7.26 (m, 2H), 7.36 (m, 2H), 7.40 - 7.49 (m, 2H), 7.77 (s, 1H), 8.90 (s, 1H).

### composé 226: 2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]acetamide

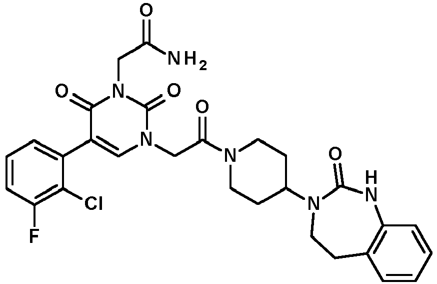

RMN ¹H (δ, DMSO) : 1.55 (m, 1H), 1.71 (m, 3H), 2.69 (m, 1H), 2.83-2.95 (m, 2H), 3.08-3.23 (m, 1H), 3.34 - 3.44 (m, 2H), 3.96 (d, J = 13.7 Hz, 1H), 4.33 (m, 1H), 4.44 (m, 3H), 4.79 (s, 2H), 6.72-6.91 (m, 1H), 6.99-7.07 (m, 3H), 7.11 (br-s, 1H), 7.19 - 7.29 (m, 1H), 7.40-7.52 (m, 2H), 7.56 (br-s, 1H), 7.87 (s, 1H), 8.52 (s, 1H).

### composé 227: 5-(2-chloro-3-fluoro-phenyl)-3-(2-methylsulfinylethyl)-1-[2-oxo-2-[4-(2-oxo-4,5-dihydro-1H-1,3-benzodiazepin-3-yl)-1-piperidyl]ethyl]pyrimidine-2,4-dione

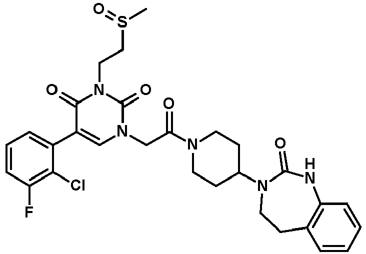

RMN ¹H (δ, DMSO) : 1.47-1.62 (m, 1H), 1.69 (m, 3H), 2.61 (s, 3H), 2.69 (m, 1H), 2.86-2.93 (m, 2H), 2.95 (m, 1H), 3.06 (m, 1H), 3.16 (m, 1H), 3.34-3.47 (m, 2H), 3.95 (d, J = 13.6 Hz, 1H), 4.23 (m, 3H), 4.43 (d, J = 12.7 Hz, 1H), 4.80 (s, 2H), 6.73-6.89 (m, 1H), 6.96-7.10 (m, 3H), 7.16-7.29 (m, 1H), 7.38-7.54 (m, 2H), 7.87 (s, 1H), 8.53 (s, 1H).

### composé 228: 5-(2-chloro-3-fluoro-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione

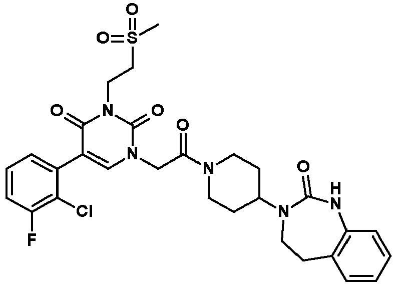

RMN ¹H (δ, DMSO) : 1.55 (m, 1H), 1.71 (m, 3H); 2.69 (m, 1H); 2.90 (m, 2H); 3.09 (s, 3H); 3.16 (m, 1H); 3.36-3.43 (m, 4H); 3.96 (d, J = 13.6 Hz, 1H); 4.29-4.33 (m, 3H); 4.43 (d, J = 12.9 Hz, 1H); 4.80 (s, 2H); 6.79-6.83 (m, 1H); 7.02-7.05 (m, 3H); 7.21-7.23 (m, 1H); 7.45-7.49 (m, 2H); 7.88 (s, 1H); 8.55 (s, 1H).

### composé 229: 5-(2-chloro-3-fluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

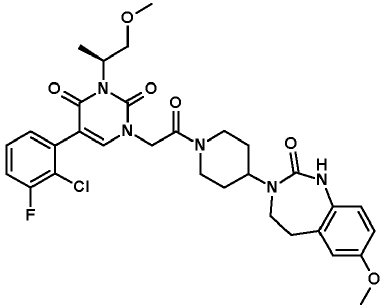

RMN ¹H (δ, DMSO) : 1.33 (dd, J = 7.0, 1.8 Hz, 3H); 1.55 (m, 1H), 1.70 (m, 3H), 2.68 (m, 1H); 2.88 (m, 2H); 3.14 (m, 1H); 3.24 (s, 3H); 3.35-3.37 (m, 2H); 3.52-3.56 (m, 1H); 3.67 (s, 3H); 3.92 (m, 2H); 4.28 (m, 1H); 4.43 (d, J = 12.8 Hz, 1H); 4.75 (m, 2H); 5.15 (m, 1H); 6.62 (d, J = 2.9 Hz, 1H), 6.67 (dd, J = 8.8, 3.0 Hz, 1H), 6.98 (d, J = 8.8 Hz, 1H); 7.20-7.22 (m, 1H); 7.43-7.47 (m, 2H); 7.80 (s, 1H); 8.33 (s, 1H).

### composé 231: 5-(2,3-difluorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione

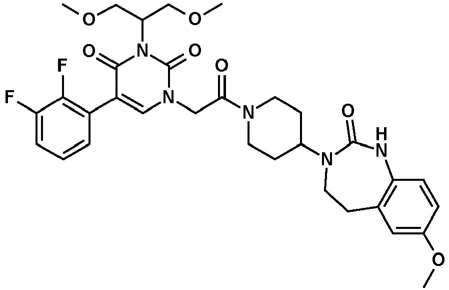

RMN ¹H (δ, DMSO) : 1.44-1.61 (m, 1H), 1.70 (m, 3H), 2.68 (m, 1H), 2.87 (m, 2H), 3.14 (m, 1H), 3.24 (s, 6H), 3.35-3.44 (m, 2H), 3.60 (m, 2H), 3.67 (s, 3H), 3.77-3.90 (m, 2H), 3.94 (d, J = 13.8 Hz, 1H), 4.28 (m, 1H), 4.43 (d, J = 13.2 Hz, 1H), 4.78 (m, 2H), 5.29 (br-s, 1H), 6.63 (d, J = 3.0 Hz, 1H), 6.67 (dd, J = 8.8, 3.0 Hz, 1H), 6.98 (d, J = 8.8 Hz, 1H), 7.12-7.34 (m, 2H), 7.39-7.54 (m, 1H), 7.90 (s, 1H), 8.33 (s, 1H).

### composé 232: 5-(2-chloro-3-fluoro-phenyl)-3-[2-méthoxy-1-(méthoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione

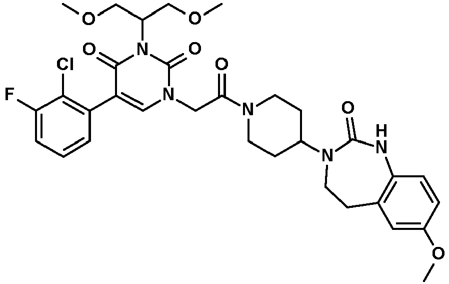

RMN ¹H (δ, DMSO) : 1.55 (m, 1 H); 1. 68 (m, 3 H); 2.68 (m, 1 H); 2.88 (m, 2 H); 3.14 (m, 1 H); 3.24 (s, 6H), 3.37 (m, 2 H); 3.60 (m, 2 H); 3.67 (s, 3 H); 3.86 (m, 2 H); 3.94 (d, J = 13.5 Hz, 1 H); 4.28 (m, 1 H); 4.43 (d, J = 12.9 Hz, 1 H); 4.78 (m, 2 H); 5.30 (br-s, 1 H); 6.62 (d, J = 3.0 Hz, 1H); 6.67 (dd, J = 8.8, 3.0 Hz, 1H), 6.98 (d, J = 8.8 Hz, 1 H); 7.21-7.23 (m, 1 H); 7.43-7.46 (m, 2 H); 7.82 (s, 1 H); 8.33 (s, 1 H).

### composé 233: 5-(2-chloro-3-fluoro-phenyl)-3-[(1S)-2-methoxy-1-methyl-ethyl]-1-[2-[4-(7-methylsulfanyl-2-oxo-4,5-dihydro-1H-1,3-benzodiazepin-3-yl)-1-piperidyl]-2-oxo-ethyl]pyrimidine-2,4-dione

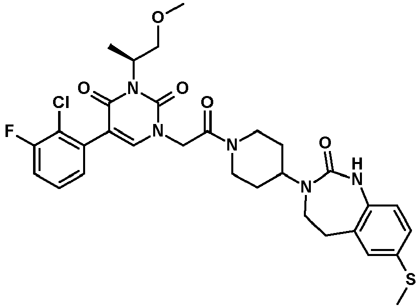

RMN ¹H (δ, DMSO) : 1.33 (m, 3H), 1.55 (m, 1H), 1.70 (m, 3H), 2.40 (s, 3H), 2.68 (m, 1H), 2.82-2.93 (m, 2H), 3.14 (m, 1H), 3.23 (s, 3H), 3.34-3.42 (m, 2H), 3.54 (m, 1H), 3.82-3.99 (m, 2H), 4.31 (m, 1H), 4.43 (d, J = 12.7 Hz, 1H), 4.75 (m, 2H), 5.14 (m, 1H), 7.01 (m, 3H), 7.21 (m, 1H), 7.36-7.55 (m, 2H), 7.79 (s, 1H), 8.57 (s, 1H).

### composé 234: 5-(2-chloro-3-fluoro-phenyl)-3-(2-hydroxy-2-methyl-propyl)-1-[2-oxo-2-[4-(2-oxo-4,5-dihydro-1H-1,3-benzodiazepin-3-yl)-1-piperidyl]ethyl]pyrimidine-2,4-dione

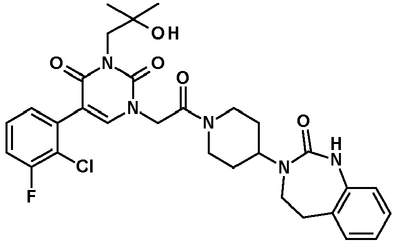

RMN ¹H (δ, DMSO) : 1.10 (s, 6H), 1.55 (m, 1H), 1.68 (m, 3H), 2.69 (m, 1H), 2.83-2.96 (m, 2H), 3.15 (m, 1H), 3.35-3.42 (m, 2H), 3.96 (m, 3H), 4.33 (m, 1H), 4.44 (m, 2H), 4.78 (m, 2H), 6.69 - 6.89 (m, 1H), 6.96-7.10 (m, 3H), 7.15-7.26 (m, 1H), 7.38-7.54 (m, 2H), 7.83 (s, 1H), 8.53 (s, 1H).

### composé 235: 5-(2-chloro-3-fluoro-phenyl)-1-[2-oxo-2-[4-(2-oxo-4,5-dihydro-1H-1,3-benzodiazepin-3-yl)-1-piperidyl]ethyl]-3-(2H-tetrazol-5-ylmethyl)pyrimidine-2,4-dione

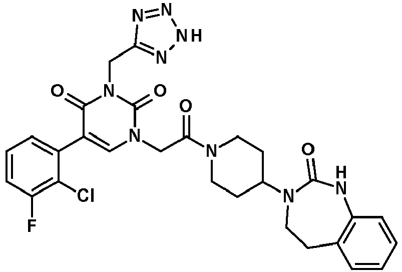

RMN ¹H (δ, DMSO) : 1.55 (m, 1H), 1.68 (m, 3H), 2.70 (m, 1H), 2.89 (m, 2H), 3.18 (m, 2H), 3.37 (m, 2H), 3.95 (d, J = 13.9 Hz, 1H), 4.33 (m, 1H), 4.43 (d, J = 13.1 Hz, 1H), 4.81 (s, 2H), 5.36 (m, 2H), 6.81 (m, 1H), 7.04 (m, 3H), 7.17-7.31 (m, 1H), 7.37-7.56 (m, 2H), 7.94 (s, 1H), 8.53 (s, 1H).

### composé 236: 5-(2,3-difluorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione

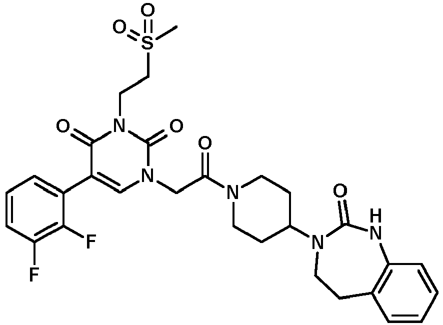

RMN ¹H (δ, DMSO) : 1.55 (m, 1H), 1.71 (m, 3H); 2.70 (m, 1H); 2.90 (m, 2H); 3.08 (s, 3H); 3.16 (m, 1H); 3.36-3.43 (m, 4H); 3.97 (d, J = 13.5 Hz, 1H); 4.32 (m, 3H); 4.43 (d, J = 12.9 Hz, 1H); 4.82 (s, 2H); 6.79-6.83 (m, 1H); 7.02-7.05 (m, 3H); 7.21-7.30 (m, 2H); 7.44-7.51 (m, 1H); 7.97 (s, 1H); 8.54 (s, 1H).

### composé 237: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1S)-1-methylpropyl]pyrimidine-2,4-dione

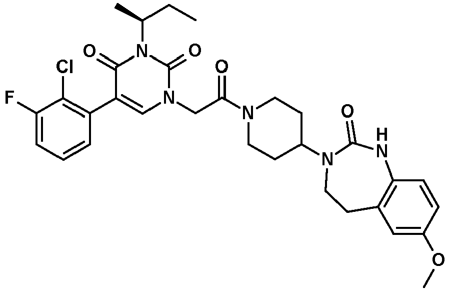

RMN ¹H (δ, DMSO) : 0. 80 (t, J = 7.4 Hz, 3H), 1.38 (d, J = 6.8 Hz, 3H), 1.54 (m, 1H), 1.70 (m, 3H), 2.00 - 2.14 (m, 1H), 2.53 (m, 1H), 2.67 (m, 1H), 2.88 (m, 2H), 3.14 (m, 1H), 3.36 (m, 2H), 3.67 (s, 3H), 3.93 (d, J = 13.4 Hz, 1H), 4.28 (m, 1H), 4.43 (d, J = 13.0 Hz, 1H), 4.65-4.84 (m, 2H), 4.89 (m, 1H), 6.62 (d, J = 3.0 Hz, 1H), 6.67 (dd, J = 8.8, 3.0 Hz, 1H), 6.98 (d, J = 8.8 Hz, 1H), 7.14-7.28 (m, 1H), 7.36-7.52 (m, 2H), 7.79 (s, 1H), 8.34 (s, 1H).

### composé 238: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1R)-1-methylpropyl]pyrimidine-2,4-dione

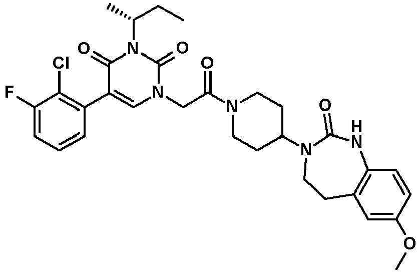

RMN ¹H (δ, DMSO) : 0. 80 (t, J = 7.4 Hz, 3H), 1.39 (d, J = 6.8 Hz, 3H), 1.54 (m, 1H), 1.67 (m, 3H), 2.05 (m, 1H), 2.67 (m, 1H), 2.88 (m, 2H), 3.14 (m, 1H), 3.36 (m, 3H), 3.67 (s, 3H), 3.93 (d, J = 13.6 Hz, 1H), 4.28 (m, 1H), 4.43 (d, J = 12.9 Hz, 1H), 4.65-4.83 (m, 2H), 4.88 (m, 1H), 6.62 (d, J = 3.0 Hz, 1H), 6.67 (dd, J = 8.7, 3.0 Hz, 1H), 6.98 (d, J = 8.8 Hz, 1H), 7.21 (m, 1H), 7.37-7.55 (m, 2H), 7.79 (s, 1H), 8.33 (s, 1H).

### composé 239: 5-(2-chloro-3-fluoro-phenyl)-3-[1-(methoxymethyl)propyl]-1-[2-[4-(7-methoxy-2-oxo-4,5-dihydro-1H-1,3-benzodiazepin-3-yl)-1-piperidyl]-2-oxo-ethyl]pyrimidine-2,4-dione

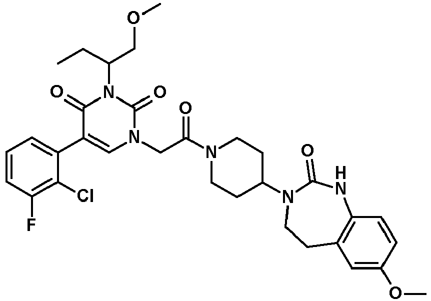

RMN ¹H (δ, DMSO) : 0.82 (t, J = 7.4 Hz, 3H); 1.55 (m, 1H); 1.65-1.75 (m, 3H); 1.97 (m, 1H); 2.53 (m, 2H), 2.67 (m, 1H); 2.88 (m, 2H); 3.14 (m, 1H); 3.23 (s, 3H); 3.36-3.36 (m, 2H); 3.56 (m, 1H); 3.67 (s, 3H); 3.92 (m, 2H); 4.28 (m, 1H); 4.43 (d, J = 12.8 Hz, 1H); 4.76 (br s, 1H); 5.02 (br s, 1H); 6.62 (d, J = 2.9 Hz, 1H); 6.67 (dd, J = 8.8, 3.0 Hz, 1H), 6.98 (d, J = 8.8 Hz, 1H); 7.21-7.23 (m, 1H); 7.43-7.46 (m, 2H); 7.81 (s, 1H); 8.33 (s, 1H).

### composé 240: 5-(2-chloro-3-fluoro-phenyl)-3-(2-methoxy-2-methyl-propyl)-1-[2-[4-(7-methoxy-2-oxo-4,5-dihydro-1H-1,3-benzodiazepin-3-yl)-1-piperidyl]-2-oxo-ethyl]pyrimidine-2,4-dione

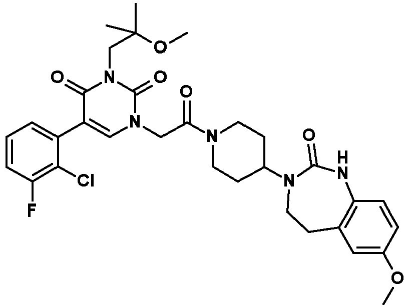

RMN ¹H (δ, DMSO) : 1.12 (s, 6H); 1.55 (m, 1H); 1.68 (m, 3H); 2.68 (m, 1H); 2.88 (m, 2H); 3.16 (m, 4H); 3.36 (m, 2H); 3.67 (s, 3H); 3.94 (d, J = 13.9 Hz, 1H); 4.01 (m, 2H); 4.28 (m, 1H); 4.43 (d, J = 12.8 Hz, 1H); 4.77 (m, 2H); 6.62 (d, J = 2.9 Hz, 1H); 6.67 (dd, J = 8.8, 2.9 Hz, 1H); 6.98 (d, J = 8.8 Hz, 1H); 7.19-7.21 (m, 1H); 7.44-7.47 (m, 2H); 7.82 (s, 1H); 8.33 (s, 1H).

### composé 241: 5-(2-chloro-3-methoxy-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione

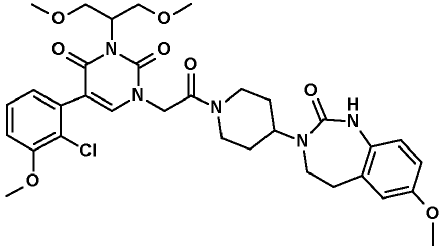

RMN ¹H (δ, DMSO) : 1.44-1.62 (m, 1H), 1.67 (m, 3H), 2.66 (m, 1H), 2.83-2.92 (m, 2H), 3.13 (m, 1H), 3.24 (s, 6H), 3.36 (m, 2H), 3.60 (m, 2H), 3.67 (s, 3H), 3.83 (m, 2H), 3.89 (s, 3H), 3.93 (d, J = 13.4 Hz, 1H), 4.28 (m, 1H), 4.43 (d, J = 12.8 Hz, 1H), 4.74 (m, 2H), 5.26 (br-s, 1H), 6.62 (d, J = 3.0 Hz, 1H), 6.67 (dd, J = 8.9, 3.0 Hz, 1H), 6.91 (dd, J = 7.5, 1.5 Hz, 1H), 6.98 (d, J = 8.7 Hz, 1H), 7.19 (dd, J = 8.5, 1.5 Hz, 1H), 7.31-7.38 (m, 1H), 7.70 (s, 1H), 8.32 (s, 1H).

### composé 242: 5-(2,3-dichlorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione

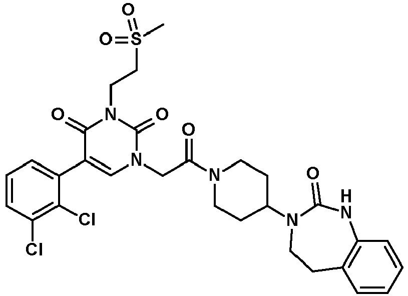

RMN ¹H (δ, DMSO) : 1.55 (m, 1H), 1.72 (m, 3H); 2.69 (m, 1H); 2.90 (m, 2H); 3.08 (s, 3H); 3.20 (m, 1H); 3.43-3.39 (m, 4H); 3.95 (d, J = 11.9 Hz, 1H) 4.31 (m, 3H); 4.44 (d, J = 11.9 Hz, 1H); 4.79 (s, 2H); 6.83-6.79 (m, 1H); 7.04 (m, 3H); 7.33 (dd, J = 7.7, 1.6 Hz, 1H); 7.44 (t, J = 7.9 Hz, 1H); 7.70 (dd, J = 8.1, 1.6 Hz, 1H); 7.87 (s, 1H); 8.53 (s, 1H).

### composé 243: 5-(2-chloro-3-methoxy-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione

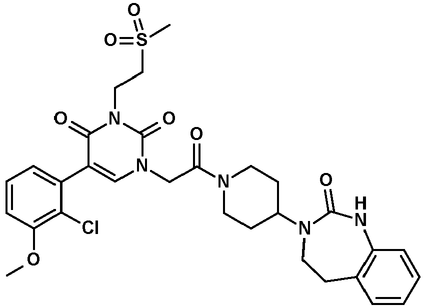

RMN ¹H (δ, DMSO) : 1.55 (m, 1H), 1.71 (m, 3H); 2.53 (m, 2H), 2.68 (m, 1H); 2.90 (m, 2H); 3.08 (s, 3H); 3.15 (m, 1H); 3.38 (m, 2H), 3.90 (s, 3H); 3.95 (d, J = 13.6 Hz, 1H); 4.28-4.32 (m, 3H); 4.43 (d, J = 13.0 Hz, 1H); 4.79 (s, 2H); 6.79-6.83 (m, 1H); 6.92 (dd, J = 7.6, 1.4 Hz, 1H); 7.02-7.05 (m, 3H); 7.19-7.21 (m, 1H); 7.36 (t, J = 8.0 Hz, 1H); 7.78 (s, 1H); 8.53 (s, 1H).

### composé 244: 5-(2-chloro-3-fluoro-phenyl)-3-[(1S)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione

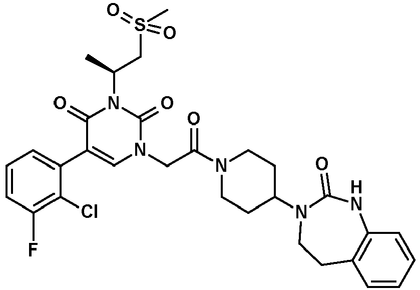

RMN ¹H (δ, DMSO) : 1.24 (d, J = 7.0 Hz, 3H), 1.57 (m, 1H), 1.69 (m, 3H), 2.70 (s, 1H), 2.84-2.95 (m, 2H), 3.06 (s, 3H), 3.16 (m, 1H), 3.35-3.43 (m, 2H), 3.53 (m, 1H), 3.95 (d, J = 13.6 Hz, 1H), 4.11-4.39 (m, 3H), 4.43 (d, J = 12.4 Hz, 1H), 4.80 (s, 2H), 6.81 (m, 1H), 7.04 (m, 3H), 7.23 (m, 1H), 7.38-7.54 (m, 2H), 7.90 (s, 1H), 8.52 (s, 1H).

### composé 245: 5-(2,3-dichlorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione

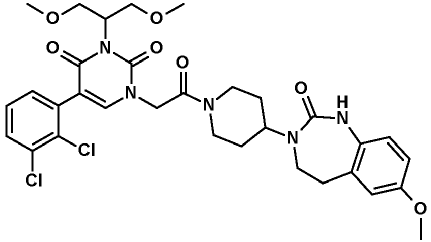

RMN ¹H (δ, DMSO) : 1.50-1.58 (m, 1H); 1.65-1.77 (m, 3H); 2.64-2.70 (m, 1H); 2.85-2.87 (m, 2H); 3.10-3.16 (m, 1H); 3.23 (s, 6H); 3.34-3.36 (m, 2H); 3.59 (m, 2H); 3.66 (s, 3H); 3.80-3.85 (m, 2H); 3.91-3.94 (m, 1H); 4.24-4.30 (m, 1H); 4.40-4.43 (m, 1H); 4.74 (m, 2H); 5.26 (br s, 1H); 6.61 (d, J = 2.9 Hz, 1H); 6.66 (dd, J = 8.8, 2.9 Hz, 1H); 6.97 (d, J = 8.8 Hz, 1H); 7.33 (dd, J = 7.7, 1.6 Hz, 1H); 7.42 (t, J = 7.9 Hz, 1H); 7.68 (dd, J = 8.1, 1.6 Hz, 1H); 7.79 (s, 1H); 8.31 (s, 1H).

### composé 246: acide 3-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propanoique

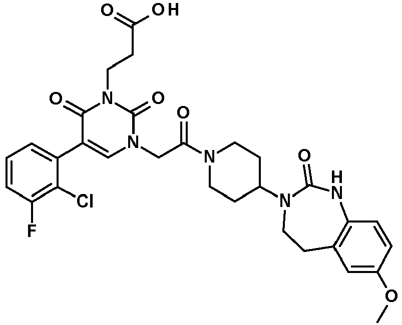

RMN ¹H (δ, DMSO) : 1.55 (m, 1H), 1.70 (m, 3H), 2.50 (m, 4H), 2.68 (m, 1H), 2.80-2.95 (m, 2H), 3.15 (m, 1H), 3.67 (s, 3H), 3.94 (d, J = 13.6 Hz, 1H), 4.02-4.17 (m, 2H), 4.28 (m, 1H), 4.43 (d, J = 12.8 Hz, 1H), 4.78 (s, 2H), 6.62 (d, J = 3.0 Hz, 1H), 6.67 (dd, J = 8.8, 3.0 Hz, 1H), 6.98 (d, J = 8.8 Hz, 1H), 7.10 - 7.30 (m, 1H), 7.37-7.54 (m, 2H), 7.84 (s, 1H), 8.32 (s, 1H), 12.45 (br-s, 1H).

### composé 247: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,3,4-oxadiazol-2-yl)ethyl]pyrimidine-2,4-dione

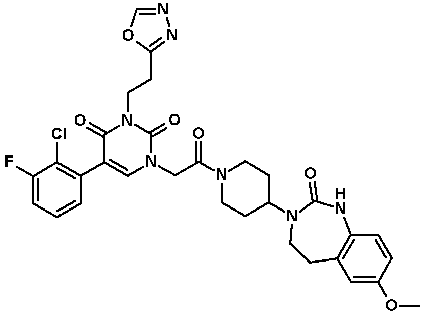

RMN ¹H (δ, DMSO) : 1.54 (m, 1H); 1.68 (m, 3H); 2.68 (m, 1H); 2.88 (m, 2H); 3.19 (m, 3H); 3.36 (m, 2H); 3.67 (s, 3H); 3.94 (d, J = 13.5 Hz, 1H); 4.25 (m, 3H); 4.43 (d, J = 12.8 Hz, 1H); 4.77 (s, 2H); 6.63 (d, J = 2.9 Hz, 1H); 6.67 (dd, J = 8.8, 2.9 Hz, 1H); 6.98 (d, J = 8.8 Hz, 1H); 7.18 (m, 1H); 7.46 (m, 2H); 7.86 (s, 1H); 8.33 (s, 1H); 9.13 (s, 1H).

### composé 248: acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]acetique

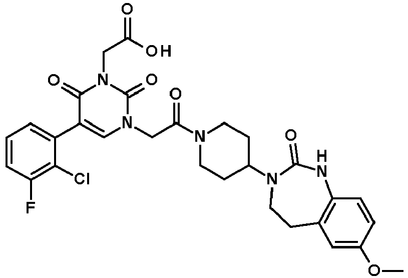

RMN ¹H (δ, DMSO) : 1.49-1.58 (m, 1H); 1.61-1.74 (m, 3H); 2.68 (m, 1H); 2.87-2.89 (m, 2H); 3.12-3.18 (m, 1H); 3.36 (m, 2H), 3.67 (s, 3H); 3.95 (d, J = 13.6 Hz, 1H); 4.25-4.31 (m, 1H); 4.43 (d, J = 12.9 Hz, 1H); 4.52 (m, 2H); 4.81 (s, 2H); 6.62 (d, J = 2.9 Hz, 1H); 6.67 (dd, J = 8.8, 2.9 Hz, 1H); 6.98 (d, J = 8.8 Hz, 1H); 7.22-7.24 (m, 1H); 7.44-7.48 (m, 2H); 7.90 (s, 1H); 8.32 (s, 1H); 13.05 (br-s, 1H).

### composé 249: 5-(2-chloro-3-fluoro-phenyl)-3-[(1R)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione

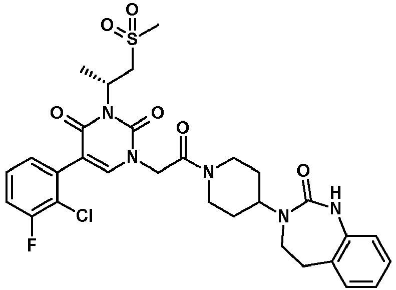

RMN ¹H (δ, DMSO) : 1.24 (d; J = 7.03 Hz; 3 H); 1.53-1.56 (m; 1 H); 1.66-1.72 (m; 3 H); 2.69 (m; 1 H); 2.89-2.91 (m; 2 H); 3.06 (s; 3 H); 3.16 (m; 1 H); 3.38 (m; 2 H); 3.53 (m; 1 H); 3.95 (d; J = 13.62 Hz; 1 H); 4.17-4.24 (m; 1 H); 4.27-4.32 (m; 2 H); 4.43 (d; J = 12.93 Hz; 1 H); 4.81 (m; 2 H); 6.81 (m; 1 H); 7.04 (m; 3 H); 7.22-7.24 (m; 1 H); 7.45-7.49 (m; 2 H); 7.90 (s; 1 H); 8.53 (s; 1 H).

### composé 251: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,2,4-triazol-4-yl)ethyl]pyrimidine-2,4-dione

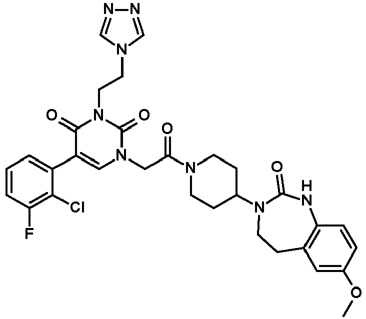

### Masse = 651

### composé 252: 5-(2-chloro-3-fluoro-phenyl)-1-[2-[4-(7-fluoro-2-oxo-4,5-dihydro-1H-1,3-benzodiazepin-3-yl)-1-piperidyl]-2-oxo-ethyl]-3-[(1R)-1-methyl-2-methylsulfonyl-ethyl]pyrimidine-2,4-dione

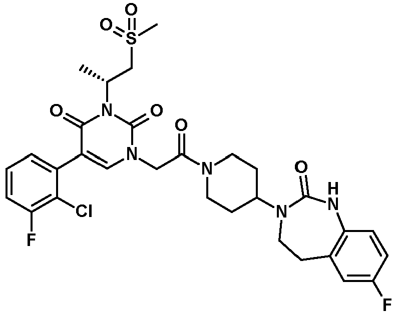

RMN ¹H (δ, DMSO) : 1.14-1.36 (m, 5H), 1.55 (m, 1H), 1.71 (m, 3H), 2.69 (m, 1H), 2.86-2.93 (m, 2H), 3.06 (s, 3H), 3.15 (m, 1H), 3.34-3.41 (m, 2H), 3.53 (m, 1H), 3.94 (d, J = 13.6 Hz, 1H), 4.15-4.33 (m, 3H), 4.43 (d, J = 13.0 Hz, 1H), 4.80 (m, 2H), 6.87-6.96 (m, 2H), 7.02-7.11 (m, 1H), 7.19-7.27 (m, 1H), 7.43-7.51 (m, 2H), 7.89 (s, 1H), 8.55 (s, 1H).

### composé 253: acide 3-[1-[2-[5-(2-chloro-3-fluoro-phenyl)-3-(2-methylsulfonylethyl)-2,4-dioxo-pyrimidin-1-yl]acetyl]-4-piperidyl]-2-oxo-4,5-dihydro-1H-1,3-benzodiazepine-7-carboxylique

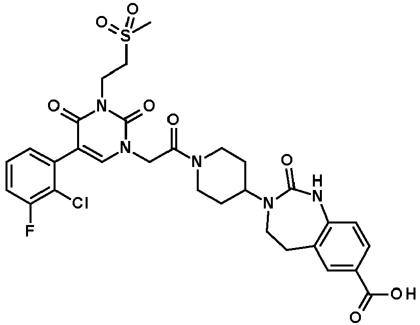

RMN ¹H (δ, DMSO) : 1.56 (m, 1H), 1.69 (m, 3H), 2.71 (m, 1H), 2.95 (m, 2H), 3.08 (s, 3H), 3.16 (m, 1H), 3.36-3.48 (m, 4H), 3.96 (d, J = 13.4 Hz, 1H), 4.31 (m, 2H), 4.35-4.51 (m, 2H), 4.80 (m, 2H), 7.11 (d, J = 8.5 Hz, 1H), 7.17 - 7.27 (m, 1H), 7.40-7.54 (m, 2H), 7.61 (dd, J = 8.5, 2.0 Hz, 1H), 7.64 (d, J = 2.0 Hz, 1H), 7.89 (s, 1H), 9.01 (s, 1H), 12.35 (br-s, 1H).

### composé 254: S-[2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethyl] ethanethioate

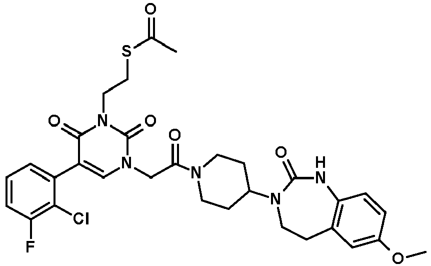

RMN ¹H (δ, DMSO) : 1.56 (m, 1H), 1.68 (m, 3H), 2.33 (m, 4H), 2.81-2.94 (m, 2H), 3.12 (m, 3H), 3.34-3.40 (m, 2H), 3.67 (s, 3H), 3.95 (d, J = 14.0 Hz, 1H), 4.07 (m, 2H), 4.28 (m, 1H), 4.43 (d, J = 12.7 Hz, 1H), 4.78 (s, 2H), 6.63 (d, J = 2.9 Hz, 1H), 6.67 (dd, J = 8.9, 2.9 Hz, 1H), 6.98 (d, J = 8.7 Hz, 1H), 7.17-7.29 (m, 1H), 7.37-7.55 (m, 2H), 7.84 (s, 1H), 8.33 (s, 1H).

### composé 255: N-[(2S)-2-[5-(2,3-dichlorophenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide

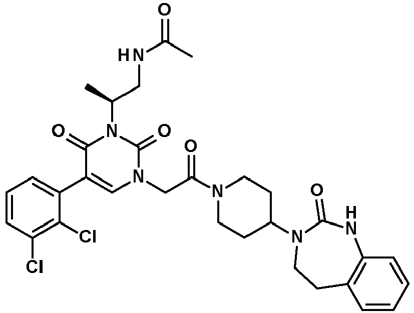

RMN ¹H (δ, DMSO) : 1.34 (3 H, d, J = 6.91 Hz), 1.57 (1 H, m), 1.69 (3 H, m), 1.77 (3 H, s), 2.68-2.72 (1 H, m), 2.90 (2 H, m), 3.15 (1 H, m), 3.38 (2 H, m), 3.44-3.57 (2 H, m), 3.95 (1 H, d, J = 13.54 Hz), 4.33 (1 H, m), 4.44 (1 H, d, J = 12.82 Hz), 4.67-4.81 (2 H, m), 4.99 (1 H, m), 6.81 (1 H, m), 7.04 (3 H, m), 7.33 (1 H, m), 7.43 (1 H, t, J = 7.87 Hz), 7.68 (1 H, dd, J = 8.04, 1.58 Hz), 7.78 (1 H, s), 7.93 (1 H, m), 8.52 (1 H, s).

### composé 256: N-[(2S)-2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide

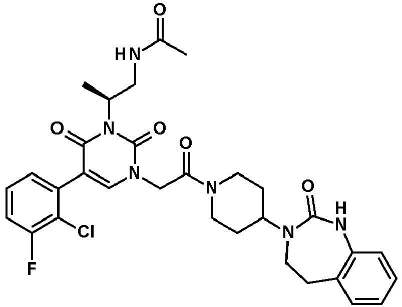

RMN ¹H (δ, DMSO) : 1.35 (d; J = 6.85 Hz; 3 H); 1.57 (m; 1 H); 1.70 (m; 3 H); 1.77 (s; 3 H); 2.69-2.72 (m; 1 H); 2.90 (m; 2 H); 3.16 (m; 1 H); 3.39 (m; 2 H); 3.49-3.57 (m; 2 H); 3.96 (d; J = 13.24 Hz; 1 H); 4.33 (m; 1 H); 4.45 (d; J = 12.80 Hz; 1 H); 4.75 (m; 2 H); 4.99 (m; 1 H); 6.82 (m; 1 H); 7.03-7.05 (m; 3 H); 7.22 (m; 1 H); 7.45 (m; 2 H); 7.79 (s; 1 H); 7.93 (m; 1 H); 8.53 (s; 1 H).

### composé 257: acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethanesulfonique

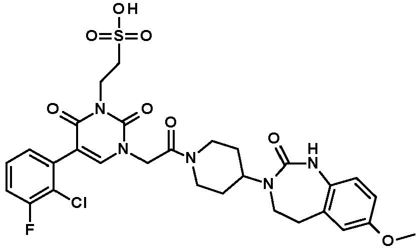

RMN ¹H (δ, DMSO) : 1.56 (m, 1H), 1.62-1.84 (m, 3H), 2.59-2.74 (m, 3H), 2.83-2.95 (m, 2H), 3.06 - 3.16 (m, 1H), 3.18 (s, 2H), 3.28-3.43 (m, 2H), 3.67 (s, 3H), 3.95 (d, J = 13.5 Hz, 1H), 4.06-4.19 (m, 2H), 4.21-4.35 (m, 1H), 4.43 (d, J = 12.8 Hz, 1H), 4.77 (m, 2H), 6.63 (d, J = 3.0 Hz, 1H), 6.66 (dd, J = 8.6, 2.9 Hz, 1H), 6.97 (d, J = 8.8 Hz, 1H), 7.22 (m, 1H), 7.39-7.50 (m, 2H), 7.80 (s, 1H), 8.31 (s, 1H).

### composé 258: 5-(2-chloro-3-fluoro-phenyl)-3-[(1S)-1-(methoxymethyl)-2-methylsulfonyl-ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione

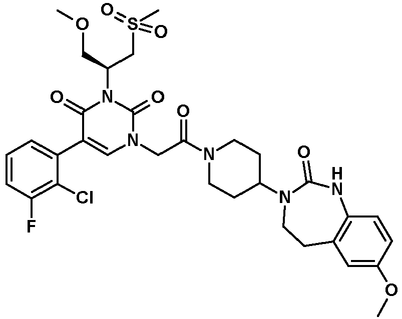

RMN ¹H (δ, DMSO) : 1.54 (m, 1H), 1.71 (m, 3H), 2.65 (m, 2H), 2.87 (m, 2H), 3.07 (s, 3H), 3.15 (m, 1H), 3.26 (s, 3H), 3.36 (m, 2H), 3.55-3.76 (m, 5H), 3.95 (m, 1H), 4.36 (m, 8.2 Hz, 4H), 4.80 (s, 2H), 6.62 (d, J = 3.0 Hz, 1H), 6.67 (dd, J = 8.6, 3. 0 Hz, 1H), 6.98 (d, J = 8.8 Hz, 1H), 7.15 - 7.28 (m, 1H), 7.38-7.53 (m, 2H), 7.89 (s, 1H), 8.32 (s, 1H).

### composé 259: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-(2-methyl-2-methylsulfonyl-propyl)pyrimidine-2,4-dione

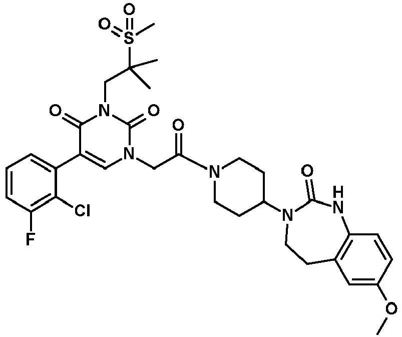

RMN ¹H (δ, DMSO) : 1.30 (s, 6 H); 1.52-1.58 (m, 1 H); 1.64-1.77 (m, 3 H); 2.68 (t, J = 12.5 Hz, 1 H); 2.88 (m, 2 H); 3.06 (s, 3 H); 3.15 (t, J = 13.0 Hz, 1 H) ; 3.36 (m, 2 H); 3.67 (s, 3 H); 3.94 (d, J = 13.5 Hz, 1 H); 4.25-4.35 (m, 3 H); 4.43 (d, J = 13.7 Hz, 1 H); 4.80 (s, 2 H); 6.62 (d, J = 3.0 Hz, 1H), 6.67 (dd, J = 8.9, 3.0 Hz, 1H),
6.98 (d, J = 8.8 Hz, 1 H); 7.22-7.24 (m, 1 H); 7.45-7.48 (m, 2 H); 7.89 (s, 1 H); 8.33 (s, 1 H).

Un autre objet de l'invention concerne les composés selon l'invention pour leur utilisation comme médicament.

Préférentiellement, les composés selon l'invention sont utilisés dans la prévention et/ou le traitement de maladies inflammatoires avec une composante neurogène.

Le terme « prévention » ou « prévenir » les maladies inflammatoires avec une composante neurogène signifie diminuer et/ou éviter l'apparition des symptômes de ces maladies.

Le terme « traitement » ou « traiter » les maladies inflammatoires avec une composante neurogène signifie diminuer et/ou inhiber le développement de ces maladies et/ou de leurs symptômes.

Par « maladies inflammatoires avec une composante neurogène », on entend préférentiellement les maladies inflammatoires de la peau avec une composante neurogène choisies parmi la rosacée de type I (Erythémateuse) et de type II (Papulopustuleuse), la dermatite atopique, l'eczema chronique des mains, le psoriasis sous ses differentes formes (vulgaire, du scalp, arthritique, pustuleux, de goutte), l'érythème facial, l'érythème pudique, l'urticaire (aigue, prurit sénile, prurigo nodularis) et l'acné, ou encore les céphalées avec une inflammation neurogène telles que les migraines.

Les maladies inflammatoires de la peau avec une composante neurogène sont préférentiellement choisies parmi la rosacée de type I (Erythémateuse), la dermatite atopique, le psoriasis vulgaire et du scalp, et l'uticaire du type prurit sénile et prurigo nodularis, encore plus préférentiellement la rosacée de type I (Erythémateuse).

L'intérêt pour les nouveaux composés hétérocycliques antagonistes du récepteur CGRP selon l'invention dans le traitement des maladies inflammatoires avec une composante neurogène est basé sur des éléments cliniques soulignant le parallèle entre notamment la rosacée et la migraine :
- la localisation faciale des lésions chez les patients atteints de rosacée est semblable aux zones d'innervation par le nerf trigéminal également impliqué dans la migraine ;
- la prévalence des migraineux chez les patients atteints de rosacée est démontrée par des études épidémiologiques (≥ 27% vs 13% dans la population générale).

Il repose également sur des données expérimentales :
- l'effet vasodilatateur de CGRP sur les vaisseaux cutanés a été démontré en clinique : l'injection de faibles doses de CGRP en sous cutanée induit un érythème persistant de plusieurs heures ;
- l'application topique de capsaïcine induit la libération de neuropeptides, parmi lesquels CGRP, qui se traduit par une vasodilatation cutanée ;
- l'injection par voie intraveineuse de αCGRP chez la souris provoque l'apparition d'un flush majoritairement mesurable au niveau de la face de l'animal. Chez des souris invalidées pour le gène mRamp1, aucune réponse vasodilatatrice à ce flush au CGRP n'est observée. De même chez ces souris, aucun œdème n'apparait dans un modèle d'inflammation neurogène local induit par l'application topique d'un agoniste TRPV1. Ces résultats semblent confirmer un rôle majeur de la libération de CGRP dans l'inflammation neurogène et dans le flush.

A cet effet, de manière générale, les composés selon l'invention présentent une bonne affinité pour le récepteur CGRP et ainsi une bonne activité antagoniste au récepteur CGRP.

Cette activité est plus particulièrement illustrée par le test de dosage cellulaire GPCR GeneBLAzer^{®} réalisé qui fournit une méthode homogène de suivi de la réponse cellulaire à des médicaments candidats.

Dans le modèle GeneBLAzer^{®} GPCR Cell-based Assay fournit par Invitrogen (Lifetechnologies), l'activation du récepteur CALCLR:RAMP1 conduit à l'expression d'un gène rapporteur (la beta-lactamase). L'activité de la beta-lactamase est quantifiée par FRET (fluorescence resonance energy transfer) en présence d'un substrat fluorescent et est directement proportionnelle à l'activation du récepteur.

Les lignées GeneBLAzer^{®} CALCRL:RAMP CRE-bla HEK293F sont des cellules HEK293F qui surexpriment stablement les récepteurs CALCRL et RAMP1 ainsi que le gène rapporteur de la beta-lactamase sous le contrôle de l'élément de réponse à l'AMPc (CRE) .

Le substrat de la beta-lactamase contient deux fluorophores (fluorescéine et coumarine) associés par un noyau beta-lactame. En l'absence de beta-lactamase et lorsqu'il est excité à λ=409nm, le substrat reste intact et la longueur d'onde d'émission de la fluorescence de la coumarine (À=460nm) excite la fluorescéine qui émet à son tour (λ=530nm). En revanche, lorsque la beta-lactamase est exprimée, le substrat est clivé, séparant les deux fluorophores et interrompant le FRET. L'émission de la coumarine sera alors proportionnelle à l'activation du récepteur.

Les cellules sont ensemencées en microplaques 384 puits à raison de 10 000 cellules par puits et incubées 18 heures à 37°C. Des dilutions sérielles verticales d'agoniste (α-Calcitonin Gene Related Peptide pour RAMP1) sont ajoutées en compétition avec des dilutions sérielles horizontales d'antagonistes à tester. Après 3 heures d'incubation à 37°C, le substrat de la beta-lactamase est ajouté à chaque puits et la microplaque est incubée 2 heures à température ambiante. La fluorescence est alors mesurée à l'aide d'un lecteur de microplaques (λ_{excitation}=409nm, λ_{lémission 1}=460nm, λ_{émission 2}=530nm).

La table suivante détaille les différentes conditions appliquées aux différentes lignées utilisées :

| **Lignée** | **Réf. Invitrogen** | **Espèce** | **Récepteur** | **Agoniste** | **Gamme agoniste (nM)** | **Gamme antagoniste (nM)** |
|---|---|---|---|---|---|---|
| HEK hRAMP1 | K1437 | Humain | CALCRL: RAMP1 | αCGRP (NEOMPS, #SC113) | 0.002-10 | 0.04-10000 |

Les données de fluorescence sont normalisées à l'aide de contrôles négatifs (cellules non stimulées) et de contrôles positifs (agoniste à concentration saturante).

Les constantes de dissociation pour chaque état du récepteur [« Mechanistic analysis of the function of agonists and allosteric modulators: reconciling two-state and operational models » David Roche, British Journal of Pharmacology (2013) 169 1189-1202*]* (KdR = affinité pour l'état repos et KdA = affinité pour l'état activé) sont calculées et le ratio KdR/KdA représente l'efficacité intrinsèque du ligand testé : si KdR=KdA alors le ligand testé est un antagoniste neutre.

Les différentes EC50 de l'agoniste obtenues à chaque concentration d'antagoniste testé sont linéarisées (régression de Schild *[*« Quantitation in receptor pharmacology » Terry P. Kenakin, Receptors and Channels (2001) 7 371-385]) afin de déterminer l'affinité apparente (Kdapp) de l'antagoniste. Cette valeur est nommée apparente car elle peut fluctuer en fonction de l'activation constitutive et de l'expression du récepteur et est assimilable au Kb dans le cas d'un antagoniste neutre.

Les résultats d'affinité ainsi obtenus pour chacun des composés selon l'invention testés sont récapitulés dans le tableau I ci-dessous dans lequel :
Kdapp représente l'affinité du composé pour le récepteur CGRP ;
A représente un Kdapp <10 nM;
B représente un Kdapp entre 10 - 100 nM;
C représente un Kdapp entre 101 - 500 nM; et
D représente un Kdapp entre 501 nM - 5000 nM.

**Tableau I :**

| Kdapp **hCGRP (nM)** | **n° Composé** |
|---|---|
| A (<10 nM) | 14; 16-19; 22-24; 28-31; 47; 51-54; 72-73; 83; 89-90; 93; 95-98; 100-101; 103-106; 108; 110; 112-124; 126-128; 130-132; 134-140; 142-146; 148; 152; 155-162; 165-166; 170; 178; 184-189; 192; 199; 202-203; 205-208; 210; 213; 215; 220-221; 225-229; 231-249; 251-259 |
| B (10 - 100 nM) | 1; 4; 8-9; 11-13; 20-21; 25-27; 33; 35; 37-38; 40; 46; 48; 55-57; 59-63; 66-69; 76; 81; 85-88; 91-92; 94; 99; 102; 109; 111; 129; 133; 147; 149-150; 153; 164; 167; 169; 171-177; 179-183; 190; 193-198; 200-201; 204; 211; 214; 250 |
| C (101 - 500 nM) | 2-3; 6; 10; 34; 41-42; 45; 49; 64-65; 70-71; 78; 84; 141; 154; 168; 216-217; 224; 230 |
| D (501 - 5000 nM) | 5; 7; 15; 32; 36; 39; 43-44; 50; 58; 74-75; 77; 79-80; 82; 107; 151; 163; 191; 209; 218-219; 222-223 |

Considérant les résultats répertoriés dans le tableau ci-dessus, les composés selon l'invention présentant un Kdapp classifié A ont une très forte affinité pour le récepteur au CGRP. De plus, ces composés présentent un ratio KdR/KdA = 1 et donc une activité antagoniste du récepteur au CGRP.

Dans la mesure où l'inflammation neurogène joue un rôle important dans la physiopathologie notamment de la rosacée et de la migraine, les antagonistes au récepteur CGRP selon l'invention permettraient ainsi de réduire ou abolir totalement cette inflammation, avec pour résultat un effet persistant à l'arrêt du traitement.

Outre leur potentiel sur le plan de l'activité biologique en tant qu'antagoniste puissant et sélectif de CGRP-R humain, ces composés, avantageusement les dérivés uraciles, présentent une instabilité métabolique au niveau hépatique et devraient ainsi présenter un risque d'hépatotoxicité limité.

Elle est plus particulièrement illustrée par le test de stabilité métabolique en présence de microsomes hépatiques humains ci-dessous.

Des microsomes hépatiques humains (Becton Dickinson) sont incubés à une concentration de protéine de 0,5 mg/mL dans le milieu réactionnel.

Le milieu réactionnel des microsomes est composé de tampon Phosphate pH : 7,4 à 100 mM, de MgCl₂ à 100 mM. (50/50), d'un système de génération d'ATP composé d'un mélange de Nicotinamide Adénine Di-Phosphate (NADP), de Glucose-6-Phosphate (G6P) à 1 mg/mL et de Glucose-6-Phosphate Deshydrogénase (G6PDH) à 4 U/mL. Les composés sont testés à 1µM (DMSO 0,1%)

Les prélèvements de milieu d'incubation après ajout des microsomes sont réalisés au temps 15 minutes. La réaction métabolique est stoppée par ajout de méthanol (1 volume milieu incubation/3 volumes de méthanol). Le pourcentage du produit parent restant après 15 minutes est mesurée par analyse LC/MS/MS.

Les résultats obtenus sont répertoriés dans le tableau 2 ci-dessous.

**Tableau 2 :**

| n° Composé | % de produit restant à 15min (microsomes hépatiques humains) |
|---|---|
| Olcegepant | 100 |
| Telcagepant | 100 |
| MK-3207 | 100 |
| 25 | 8 |
| 30 | 16 |
| 63 | 45 |
| 110 | 1 |
| 112 | 0 |
| 114 | 41 |
| 115 | 0 |
| 120 | 1 |
| 142 | 65 |
| 143 | 42 |
| 149 | 26 |
| 150 | 0 |
| 158 | 0 |
| 165 | 0 |
| 193 | 18 |
| 206 | 1 |
| 232 | 1 |
| 237 | 3 |
| 240 | 0 |
| 251 | 4 |

D'après les résultats obtenus, les composés de formule (I) selon l'invention présentent une instabilité métabolique au niveau hépatique par rapport aux composés connus de l'art antérieur testés et devraient ainsi présenter un risque d'hépatotoxicité limité.

Les composés selon l'invention peuvent également être formulés dans des compositions pharmaceutiques à usage humain. Lesdites compositions comprennent dans un véhicule pharmaceutiquement acceptable au moins un composé de formule générale (I) selon l'invention.

Par véhicule pharmaceutiquement acceptable, on entend un véhicule adapté pour une utilisation en contact avec des cellules d'humains, compatible avec la peau, les muqueuses et les phanères, sans toxicité, irritation, réponse allergique indue, et proportionné à un rapport avantage/risque raisonnable.

De préférence, le composé de formule générale (I) selon l'invention est présent dans une composition pharmaceutique pour application topique, ce qui réduit considérablement tout effet secondaire au niveau hépatique.

## Revendications

1. Composé hétérocyclique choisi parmi les composés de formule générale (I) : 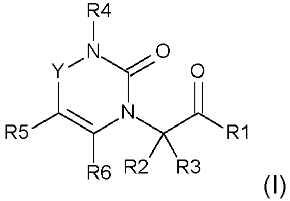 dans laquelle,
- Y est choisi parmi -CH₂, -C(O), -C(CH₃)₂, ou un spirocyclopropyle ;
- R1 est choisi parmi les groupements (1-1) à (1-12) suivants : 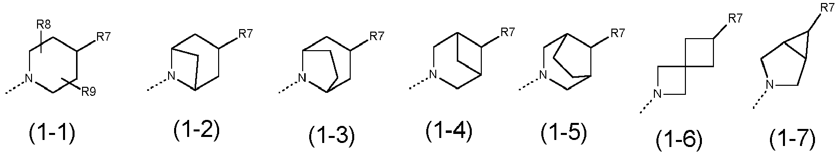 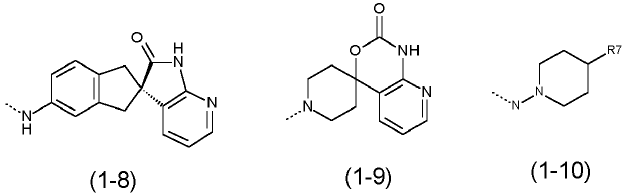 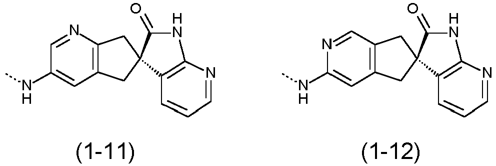
- R2, R3, R6 sont identiques ou différents, et choisis parmi un atome d'hydrogène, F ou -CH₃;
- R4 est choisi parmi un atome d'hydrogène, un alkyle, un alcène, un alcyne, un cycloalkyle, un cycloalcène, un aralkyle, un hétéroaralkyle, ou les groupements (4-1) à (4-55) suivants : 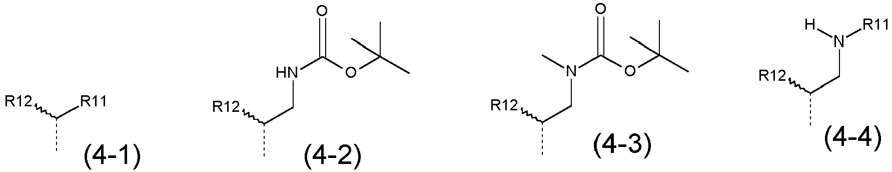 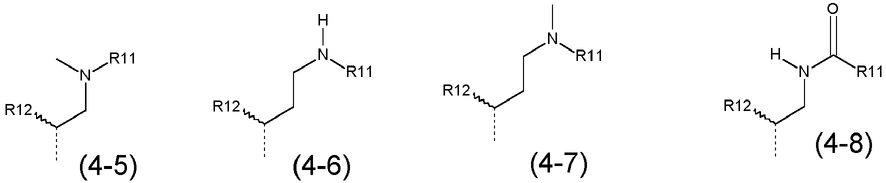 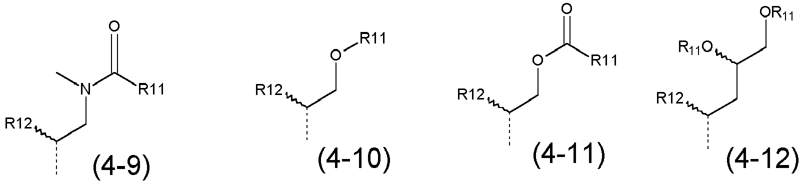 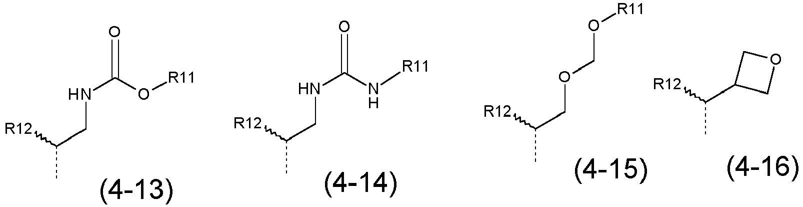 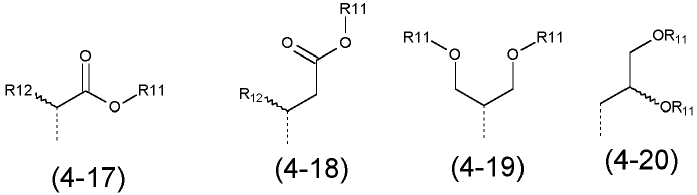 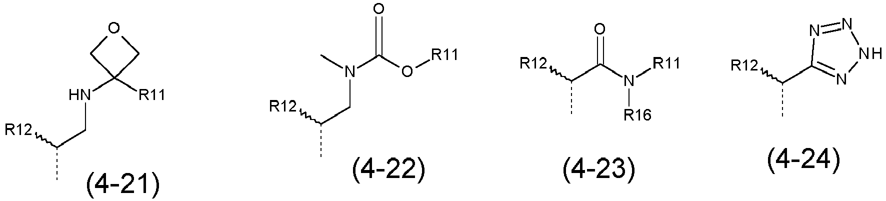 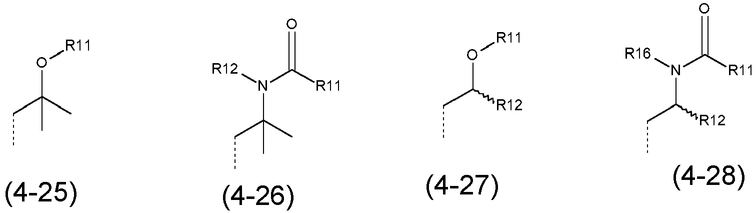 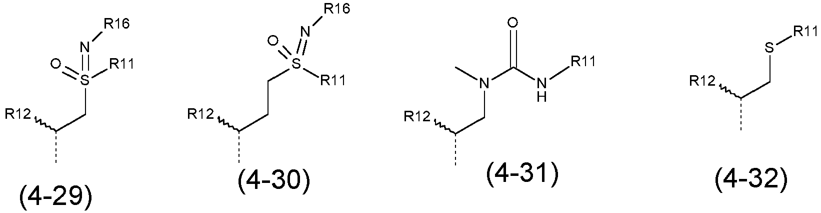 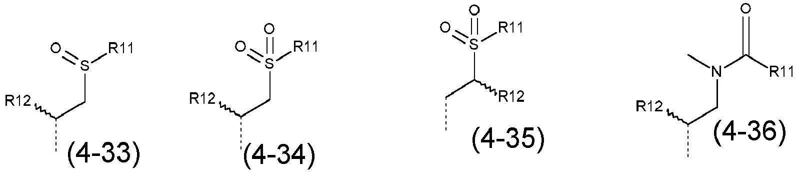 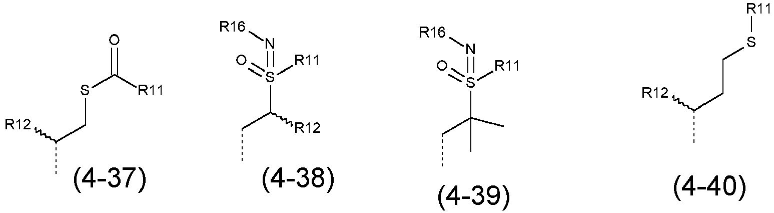 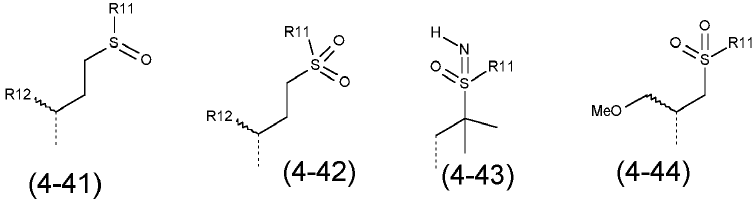 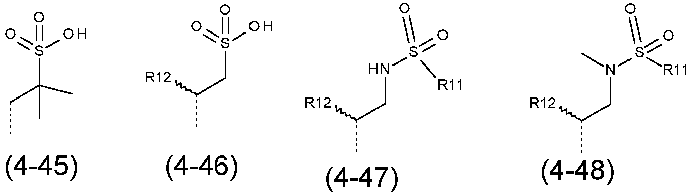 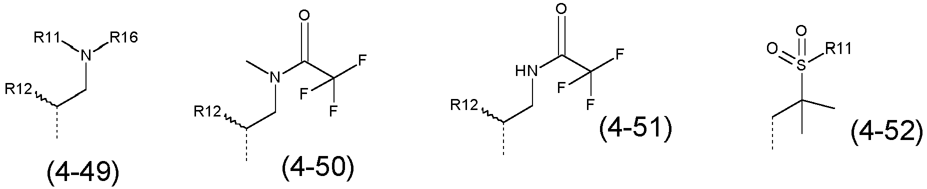 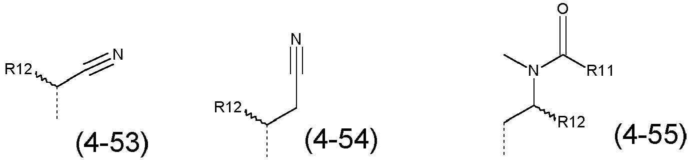
- R5 est choisi parmi un halogène, un alkyle, un cycloalkyle éventuellement substitué par R13, R14 et/ou R15, un alcène, un cycloalcène, un alcyne, un éther ou les groupements (5-1) à (5-5) suivants : 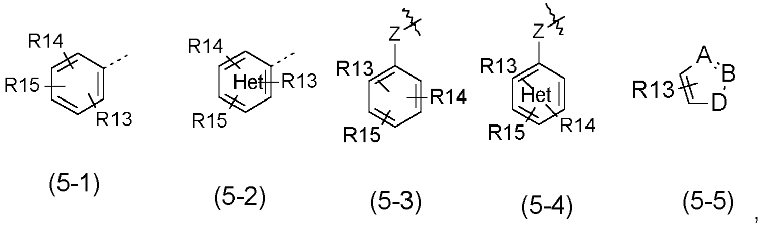 avec Het représentant 1 à 3 atomes d'azote parmi les 6 atomes du cycle aromatique, ces atomes d'azote pouvant, indépendamment les uns des autres, être substitués par un atome d'oxygène pour former un groupe N-oxyde ;
- R7 est choisi parmi les groupements (7-1) à (7-28) suivants : 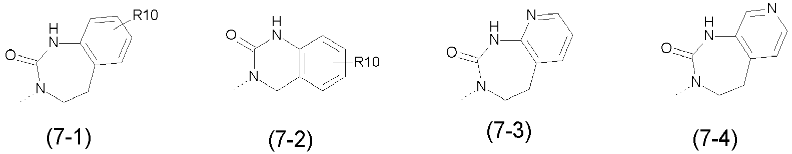 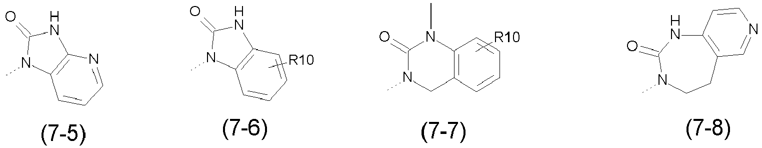 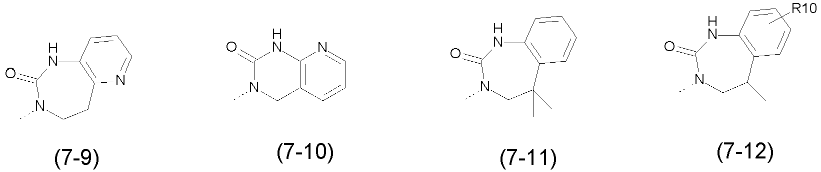 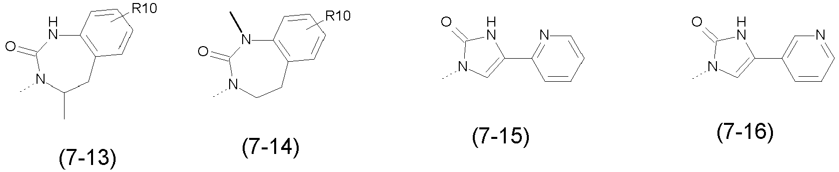 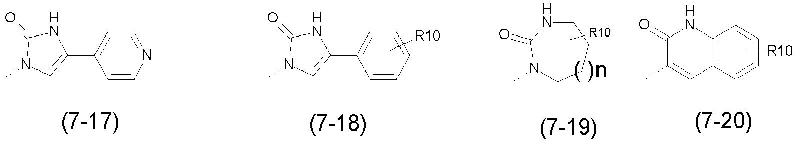 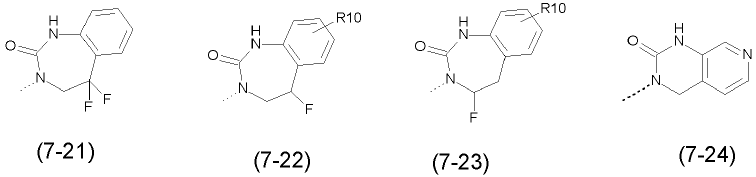 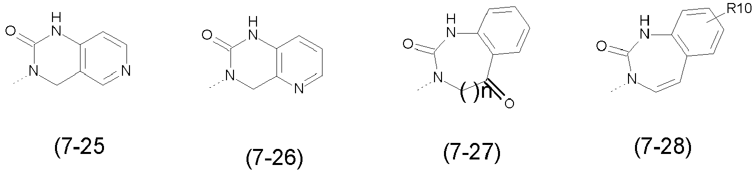 avec n = 0 ou 1 ;
- R8, R9 sont identiques ou différents, et choisis parmi un atome d'hydrogène, F ou -CH₃;
- R10 est choisi parmi un atome d'hydrogène, -OR11, -SR11, -NR11R12, - S(O)R11, -SO₂R11, -OC(O)R11, -CO₂R11, un halogène, -NO₂, -CN, -C(O)NR11R12, -CF₃ ou -OCF₃;
- R11, R12 sont identiques ou différents, et choisis parmi un atome d'hydrogène, un alkyle en C1-C6, CF₃ ou un éther ;
- R13, R14, R15 sont identiques ou différents, et choisis parmi un atome d'hydrogène, un alkyle C1-C6, un cycloalkyle, -OR16, -NR16R17, un halogène, -OCF₃, -CF₃, -CN, -CO₂R16, -CONR16R17, -NO₂, -OCH₂OR16, -SR16, -S(O)R16, -SO₂R16 ou un éther ;
- A, B, D sont identiques ou différents, et choisis parmi un atome C, N, O ou S ;
- R16 et R17 sont identiques ou différents, et choisis parmi un atome d'hydrogène, un alkyle C1-C6 ou -C(O)R18 ;
- Z est choisi parmi -CH₂, O ou -NR18 ; et
- R18 est choisi parmi un atome d'hydrogène, un alkyle C1-C3,
ainsi que leurs sels pharmaceutiquement acceptables, et leurs énantiomères,
et dans laquelle
- un alkyle est une chaîne hydrocarbonée saturée, linéaire ou ramifiée, comprenant de 1 à 8 atomes de carbone, éventuellement substituée par un ou plusieurs atomes de fluor ;
- un alcène est une chaîne hydrocarbonée insaturée, linéaire ou ramifiée, comprenant de 2 à 8 atomes de carbone et comprenant une ou deux doubles liaisons, éventuellement substituée par un ou plusieurs atomes de fluor ;
- un alcyne est une chaîne hydrocarbonée insaturée, linéaire ou ramifiée, comprenant de 2 à 8 atomes de carbone et comprenant une ou deux triples liaisons, éventuellement substituée par un ou plusieurs atomes de fluor ;
- un cycloalkyle est une chaine hydrocarbonée, saturée, cyclique comprenant de 3 à 7 atomes de carbones, éventuellement substituée par un ou plusieurs atomes de fluor ;
- un cycloalcène est une chaine hydrocarbonée, insaturée, cyclique comprenant de 4 à 7 atomes de carbones et comprenant une ou deux doubles liaisons, éventuellement substituée par un ou plusieurs atomes de fluor ;
- un aryle est un cycle hydrocarboné aromatique ou deux cycles fusionnés hydrocarbonés aromatiques ;
- un aralkyle est un alkyle substitué par un aryle ;
- un hétéroaryle est un radical hétérocyclique aromatique comprenant un ou plusieurs hétéroatomes choisis parmi O, S et N, éventuellement substituée par un ou plusieurs groupes d'atomes choisis par exemple parmi un alkyle, un alkoxy, un aryle, un aryle substitué, un halogène, un hydroxy, un cyano, un trifluorométhyle, un sulfure, un pentafluorosulfure, un sulfoxyde, un sulfone, un acide carboxylique, un ester et un nitro ;
- un hétéroaralkyle est un alkyle substitué par un hétéroaryle ;
- un hétérocycle désigne, en particulier, une chaine hydrocarbonée cyclique ou bicylique, saturée ou insaturée, comprenant un ou plusieurs hétéroatomes choisis parmi O, S et N, éventuellement substitué par un ou plusieurs groupes choisis parmi un alkyle, un alkoxyle, un halogène, un hydroxyle, un cyano, un trifluorométhyle, un sulfure, un pentafluorosulfure, un sulfoxyde, un sulfone, un acide carboxylique, un ester et un nitro ;
- un ether est une chaîne hydrocarbonée saturée, linéaire ou ramifiée, comprenant de 1 à 8 atomes de carbone, et dans laquelle 1 à 3 atomes de carbone sont remplacés respectivement par 1 à 3 atomes d'oxygène, deux atomes d'oxygène étant toujours séparés par au moins un atome de carbone, ladite chaine étant éventuellement substituée par un ou plusieurs atomes de fluor.

2. Composé de formule générale (I) selon la revendication 1, dans laquelle :
- Y est choisi parmi -CH₂ ou -C(O) ;
- R1 est choisi parmi les groupements suivants : 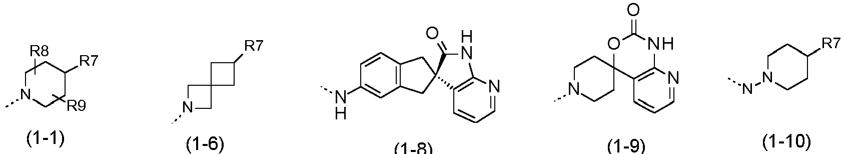
- R2, R3, R6 sont identiques ou différents, et choisis parmi un atome d'hydrogène, F ou -CH₃;
- R4 est choisi parmi un alkyle C1-C4, un aralkyle, un hétéroaralkyle, ou les groupements suivants : 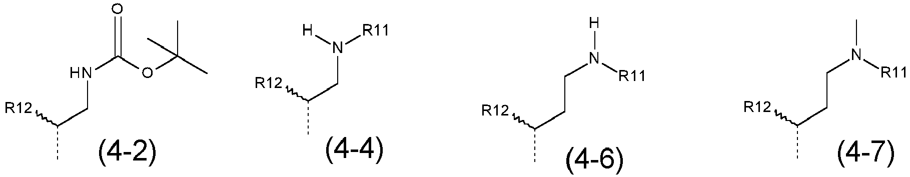 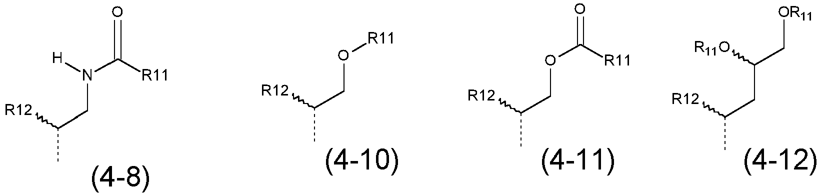 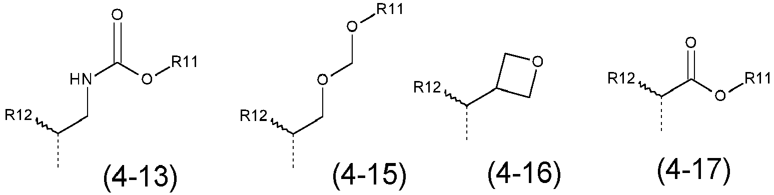 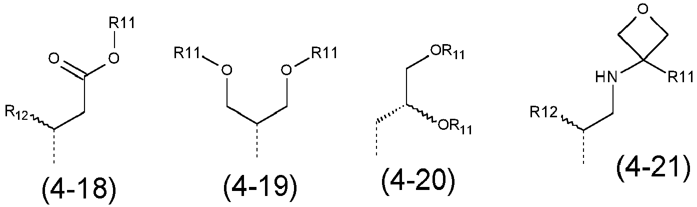 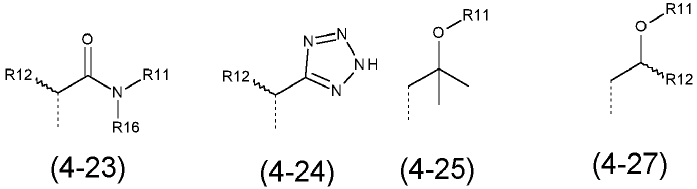 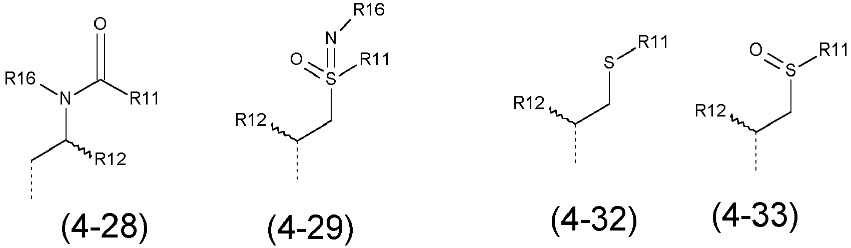 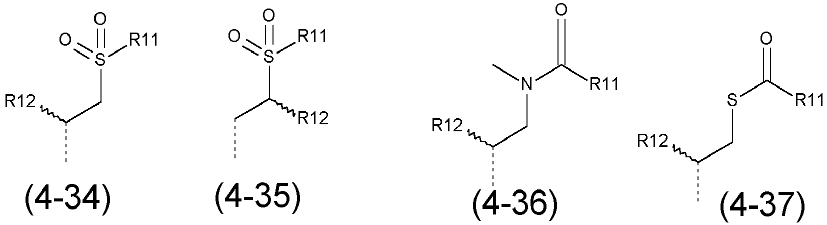 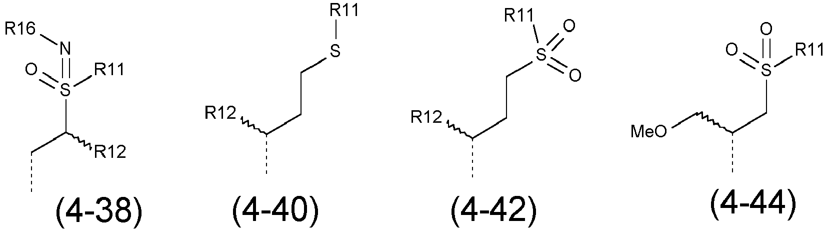 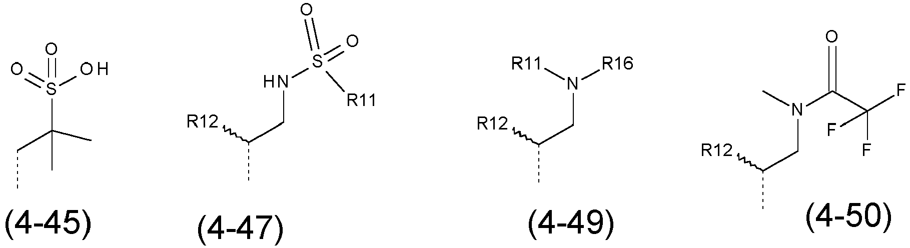 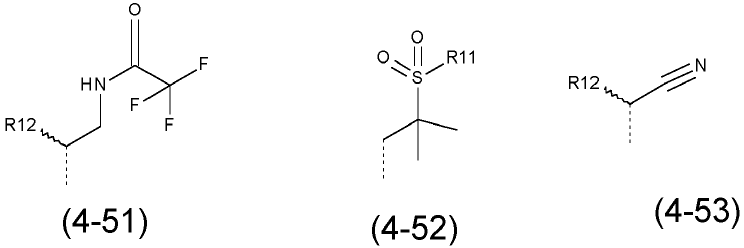
- R5 est choisi parmi Br, -CH₃, un cyclohexène ou les groupements suivants : 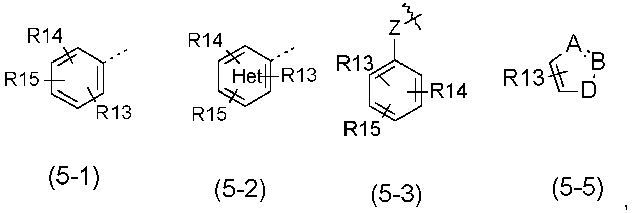 avec Het représentant 1 à 2 atomes d'azote parmi les 6 atomes du cycle aromatique, ces atomes d'azote pouvant, indépendamment les uns des autres, être substitués par un atome d'oxygène pour former un groupe N-oxyde ;
- R7 est choisi parmi les groupements suivants : 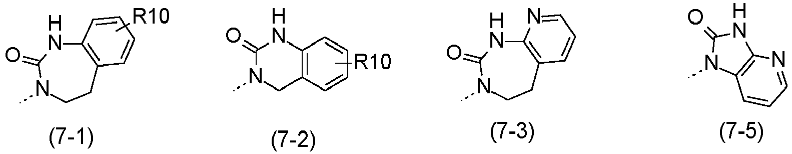 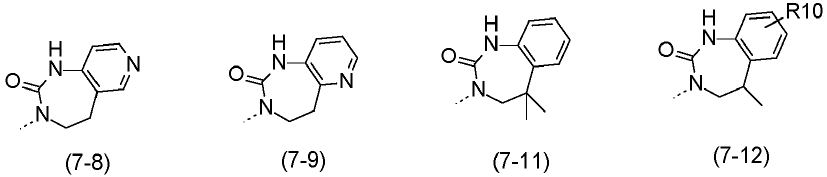 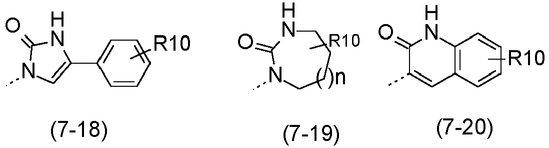 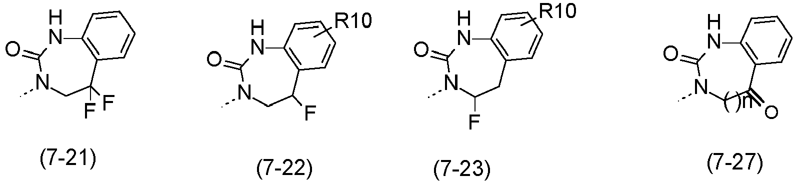 avec n = 1 ;
- R8, R9 sont identiques ou différents, et choisis parmi un atome d'hydrogène, F ou - CH₃ ;
- R10 est choisi parmi un atome d'hydrogène, -OR11, -SR11, -S(O)R11, -SO₂R11, - CO₂H ou un halogène choisi parmi Br ou F ;
- R11, R12 sont identiques ou différents, et choisis parmi un atome d'hydrogène ou un alkyle en C1-C3;
- R13, R14, R15 sont identiques ou différents, et choisis parmi un atome d'hydrogène,
- CH₃, -OR16, un halogène, -OCF₃, -CN, -CO₂R16, -SR16, -S(O)R16, ou -SO₂R16 ;
- A, B, D sont identiques ou différents, et choisis parmi un atome C, N ou S ;
- R16 est choisi parmi un atome d'hydrogène ou un alkyle ; et
- Z est choisi parmi -CH₂ ou O.

3. Composé de formule générale (I) selon la revendication 1 ou 2, dans laquelle :
- Y est choisi parmi -CH₂ ou -C(O) ;
- R1 est choisi parmi les groupements suivants : 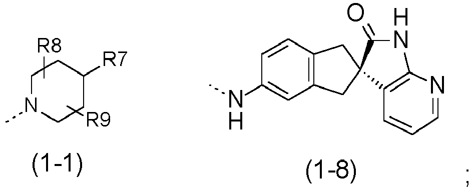
- R2, R3, R6 sont un atome d'hydrogène ;
- R4 est choisi parmi un alkyle C1-C4, un hétéroaralkyle de formule 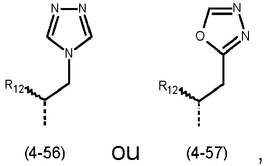 ou les groupements suivants : 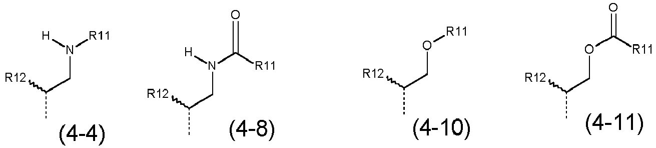 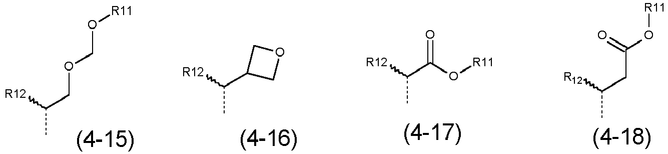 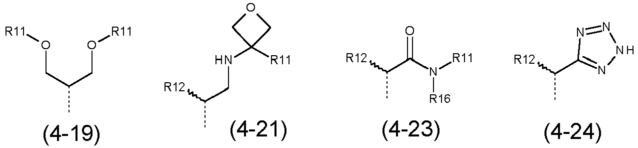 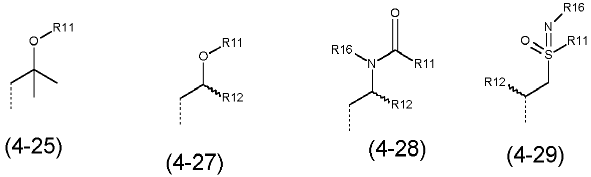 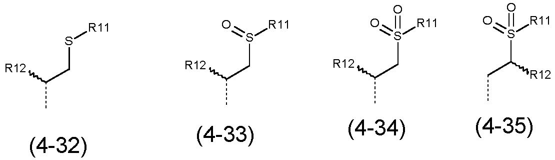 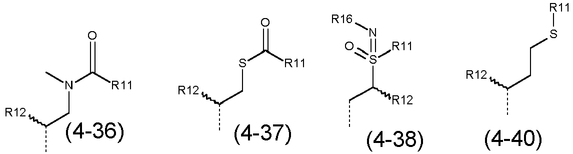 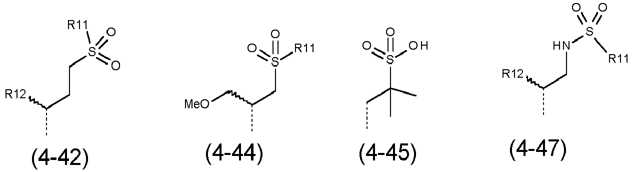 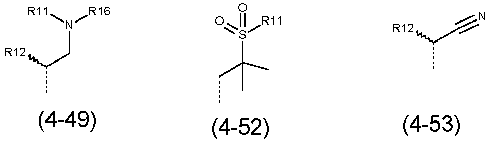
- R5 est 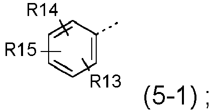
- R7 est choisi parmi les groupements suivants : 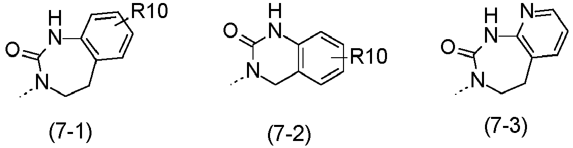 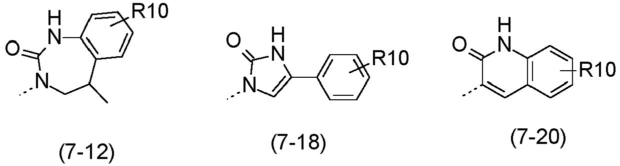
- R8, R9 sont un atome d'hydrogène ;
- R10 est choisi parmi un atome d'hydrogène, -OR11, -SR11, -S(O)R11, -SO₂R11,-CO₂H ou un halogène choisi parmi Br ou F ;
- R11, R12 sont identiques ou différents, et choisis parmi un atome d'hydrogène ou un alkyle en C1-C2 ;
- R13, R14, R15 sont identiques ou différents, et choisis parmi un atome d'hydrogène,
- CH₃, -OR16, un halogène, ou -OCF₃ ; et
- R16 est choisi parmi un atome d'hydrogène ou un alkyle.

4. Composé de formule générale (I) selon l'une des revendications 1 à 3, dans laquelle :
- Y est -C(O) ;
- R1 est choisi parmi les groupements suivants : 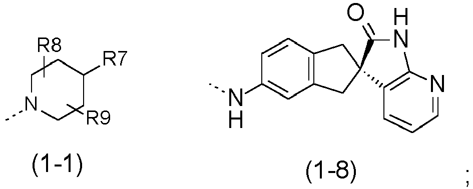
- R2, R3, R6 sont un atome d'hydrogène ;
- R4 est choisi parmi les groupements suivants : 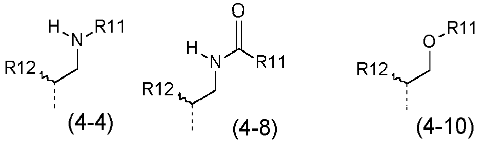 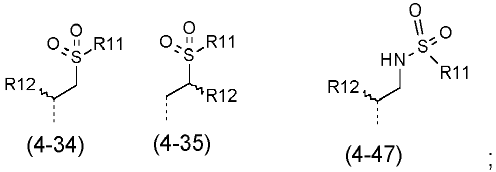
- R5 est 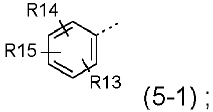
- R7 est 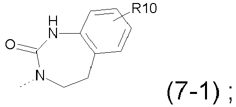
- R8, R9 sont un atome d'hydrogène ;
- R10 est choisi parmi un atome d'hydrogène, -OR11, -SR11 ou Br ;
- R11, R12 sont identiques ou différents, et choisis parmi un atome d'hydrogène ou un alkyle en C1-C2 ;
- R13, R14, R15 sont identiques ou différents, et choisis parmi un atome d'hydrogène,
- OR16, ou un halogène choisi parmi F et Cl ; et
- R16 est un alkyle C1-C3.

5. Composé de formule générale (I) selon la revendication 1, dans laquelle ledit composé est choisi parmi:
composé 14: 5-(2-chloro-3-fluoro-phényl)-3-((S)-2-méthoxy-1-méthyl-éthyl)-1-{2-oxo-2-[4-(2-oxo-1,4-dihydro-2Hquinazolin-3-yl)-pipéridin-1-yl]-éthyl}-1H-pyrimidine-2,4-dione ;
composé 16: 5-(2,3-difluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 17: 5-(2,3-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 18: 5-(2-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 19 : 3-méthyl-1-{2-oxo-2-[4-(2-oxo-1,2,4,5-tétrahydro-benzo[d][1,3]diazépin-3-yl)-pipéridin-1-yl]-éthyl}-5-(2-trifluorométhoxy-phényl)-1H-pyrimidine-2,4-dione ;
composé 22 : 5-(3-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 23 5-(3-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 24 : 5-(3-chloro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 28 5-(3-fluoro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 29 5-(3-chloro-2-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 30 5-(2,3-diméthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 31 5-(2-chloro-3-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 47 : 5-(3,4-dichloro-phényl)-3-méthyl-1-{2-oxo-2-[4-(2-oxo-1,2,4,5-tétrahydro-benzo[d][1,3]diazépin-3- yl)-pipéridin-1-yl]-éthyl}-1H-pyrimidine-2,4-dione ;
composé 51 : 5-(2,3-diméthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 52 5-(3-chloro-2-méthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 53 : 5-(2,3-dichloro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 54 5-(2-chloro-3-fluoro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 72 : (5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-acetonitrile ;
composé 73 : acide 3-(5-(2-Chloro-3-fluoro-phenyl)-2,6-dioxo-3-{2-oxo-2-[4-(2-oxo-1,2,4,5-tetrahydrobenzo[d][1,3]diazepin-3-yl)-piperidin-1-yl]-ethyl}-3,6-dihydro-2H-pyrimidin-1-yl)-propanoïque ;
composé 83: 5-(2,3-dichloro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 89: 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 90 : 5-(2,3-dichloro-phényl)-3-(2-méthylaminoéthyl)-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione ;
composé 93; 5-(2,3-diméthyl-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 95: 5-(3-chloro-2-méthoxy-phényl)-3-(2-méthoxyéthyl)-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione ;
composé 96 : 5-(2-chloro-3-fluoro-phényl)-3-(2-méthoxyéthyl)-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione ;
composé 97 5-(2-chloro-3-méthoxy-phényl)-3-(2-méthoxyéthyl)-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione ;
composé 98 5-(2,3-diméthyl-phényl)-3-(2-hydroxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 100: 5-(2,3-diméthyl-phényl)-3-(2-méthoxyméthoxy-éthyl)-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione ;
composé 101 : N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétam ide ;
composé 103: (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle ;
composé 104 : acide (5-(2-chloro-3-fluoro-phényl)-2,6-dioxo-3-{2-oxo-2-[4-(2-oxo-1,2,4,5-tétrahydrobenzo[d][1,3]diazépin-3-yl)-piperidin-1-yl]-éthyl}-3,6-dihydro-2H-pyrimidin-1-yl)-acétique ;
composé 105 : 5-(2-chloro-3-fluoro-phényl)-3-oxétan-3-ylméthyl-1-{2-oxo-2-[4-(2-oxo-1,2,4,5-tétrahydroben zo[d][1,3]diazépin-3-yl)-pipéridin-1-yl]-éthyl}-1H-pyrimidine-2,4-dione ;
composé 106: 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 108 : 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 110: 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
composé 112: 5-(2-chloro-3-fluoro-phényl)-3-(2-méthylsulfanyl-éthyl)-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione ;
composé 113 : 5-(2,3-difluoro-phényl)-3-(2-méthylsulfanyl-éthyl)-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione ;
composé 114 : 3-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle ;
composé 115 N-[(S)-2-(5-(2-chloro-3-fluoro-phényl)-3-{2-[4-(7-méthoxy-2-oxo-1,2,4,5-tétrahydrobenzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
composé 116 : N-[(S)-2-(5-(2-chloro-3-fluoro-phényl)-3-{2-[4-(7-méthoxy-5-méthyl-2-oxo-1,2,4,5-tétrahydrobenzo[d][1,3]diazépin-3-yl)-pipéridin-1-yl]-2-oxo-éthyl}-2,6-dioxo-3,6-dihydro-2H-pyrimidin-1 -yl)-propyl]-acétamide ;
composé 117 : N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide ;
composé 118 : N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide ;
composé 119 : 3-((S)-2-amino-1-méthyl-éthyl)-5-(2-chloro-3-fluoro-phényl)-1-{2-[4-(7-méthoxy-2-oxo-1,2,4,5-tétrahydrobenzo[d][1,3]diazépin-3-yl)-pipéridin-1-yl]-2-oxo-éthyl}-1H-pyrimidine-2,4-dione ;
composé 120 N-[(S)-2-(5-(2-chloro-3-fluoro-phényl)-3-{2-[4-(7-méthoxy-2-oxo-1,2,4,5-tétrahydrobenzo[d][1,3]diazépin-3-yl)-pipéridin-1-yl]-2-oxo-éthyl}-2,6-dioxo-3,6-dihydro-2H-pyrimidin-1-yl)-propyl]-méthanesulfonamide ;
composé 121 5-(2-chloro-3-fluoro-phényl)-1-{2-[4-(7-méthoxy-2-oxo-1,2,4,5-tétrahydro-benzo[d][1,3]diazépin-3-yl)-pipéridin-1 -yl]-2-oxo-éthyl}-3-[(S)-1 -méthyl-2-(3-méthyloxétan-3-ylamino)-éthyl]-1H-pyrimidine-2,4-dione ;
composé 122 : 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 123 : (S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-acétate de propyle ;
composé 124 : 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H pyrimidine-2,4-dione ;
composé 126 : 5-(2,3-difluoro-phényl)-3-((S)-2-méthoxy-1-méthyl-éthyl)-1-{2-[4-(7-méthoxy-2-oxo-1,2,4,5-tétrahydrobenzo[d][1,3]diazépin-3-yl)-pipéridin-1-yl]-2-oxo-éthyl}-1H-pyrimidine-2,4-dione ;
composé 130 : 5-(2-chloro-3-méthoxy-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 131 : 5-(2-chloro-3-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 132 : 5-(2,3-dichloro-phényl)-3-((S)-2-méthoxy-1-méthyl-éthyl)-1-{2-[4-(7-méthoxy-2-oxo-1,2,4,5-tétrahydrobenzo[d][1,3]diazépin-3-yl)-pipéridin-1-yl]-2-oxo-éthyl}-1H-pyrimidine-2,4-dione ;
composé 134 : 5-(3-bromo-2-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 135 : 5-(2-chloro-3-fluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 136 5-(2,3-difluoro-phényl)-3-(2-méthanesulfonyl-éthyl)-1-{2-[4-(7-méthoxy-2-oxo-1,2,4,5-tétrahydrobenzo[d][1,3]diazépin-3-yl)-pipéridin-1-yl]-2-ozoéthyl}-1H-pyrimidine-2,4-dione ;
composé 137 5-(2,3-dichloro-phényl)-3-(2-méthanesulfonyl-éthyl)-1-{2-[4-(7-méthoxy-2-oxo-1,2,4,5-tétrahydrobenzo[d][1,3]diazépin-3-yl)-pipéridin-1-yl]-2-oxoéthyl}-1H-pyrimidine-2,4-dione ;
composé 138 5-(2,3-diméthyl-phényl)-3-(2-méthanesulfonyl-éthyl)-1-{2-[4-(7-méthoxy-2-oxo-1,2,4,5-tétrahydrobenzo[d][1,3]diazépin-3-yl)-pipéridin-1-yl]-2-oxoéthyl}-1H-pyrimidine-2,4-dione ;
composé 139 5-(2,3-diméthoxy-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 140 : 5-(2-chloro-3-méthoxy-phényl)-3-(2-methanesulfonyl-éthyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 142 : 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
composé 143 : 5-(2-chloro-3-fluoro-phényl)-3-((R)-2-méthanesulfonyl-1-méthyl-ethyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
composé 144 : 5-(2,3-dichloro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
composé 145 : 5-(2,3-dichloro-phényl)-3-((R)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
composé 146 5-(2-chloro-3-fluoro-phényl)-3-isopropyl-1-{2-[4-(7-méthoxy-2-oxo-1,2,4,5-tétrahydrobenzo[d][1,3]diazépin-3-yl)-pipéridin-1-yl]-2-oxo-éthyl}-1H-pyrimidine-2,4-dione ;
composé 148 : 5-(2-chloro-3-méthoxy-phényl)-3-isopropyl-1-{2-[4-(7-méthoxy-2-oxo-1,2,4,5-tétrahydrobenzo[d][1,3]diazépin-3-yl)-pipéridin-1-yl]-2-oxo-éthyl}-1H-pyrimidine-2,4-dione ;
composé 152 : 3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-5-(3-méthoxy-2-trifluorométhoxy-phényl)-1H-pyrimidine-2,4-dione ;
composé 155 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione ;
composé 156 : 5-(2-chloro-3-fluoro-phényl)-3-(3-méthanesulfonyl-propyl)-1-{2-[4-(7-méthoxy-2-oxo-1,2,4,5-tétrahydro-benzo[d][1,3]diazépin-3-yl)-pipéridin-1-yl]-2-oxoéthyl}-1H-pyrimidine-2,4-dione ;
composé 157 : 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione ;
composé 158 N-[(S)-2-(5-(2-chloro-3-fluoro-phényl)-3-{2-[4-(7-méthoxy-2-oxo-1,2,4,5-tétrahydrobenzo[d][1,3]diazepin-3-yl)-pipéridin-1-yl]-2-oxo-éthyl}-2,6-dioxo-3,6-dihydro-2H-pyrimidin-1-yl)-propyl]-propionamide ;
composé 159 : 5-(2-Chloro-3-fluoro-phenyl)-1-{2-[4-(7-fluoro-2-oxo-1,2,4,5-tetrahydro-benzo[d][1, 3]diazepin-3-yl)-piperid in-1-yl]-2-oxo-ethyl}-3-((S)-2-methoxy-1-methyl-ethyl)-1H-pyrimidine-2,4-dione ;
composé 160 : 5-(2-Chloro-3-fluoro-phenyl)-1-{2-[4-(7-fluoro-2-oxo-1,2,4,5-tetrahydro-benzo[d][1, 3]diazepin-3-yl)-piperidin-1-yl]-2-oxo-ethyl}-3-(2-methanesulfonyl-ethyl)-1H-pyrimidine-2,4-dione ;
composé 161 : N-[(S)-2-(5-(2-Chloro-3-fluoro-phenyl)-3-{2-[4-(7-fluoro-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidin-1-yl)-propyl]-acetamide ;
composé 162 N-[(S)-2-(5-(2,3-Dichloro-phenyl)-3-{2-[4-(7-fluoro-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide ;
composé 165 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H pyrrolo[2,3-b]pyridine]-5-yl]-acetamide ;
Composé 166 : 5-(2-chloro-3-fluoro-phényl)-3-isopropylpyrimidine-2,4-dione-1-[(2R)-2'-oxospiro[1,3-dihydroindene-2,3'-1H-pyrrolo[2,3-b]pyridine]-5-yl]-acetamide ;
composé 170 : 5-(2,3-dichlorophenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide;
composé 178:5-(2,3-dichlorophenyl)-3-[(1S)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
composé 184 : 5-(2-Chloro-3-fluoro-phényl)-3-(2-méthanesulfonyl-éthyl)-1-{2-[4-(7-méthanesulfonyl-2-oxo-1,2,4,5-tétrahydro-benzo[d][1,3]diazepin-3-yl)-piperidin-1-yl]-2-oxo-ethyl}-1H-pyrimidine-2,4-dione ;
composé 185 : 5-(2-Chloro-3-fluoro-phenyl)-3-isopropyl-1-{2-[4-(7-methanesulfonyl-2-oxo-1,2,4,5-tetrahydro-benzo[d][1,3]diazepin-3-yl)-piperidin-1-yl]-2-oxo-ethyl}-1H-pyrimidine-2,4-dione ;
composé 186 : 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d][1,3]diazepin-3-yl)-piperidin-1-yl]-2-oxo-éthyl}-1H-pyrimidine-2,4-dione ;
composé 187 : 5-(2-Chloro-3-fluoro-phenyl)-3-((R)-2-methoxy-1-methyl-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione ;
composé 188 5-(2-Chloro-3-fluoro-phenyl)-3-(2-methoxy-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione ;
composé 189 : 5-(2-Chloro-3-fluoro-phenyl)-3-isopropyl-1-{2-[4-(7-methylsulfanyl-2-ozo-1,2,4,5-tetrahydro-benzo[d][1,3]diazepin-3-yl)-piperidin-1-yl]-2-oxo-ethyl}-1H-pyrimidine-2,4-dione ;
composé 192: 3-(1-{2-[5-(2-chloro-3-fluoro-phenyl)-3-(2-methoxy-ethyl)-2-oxo-3,4-dihydro-2H-pyrimidin-1-yl]-acetyl}-piperidin-4-yl)-1,3,4,5-tetrahydro-benzo[d][1,3]diazepin-2-one ;
composé 199: 3-(1-{2-[5-(2,3-Dichloro-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-one ;
composé 202 : N-[2-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide ;
composé 203 N-[(R)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
composé 205 : N-[(S)-2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
composé 206 : N-[(S)-2-(5-(2-chloro-3-méthoxy-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
composé 207 N-[2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide ;
composé 208 5-(2,3-dichlorophenyl)-3-methyl-1-[2-oxo-2-[4-(2-oxo-5-phenyl-1H-imidazol-3-yl)-1-piperidyl]ethyl]pyrimidine-2,4-dione ;
composé 210: 5-(2,3-dichlorophenyl)-3-isopropyl-1-[2-oxo-2-[4-(2-oxo-4,5-dihydro-1H-1,3-benzodiazepin-3-yl)-1-piperidyl]ethyl]pyrimidine-2,4-dione ;
composé 213 : 5-(2,3-dichlorophenyl)-3-methyl-1-[2-oxo-2-[4-(2-oxo-4,5-dihydro-1H-pyrido[2,3-d][1,3]diazepin-3-yl)-1-piperidyl]ethyl]pyrimidine-2,4-dione ;
composé 215 : 5-(2,3-dichlorophenyl)-3-methyl-1-[2-oxo-2-[4-(2-oxo-1H-quinolin-3-yl)-1-piperidyl]ethyl]pyrimidine-2,4-dione ;
composé 220 : 5-(3,4-dichlorophenyl)-3-methyl-1-[2-oxo-2-[4-(2-oxo-4,5-dihydro-1H-1,3-benzodiazepin-3-yl)-1-piperidyl]ethyl]pyrimidine-2,4-dione ;
composé 221 : 1-[2-[4-(7-bromo-2-oxo-4,5-dihydro-1H-1,3-benzodiazepin-3-yl)-1-piperidyl]-2-oxo-ethyl]-5-(2-chloro-3-fluoro-phenyl)-3-isopropyl-pyrimidine-2,4-dione ;
composé 225 : 5-(2-chloro-3-fluoro-phenyl)-3-isopropyl-1-[2-[4-(7-methylsulfinyl-2-oxo-4,5-dihydro-1H-1,3-benzodiazepin-3-yl)-1-piperidyl]-2-oxo-ethyl]pyrimidine-2,4-dione ;
composé 226 2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]acetamide ;
composé 227 : 5-(2-chloro-3-fluoro-phenyl)-3-(2-methylsulfinylethyl)-1-[2-oxo-2-[4-(2-oxo-4,5-dihydro-1H-1,3-benzodiazepin-3-yl)-1-piperidyl]ethyl]pyrimidine-2,4-dione ;
composé 228 : 5-(2-chloro-3-fluoro-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
composé 229 : 5-(2-chloro-3-fluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
composé 231 : 5-(2,3-difluorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
composé 232 : 5-(2-chloro-3-fluoro-phenyl)-3-[2-méthoxy-1-(méthoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
composé 233 : 5-(2-chloro-3-fluoro-phenyl)-3-[(1S)-2-methoxy-1-methyl-ethyl]-1-[2-[4-(7-methylsulfanyl-2-oxo-4,5-dihydro-1H-1,3-benzodiazepin-3-yl)-1-piperidyl]-2-oxo-ethyl]pyrimidine-2,4-dione ;
composé 234 : 5-(2-chloro-3-fluoro-phenyl)-3-(2-hydroxy-2-methyl-propyl)-1-[2-oxo-2-[4-(2-oxo-4,5-dihydro-1H-1,3-benzodiazepin-3-yl)-1-piperidyl]ethyl]pyrimidine-2,4-dione ;
composé 235 : 5-(2-chloro-3-fluoro-phenyl)-1-[2-oxo-2-[4-(2-oxo-4,5-dihydro-1H-1,3-benzodiazepin-3-yl)-1-piperidyl]ethyl]-3-(2H-tetrazol-5-ylmethyl)pyrimidine-2,4-dione ;
composé 236 : 5-(2,3-difluorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
composé 237 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1S)-1-methylpropyl]pyrimidine-2,4-dione ;
composé 238 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1R)-1-methylpropyl]pyrimidine-2,4-dione ;
composé 239 : 5-(2-chloro-3-fluoro-phenyl)-3-[1-(methoxymethyl)propyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
composé 240 : 5-(2-chloro-3-fluoro-phenyl)-3-(2-methoxy-2-methyl-propyl)-1-[2-[4-(7-methoxy-2-oxo-4,5-dihydro-1H-1,3-benzodiazepin-3-yl)-1-piperidyl]-2-oxo-ethyl]pyrimidine-2,4-dione ;
composé 241 : 5-(2-chloro-3-methoxy-phenyl)-3-[2-methoxy-1-(methoxymethyl)ethyl]-1-[2-[4-(7-methoxy-2-oxo-4,5-dihydro-1H-1 ,3-benzodiazepin-3-yl)-1-piperidyl]-2-oxo-ethyl]pyrimidine-2,4-dione ;
composé 242 : 5-(2,3-dichlorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
composé 243 : 5-(2-chloro-3-methoxy-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 244 : 5-(2-chloro-3-fluoro-phenyl)-3-[(1S)-1-methyl-2-methylsulfonyl-ethyl]-1-[2-oxo-2-[4-(2-oxo-4,5-dihydro-1H-1,3-benzodiazepin-3-yl)-1 piperidyl]ethyl]pyrimidine-2,4-dione ;
composé 245 : 5-(2,3-dichlorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
composé 246 : acide 3-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propanoique ;
composé 247 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,3,4-oxadiazol-2-yl)ethyl]pyrimidine-2,4-dione ;
composé 248 : acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]acetique ;
composé 249 : 5-(2-chloro-3-fluoro-phenyl)-3-[(1R)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione ;
composé 251 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,2,4-triazol-4-yl)ethyl]pyrimidine-2,4-dione ;
composé 252 : 5-(2-chloro-3-fluoro-phenyl)-1-[2-[4-(7-fluoro-2-oxo-4,5-dihydro-1H-1,3-benzodiazepin-3-yl)-1-piperidyl]-2-oxo-ethyl]-3-[(1R)-1-methyl-2-methylsulfonyl-ethyl]pyrimidine-2,4-dione ;
composé 253 : acide 3-[1-[2-[5-(2-chloro-3-fluoro-phenyl)-3-(2-methylsulfonylethyl)-2,4-dioxo-pyrimidin-1-yl]acetyl]-4-piperidyl]-2-oxo-4,5-dihydro-1H-1,3-benzodiazepine-7-carboxylique ;
composé 254 S-[2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethyl]ethanethioate ;
composé 255 : N-[(2S)-2-[5-(2,3-dichlorophenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide ;
composé 256 : N-[(2S)-2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide ;
composé 257 : acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethanesulfonique ;
composé 258 : 5-(2-chloro-3-fluoro-phenyl)-3-[(1S)-1-(methoxymethyl)-2-methylsulfonyl-ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ; et
composé 259 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-(2-methyl-2-methylsulfonyl-propyl)pyrimidine-2,4-dione.

6. Composé de formule générale (I) selon l'une des revendications précédentes, pour son utilisation comme médicament.

7. Composé de formule générale (I) selon l'une quelconque des revendications 1 à 5, pour son utilisation dans la prévention et/ou le traitement de maladies inflammatoires avec une composante neurogène.

8. Composé de formule générale (I) pour son utilisation selon la revendication 7 dans laquelle les maladies inflammatoires de la peau avec une composante neurogène sont choisies parmi la rosacée de type I (Erythémateuse) et de type Il (Papulopustuleuse), la dermatite atopique, l'eczema chronique des mains, le psoriasis sous ses differentes formes (vulgaire, du scalp, arthritique, pustuleux, de goutte), l'érythème facial, l'érythème pudique, l'urticaire (aigue, prurit sénile, prurigo nodularis) et l'acné.

9. Composé de formule générale (I) pour son utilisation selon la revendication 8 dans la prévention et/ou le traitement de la rosacée de type I (Erythémateuse), la dermatite atopique, le psoriasis vulgaire et du scalp, et l'uticaire du type prurit sénile et prurigo nodularis.

10. Composé de formule générale (I) pour son utilisation selon la revendication 9 dans la prévention et/ou le traitement de la rosacée de type I (Erythémateuse).

11. Composé de formule générale (I) pour son utilisation selon la revendication 7 dans le traitement de la migraine.

12. Composition pharmaceutique comprenant au moins un composé de formule générale (I) tel que défini dans l'une quelconque des revendications 1 à 4 et un véhicule pharmaceutiquement acceptable, de préférence adapté pour une administration par voie topique.

13. Utilisation cosmétique d'un composé de formule générale (I) selon l'une des revendications 1 à 4.

## Patentansprüche

1. Heterocyclische Verbindung, ausgewählt aus den Verbindungen der allgemeinen Formel (I): 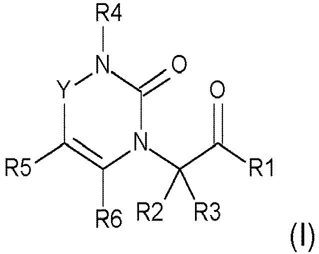 wobei
- Y aus -CH₂, -C(O), -C(CH₃)₂ oder einem Spirocyclopropyl ausgewählt ist;
- R1 aus den folgenden Gruppen (1-1) bis (1-12) ausgewählt ist: 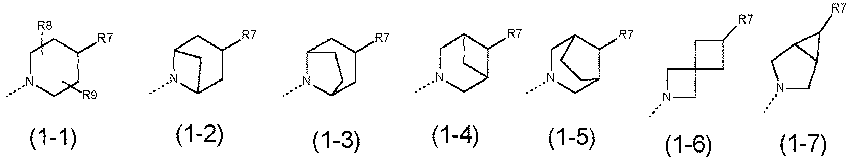 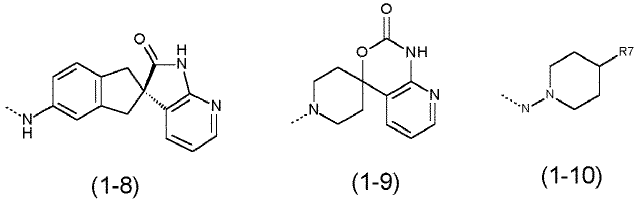 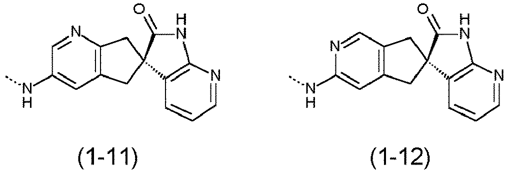
- R2, R3, R6 identisch oder verschieden sind und aus einem Wasserstoffatom, F oder -CH₃ ausgewählt sind;
- R4 aus einem Wasserstoffatom, einem Alkyl, einem Alken, einem Alkin, einem Cycloalkyl, einem Cycloalken, einem Aralkyl, einem Heteroaralkyl oder den folgenden Gruppen (4-1) bis (4-55) ausgewählt ist: 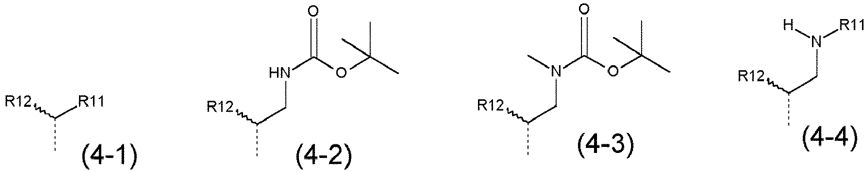 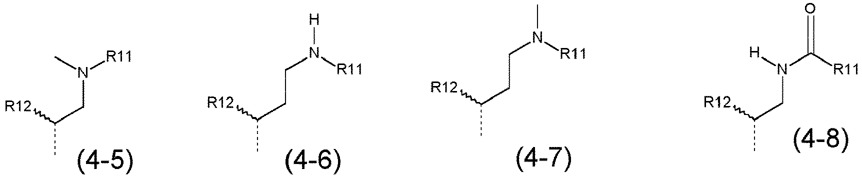 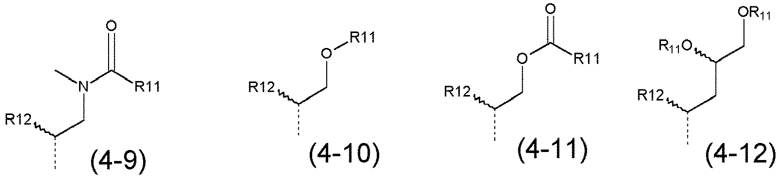 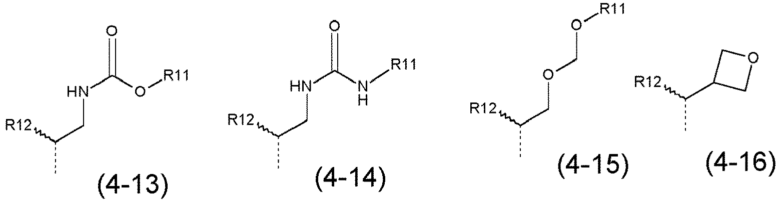 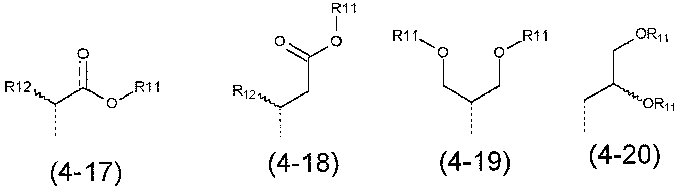 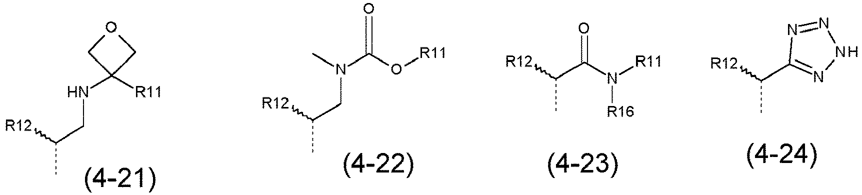 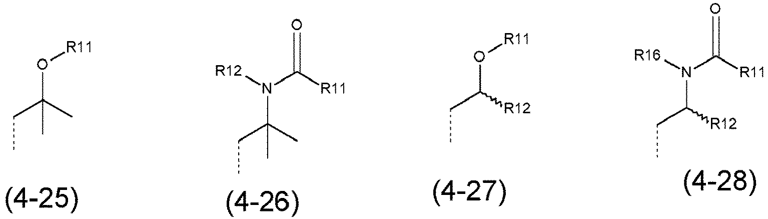 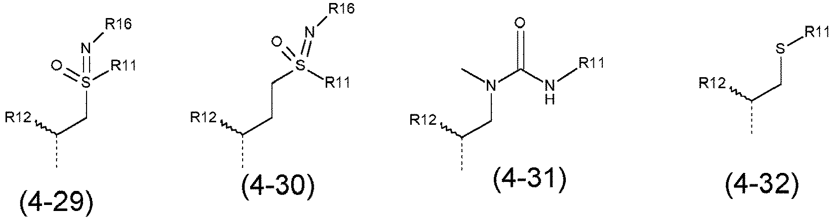 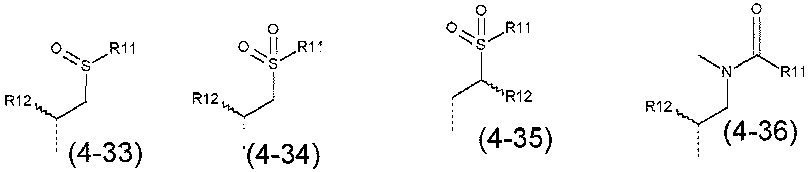 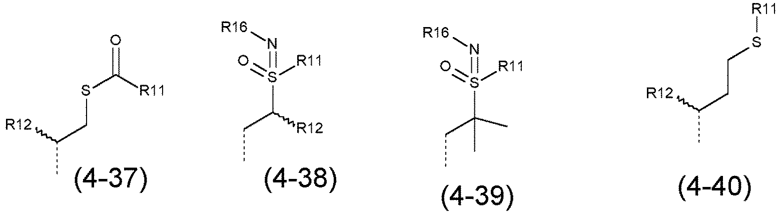 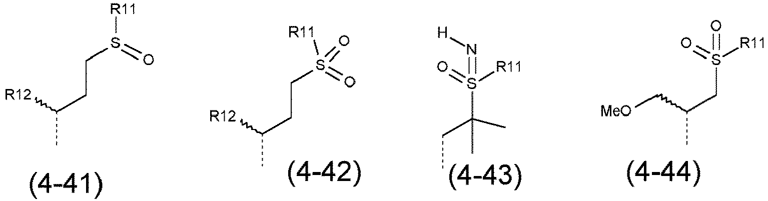 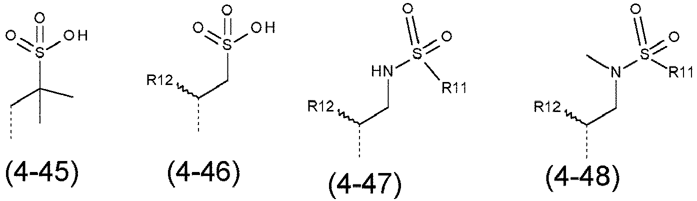 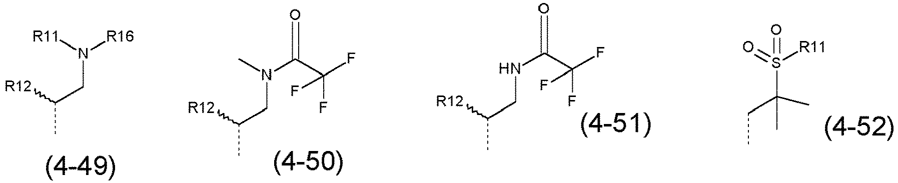 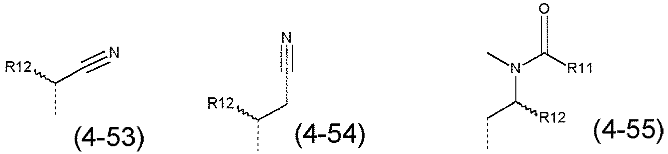
- R5 aus einem Halogen, einem Alkyl, einem gegebenenfalls mit R13, R14 und/oder R15 substituierten Cycloalkyl, einem Alken, einem Cycloalken, einem Alkin, einem Ether oder den folgenden Gruppen (5-1) bis (5-5) ausgewählt ist: 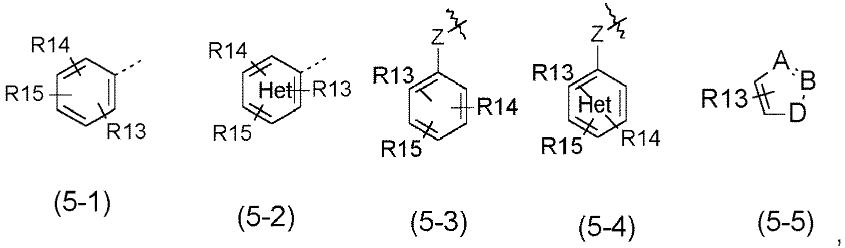 wobei Het 1 bis 3 Stickstoffatome aus den 6 Atomen des aromatischen Rings repräsentiert, wobei diese Stickstoffatome unabhängig voneinander mit einem Sauerstoffatom substituiert sein können, um eine N-Oxid-Gruppe zu bilden;
- R7 aus den folgenden Gruppen (7-1) bis (7-28) ausgewählt ist: 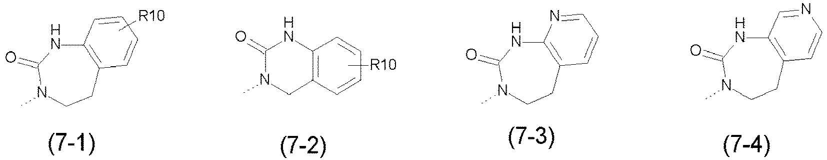 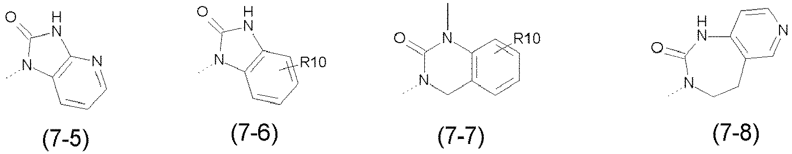 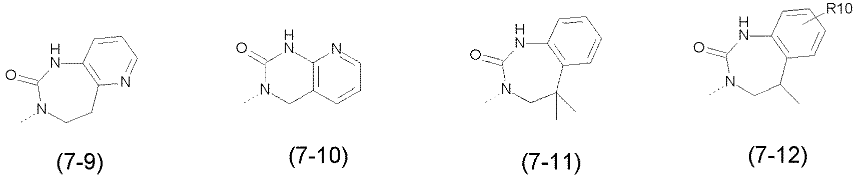 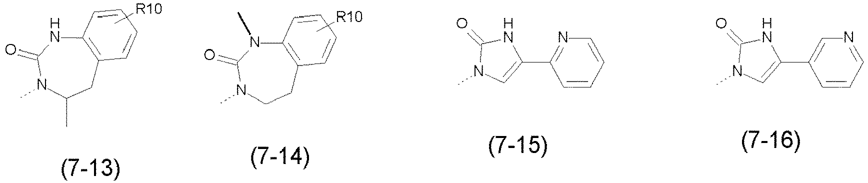 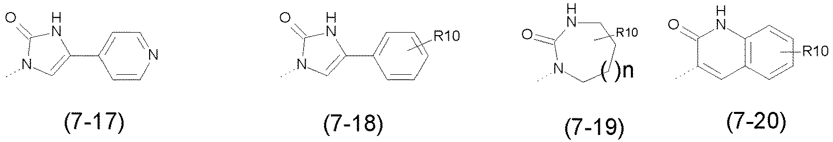 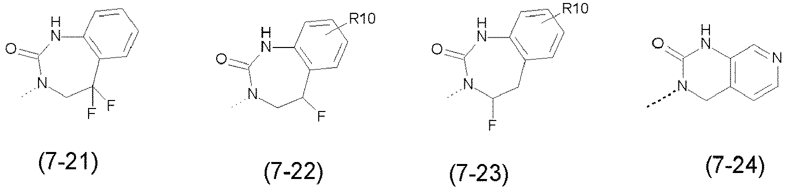 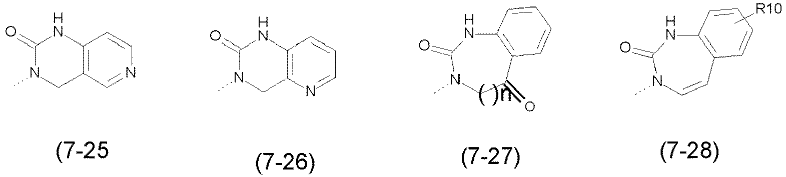 wobei n = 0 oder 1 ist;
- R8, R9 identisch oder verschieden sind und aus einem Wasserstoffatom, F oder -CH₃ ausgewählt sind;
- R10 aus einem Wasserstoffatom, -OR11, -SR11, -NR11R12, -S(O)R11, - SO₂R11, -OC(O)R11, -CO₂R11, einem Halogen, -NO₂, -CN, -C(O)NR11R12, -CFsoder -OCF₃ ausgewählt ist;
- R11, R12 identisch oder verschieden sind und aus einem Wasserstoffatom, einem C1-C6-Alkyl, CF₃ oder einem Ether ausgewählt sind;
- R13, R14, R15 identisch oder verschieden sind und aus einem Wasserstoffatom, einem C1-C6-Alkyl, einem Cycloalkyl, -OR16, -NR16R17, einem Halogen, -OCF₃, -CF₃, -CN, -CO₂R16, -CONR16R17, -NO₂, -OCH₂OR16, -SR16,-S(O)R16, -SO₂R16 oder einem Ether ausgewählt sind;
- A, B, D identisch oder verschieden sind und aus einem C-, N-, O- oder S-Atom ausgewählt sind;
- R16 und R17 identisch oder verschieden sind und aus einem Wasserstoffatom, einem C1-C6-Alkyl oder -C(O)R18 ausgewählt sind;
- Z aus -CH₂, O oder -NR18 ausgewählt ist; und
- R18 aus einem Wasserstoffatom, einem C1-C3-Alkyl sowie deren pharmazeutisch verträglichen Salze und deren Enantiomere ausgewählt ist,
und wobei
- ein Alkyl eine gesättigte, lineare oder verzweigte Kohlenwasserstoffkette ist, die 1 bis 8 Kohlenstoffatome umfasst, die gegebenenfalls mit einem oder mehreren Fluoratomen substituiert ist;
- ein Alken eine ungesättigte, lineare oder verzweigte Kohlenwasserstoffkette ist, die 2 bis 8 Kohlenstoffatome umfasst und eine oder zwei Doppelbindungen umfasst, die gegebenenfalls mit einem oder mehreren Fluoratomen substituiert ist;
- ein Alkin eine ungesättigte, lineare oder verzweigte Kohlenwasserstoffkette ist, die 2 bis 8 Kohlenstoffatome umfasst und eine oder zwei Dreifachbindungen umfasst, die gegebenenfalls mit einem oder mehreren Fluoratomen substituiert ist;
- ein Cycloalkyl eine gesättigte, cyclische Kohlenwasserstoffkette ist, die 3 bis 7 Kohlenstoffatome umfasst, die gegebenenfalls mit einem oder mehreren Fluoratomen substituiert ist;
- ein Cycloalken eine ungesättigte, cyclische Kohlenwasserstoffkette ist, die 4 bis 7 Kohlenstoffatome umfasst und eine oder zwei Doppelbindungen umfasst, die gegebenenfalls mit einem oder mehreren Fluoratomen substituiert ist;
- ein Aryl ein aromatischer Kohlenwasserstoffring oder zwei kondensierte aromatische Kohlenstoffwasserstoffringe sind;
- ein Aralkyl ein Alkyl ist, das mit einem Aryl substituiert ist;
- ein Heteroaryl ein aromatischer heterocyclischer Rest ist, der ein oder mehrere Heteroatome umfasst, die aus O, S und N ausgewählt sind, der gegebenenfalls mit einer oder mehreren Atomgruppen substituiert ist, die zum Beispiel aus einem Alkyl, einem Alkoxy, einem Aryl, einem substituierten Aryl, einem Halogen, einem Hydroxy, einem Cyano, einem Trifluormethyl, einem Sulfid, einem Pentafluorsulfid, einem Sulfoxid, einem Sulfon, einer Carbonsäure, einem Ester und einem Nitro ausgewählt sind;
- ein Heteroaralkyl ein Alkyl ist, das mit einem Heteroaryl substituiert ist;
- ein Heterocyclus insbesondere eine gesättigte oder ungesättigte cyclische oder bicyclische Kohlenwasserstoffkette bezeichnet, die ein oder mehrere Heteroatome umfasst, die aus O, S und N ausgewählt sind, der gegebenenfalls mit einer oder mehreren Gruppen substituiert ist, die aus einem Alkyl, einem Alkoxyl, einem Halogen, einem Hydroxyl, einem Cyano, einem Trifluormethyl, einem Sulfid, einem Pentafluorsulfid, einem Sulfoxid, einem Sulfon, einer Karbonsäure, einem Ester und einem Nitro ausgewählt sind;
- ein Ether eine gesättigte, lineare oder verzweigte Kohlenwasserstoffkette ist, die 1 bis 8 Kohlenstoffatome umfasst, und wobei 1 bis 3 Kohlenstoffatome jeweils durch 1 bis 3 Sauerstoffatome ersetzt sind, wobei zwei Sauerstoffatome immer durch mindestens ein Kohlenstoffatom getrennt sind, wobei die besagte Kette gegebenenfalls mit einem oder mehreren Fluoratomen substituiert ist.

2. Verbindung der allgemeinen Formel (I) nach Anspruch 1, wobei:
- Y aus -CH₂ oder -C(O) ausgewählt ist;
- R1 aus den folgenden Gruppen ausgewählt ist: 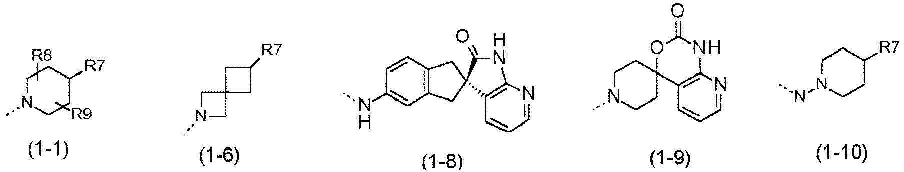
- R2, R3, R6 identisch oder verschieden sind und aus einem Wasserstoffatom, F oder -CH₃ ausgewählt sind;
- R4 aus einem C1-C4-Alkyl, einem Aralkyl, einem Heteroaralkyl oder den folgenden Gruppen ausgewählt ist: 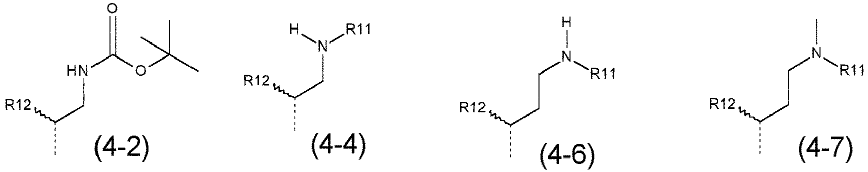 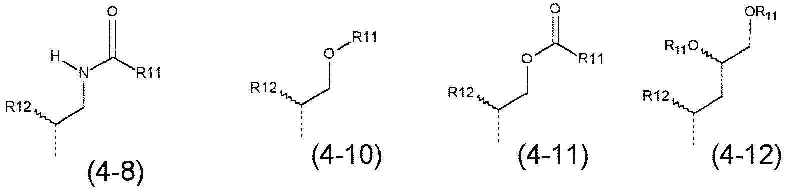 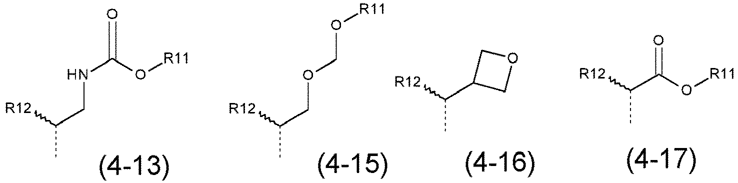 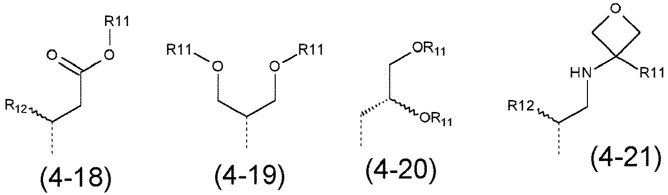 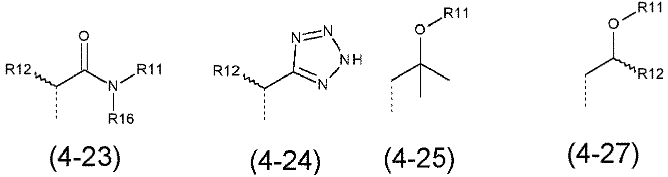 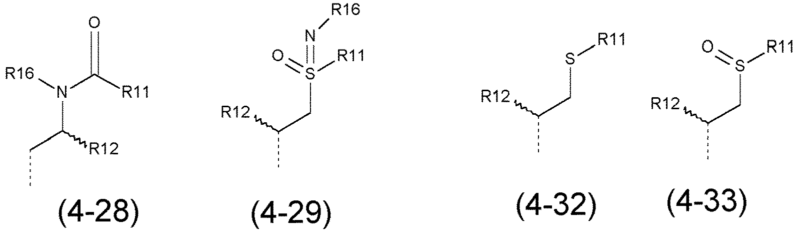 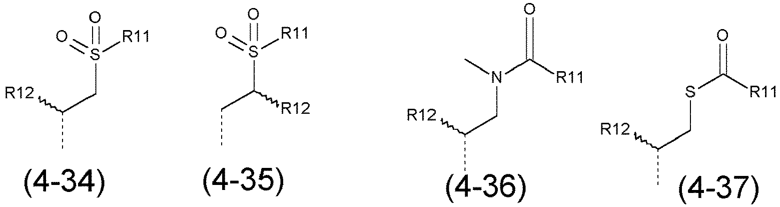 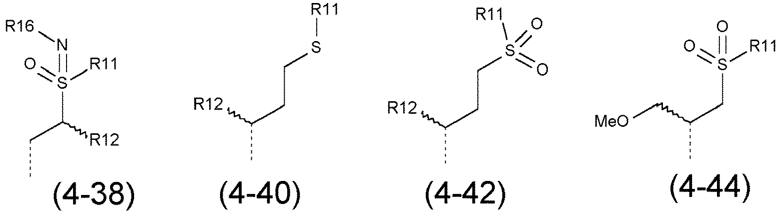 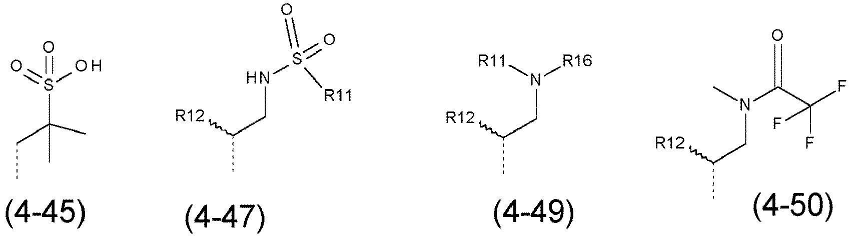 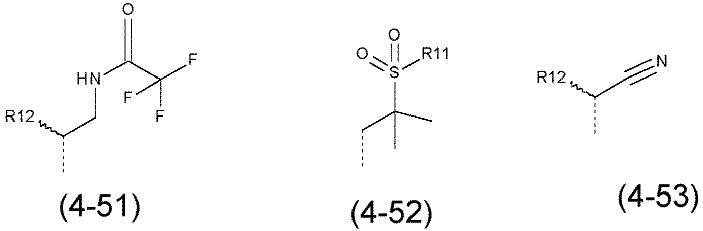
- R5 aus Br, -CH₃, einem Cyclohexen oder den folgenden Gruppen ausgewählt ist: 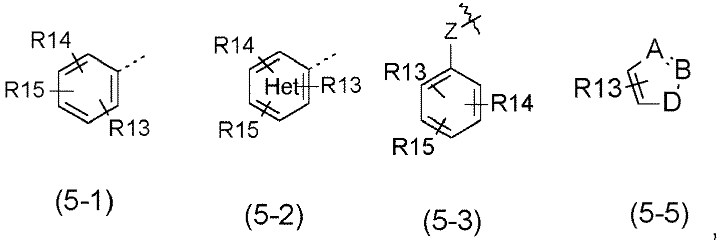 wobei Het 1 bis 2 Stickstoffatome aus den 6 Atomen des aromatischen Rings repräsentiert, wobei diese Stickstoffatome unabhängig voneinander mit einem Sauerstoffatom substituiert werden können, um eine N-Oxid-Gruppe zu bilden;
- R7 aus den folgenden Gruppen ausgewählt ist: 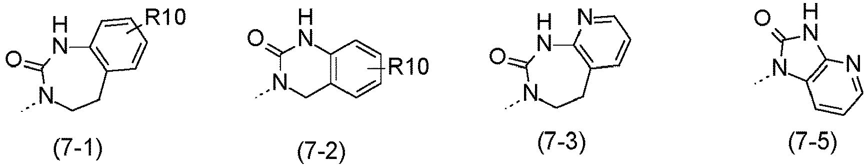 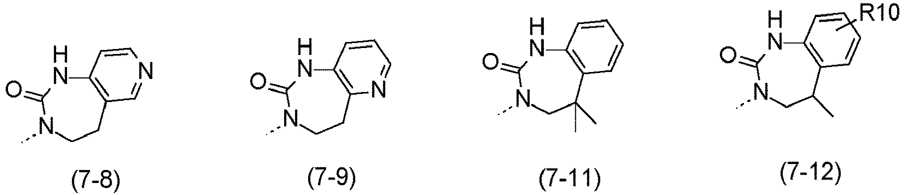 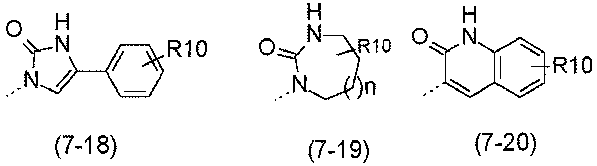 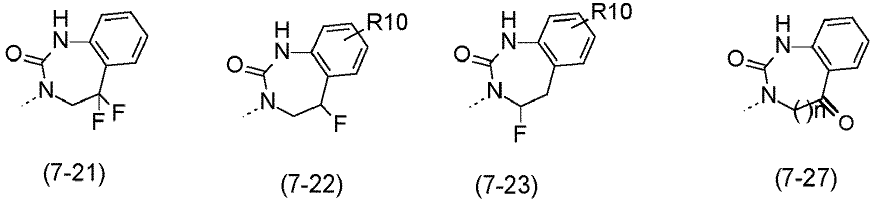 wobei n = 1 ist;
- R8, R9 identisch oder verschieden sind und aus einem Wasserstoffatom, F oder -CH₃ ausgewählt sind;
- R10 aus einem Wasserstoffatom, -OR11, -SR11, -S(O)R11, -SO₂R11, CO₂H oder einem Halogen ausgewählt ist, das aus Br oder F ausgewählt ist;
- R11, R12 identisch oder verschieden sind und aus einem Wasserstoffatom oder einem C1-C3-Alkyl ausgewählt sind;
- R13, R14, R15 identisch oder verschieden sind und aus einem Wasserstoffatom,
- CH₃, -OR16, einem Halogen, -OCF₃, -CN, -CO₂R16, -SR16, -S(O)R16 oder -SO₂R16 ausgewählt sind;
- A, B, D identisch oder verschieden sind und aus einem C-, N- oder S-Atom ausgewählt sind;
- R16 aus einem Wasserstoffatom oder einem Alkyl ausgewählt ist; und
- Z aus -CH₂ oder O ausgewählt ist.

3. Verbindung der allgemeinen Formel (I) nach Anspruch 1 oder 2, wobei:
- Y aus -CH₂ oder -C(O) ausgewählt ist;
- R1 aus den folgenden Gruppen ausgewählt ist: 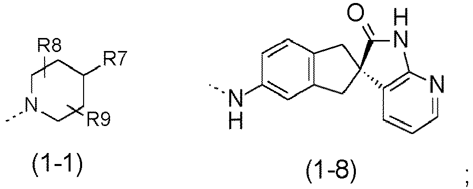
- R2, R3, R6 ein Wasserstoffatom sind;
- R4 aus einem C1-C4-Alkyl, einem Heteroaralkyl der Formel 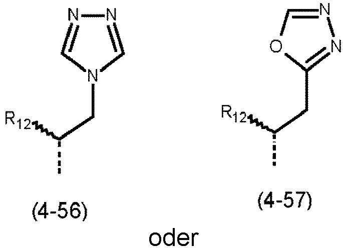 oder den folgenden Gruppen ausgewählt ist: 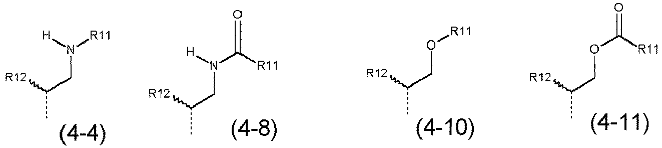 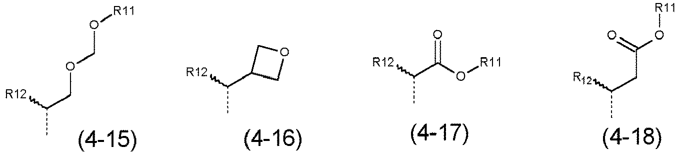 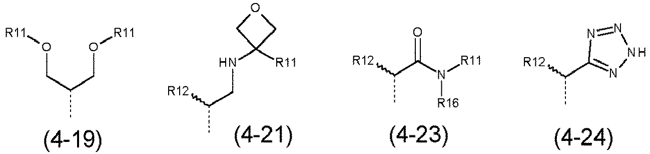 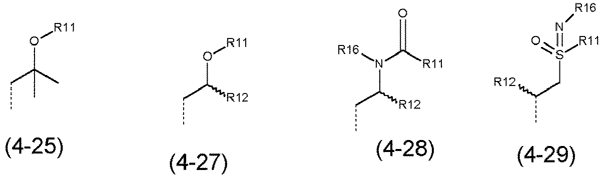 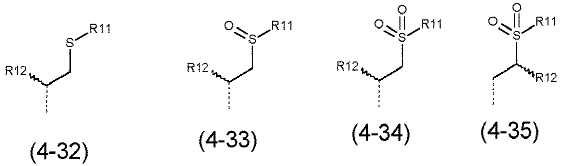 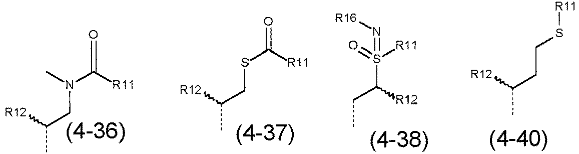 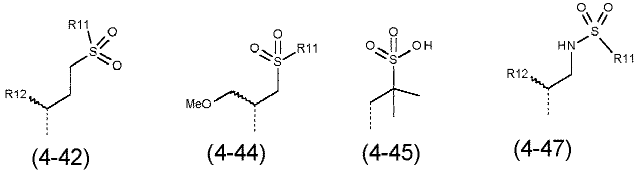 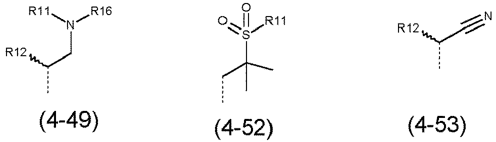
- R5 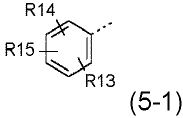 ist;
- R7 aus den folgenden Gruppen ausgewählt ist: 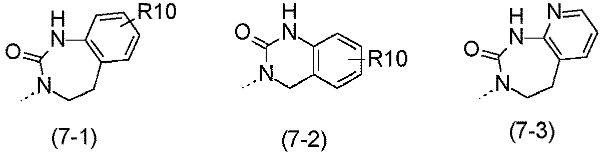 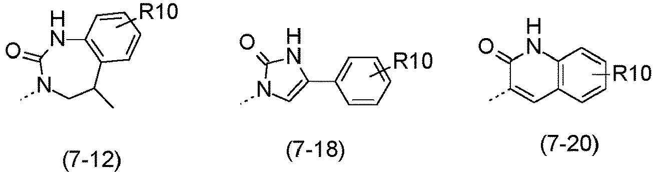
- R8, R9 ein Wasserstoffatom sind;
- R10 aus einem Wasserstoffatom, -OR11, -SR11, -S(O)R11, -SO₂R11, CO₂H oder einem Halogen ausgewählt ist, das aus Br oder F ausgewählt ist;
- R11, R12 identisch oder verschieden sind und aus einem Wasserstoffatom oder einem C1-C2-Alkyl ausgewählt sind;
- R13, R14, R15 identisch oder verschieden sind und aus einem Wasserstoffatom,
- CH₃, -OR16, einem Halogen oder -OCF₃ ausgewählt sind; und
- R16 aus einem Wasserstoffatom oder einem Alkyl ausgewählt ist.

4. Verbindung der allgemeinen Formel (I) nach einem der Ansprüche 1 bis 3, wobei:
- Y -C(O) ist;
- R1 aus den folgenden Gruppen ausgewählt ist: 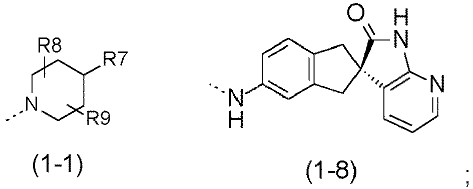
- R2, R3, R6 ein Wasserstoffatom sind;
- R4 aus den folgenden Gruppen ausgewählt ist: 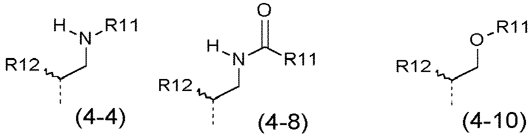 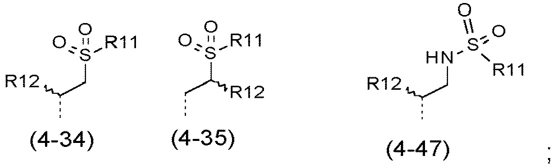
- R5 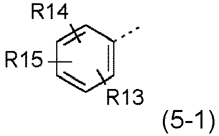 ist;
- R7 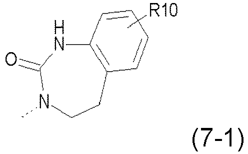 ist;
- R8, R9 ein Wasserstoffatom sind;
- R10 aus einem Wasserstoffatom, -OR11, SR11 oder Br ausgewählt ist;
- R11, R12 identisch oder verschieden sind und aus einem Wasserstoffatom oder einem C1-C2-Alkyl ausgewählt sind;
- R13, R14, R15 identisch oder verschieden sind und aus einem Wasserstoffatom, - OR16 oder einem Halogen ausgewählt sind, das aus F und Cl ausgewählt ist; und
- R16 ein C1-C3-Alkyl ist.

5. Verbindung der allgemeinen Formel (I) nach Anspruch 1, wobei die besagte Verbindung aus Folgendem ausgewählt ist:
Verbindung 14: 5-(2-chlor-3-fluor-phenyl)-3-((S)-2-methoxy-1-methyl-ethyl)-1-{2-oxo-2-[4-(2-oxo-1,4-dihydro-2Hquinazolin-3-yl)-piperidin-1-yl]-ethyl}-1H-pyrim idin-2,4-dion;
Verbindung 16: 5-(2,3-difluor-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 17: 5-(2,3-dichlor-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 18: 5-(2-chlor-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 19: 3-methyl-1-{2-oxo-2-[4-(2-oxo-1,2,4,5-tetrahydro-benzo[d][1,3]diazepin-3-yl)-piperidin-1-yl]-ethyl}-5-(2-trifluormethoxy-phenyl)-1H-pyrimidin-2,4-dion;
Verbindung 22: 5-(3-chlor-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidin-2,4-dion;
Verbindung 23: 5-(3-chlor-2-methyl-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 24: 5-(3-chlor-2-methoxy-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 28: 5-(3-fluor-2-methyl-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 29: 5-(3-chlor-2-fluor-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 30: 5-(2,3-dimethyl-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 31: 5-(2-chlor-3-fluor-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 47: 5-(3,4-dichlor-phenyl)-3-methyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 51: 5-(2,3-dimethyl-phenyl)-3-ethyl-1-{2-oxo-2-[4-(2-oxo-1,2,4,5-tetrahydro-benzo[d][1,3]diazepin-3-yl)-piperidin-1-yl]-ethyl}-1H-pyrimidin-2,4-dion;
Verbindung 52: 5-(3-chlor-2-methyl-phenyl)-3-ethyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 53: 5-(2,3-dichlor-phenyl)-3-ethyl-1-{2-oxo-2-[4-(2-oxo-1,2,4,5-tetrahydro-benzo[d][1,3]diazepin-3-yl)-piperidin-1-yl]-ethyl}-1H-pyrimidin-2,4-dion;
Verbindung 54: 5-(2-chlor-3-fluor-phenyl)-3-ethyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 72: (5-(2-Chlor-3-fluor-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-acetonitril;
Verbindung 73: 3-(5-(2-Chlor-3-fluor-phenyl)-2,6-dioxo-3-{2-oxo-2-[4-(2-oxo-1,2,4,5-tetrahydrobenzo[d][1,3]diazepin-3-yl)-piperidin-1-yl]-ethyl}-3,6-dihydro-2H-pyrim idin-1-yl)-Propionsäure;
Verbindung 83: 5-(2,3-dichlor-phenyl)-3-isobutyl-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 89: 3-(2-amino-ethyl)-5-(2,3-dichlor-phenyl)-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 90: 5-(2,3-dichlor-phenyl)-3-(2-methylaminoethyl)-1-{2-oxo-2-[4-(2-oxo-1,2,4,5-tetrahydrobenzo[d][1,3]diazepin-3-yl)-piperidin-1-yl]-ethyl}-1H-pyrimidin-2,4-dion;
Verbindung 93; 5-(2,3-dimethyl-phenyl)-3-(2-methoxy-ethyl)-1-{2-oxo-2-[4-(2-oxo-1,2,4,5-tetrahydro-benzo[d][1,3]diazepin-3-yl)-piperidin-1-yl]-ethyl}-1H-pyrimidin-2,4-dion;
Verbindung 95: 5-(3-chlor-2-methoxy-phenyl)-3-(2-methoxyethyl)-1-{2-oxo-2-[4-(2-oxo-1,2,4,5-tetrahydrobenzo[d][1,3]diazepin-3-yl)-piperidin-1-yl]-ethyl}-1H-pyrimidin-2,4-dion;
Verbindung 96: 5-(2-chlor-3-fluor-phenyl)-3-(2-methoxyethyl)-1-{2-oxo-2-[4-(2-oxo-1,2,4,5-tetrahydrobenzo[d][1,3]diazepin-3-yl)-piperidin-1-yl]-ethyl}-1H-pyrimidin-2,4-dion;
Verbindung 97: 5-(2-chlor-3-methoxy-phenyl)-3-(2-methoxyethyl)-1-{2-oxo-2-[4-(2-oxo-1,2,4,5-tetrahydrobenzo[d][1,3]diazepin-3-yl)-piperidin-1-yl]-ethyl}-1H-pyrimidin-2,4-dion;
Verbindung 98: 5-(2,3-dimethyl-phenyl)-3-(2-hydroxy-ethyl)-1-{2-oxo-2-[4-(2-oxo-1,2,4,5-tetrahydro-benzo[d][1,3]diazepin-3-yl)-piperidin-1-yl]-ethyl}-1H-pyrimidin-2,4-dion;
Verbindung 100: 5-(2,3-dimethyl-phenyl)-3-(2-methoxymethoxy-ethyl)-1-{2-oxo-2-[4-(2-oxo-1,2,4,5-tetrahydrobenzo[d][1,3]diazepin-3-yl)-piperidin-1-yl]-ethyl}-1H-pyrimidin-2,4-dion;
Verbindung 101: N-[2-(5-(2,3-dimethyl-phenyl)-2,6-dioxo-3-{2-oxo-2-[4-(2-oxo-1,2,4,5-tetrahydro-benzo[d][1, 3]diazepin-3-yl)-piperidin-1-yl]-ethyl}-3,6-dihydro-2H-pyrim idin-1-yl)-ethyl]-acetamid;
Verbindung 103: (5-(2-chlor-3-fluor-phenyl)-2,6-dioxo-3-{2-oxo-2-[4-(2-oxo-1,2,4,5-tetrahydro-benzo[d][1, 3]diazepin-3-yl)-piperidin-1-yl]-ethyl}-3,6-dihydro-2H-pyrim idin-1-yl)-Methylacetat;
Verbindung 104: (5-(2-chlor-3-fluor-phenyl)-2,6-dioxo-3-{2-oxo-2-[4-(2-oxo-1,2,4,5-tetrahydrobenzo[d][1,3]diazepin-3-yl)-piperidin-1-yl]-ethyl}-3,6-dihydro-2H-pyrimidin-1-yl)-Essigsäure;
Verbindung 105: 5-(2-chlor-3-fluor-phenyl)-3-oxetan-3-ylmethyl-1-{2-oxo-2-[4-(2-oxo-1,2,4,5-tetrahydroben zo[d][1,3]diazepin-3-yl)-piperidin-1-yl]-ethyl}-1H-pyrimidin-2,4-dion;
Verbindung 106: 5-(2-chlor-3-fluor-phenyl)-3-((S)-2-methoxy-1-methyl-ethyl)-1-{2-oxo-2-[4-(2-oxo-1,2,4,5-tetrahydro-benzo[d][1,3]diazepin-3-yl)-piperidin-1-yl]-ethyl}-1H-pyrimidin-2,4-dion;
Verbindung 108: 5-(2-chlor-3-methoxy-phenyl)-3-((S)-2-methoxy-1-methyl-ethyl)-1-{2-oxo-2-[4-(2-oxo-1,2,4,5-tetrahydro-benzo[d][1,3]diazepin-3-yl)-piperidin-1-yl]-ethyl}-1H-pyrimidin-2,4-dion;
Verbindung 110: 5-(2-chlor-3-fluor-phenyl)-3-((R)-2-methoxy-1-methyl-ethyl)-1-{2-oxo-2-[4-(2-oxo-1,2,4,5-tetrahydro-benzo[d][1,3]diazepin-3-yl)-piperidin-1 -yl]-ethyl}-1H-pyrimidin-2,4-dion;
Verbindung 112: 5-(2-chlor-3-fluor-phenyl)-3-(2-methylsulfanyl-ethyl)-1-{2-oxo-2-[4-(2-oxo-1,2,4,5-tetrahydrobenzo[d][1,3]diazepin-3-yl)-piperidin-1-yl]-ethyl}-1H-pyrimidin-2,4-dion;
Verbindung 113: 5-(2,3-difluor-phenyl)-3-(2-methylsulfanyl-ethyl)-1-{2-oxo-2-[4-(2-oxo-1,2,4,5-tetrahydrobenzo[d][1,3]diazepin-3-yl)-piperidin-1-yl]-ethyl}-1H-pyrimidin-2,4-dion;
Verbindung 114: 3-(5-(2-chlor-3-fluor-phenyl)-2,6-dioxo-3-{2-oxo-2-[4-(2-oxo-1,2,4,5-tetrahydro-benzo[d][1, 3]diazepin-3-yl)-piperidin-1-yl]-ethyl}-3,6-dihydro-2H-pyrim idin-1-yl)-Methylpropanoat;
Verbindung 115: N-[(S)-2-(5-(2-chlor-3-fluor-phenyl)-3-{2-[4-(7-methoxy-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidin-1-yl)-propyl]-acetamid;
Verbindung 116: N-[(S)-2-(5-(2-chlor-3-fluor-phenyl)-3-{2-[4-(7-methoxy-5-methyl-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidin-1-yl)-propyl]-acetamid;
Verbindung 117: N-[2-(5-(2-chlor-3-fluor-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-ethyl]-acetamid;
Verbindung 118: N-[2-(5-(2-chlor-3-fluor-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-ethyl]-N-methyl-acetamid;
Verbindung 119: 3-((S)-2-amino-1-methyl-ethyl)-5-(2-chlor-3-fluor-phenyl)-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidin-2,4-dion;
Verbindung 120: N-[(S)-2-(5-(2-chlor-3-fluor-phenyl)-3-{2-[4-(7-methoxy-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidin-1-yl)-propyl]-methansulfonamid;
Verbindung 121: 5-(2-chlor-3-fluor-phenyl)-1 -{2-[4-(7-methoxy-2-oxo-1,2,4,5-tetrahydro-benzo[d][1,3]diazepin-3-yl)-piperidin-1 -yl]-2-oxo-ethyl}-3-[(S)-1 -methyl-2-(3-methyloxetan-3-ylamino)-ethyl]-1H-pyrimidin-2,4-dion;
Verbindung 122: 5-(2-chlor-3-fluor-phenyl)-3-((S)-2-hydroxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 123: (S)-2-(5-(2-chlor-3-fluor-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-Propylacetat;
Verbindung 124: 5-(2-chlor-3-methoxy-phenyl)-3-((S)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H pyrimidin-2,4-dion;
Verbindung 126: 5-(2,3-difluor-phenyl)-3-((S)-2-methoxy-1-methyl-ethyl)-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidin-2,4-dion;
Verbindung 130: 5-(2-chlor-3-methoxy-phenyl)-3-((R)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 131: 5-(2-chlor-3-fluor-phenyl)-3-((R)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 132: 5-(2,3-dichlor-phenyl)-3-((S)-2-methoxy-1-methyl-ethyl)-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidin-2,4-dion;
Verbindung 134: 5-(3-brom-2-fluor-phenyl)-3-((R)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 135: 5-(2-chlor-3-fluor-phenyl)-3-(2-methansulfonyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 136: 5-(2,3-difluor-phenyl)-3-(2-methansulfonyl-ethyl)-1-{2-[4-(7-methoxy-2-oxo-1,2,4,5-tetrahydrobenzo[d][1,3]diazepin-3-yl)-piperidin-1-yl]-2-ozoethyl}-1H-pyrimidin-2,4-dion;
Verbindung 137: 5-(2,3-dichlor-phenyl)-3-(2-methansulfonyl-ethyl)-1-{2-[4-(7-methoxy-2-oxo-1,2,4,5-tetrahydrobenzo[d][1,3]diazepin-3-yl)-piperidin-1-yl]-2-oxoethyl}-1H-pyrimidin-2,4-dion;
Verbindung 138: 5-(2,3-dimethyl-phenyl)-3-(2-methansulfonyl-ethyl)-1-{2-[4-(7-methoxy-2-oxo-1,2,4,5-tetrahydrobenzo[d][1,3]diazepin-3-yl)-piperidin-1-yl]-2-oxoethyl}-1H-pyrimidin-2,4-dion;
Verbindung 139: 5-(2,3-dimethoxy-phenyl)-3-(2-methansulfonyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 140: 5-(2-chlor-3-methoxy-phenyl)-3-(2-methansulfonyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 142: 5-(2-chlor-3-fluor-phenyl)-3-((S)-2-methansulfonyl-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 143: 5-(2-chlor-3-fluor-phenyl)-3-((R)-2-methansulfonyl-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 144: 5-(2,3-dichlor-phenyl)-3-((S)-2-methansulfonyl-1-methyl-ethyl)-1-{2-[4-(7 -methoxy-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 145: 5-(2,3-dichlor-phenyl)-3-((R)-2-methansulfonyl-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 146: 5-(2-chlor-3-fluor-phenyl)-3-isopropyl-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidin-2,4-dion;
Verbindung 148: 5-(2-chlor-3-methoxy-phenyl)-3-isopropyl-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidin-2,4-dion;
Verbindung 152: 3-isopropyl-1-{2-[4-(7-methoxy-2-oxo-1,2,4,5-tetrahydro-benzo[d][1,3]diazepin-3-yl)-piperidin-1-yl]-2-oxo-ethyl}-5-(3-methoxy-2-trifluormethoxy-phenyl)-1H-pyrimidin-2,4-dion;
Verbindung 155: 5-(2-chlor-3-fluor-phenyl)-1-{2-[4-(7-methoxy-2-oxo-1,2,4,5-tetrahydro-benzo[d][1,3]diazepin-3-yl)-piperidin-1-yl]-2-oxo-ethyl}-3-(2-methylsulfanyl-ethyl)-1H-pyrimidin-2,4-dion;
Verbindung 156: 5-(2-chlor-3-fluor-phenyl)-3-(3-methansulfonyl-propyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidin-2,4-dion;
Verbindung 157: 5-(2-chlor-3-fluor-phenyl)-1-[2-[4-(7-methoxy-2-oxo-4,5-dihydro-1H-1,3-benzodiazepin-3-yl)-1-piperidyl]-2-oxo-ethyl]-3-[2-(methylsulfonimidoyl)ethyl]pyrimidin-2,4-dion;
Verbindung 158: N-[(S)-2-(5-(2-chlor-3-fluor-phenyl)-3-{2-[4-(7-methoxy-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidin-1-yl)-propyl]-propionamid;
Verbindung 159: 5-(2-Chlor-3-fluor-phenyl)-1-{2-[4-(7-fluor-2-oxo-1,2,4,5-tetrahydro-benzo[d][1,3]diazepin-3-yl)-piperidin-1-yl]-2-oxo-ethyl}-3-((S)-2-methoxy-1-methyl-ethyl)-1H-pyrimidin-2,4-dion;
Verbindung 160: 5-(2-Chlor-3-fluor-phenyl)-1-{2-[4-(7-fluor-2-oxo-1,2,4,5-tetrahydro-benzo[d][1,3]diazepin-3-yl)-piperidin-1-yl]-2-oxo-ethyl}-3-(2-methansulfonyl-ethyl)-1H-pyrimidin-2,4-dion;
Verbindung 161: N-[(S)-2-(5-(2-Chlor-3-fluor-phenyl)-3-{2-[4-(7-fluor-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidin-1-yl)-propyl]-acetamid;
Verbindung 162: N-[(S)-2-(5-(2,3-Dichlor-phenyl)-3-{2-[4-(7-fluor-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamid;
Verbindung 165: 5-(2-chlor-3-fluor-phenyl)-3-((S)-2-methoxy-1-methyl-ethyl)-2,4-dioxo-3,4-dihydro-2H-pyrimidin-1-yl]-1-[(2R)-2'-oxospiro[1,3-dihydroinden-2,3'-1H-pyrrol[2,3-b]pyridin]-5-yl]-acetamid;
Verbindung 166: 5-(2-chlor-3-fluor-phenyl)-3-isopropylpyrimidin-2,4-dion-1-[(2R)-2'-oxospiro[1,3-dihydroinden-2,3'-1H-pyrrol[2,3-b]pyridin]-5-yl]-acetamid;
Verbindung 170: 5-(2,3-dichlorphenyl)-3-(2-methylsulfonylethyl)-pyrimidin-2,4-dion-1-[(2R)-2'-oxospiro[1,3-dihydroinden-2,3'-1H-pyrrol[2,3-b]pyridin]-5-yl]-acetamid;
Verbindung 178: 5-(2,3-dichlorphenyl)-3-[(1S)-2-methoxy-1-methyl-ethyl]-pyrimidin-2,4-dion-1-[(2R)-2'-oxospiro[1,3-dihydroinden-2,3'-1H-pyrrol[2,3-b]pyridin]-5-yl]-acetamid;
Verbindung 184: 5-(2-Chlor-3-fluor-phenyl)-3-(2-methansulfonyl-ethyl)-1-{2-[4-(7-methansulfonyl-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 185: 5-(2-Chlor-3-fluor-phenyl)-3-isopropyl-1-{2-[4-(7-methansulfonyl-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 186: 5-(2-chlor-3-fluor-phenyl)-3-(2-methansulfonyl-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 187: 5-(2-Chlor-3-fluor-phenyl)-3-((R)-2-methoxy-1-methyl-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 188: 5-(2-Chlor-3-fluor-phenyl)-3-(2-methoxy-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 189: 5-(2-Chlor-3-fluor-phenyl)-3-isopropyl-1-{2-[4-(7-methylsulfanyl-2-ozo-1,2,4,5-tetrahydro-benzo[d][1,3]diazepin-3-yl)-piperidin-1-yl]-2-oxo-ethyl}-1H-pyrimidin-2,4-dion;
Verbindung 192: 3-(1-{2-[5-(2-chlor-3-fluor-phenyl)-3-(2-methoxy-ethyl)-2-oxo-3,4-dihydro-2H-pyrimidin-1-yl]-acetyl}-piperidin-4-yl)-1,3,4,5-tetrahydro-benzo[d][1,3]diazepin-2-on;
Verbindung 199: 3-(1-{2-[5-(2,3-Dichlor-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-on;
Verbindung 202: N-[2-(5-(2-chlor-3-fluor-phenyl)-2,6-dioxo-3-{2-oxo-2-[4-(2-oxo-1,2,4,5-tetrahydro-benzo[d][1, 3]diazepin-3-yl)-piperidin-1-yl]-ethyl}-3,6-dihydro-2H-pyrim idin-1-yl)-ethyl]-acetamid;
Verbindung 203: N-[(R)-2-(5-(2-chlor-3-fluor-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamid;
Verbindung 205: N-[(S)-2-(5-(2,3-dichlor-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamid;
Verbindung 206: N-[(S)-2-(5-(2-chlor-3-methoxy-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamid;
Verbindung 207: N-[2-(5-(2,3-dichlor-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-ethyl]-acetamid;
Verbindung 208: 5-(2,3-dichlorphenyl)-3-methyl-1-[2-oxo-2-[4-(2-oxo-5-phenyl-1H-imidazol-3-yl)-1-piperidyl]ethyl]pyrimidin-2,4-dion;
Verbindung 210: 5-(2,3-dichlorphenyl)-3-isopropyl-1-[2-oxo-2-[4-(2-oxo-4,5-dihydro-1H-1,3-benzodiazepin-3-yl)-1-piperidyl]ethyl]pyrimidin-2,4-dion;
Verbindung 213: 5-(2,3-dichlorphenyl)-3-methyl-1-[2-oxo-2-[4-(2-oxo-4,5-dihydro-1H-pyrido[2,3-d][1,3]diazepin-3-yl)-1-piperidyl]ethyl]pyrimidin-2,4-dion;
Verbindung 215: 5-(2,3-dichlorphenyl)-3-methyl-1-[2-oxo-2-[4-(2-oxo-1H-quinolin-3-yl)-1-piperidyl]ethyl]pyrimidin-2,4-dion;
Verbindung 220: 5-(3,4-dichlorphenyl)-3-methyl-1-[2-oxo-2-[4-(2-oxo-4,5-dihydro-1H-1,3-benzodiazepin-3-yl)-1-piperidyl]ethyl]pyrimidin-2,4-dion;
Verbindung 221: 1-[2-[4-(7-brom-2-oxo-4,5-dihydro-1H-1,3-benzodiazepin-3-yl)-1-piperidyl]-2-oxo-ethyl]-5-(2-chlor-3-fluor-phenyl)-3-isopropyl-pyrim idin-2,4-dion;
Verbindung 225: 5-(2-chlor-3-fluor-phenyl)-3-isopropyl-1-[2-[4-(7-methylsulfinyl-2-oxo-4,5-dihydro-1H-1,3-benzodiazepin-3-yl)-1-piperidyl]-2-oxo-ethyl]pyrimidin-2,4-dion;
Verbindung 226: 2-[5-(2-chlor-3-fluor-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]acetamid;
Verbindung 227: 5-(2-chlor-3-fluor-phenyl)-3-(2-methylsulfinylethyl)-1-[2-oxo-2-[4-(2-oxo-4,5-dihydro-1H-1,3-benzodiazepin-3-yl)-1-piperidyl]ethyl]pyrimidin-24-dion;
Verbindung 228: 5-(2-chlor-3-fluor-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidin-2,4-dion;
Verbindung 229: 5-(2-chlor-3-fluor-phenyl)-3-((S)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidin-2,4-dion;
Verbindung 231: 5-(2,3-difluorphenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidin-2,4-dion;
Verbindung 232: 5-(2-chlor-3-fluor-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidin-2,4-dion;
Verbindung 233: 5-(2-chlor-3-fluor-phenyl)-3-[(1S)-2-methoxy-1-methyl-ethyl]-1-[2-[4-(7-methylsulfanyl-2-oxo-4,5-dihydro-1H-1,3-benzodiazepin-3-yl)-1-piperidyl]-2-oxo-ethyl]pyrimidin-2,4-dion;
Verbindung 234: 5-(2-chlor-3-fluor-phenyl)-3-(2-hydroxy-2-methyl-propyl)-1-[2-oxo-2-[4-(2-oxo-4,5-dihydro-1H-1,3-benzodiazepin-3-yl)-1-piperidyl]ethyl]pyrimidin-2,4-dion;
Verbindung 235: 5-(2-chlor-3-fluor-phenyl)-1-[2-oxo-2-[4-(2-oxo-4,5-dihydro-1H-1,3-benzodiazepin-3-yl)-1-piperidyl]ethyl]-3-(2H-tetrazol-5-ylmethyl)pyrim idin-2,4-dion;
Verbindung 236: 5-(2,3-difluorphenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidin-2,4-dion;
Verbindung 237: 5-(2-chlor-3-fluor-phenyl)-1-[2-[4-(7-methoxy-2-oxo-4,5-dihydro-1H-1,3-benzodiazepin-3-yl)-1-piperidyl]-2-oxo-ethyl]-3-[(1S)-1-methylpropyl]pyrimidin-2,4-dion;
Verbindung 238: 5-(2-chlor-3-fluor-phenyl)-1-[2-[4-(7-methoxy-2-oxo-4,5-dihydro-1H-1,3-benzodiazepin-3-yl)-1-piperidyl]-2-oxo-ethyl]-3-[(1R)-1-methylpropyl]pyrimidin-2,4-dion;
Verbindung 239: 5-(2-chlor-3-fluor-phenyl)-3-[1-(methoxymethyl)propyl]-1-[2-[4-(7-methoxy-2-oxo-4,5-dihydro-1H-1,3-benzodiazepin-3-yl)-1-piperidyl]-2-oxo-ethyl]pyrimidin-2,4-dion;
Verbindung 240: 5-(2-chlor-3-fluor-phenyl)-3-(2-methoxy-2-methyl-propyl)-1-[2-[4-(7-methoxy-2-oxo-4,5-dihydro-1H-1,3-benzodiazepin-3-yl)-1-piperidyl]-2-oxo-ethyl]pyrimidin-2,4-dion;
Verbindung 241: 5-(2-chlor-3-methoxy-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidin-2,4-dion;
Verbindung 242: 5-(2,3-dichlorphenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidin-2,4-dion;
Verbindung 243: 5-(2-chlor-3-methoxy-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidin-2,4-dion;
Verbindung 244: 5-(2-chlor-3-fluor-phenyl)-3-[(1S)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidin-2,4-dion;
Verbindung 245: 5-(2,3-dichlorphenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidin-2,4-dion;
Verbindung 246: 3-[5-(2-chlor-3-fluor-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]Propionsäure;
Verbindung 247: 5-(2-chlor-3-fluor-phenyl)-1-[2-[4-(7-methoxy-2-oxo-4,5-dihydro-1H-1,3-benzodiazepin-3-yl)-1-piperidyl]-2-oxo-ethyl]-3-[2-(1,3,4-oxadiazol-2-yl)ethyl]pyrimidin-2,4-dion;
Verbindung 248: 2-[5-(2-chlor-3-fluor-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]Essigsäure;
Verbindung 249: 5-(2-chlor-3-fluor-phenyl)-3-[(1R)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidin-2,4-dion;
Verbindung 251: 5-(2-chlor-3-fluor-phenyl)-1-[2-[4-(7-methoxy-2-oxo-4,5-dihydro-1H-1,3-benzodiazepin-3-yl)-1-piperidyl]-2-oxo-ethyl]-3-[2-(1,2,4-triazol-4-yl)ethyl]pyrimidin-2,4-dion;
Verbindung 252: 5-(2-chlor-3-fluor-phenyl)-1-[2-[4-(7-fluor-2-oxo-4,5-dihydro-1H-1,3-benzodiazepin-3-yl)-1-piperidyl]-2-oxo-ethyl]-3-[(1R)-1 -methyl-2-methylsulfonyl-ethyl]pyrimidin-2,4-dion;
Verbindung 253: 3-[1-[2-[5-(2-chlor-3-fluor-phenyl)-3-(2-methylsulfonylethyl)-2,4-dioxo-pyrimidin-1-yl]acetyl]-4-piperidyl]-2-oxo-4,5-dihydro-1H-1,3-benzodiazepin-7-Carbonsäure;
Verbindung 254: S-[2-[5-(2-chlor-3-fluor-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethyl]ethanthioat;
Verbindung 255: N-[(2S)-2-[5-(2,3-dichlorphenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamid;
Verbindung 256: N-[(2S)-2-[5-(2-chlor-3-fluor-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamid;
Verbindung 257: 2-[5-(2-chlor-3-fluor-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]Ethansulfonsäure;
Verbindung 258: 5-(2-chlor-3-fluor-phenyl)-3-[(1S)-1-(methoxymethyl)-2-methylsulfonyl-ethyl]-1-[2-[4-(7-methoxy-2-oxo-4,5-dihydro-1H-1,3-benzodiazepin-3-yl)-1-piperidyl]-2-oxo-ethyl]pyrimidin-2,4-dion; und
Verbindung 259: 5-(2-chlor-3-fluor-phenyl)-1-[2-[4-(7-methoxy-2-oxo-4,5-dihydro-1H-1,3-benzodiazepin-3-yl)-1-piperidyl]-2-oxo-ethyl]-3-(2-methyl-2-methylsulfonyl-propyl)pyrimidin-2,4-dion.

6. Verbindung der allgemeinen Formel (I) nach einem der vorherigen Ansprüche zu ihrer Verwendung als Medikament.

7. Verbindung der allgemeinen Formel (I) nach einem der Ansprüche 1 bis 5 zu ihrer Verwendung bei der Vorbeugung und/oder der Behandlung von entzündlichen Krankheiten mit einer neurogenen Komponente.

8. Verbindung der allgemeinen Formel (I) zu ihrer Verwendung nach Anspruch 7, wobei die entzündlichen Krankheiten der Haut mit einer neurogenen Komponente aus der Rosacea Typ I (erythematös) und Typ II (papulopustulös), der atopischen Dermatitis, dem chronischen Handekzem, der Psoriasis in ihren verschiedenen Formen (vulgär, Kopfhaut, arthritisch, pustulös, guttata), dem Gesichtserythem, dem Schamerythem, der Urtikaria (akut, seniler Pruritus, Prurigo nodularis) und der Akne ausgewählt sind.

9. Verbindung der allgemeinen Formel (I) zu ihrer Verwendung nach Anspruch 8 bei der Vorbeugung und/oder der Behandlung der Rosacea Typ I (erythematös), der atopischen Dermatitis, der vulgären und Kopfhaut-Psoriasis und der Urtikaria des Typs seniler Pruritus und Prurigo nodularis.

10. Verbindung der allgemeinen Formel (I) zu ihrer Verwendung nach Anspruch 9 bei der Vorbeugung und/oder der Behandlung der Rosacea Typ I (erythematös).

11. Verbindung der allgemeinen Formel (I) zu ihrer Verwendung nach Anspruch 7 bei der Behandlung der Migräne.

12. Pharmazeutische Zusammensetzung, die mindestens eine Verbindung der allgemeinen Formel (I), wie in einem der Ansprüche 1 bis 4 definiert, und einen pharmazeutisch annehmbaren Träger umfasst, der vorzugsweise für eine topische Verabreichung geeignet ist.

13. Kosmetische Verwendung einer Verbindung der allgemeinen Formel (I) nach einem der Ansprüche 1 bis 4.

## Claims

1. Heterocyclic compound selected from the compounds of general formula (I): 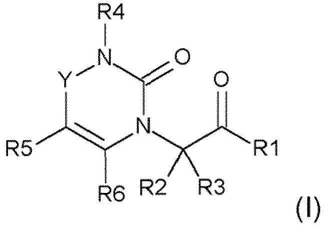 wherein
- Y is selected from -CH₂, -C(O), -C(CH₃)₂, or a spirocyclopropyl;
- R1 is selected from the following groups (1-1) to (1-12): 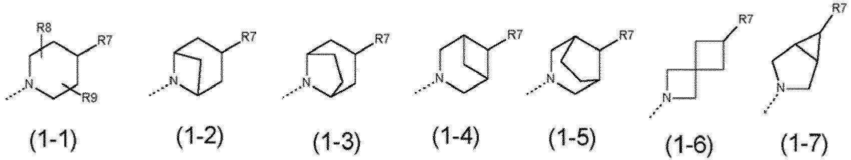 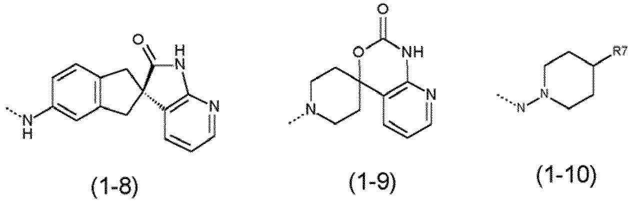 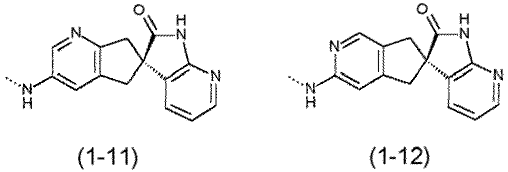
- R2, R3, R6 are identical or different, and selected from a hydrogen atom, F or -CH₃;
- R4 is selected from a hydrogen atom, an alkyl, an alkene, an alkyne, a cycloalkyl, a cycloalkene, an aralkyl, a heteroaralkyl, or the following groups (4-1) to (4-55): 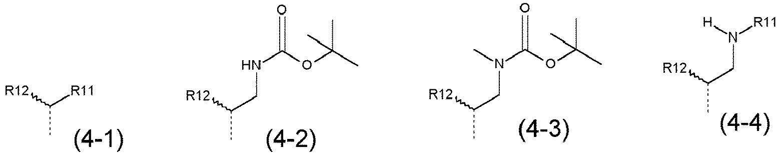 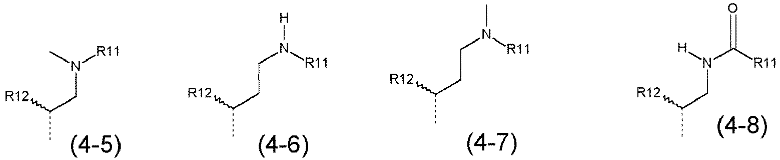 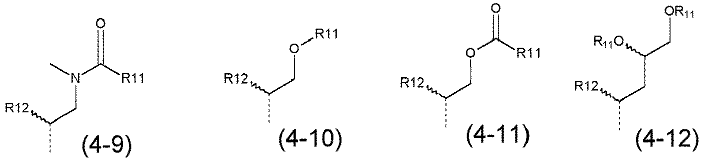 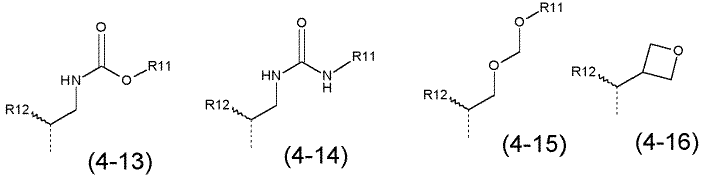 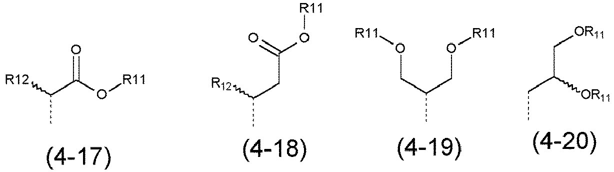 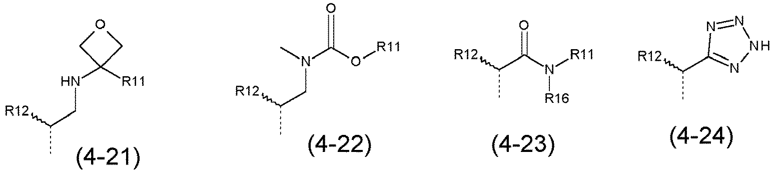 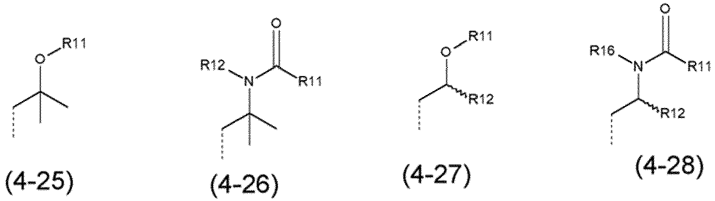 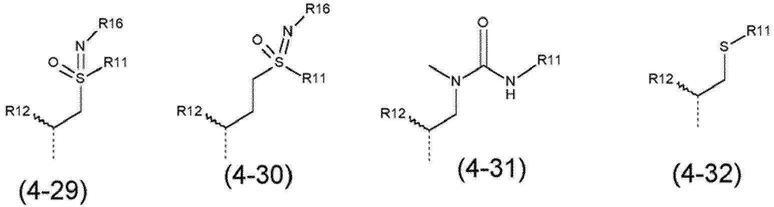 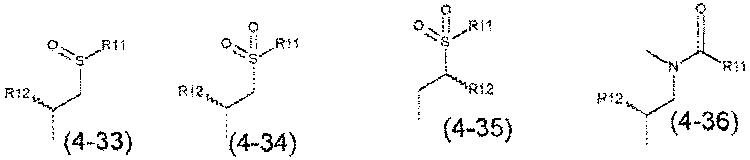 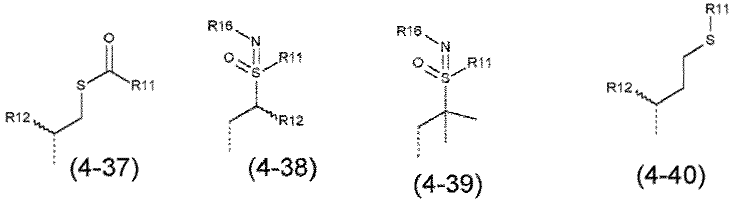 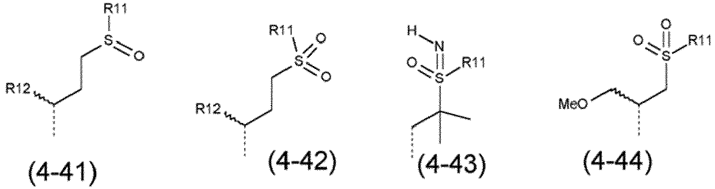 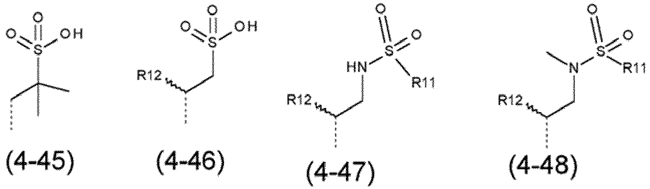 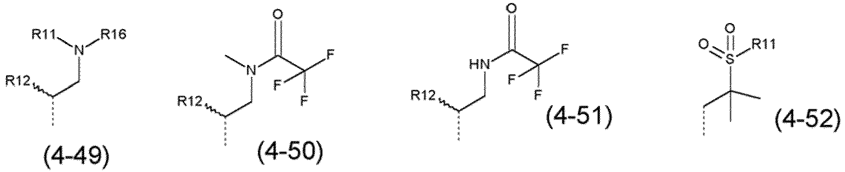 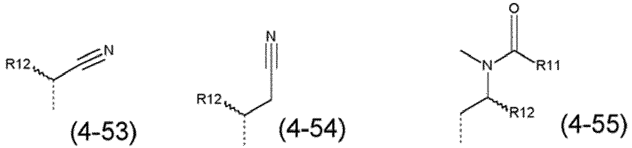
- R5 is selected from a halogen, an alkyl, a cycloalkyl possibly substituted by R13, R14 and/or R15, an alkene, a cycloalkene, an alkyne, an ether or the following groups (5-1) to (5-5): 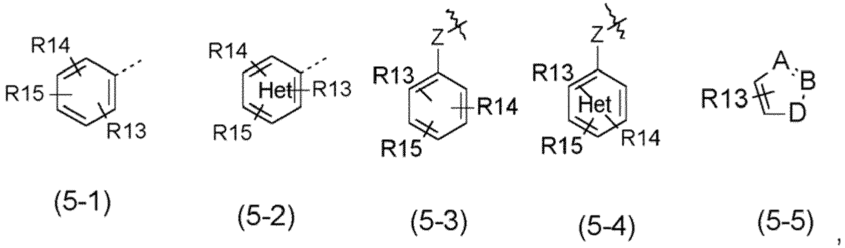 wherein Het represents 1 to 3 nitrogen atoms among the 6 atoms of the aromatic ring, wherein these nitrogen atoms can, independently of each other, be substituted by an oxygen atom to form an N-oxide group;
- R7 is selected from the following groups (7-1) to (7-28): 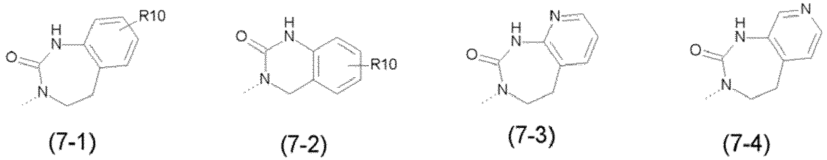 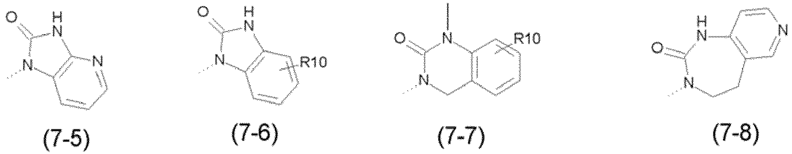 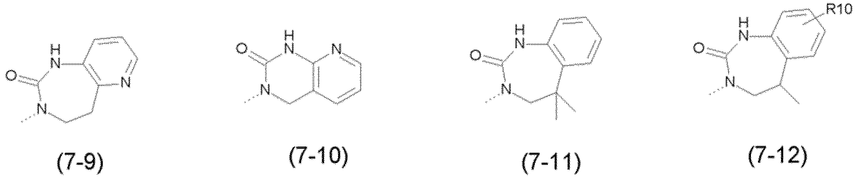 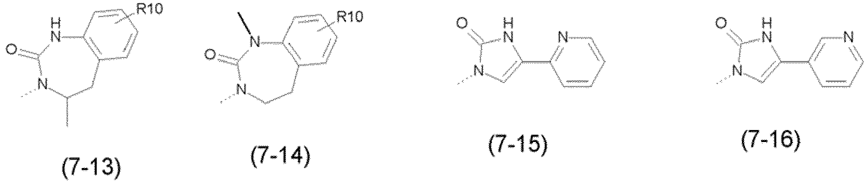 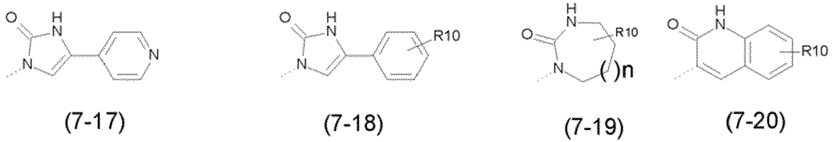 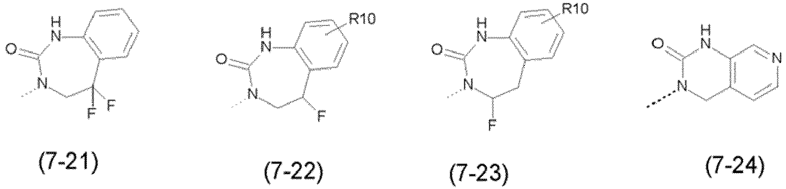 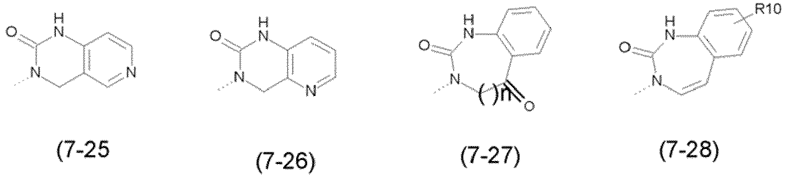 wherein n = 0 or 1;
- R8, R9 are identical or different, and selected from a hydrogen atom, For -CH₃;
- R10 is selected from a hydrogen atom, -OR11, -SR11, -NR11R12, -S(O)R11, -SO₂R11, -OC(O)R11, -CO₂R11, a halogen, -NO₂, -CN, -C(O)NR11R12, -CFs or-OCF₃;
- R11, R12 are identical or different, and selected from a hydrogen atom, a C1-C6 alkyl, CF₃ or an ether;
- R13, R14, R15 are identical or different, and selected from a hydrogen atom, a C1-C6 alkyl, a cycloalkyl, -OR16, -NR16R17, a halogen, -OCF₃, -CF₃, -CN, -CO₂R16, -CONR16R17, -NO₂, -OCH₂OR16, -SR16, -S(O)R16, -SO₂R16 or an ether;
- A, B, D are identical or different, and selected from a C, N, O or S atom;
- R16 and R17 are identical or different, and selected from a hydrogen atom, a C1-C6 alkyl or -C(O)R18;
- Z is selected from -CH₂, O or -NR18; and
- R18 is selected from a hydrogen atom, a C1-C3 alkyl, as well as their pharmaceutically acceptable salts, and their enantiomers, and wherein
- an alkyl is a saturated, linear or branched hydrocarbon chain comprising from 1 to 8 carbon atoms, optionally substituted by one or more fluorine atoms;
- an alkene is an unsaturated, linear or branched hydrocarbon chain comprising 2 to 8 carbon atoms and comprising one or two double bonds, optionally substituted by one or more fluorine atoms;
- an alkyne is an unsaturated, linear or branched hydrocarbon chain comprising 2 to 8 carbon atoms and comprising one or two triple bonds, optionally substituted by one or more fluorine atoms;
- a cycloalkyl is a saturated, cyclic hydrocarbon chain comprising 3 to 7 carbon atoms, optionally substituted by one or more fluorine atoms;
- a cycloalkene is an unsaturated, cyclic hydrocarbon chain comprising 4 to 7 carbon atoms and comprising one or two double bonds, optionally substituted by one or more fluorine atoms;
- an aryl is an aromatic hydrocarbon ring or two fused aromatic hydrocarbon rings;
- an aralkyl is an alkyl substituted by an aryl;
- a heteroaryl is an aromatic heterocyclic radical comprising one or more heteroatoms selected from O, S and N, optionally substituted by one or more groups of atoms selected, for example from an alkyl, an alkoxy, an aryl, a substituted aryl, a halogen, a hydroxy, a cyano, a trifluoromethyl, a sulfide, a pentafluorosulfide, a sulfoxide, a sulfone, a carboxylic acid, an ester and a nitro;
- a heteroaralkyl is an alkyl substituted by a heteroaryl;
- a heterocycle designates, in particular, a cyclic or bicyclic, saturated or unsaturated hydrocarbon chain comprising one or more heteroatoms selected from O, S and N, optionally substituted by one or more groups selected from an alkyl, an alkoxy, a halogen, a hydroxyl, a cyano, a trifluoromethyl, a sulfide, a pentafluorosulfide, a sulfoxide, a sulfone, a carboxylic acid, an ester and a nitro;
- an ether is a saturated, linear or branched hydrocarbon chain comprising from 1 to 8 carbon atoms, and wherein 1 to 3 carbon atoms are replaced respectively by 1 to 3 oxygen atoms, wherein two oxygen atoms are always separated by at least one carbon atom, wherein said chain is optionally substituted by one or more fluorine atoms.

2. Compound of general formula (I) according to claim 1, wherein:
- Y is selected from -CH₂ or -C(O);
- R1 is selected from the following groups: 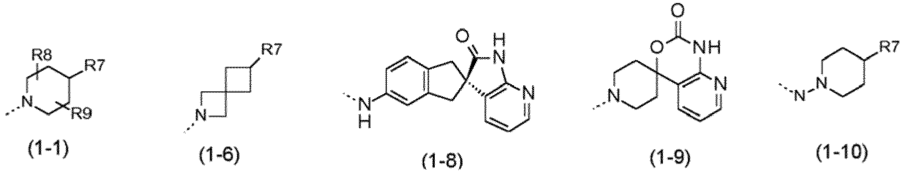
- R2, R3, R6 are identical or different, and selected from a hydrogen atom, F or -CH₃;
- R4 is selected from a C1-C4 alkyl, an aralkyl, a heteroaralkyl, or the following groups: 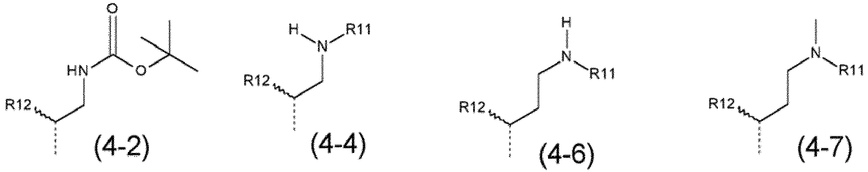 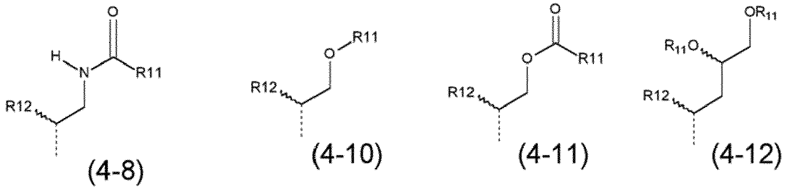 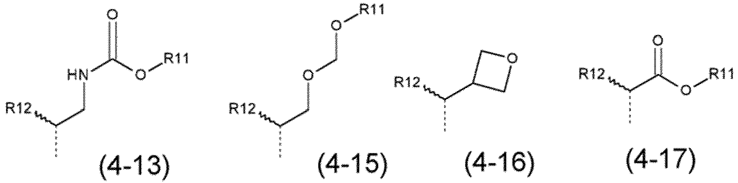 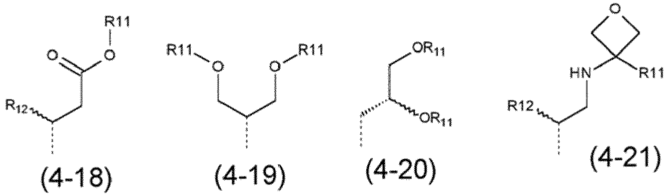 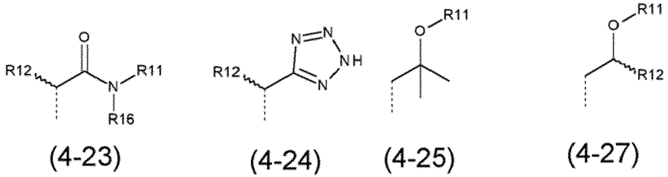 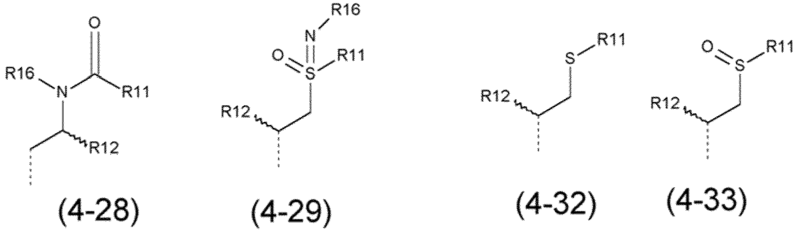 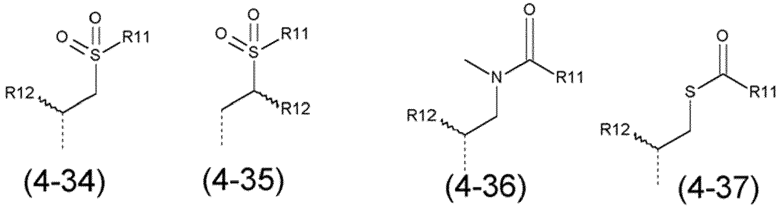 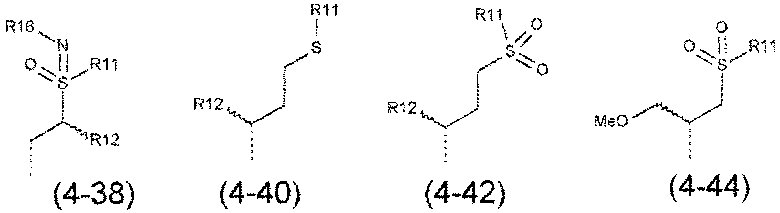 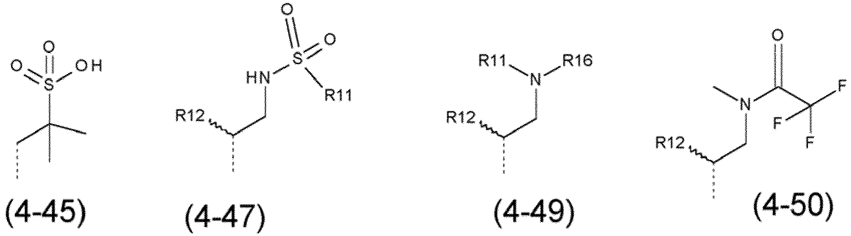 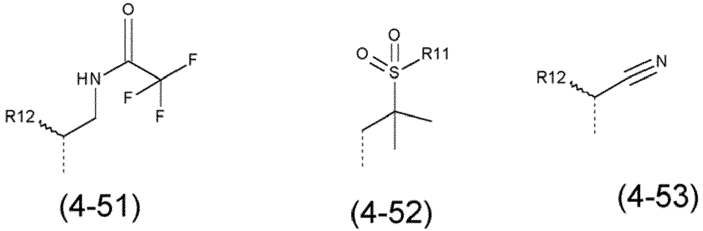
- R5 is selected from Br, -CH₃, a cyclohexene or the following groups: 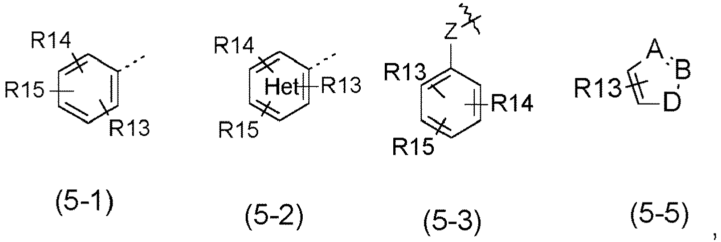 wherein Het represents 1 to 2 nitrogen atoms from the 6 atoms of the aromatic ring, wherein these nitrogen atoms are able to, independently of each other, be substituted by an oxygen atom to form an N-oxide group;
- R7 is selected from the following groups: 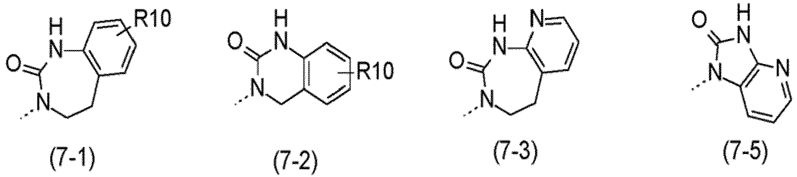 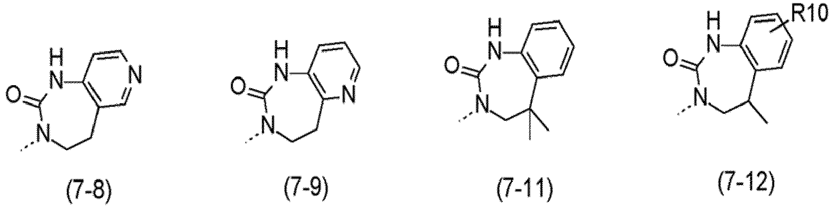 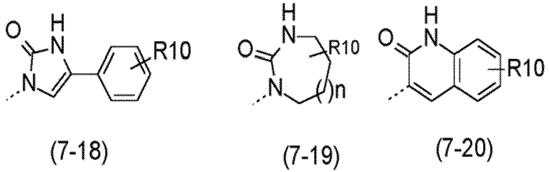 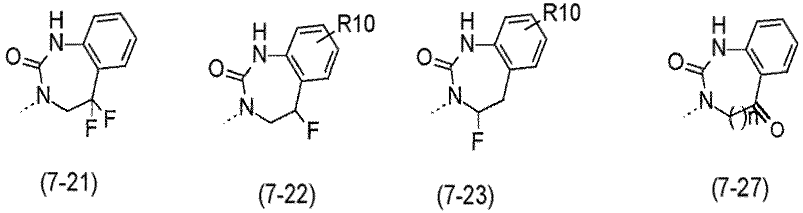 wherein n = 1;
- R8, R9 are identical or different, and selected from a hydrogen atom, F or -CH₃;
- R10 is selected from a hydrogen atom, -OR11, -SR11, -S(O)R11, -SO₂R11, -CO₂H, or a halogen selected from Br or F;
- R11, R12 are identical or different, and selected from a hydrogen atom or a C1-C3 alkyl;
- R13, R14, R15 are identical or different, and selected from a hydrogen atom,
- CH₃, -OR16, a halogen, -OCF₃, -CN, -CO₂R16, -SR16, -S(O)R16, or -SO₂R16;
- A, B, D are identical or different, and selected from a C, N or S atom;
- R16 is selected from a hydrogen atom or an alkyl; and
- Z is selected from -CH₂ or O.

3. Compound of general formula (I) according to claim 1 or 2, wherein:
- Y is selected from -CH₂ or -C(O);
- R1 is selected from the following groups: 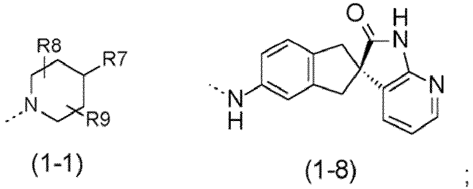
- R2, R3, R6 are a hydrogen atom;
- R4 is selected from a C1-C4 alkyl, a heteroaralkyl of formula 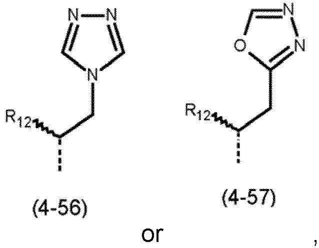 or the following groups: 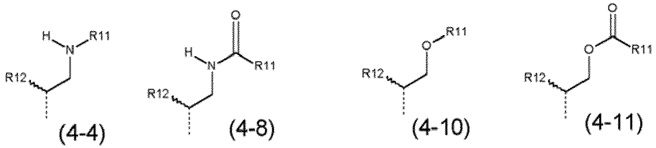 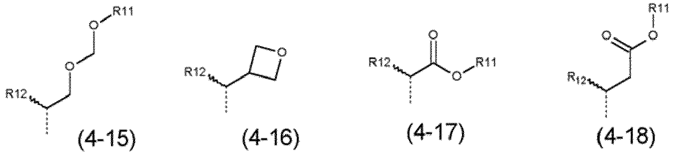 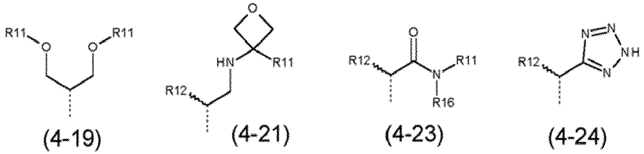 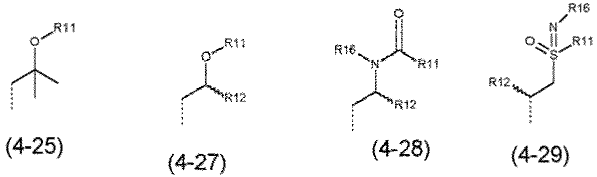 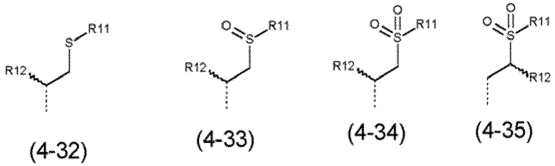 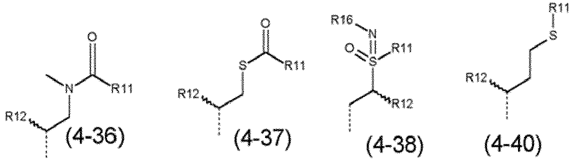 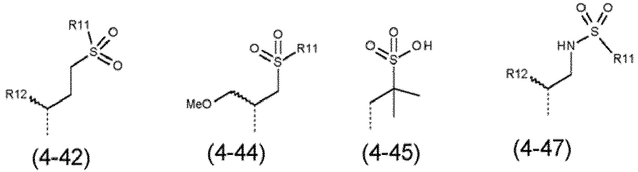 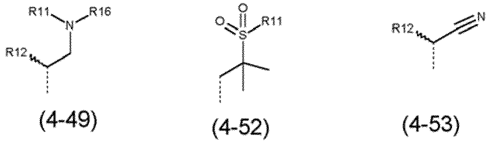
- R5 is 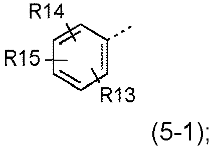
- R7 is selected from the following groups: 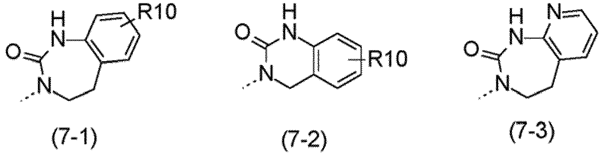 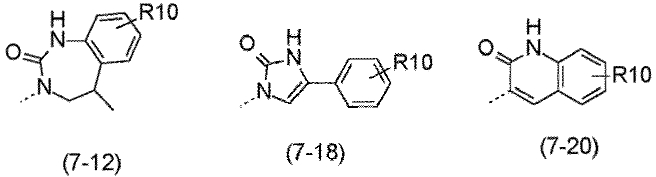
- R8, R9 are a hydrogen atom;
- R10 is selected from a hydrogen atom, -OR11, -SR11, -S(O)R11, -SO₂R11, CO₂H, or a halogen selected from Br or F;
- R11, R12 are identical or different, and selected from a hydrogen atom or a C1-C2 alkyl;
- R13, R14, R15 are identical or different, and selected from a hydrogen atom, -CH₃, - OR16, a halogen, or -OCF₃; and
- R16 is selected from a hydrogen atom or an alkyl.

4. Compound of general formula (I) according to one of claims 1 to 3, wherein:
- Y is -C(O);
- R1 is selected from the following groups: 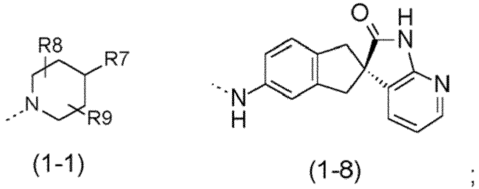
- R2, R3, R6 are a hydrogen atom;
- R4 is selected from the following groups: 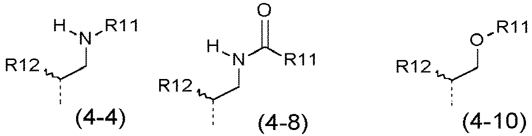 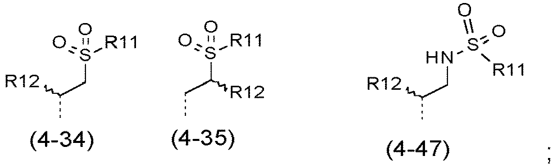 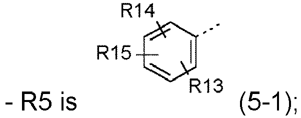
- R7 is 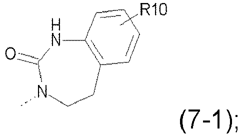
- R8, R9 are a hydrogen atom;
- R10 is selected from a hydrogen atom, -OR11, SR11 or Br;
- R13, R14, R15 are identical or different, and selected from a hydrogen atom, -OR16, or a halogen selected from F and Cl; and
- R16 is a C1-C3 alkyl.

5. Compound of general formula (I) according to claim 1, wherein the said compound is selected from:
compound 14: 5-(2-chloro-3-fluoro-phenyl)-3-((S)-2-methoxy-1-methyl-ethyl)-1-{2-oxo-2-[4-(2-oxo-1,4-dihydro-2Hquinazolin-3-yl)-piperidin-1-yl]-ethyl}-1H-pyrimidine-2,4-dione;
compound 16: 5-(2,3-difluoro-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 17: 5-(2,3-dichloro-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 18: 5-(2-chloro-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 19: 3-methyl-1-{2-oxo-2-[4-(2-oxo-1,2,4,5-tetrahydro-benzo[d][1,3]diazepin-3-yl)-piperidin-1-yl]-ethyl}-5-(2-trifluoromethoxy-phenyl)-1H-pyrimidine-2,4-dione;
compound 22: 5-(3-chloro-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 23: 5-(3-chloro-2-methyl-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 24: 5-(3-chloro-2-methoxy-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 28: 5-(3-fluoro-2-methyl-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 29: 5-(3-chloro-2-fluoro-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 30: 5-(2,3-dimethyl-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 31: 5-(2-chloro-3-fluoro-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 47: 5-(3,4-dichloro-phenyl)-3-methyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 51: 5-(2,3-dimethyl-phenyl)-3-ethyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 52: 5-(3-chloro-2-methyl-phenyl)-3-ethyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 53: 5-(2,3-dichloro-phenyl)-3-ethyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 54: 5-(2-chloro-3-fluoro-phenyl)-3-ethyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 72: (5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidine-1-yl)-acetonitrile;
compound 73: 3-(5-(2-Chloro-3-fluoro-phenyl)-2,6-dioxo-3-{2-oxo-2-[4-(2-oxo-1,2,4,5-tetrahydrobenzo[d][1,3]diazepin-3-yl)-piperidin-1-yl]-ethyl}-3,6-dihydro-2H-pyrimidine-1-yl)-propanoic acid;
compound 83: 5-(2,3-dichloro-phenyl)-3-isobutyl-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 89: 3-(2-amino-ethyl)-5-(2,3-dichloro-phenyl)-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 90: 5-(2,3-dichloro-phenyl)-3-(2-methylaminoethyl)-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 93; 5-(2,3-dimethyl-phenyl)-3-(2-methoxy-ethyl)-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 95: 5-(3-chloro-2-methoxy-phenyl)-3-(2-methoxyethyl)-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 96: 5-(2-chloro-3-fluoro-phenyl)-3-(2-methoxyethyl)-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 97: 5-(2-chloro-3-methoxy-phenyl)-3-(2-methoxyethyl)-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 98: 5-(2,3-dimethyl-phenyl)-3-(2-hydroxy-ethyl)-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 100: 5-(2,3-dimethyl-phenyl)-3-(2-methoxymethoxy-ethyl)-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 101: N-[2-(5-(2,3-dimethyl-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidine-1-yl)-ethyl]-acetamide;
compound 103: (5-(2-chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidine-1-yl)-methyl acetate;
compound 104: (5-(2-chloro-3-fluoro-phenyl)-2,6-dioxo-3-{2-oxo-2-[4-(2-oxo-1,2,4,5-tetrahydrobenzo[d][1,3]diazepin-3-yl)-piperidin-1-yl]-ethyl}-3,6-dihydro-2H-pyrimidine-1-yl)-acetic acid;
compound 105: 5-(2-chloro-3-fluoro-phenyl)-3-oxetan-3-ylmethyl-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 106: 5-(2-chloro-3-fluoro-phenyl)-3-((S)-2-methoxy-1-methyl-ethyl)-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 108: 5-(2-chloro-3-methoxy-phenyl)-3-((S)-2-methoxy-1-methyl-ethyl)-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 110: 5-(2-chloro-3-fluoro-phenyl)-3-((R)-2-methoxy-1-methyl-ethyl)-1-{2-oxo-2-[4-(2-oxo-1,2,4,5-tetrahydro-benzo[d][1,3]diazepin-3-yl)-piperidin-1-yl]-ethyl}-1H-pyrimidine-2,4-dione;
compound 112: 5-(2-chloro-3-fluoro-phenyl)-3-(2-methylsulfanyl-ethyl)-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 113: 5-(2,3-difluoro-phenyl)-3-(2-methylsulfanyl-ethyl)-1-{2-oxo-2-[4-(2-oxo-1,2,4,5-tetrahydrobenzo[d][1,3]diazepin-3-yl)-piperidin-1-yl]-ethyl}-1H-pyrimidine-2,4-dione;
compound 114: 3-(5-(2-chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidine-1-yl)- methyl propanoate;
compound 115: N-[(S)-2-(5-(2-chloro-3-fluoro-phenyl)-3-{2-[4-(7-methoxy-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidine-1-yl)-propyl]-acetam ide;
compound 116: N-[(S)-2-(5-(2-chloro-3-fluoro-phenyl)-3-{2-[4-(7-methoxy-5-methyl-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidine-1-yl)-propyl]-acetamide;
compound 117: N-[2-(5-(2-chloro-3-fluoro-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-ethyl]-acetamide;
compound 118: N-[2-(5-(2-chloro-3-fluoro-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-ethyl]-N-methyl-acetamide;
compound 119: 3-((S)-2-amino-1-methyl-ethyl)-5-(2-chloro-3-fluoro-phenyl)-1-{2-[4-(7-methoxy-2-oxo-1,2,4,5-tetrahydrobenzo[d][1,3]diazepin-3-yl)-piperidin-1-yl]-2-oxoethyl}-1H-pyrimidine-2,4-dione;
compound 120: N-[(S)-2-(5-(2-chloro-3-fluoro-phenyl)-3-{2-[4-(7-methoxy-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidine-1-yl)-propyl]-methanesulfonamide;
compound 121: 5-(2-chloro-3-fluoro-phenyl)-1-{2-[4-(7-methoxy-2-oxo-1,2,4,5-tetrahydro-benzo[d][1,3]diazepin-3-yl)-piperidin-1-yl]-2-oxo-ethyl}-3-[(S)-1-methyl-2-(3-methyloxetan-3-ylamino)-ethyl]-1H-pyrimidine-2,4-dione;
compound 122: 5-(2-chloro-3-fluoro-phenyl)-3-((S)-2-hydroxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidine-2,4-dione;
compound 123: (S)-2-(5-(2-chloro-3-fluoro-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-propyl acetate;
compound 124: 5-(2-chloro-3-methoxy-phenyl)-3-((S)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H pyrimidine-2,4-dione;
compound 126: 5-(2,3-difluoro-phenyl)-3-((S)-2-methoxy-1-methyl-ethyl)-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidine-2,4-dione;
compound 130: 5-(2-chloro-3-methoxy-phenyl)-3-((R)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidine-2,4-dione;
compound 131: 5-(2-chloro-3-fluoro-phenyl)-3-((R)-2-methoxy-1-methyl-ethyl)-1-{2-[4-(7-methoxy-2-oxo-1 2,4,5-tetrahydro-benzo[d][1 3]diazepin-3-yi)-piperidin-1 -yi]-2-oxoethyl}-1H-pyrimidine-2,4-dione;
compound 132: 5-(2,3-dichloro-phenyl)-3-((S)-2-methoxy-1-methyl-ethyl)-1-{2-[4-(7-methoxy-2-oxo-1,2,4,5-tetrahydrobenzo[d][1,3]diazepin-3-yl)-piperidin-1-yl]-2-oxoethyl}-1H-pyrimidine-2,4-dione;
compound 134: 5-(3-bromo-2-fluoro-phenyl)-3-((R)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidine-2,4-dione;
compound 135: 5-(2-chloro-3-fluoro-phenyl)-3-(2-methanesulfonyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidine-2,4-dione;
compound 136: 5-(2,3-difluoro-phenyl)-3-(2-methanesulfonyl-ethyl)-1-{2-[4-(7-methoxy-2-oxo-1,2,4,5-tetrahydrobenzo[d][1,3]diazepin-3-yl)-piperidin-1-yl]-2-ozoethyl}-1H-pyrimidine-2,4-dione;
compound 137: 5-(2,3-dichloro-phenyl)-3-(2-methanesulfonyl-ethyl)-1-{2-[4-(7-methoxy-2-oxo-1,2,4,5-tetrahydrobenzo[d][1,3]diazepin-3-yl)-piperidin-1-yl]-2-oxoethyl}-1H-pyrimidine-2,4-dione;
compound 138: 5-(2,3-dimethyl-phenyl)-3-(2-methanesulfonyl-ethyl)-1-{2-[4-(7-methoxy-2-oxo-1,2,4,5-tetrahydrobenzo[d][1,3]diazepin-3-yl)-piperidin-1-yl]-2-oxoethyl}-1H-pyrimidine-2,4-dione;
compound 139: 5-(2,3-dimethoxy-phenyl)-3-(2-methanesulfonyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidine-2,4-dione;
compound 140: 5-(2-chloro-3-methoxy-phenyl)-3-(2-methanesulfonyl-ethyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidine-2,4-dione;
compound 142: 5-(2-chloro-3-fluoro-phenyl)-3-((S)-2-methanesulfonyl-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidine-2,4-dione;
compound 143: 5-(2-chloro-3-fluoro-phenyl)-3-((R)-2-methanesulfonyl-1-methylethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidine-2,4-dione;
compound 144: 5-(2,3-dichloro-phenyl)-3-((S)-2-methanesulfonyl-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidine-2,4-dione;
compound 145: 5-(2,3-dichloro-phenyl)-3-((R)-2-methanesulfonyl-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidine-2,4-dione;
compound 146: 5-(2-chloro-3-fluoro-phenyl)-3-isopropyl-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidine-2,4-dione;
compound 148: 5-(2-chloro-3-methoxy-phenyl)-3-isopropyl-1-{2-[4-(7-methoxy-2-oxo-1,2,4,5-tetrahydrobenzo[d][1,3]diazepin-3-yl)-piperidin-1-yl]-2-oxo-ethyl}-1H-pyrimidine-2,4-dione;
compound 152: 3-isopropyl-1-{2-[4-(7-methoxy-2-oxo-1,2,4,5-tetrahydro-benzo[d][1,3]diazepin-3-yl)-piperidin-1-yl]-2-oxo-ethyl}-5-(3-methoxy-2-trifluoromethoxy-phenyl)-1H-pyrimidine-2,4-dione;
compound 155: 5-(2-chloro-3-fluoro-phenyl)-1-{2-[4-(7-methoxy-2-oxo-1,2,4,5-tetrahydro-benzo[d][1,3]diazepin-3-yl)-piperidin-1-yl]-2-oxo-ethyl}-3-(2methylsulfanyl-ethyl)-1H-pyrimidine-2,4-dione;
compound 156: 5-(2-chloro-3-fluoro-phenyl)-3-(3-methanesulfonyl-propyl)-1-{2-[4-(7-methoxy-2-oxo-1,2,4,5-tetrahydro-benzo[d][1,3]diazepin-3-yl)-piperidin-1-yl]-2-oxoethyl}-1H-pyrimidine-2,4-dione;
compound 157: 5-(2-chloro-3-fluoro-phenyl)-1-[2-[4-(7-methoxy-2-oxo-4,5-dihydro-1H-1,3-benzodiazepin-3-yl)-1 -piperidyl]-2-oxo-ethyl]-3-[2-(methylsulfonimidoyl)ethyl]pyrimidine-2,4-dione;
compound 158: N-[(S)-2-(5-(2-chloro-3-fluoro-phenyl)-3-{2-[4-(7-methoxy-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidine-1-yl)-propyl]-propionamide;
compound 159: 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-((S)-2-methoxy-1-methyl-ethyl)-1H-pyrimidine-2,4-dione;
compound 160: 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-(2-methanesulfonyl-ethyl)-1H-pyrimidine-2,4-dione;
compound 161: N-[(S)-2-(5-(2-Chloro-3-fluoro-phenyl)-3-{2-[4-(7-fluoro-2-oxo-1,2,4,5-tetrahydrobenzo[d][1,3]diazepin-3-yl)-piperidin-1-yl]-2-oxo-ethyl}-2,6-dioxo-3,6-dihydro-2H-pyrimidine-1-yl)-propyl]-acetamide;
compound 162: N-[(S)-2-(5-(2,3-Dichloro-phenyl)-3-{2-[4-(7-fluoro-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-propyl]-acetamide;
compound 165: 5-(2-chloro-3-fluoro-phenyl)-3-((S)-2-methoxy-1-methyl-ethyl)-2,4-dioxo-3,4-dihydro-2H-pyrimidine-1-yl]-1-[(2R)-2'-oxospiro[1,3-dihydroindene-2,3'-1H-pyrrolo[2,3-b]pyridine]-5-yl]-acetamide;
compound 166: 5-(2-chloro-3-fluoro-phenyl)-3-isopropylpyrimidine-2,4-dione-1-[(2R)-2'-oxospiro[1,3-dihydroindene-2,3'-1H-pyrrolo[2,3-b]pyridine]-5-yl]-acetamide;
compound 170: 5-(2,3-dichlorophenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide;
compound 178: 5-(2,3-dichlorophenyl)-3-[(1S)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide;
compound 184: 5-(2-Chloro-3-fluoro-phenyl)-3-(2-methanesulfonyl-ethyl)-1-{2-[4-(7-methanesulfonyl-2-oxo-1,2,4,5-tetrahydro-benzo[d][1,3]diazepin-3-yl)-piperidin-1-yl]-2-oxo-ethyl}-1H-pyrimidine-2,4-dione;
compound 185: 5-(2-Chloro-3-fluoro-phenyl)-3-isopropyl-1-{2-[4-(7-methanesulfonyl-2-oxo-1,2,4,5-tetrahydro-benzo[d][1,3]diazepin-3-yl)-piperidin-1-yl]-2-oxo-ethyl}-1H-pyrimidine-2,4-dione;
compound 186: 5-(2-chloro-3-fluoro-phenyl)-3-(2-methanesulfonyl-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione;
compound 187: 5-(2-Chloro-3-fluoro-phenyl)-3-((R)-2-methoxy-1-methyl-ethyl)-1-{2-[4-(7-methylsulfanyl-2-oxo-1 2,4,5-tetrahydro-benzo[d][1 3]diazepin-3-yi)-piperidin-1 - yl]-2-oxo-ethyl}-1H-pyrimidine-2,4-dione;
compound 188: 5-(2-Chloro-3-fluoro-phenyl)-3-(2-methoxy-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione;
compound 189: 5-(2-Chloro-3-fluoro-phenyl)-3-isopropyl-1-{2-[4-(7-methylsulfanyl-2-ozo-1,2,4,5-tetrahydro-benzo[d][1,3]diazepin-3-yl)-piperidin-1-yl]-2-oxo-ethyl}-1H-pyrimidine-2,4-dione;
compound 192: 3-(1-{2-[5-(2-chloro-3-fluoro-phenyl)-3-(2-methoxy-ethyl)-2-oxo-3,4-dihydro-2H-pyrimidine-1-yl]-acetyl}-piperidin-4-yl)-1,3,4,5-tetrahydro-benzo[d][1,3]diazepin-2-one;
compound 199: 3-(1-{2-[5-(2,3-Dichloro-phenyl)-3-ethyl-2-oxo-3,4-dihydro-2H-pyrimidine-1-yl]-acetyl}-piperidin-4-yl)-1,3,4,5-tetrahydro-benzo[d][1,3]diazepin-2-one;
compound 202: N-[2-(5-(2-chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidine-1-yl)-ethyl]-acetamide;
compound 203: N-[(R)-2-(5-(2-chloro-3-fluoro-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-propyl]-acetamide;
compound 205: N-[(S)-2-(5-(2,3-dichloro-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-propyl]-acetamide;
compound 206: N-[(S)-2-(5-(2-chloro-3-methoxy-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-propyl]-acetamide;
compound 207: N-[2-(5-(2,3-dichloro-phenyl)-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidine-1-yl)-ethyl]-acetamide;
compound 208: 5-(2,3-dichlorophenyl)-3-methyl-1-[2-oxo-2-[4-(2-oxo-5-phenyl-1H-imidazol-3-yl)-1-piperidyl]ethyl]pyrimidine-2,4-dione;
compound 210: 5-(2,3-dichlorophenyl)-3-isopropyl-1-[2-oxo-2-[4-(2-oxo-4,5-dihydro-1H-1,3-benzodiazepin-3-yl)-1-piperidyl]ethyl]pyrimidine-2,4-dione;
compound 213: 5-(2,3-dichlorophenyl)-3-methyl-1-[2-oxo-2-[4-(2-oxo-4,5-dihydro-1H-pyrido[2,3-d][1,3]diazepin-3-yl)-1-piperidyl]ethyl]pyrimidine-2,4-dione;
compound 215: 5-(2,3-dichlorophenyl)-3-methyl-1-[2-oxo-2-[4-(2-oxo-1H-quinolin-3-yl)-1-piperidyl]ethyl]pyrimidine-2,4-dione;
compound 220: 5-(3,4-dichlorophenyl)-3-methyl-1-[2-oxo-2-[4-(2-oxo-4,5-dihydro-1H-1,3-benzodiazepin-3-yl)-1-piperidyl]ethyl]pyrimidine-2,4-dione;
compound 221: 1-[2-[4-(7-bromo-2-oxo-4,5-dihydro-1H-1,3-benzodiazepin-3-yl)-1-piperidyl]-2-oxo-ethyl]-5-(2-chloro-3-fluoro-phenyl)-3-isopropyl-pyrimidine-2,4-dione;
compound 225: 5-(2-chloro-3-fluoro-phenyl)-3-isopropyl-1-[2-[4-(7-methylsulfinyl-2-oxo-4,5-dihydro-1H-1,3-benzodiazepin-3-yl)-1-piperidyl]-2-oxo-ethyl]pyrimidine-2,4-dione;
compound 226: 2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidine-1-yl]acetamide;
compound 227: 5-(2-chloro-3-fluoro-phenyl)-3-(2-methylsulfinylethyl)-1-[2-oxo-2-[4-(2-oxo-4,5-dihydro-1H-1,3-benzodiazepin-3-yl)-1 -piperidyl]ethyl]pyrimidine-24-dione;
compound 228: 5-(2-chloro-3-fluoro-phenyl)-3-(2-methylsulfonylethyl)-1-[2-oxo-2-[4-(2-oxo-4,5-dihydro-1H-1,3-benzodiazepin-3-yl)-1 -piperidyl]ethyl]pyrimidine-2,4-dione;
compound 229: 5-(2-chloro-3-fluoro-phenyl)-3-((S)-2-methoxy-1-methyl-ethyl)-1-{2-[4-(7-methoxy-2-oxo-1,2,4,5-tetrahydro-benzo[d][1,3]diazepin-3-yl)-piperidin-1-yl]-2-oxo-ethyl}-1H-pyrimidine-2,4-dione;
compound 231: 5-(2,3-difluorophenyl)-3-[2-methoxy-1-(methoxymethyl)ethyl]-1-[2-[4-(7-methoxy-2-oxo-4,5-dihydro-1H-1,3-benzodiazepin-3-yl)-1 -piperidyl]-2-oxoethyl]pyrimidine-2,4-dione;
compound 232: 5-(2-chloro-3-fluoro-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxoethyl]pyrimidine-2,4-dione;
compound 233: 5-(2-chloro-3-fluoro-phenyl)-3-[(1S)-2-methoxy-1-methyl-ethyl]-1-[2-[4-(7-methylsulfanyl-2-oxo-4,5-dihydro-1H-1,3-benzodiazepin-3-yl)-1-piperidyl]-2-oxoethyl]pyrimidine-2,4-dione;
compound 234: 5-(2-chloro-3-fluoro-phenyl)-3-(2-hydroxy-2-methyl-propyl)-1-[2-oxo-2-[4-(2-oxo-4,5-dihydro-1H-1,3-benzodiazepin-3-yl)-1 -piperidyl]ethyl]pyrimidine-2,4-dione;
compound 235: 5-(2-chloro-3-fluoro-phenyl)-1-[2-oxo-2-[4-(2-oxo-4,5-dihydro-1H-1,3-benzodiazepin-3-yl)-1-piperidyl]ethyl]-3-(2H-tetrazol-5-ylmethyl)pyrimidine-2,4-dione;
compound 236: 5-(2,3-difluorophenyl)-3-(2-methylsulfonylethyl)-1-[2-oxo-2-[4-(2-oxo-4,5-dihydro-1H-1,3-benzodiazepin-3-yl)-1 -piperidyl]ethyl]pyrimidine-2,4-dione;
compound 237: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1S)-1 -methylpropyl]pyrimidine-2,4-dione;
compound 238: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1R)-1-methylpropyl]pyrimidine-2,4-dione;
compound 239: 5-(2-chloro-3-fluoro-phenyl)-3-[1-(methoxymethyl)propyl]-1-[2-[4-(7-methoxy-2-oxo-4,5-dihydro-1H-1,3-benzodiazepin-3-yl)-1-piperidyl]-2-oxoethyl]pyrim idine-2,4-dione;
compound 240: 5-(2-chloro-3-fluoro-phenyl)-3-(2-methoxy-2-methyl-propyl)-1-[2-[4-(7-methoxy-2-oxo-4,5-dihydro-1H-1,3-benzodiazepin-3-yl)-1-piperidyl]-2-oxoethyl]pyrimidine-2,4-dione;
compound 241: 5-(2-chloro-3-methoxy-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione;
compound 242: 5-(2,3-dichlorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione;
compound 243: 5-(2-chloro-3-methoxy-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione;
compound 244: 5-(2-chloro-3-fluoro-phenyl)-3-[(1S)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione;
compound 245: 5-(2,3-dichlorophenyl)-3-[2-methoxy-1-(methoxymethyl)ethyl]-1-[2-[4-(7-methoxy-2-oxo-4,5-dihydro-1H-1,3-benzodiazepin-3-yl)-1 -piperidyl]-2-oxo-ethyl]pyrimidine-2,4-dione;
compound 246: 3-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidine-1-yl]propanoic acid;
compound 247: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,3,4-oxadiazol-2-yl)ethyl]pyrimidine-2,4-dione;
compound 248: 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidine-1-yl]acetic acid;
compound 249: 5-(2-chloro-3-fluoro-phenyl)-3-[(1R)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione;
compound 251: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,2,4-triazol-4-yl)ethyl]pyrimidine-2,4-dione;
compound 252: 5-(2-chloro-3-fluoro-phenyl)-1-[2-[4-(7-fluoro-2-oxo-4,5-dihydro-1H-1,3-benzodiazepin-3-yl)-1-piperidyl]-2-oxo-ethyl]-3-[(1R)-1-methyl-2-methylsulfonylethyl]pyrimidine-2,4-dione;
compound 253: 3-[1-[2-[5-(2-chloro-3-fluoro-phenyl)-3-(2-methylsulfonylethyl)-2,4-dioxo-pyrimidine-1-yl]acetyl]-4-piperidyl]-2-oxo-4,5-dihydro-1H-1,3-benzodiazepine-7-carboxylic acid;
compound 254: S-[2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidine-1-yl]ethyl]ethanethioate;
compound 255: N-[(2S)-2-[5-(2,3-dichlorophenyl)-2,6-dioxo-3-[2-oxo-2-[4-(2-oxo-4,5-dihydro-1H-1,3-benzodiazepin-3-yl)-1-piperidyl]ethyl]pyrimidine-1-yl]propyl]acetamide;
compound 256: N-[(2S)-2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidine-1-yl]propyl]acetamide;
compound 257: 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidine-1-yl]ethanesulfonic acid;
compound 258: 5-(2-chloro-3-fluoro-phenyl)-3-[(1S)-1-(methoxymethyl)-2-methylsulfonyl-ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione; and
compound 259: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-(2-methyl-2-methylsulfonyl-propyl)pyrimidine-2,4-dione.

6. Compound of general formula (I) according to one of the preceding claims for use as a medicament.

7. Compound of general formula (I) according to any of the claims 1 to 5 for use in the prevention and/or treatment of inflammatory diseases with a neurogenic component.

8. Compound of general formula (I) for use according to claim 7 wherein the inflammatory skin diseases with a neurogenic component are selected from type 1 (Erythematous) and type 2 (Papulopustular) rosacea, atopic dermatitis, chronic hand eczema, psoriasis in its various forms (vulgar, scalp, arthritic, pustular, guttate), facial erythema, blushing, hives (acute, senile pruritus, prurigo nodularis) and acne.

9. Compound of general formula (I) for use according to claim 8 in the prevention and/or treatment of type 1 (Erythematous) rosacea, atopic dermatitis, vulgar and scalp psoriasis, and hives of the senile pruritus and prurigo nodularis types.

10. Compound of general formula (I) for use according to claim 9 in the prevention and/or treatment of type 1 (Erythematous) rosacea.

11. Compound of general formula (I) for use according to claim 7 in the treatment of migraine.

12. Pharmaceutical composition comprising at least one compound of general formula (I) as defined in any of claims 1 to 4 and a pharmaceutically acceptable vehicle, preferably adapted for a topical administration.

13. Cosmetic use of a compound of general formula (I) according to one of claims 1 to 4.
